# EUROPEAN PATENT SPECIFICATION

(11) **EP 3 131 931 B1**
(45) Date of publication and mention of the grant of the patent: **21.10.2020**
(21) Application number: 15779814.1
(22) Date of filing: 17.04.2015
(51) Int. Cl.: C07K 16/46, C07K 16/22, A61K 38/17, A61K 38/18, A61P 7/06

(54) **METHODS FOR INCREASING RED BLOOD CELL LEVELS AND TREATING SICKLE-CELL DISEASE**
VERFAHREN ZUR ERHÖHUNG DER ERYTHROZYTENKONZENTRATION UND BEHANDLUNG DER SICHELZELLENANÄMIE
PROCÉDÉS D'AUGMENTATION DES TAUX DE GLOBULES ROUGES ET DE TRAITEMENT DE LA DRÉPANOCYTOSE

(30) Priority: 18.04.2014 US 201461981519 P; 25.04.2014 US 201461984393 P; 12.06.2014 US 201462011482 P; 11.08.2014 US 201462036066 P; 05.12.2014 US 201462088374 P
(43) Date of publication of application: 22.02.2017
(73) Proprietor: Acceleron Pharma Inc., Cambridge, MA 02139 (US)
(72) Inventor: KUMAR, Ravindra, Acton, MA 01720 (US); SURAGANI, Naga Venkata Sai, Rajasekhar, Wrentham, MA 02093 (US)
(74) Representative: Rashid, Jeremy Suhail
(86) International application number: PCT/US2015/026415
(87) International publication number: WO 2015/161220

(56) References cited:
- WO-A1-2013/059347
- WO-A2-2009/158015
- WO-A2-2014/066486
- US-A1- 2010 028 331
- US-A1- 2010 068 215
- US-B2- 8 895 016
- "Review of Data Presented at the European Hematology Association 19 th Annual Meeting", , 16 June 2014 (2014-06-16), XP55243916, Retrieved from the Internet: URL:http://files.shareholder.com/downloads /AMDA-23MZWJ/0x0x762136/20a51a29-0a0f-4a35 -965e-4e299efd7d12/v11 EHA Investor Call June 16 2014.pdf
- Rajasekhar Nvs Suragani ET AL: "Modified ActRIIB-mFc Fusion Protein (murine ortholog of Luspatercept) Mitigates Sickling and Red Cell Pathology in a Murine Model of Sickle Cell Disease", , 8 December 2014 (2014-12-08), XP055410706, Retrieved from the Internet: URL:http://acceleronpharma.com/wp-content/ uploads/2017/03/20141208-RAP-536-SCD-ASH-2 014-Final-poster1-2.pdf [retrieved on 2017-09-27]
- Rajasekhar Nvs Suragani ET AL: "Modified ActRIIB-Fc Fusion Protein (ACE-536) Decreases Irreversible Sickle Cells in a Murine Model of Sickle Cell Disease", , 14 June 2014 (2014-06-14), XP055410708, Retrieved from the Internet: URL:http://acceleronpharma.com/wp-content/ uploads/2017/03/20140614-ACE-536-20140613- Modified-ActRIIB-Fc-Fusion-Protein-Decreas es-Irreversible-Sickle-Cells-in-a-Murine-M odel-of-1.pdf [retrieved on 2017-09-27]
- KWIATKOWSKI, J.L ET AL.: 'Iron chelation therapy in sickle- cell disease and other transfusion-dependent anemias' HEMATOL ONCOL CLIN N AM vol. 18, 2004, pages 1355 - 1377, XP008184724
- WARE, R.E ET AL.: 'How I use hydroxyurea to treat young patients with sickle cell anemia' BLOOD vol. 115, no. 26, 2010, ISSN 0006-4971 pages 5300 - 5311, XP055230634

## Description

### CROSS-REFERENCE TO RELATED APPLICATIONS

This application claims the benefit of priority to U.S. Provisional Application Serial Nos., 61/981,519, filed April 18, 2014, 61/984,393, filed April 25, 2014, 62/011,482, filed June 12, 2014, 62/036,066, filed August 11, 2014, and 62/088,374, filed December 5, 2014.

### BACKGROUND

Hematopoiesis is the formation of cellular components of the blood from self-renewing hematopoietic stem cells located mainly in the bone marrow, spleen, or lymph nodes during postnatal life. Blood cells can be classified as belonging to the lymphocytic lineage, myelocytic lineage, or erythroid lineage. By a process known as lymphopoiesis, common lymphoid progenitor cells give rise to T-cells, B-cells, natural killer cells, and dendritic cells. By a process termed myelopoiesis, common myeloid progenitor cells give rise to macrophages, granulocytes (basophils, neutrophils, eosinophils, and mast cells), and thrombocytes (platelets). Finally, by a process known as erythropoiesis, erythroid progenitor cells give rise to red blood cells (erythrocytes).

Postnatal erythropoiesis occurs primarily in the bone marrow and in the red pulp of the spleen. The coordinated action of various signaling pathways controls the balance of cell proliferation, differentiation, survival, and death. Under normal conditions, red blood cells are produced at a rate that maintains a constant red cell mass in the body, and production may increase or decrease in response to various stimuli, including increased or decreased oxygen tension or tissue demand. The process of erythropoiesis begins with the formation of lineage-committed precursor cells and proceeds through a series of distinct precursor cell types. The final stages of erythropoiesis occur as reticulocytes are released into the bloodstream and lose their mitochondria and ribosomes while assuming the morphology of mature red blood cell. An elevated level of reticulocytes, or an elevated reticulocyte:erythrocyte ratio, in the blood is indicative of increased red blood cell production rates. The mature red blood cell (RBC) is responsible for oxygen transport in the circulatory systems of vertebrates. Red blood cells contain high concentrations of hemoglobin, a protein that binds to oxygen in the lungs at relatively high partial pressure of oxygen (pO₂) and delivers oxygen to areas of the body with relatively low pO₂.

Erythropoietin (EPO) is widely recognized as a significant positive regulator of postnatal erythropoiesis in vertebrates. EPO regulates the compensatory erythropoietic response to reduced tissue oxygen tension (hypoxia) and low red blood cell levels or low hemoglobin levels. In humans, elevated EPO levels promote red blood cell formation by stimulating the generation of erythroid progenitors in the bone marrow and spleen. In the mouse, EPO enhances erythropoiesis primarily in the spleen.

Effects of EPO are mediated by a cell-surface receptor belonging to the cytokine receptor superfamily. The human EPO receptor gene encodes a 483 amino acid transmembrane protein; however, the active EPO receptor is thought to exist as a multimeric complex even in the absence of ligand (see, *e.g.,* U.S. Pat. No. 6,319,499). The cloned full-length EPO receptor expressed in mammalian cells binds EPO with an affinity similar to that of the native receptor on erythroid progenitor cells. Binding of EPO to its receptor causes a conformational change resulting in receptor activation and biological effects including increased proliferation of immature erythroblasts, increased differentiation of immature erythroblasts, and decreased apoptosis in erythroid progenitor cells [see, *e.g.,* Liboi et al. (1993) Proc Natl Acad Sci USA 90:11351-11355; Koury et al. (1990) Science 248:378-381].

Various forms of recombinant EPO are used by physicians to increase red blood cell levels in a variety of clinical settings, particularly in the treatment of anemia. Anemia is a broadly-defined condition characterized by lower than normal levels of hemoglobin or red blood cells in the blood. In some instances, anemia is caused by a primary disorder in the production or survival of red blood cells (*e.g.,* sickle-cell anemia). More commonly, anemia is secondary to diseases of other systems [see, *e.g.,* Weatherall & Provan (2000) Lancet 355, 1169-1175]. Anemia may result from a reduced rate of production or increased rate of destruction of red blood cells or by loss of red blood cells due to bleeding. Anemia may result from a variety of disorders that include, for example, acute or chronic renal failure or end stage renal disease, chemotherapy treatment, a myelodysplastic syndrome, rheumatoid arthritis, and bone marrow transplantation.

Treatment with EPO typically causes a rise in hemoglobin by about 1-3 g/dL in healthy humans over a period of weeks. When administered to anemic individuals, this treatment regimen often provides substantial increases in hemoglobin and red blood cell levels and leads to improvements in quality of life and prolonged survival. However, EPO is not uniformly effective, and many individuals are refractory to even high doses [see, *e.g.,* Horl et al. (2000) Nephrol Dial Transplant 15, 43-50]. For example, over 50% of patients with cancer have an inadequate response to EPO, and approximately 10% with end-stage renal disease are hyporesponsive to EPO [see, *e.g.,* Glaspy et al. (1997) J Clin Oncol 15, 1218-1234; Demetri et al. (1998) J Clin Oncol 16, 3412-3425], and less than 10% with myelodysplastic syndromes respond favorably to EPO [see, *e.g.,* Estey (2003) Curr Opin Hematol 10, 60-670]. Although the molecular mechanisms of resistance to EPO are as yet unclear, several factors, including inflammation, iron and vitamin deficiency, inadequate dialysis, aluminum toxicity, and hyperparathyroidism, may predict a poor therapeutic response. In addition, recent evidence suggests that higher doses of EPO may be associated with an increased risk of cardiovascular morbidity, tumor growth, and mortality in some patient populations [see, *e.g.,* Krapf et al. (2009) Clin J Am Soc Nephrol 4:470-480; Glaspy (2009) Annu Rev Med 60:181-192]. Therefore, it has been recommended that EPO-based therapeutic compounds (*e.g*., erythropoietin-stimulating agents, ESAs) be administered at the lowest dose that allows a patient to avoid red blood cell transfusions [see, *e.g.,* Jelkmann et al. (2008) Crit Rev Oncol. Hematol 67:39-61].

Sickle-cell disease is a hereditary blood disorder characterized by red blood cells that assume an abnormal, rigid, sickle shape [see, *e.g.,* Eaton et al. (1990) Adv Protein Chem, 40: 63-279; Steinberg, MH (1999) N Engl J Med 340(13): 1021-1030; and Ballas et al. (1992) Blood, 79(8) 2154-63]. The loss of red blood cell elasticity is believed to be central to the pathophysiology of sickle-cell disease. Normal red blood cells are quite elastic, which allows the red blood cells to deform while passing through capillaries. In sickle-cell disease, low-oxygen tension promotes red blood cell sickling, and repeated episodes of sickling damage the cell membrane and thus decreases the cell's elasticity. Furthermore, sickle-cells often fail to return to a normal shape when normal oxygen tension is restored. As a consequence, these rigid blood cells are unable to deform as they pass through narrow capillaries, resulting in vascular (vaso) occlusion and ischemia. Furthermore, sickle-cells are more prone to hemolysis than normal red blood cells, resulting in a high incidence of anemia in subjects with sickle-cell disease.

Sickle-cell disease is characterized by various acute and chronic complications, which are associated with significant morbidity and mortality in afflicted subject [see, *e.g.,* Kassim et al. (2013) Annu Rev Med, 64: 451-466]. The terms "sickle-cell crisis" or "sickling crisis" may be used to describe several independent acute complications occurring in patients with sickle-cell disease including, for example, pain crisis, painful crisis, anemia crisis, vaso-occlusive crisis, aplastic crisis, sequestration crisis (*e.g*., splenic and/or liver), and hemolytic crisis. These complications of sickle-cell crises are associated with a high incidence of, *e.g.,* stroke, pulmonary hypertension, (acute) chest syndrome, splenomegaly, iron overload, organ damage, renal failure, anemia, and requirements for blood transfusion and pain management. Such complications contribute to shortened life expectancy and increased morbidity in subjects with sickle-cell disease.

There is high unmet need for effective therapies for sickle-cell disease and its complications. It has been noted that endogenous EPO levels are commonly elevated in patients with sickle-cell disease [see, *e.g.,* Dale et al. (1998) Lancet, 352: 566-567]. Therefore, it is not surprising that EPO-based therapeutics have had mixed results with respect to treating sickle-cell disease. For example, some patients appear to be unresponsive to EPO with respect to the treatment of sickle-cell induced anemia, particularly patients having end-stage renal disease [see, *e.g.,* Zumrutdal et al. (2010) NDT Plus, 3(3): 328-330]. Furthermore, EPO-based therapeutics have been observed to aggravate other aspects of sickle-cell disease, such as vaso-occlusive crisis (*e.g*., leading to an increase in vaso-occlusive pain and/or hypertension) [see, *e.g.,* Little et al. (2006) Haematologica, 91(8): 1076-1083].

Thus, it is an object of the present disclosure to provide alternative methods for increasing red blood cell levels and/or addressing other complications of sickle-cell disease.

### SUMMARY OF THE PRESENT DISCLOSURE

In this regard, the present disclosure provides a composition for use in a method of treating or preventing a complication of sickle-cell disease in a subject, the method comprising administering the composition to a subject, wherein the composition comprises an ActRII antagonist, wherein the complication is selected from the group consisting of pain crisis, vaso-occlusion and vaso-occlusive crisis, wherein the ActRII antagonist comprises a polypeptide that comprises an amino acid sequence that is at least 90% identical to the sequence of amino acids 29-109 of SEQ ID NO: 1, wherein the polypeptide comprises an acidic amino acid at the position corresponding to position 79 of SEQ ID NO: 1; wherein the polypeptide binds to GDF8 and/or GDF11. In one instance, the complication is pain crisis. In one instance, the complication of sickle-cell disease is vaso-occlusive crisis. In one instance, the polypeptide ccomprises an amino acid sequence that is at least 95%, 96%, 97%, 98%, or 99% identical to the sequence of amino acids 29-109 of SEQ ID NO:1. In one instance, the acidic amino acid is a glutamic acid or an aspartic acid. In one instance, the acidic amino acid is a glutamic acid. In one instance, the acidic amino acid is an aspartic acid. In one instance, the polypeptide is: a polypeptide comprising the amino acid sequence of SEQ ID NO: 44 or comprising an amino acid sequence that is at least 95% identical to the amino acid sequence of SEQ ID NO: 44; or a polypeptide comprising the amino acid sequence of SEQ ID NO: 45 or comprising an amino acid sequence that is at least 95% identical to the amino acid sequence of SEQ ID NO: 45. In one instance, the polypeptide comprises the amino acid sequence of SEQ ID NO: 45. In one instance, the polypeptide comprises the amino acid sequence of SEQ ID NO: 44. In one instance, the polypeptide is a fusion protein further comprising one or more heterologous polypeptide domains that enhance one or more of: *in vivo* half-life, *in vitro* half-life, administration, tissue localization or distribution, formation of protein complexes, and purification, wherein the heterologous polypeptide domain is selected from: an immunoglobulin Fc domain and a serum albumin. In one instance, the immunoglobulin Fc domain: a) is an IgG1 Fc domain; or b) comprises an amino acid sequence selected from SEQ ID NO: 15 or 16. In one instance, the fusion protein further comprises a linker domain positioned between the polypeptide domain and the immunoglobulin Fc domain. In one instance, the polypeptide comprises one or more amino acid modifications selected from: a glycosylated amino acid, a PEGylated amino acid, a farnesylated amino acid, an acetylated amino acid, a biotinylated amino acid, an amino acid conjugated to a lipid moiety, and amino acid conjugated to an organic derivatizing agent. In one instance, the method further comprises administering one or more supportive therapy for sickle-cell disease, wherein the supportive therapy: a) is transfusion with red blood cells; b) comprises administration of an iron-chelating agent or multiple iron-chelating agents; c) comprises administration of erythropoietin, epoetin alfa, epoetin beta, epoetin delta, epoetin omega, darbepoetin alfa, or methoxy-polyethylene-glycol epoetin beta; and/or d) comprises administration of hydroxyurea. In one instance, the supportive therapy comprises administration of hydroxyurea.

In part, the disclosure provides methods of treating sickle-cell disease (SCD), particularly treating or preventing one or more complications of SCD, with one or more ActRII antagonists. ActRII antagonists of the disclosure include, for example, agents that can inhibit ActRII receptor (*e.g.,* an ActRIIA and/or ActRIIB receptor) mediated activation of a signal transduction pathway (*e.g*., activation of signal transduction via intracellular mediators, such as SMAD 1, 2, 3, 5, and/or 8); agents that can inhibit one or more ActRII ligands (*e.g.,* activin A, activin B, activin AB, activin C, activin E, GDF11, GDF8, BMP6, BMP7, Nodal, *etc.)* from, *e.g.,* binding to and/or activating an ActRII receptor; agents that inhibit expression (*e.g*., transcription, translation, cellular secretion, or combinations thereof) of an ActRII ligand and/or an ActRII receptor; and agents that can inhibit one or more intracellular mediators of the ActRII signaling pathway (*e.g*., SMADs 1, 2, 3, 5, and/or 8).

In some instances, the disclosure concerns an ActRII antagonist for use in a composition for use in treating sickle-cell disease in a subject in need thereof. According to one instance, the disclosure concerns an ActRII antagonist for the manufacture of a medicament for treating sickle-cell disease in a subject in need thereof. In some instances, the disclosure concerns an ActRII antagonist for use in a composition for use in treating one or more complications of sickle-cell disease. According to one instance, the disclosure concerns an ActRII antagonist for the manufacture of a medicament for treating one or more complications sickle-cell disease in a subject in need thereof. In some instances, the disclosure concerns an ActRII antagonist for use in a composition for use in preventing one or more complications of sickle-cell disease. According to one instance, the disclosure concerns an ActRII antagonist for the manufacture of a medicament for preventing one or more complications sickle-cell disease in a subject in need thereof.

In particular, the disclosure provides methods for using an ActRII antagonist, or combination of ActRII antagonists, to treat or prevent one or more complications of sickle-cell disease including, for example, anemia, anemia crisis, splenomegaly, pain crisis, chest syndrome, acute chest syndrome, blood transfusion requirement, organ damage, pain medicine (management) requirement, splenic sequestration crises, hyperhemolytic crisis, vaso-occlusion, vaso-occlusion crisis, acute myocardial infarction, sickle-cell chronic lung disease, thromboemboli, hepatic failure, hepatomegaly, hepatic sequestration, iron overload and complications of iron overload (*e.g*., congestive heart failure, cardiac arrhythmia, myocardial infarction, other forms of cardiac disease, diabetes mellitus, dyspnea, hepatic disease and adverse effects of iron chelation therapy), splenic infarction, acute and/or chronic renal failure, pyelonephritis, aneurysm, ischemic stroke, intraparenchymal hemorrhage, subarachnoid hemorrhage, intraventricular hemorrhage, peripheral retinal ischemia, proliferative sickle retinopathy, vitreous hemorrhage, and/or priapism. In some instances, the disclosure provides methods for using an ActRII antagonist, or combination of ActRII antagonists, to treat or prevent vascular occlusion (vaso-occlusion) in a sickle-cell disease patient in need thereof as well as various complications associated with vaso-occlusion in a sickle-cell disease patient (*e.g.,* vaso-occlusion crisis, pain crisis, *etc*.). In some instances, the disclosure provides methods for using an ActRII antagonist, or combination of ActRII antagonists, to treat or prevent anemia in a sickle-cell disease patient in need thereof as well as various complications associated with anemia in a sickle-cell disease patient (*e.g*., aplastic crisis, hyperhemolytic crisis, *etc*.). In such methods, ActRII antagonists can be used to increase red blood cell levels while reducing the need for red blood cell transfusions and/or iron chelation therapy, and thereby reduce morbidity and mortality associated with iron accumulation in vulnerable tissues/organs. In such methods, ActRII antagonists can also be used to reduce the need for other supportive therapies for treating sickle-cell disease [*e.g*., treatment with hydroxyurea, treatment with an EPO or other EPO agonist, and/or pain management (*e.g*., treatment with one or more of opioid analgesic agents, non-steroidal antiinflammatory drugs, and/or corticosteroids)]. In part, ActRII antagonists can be used in combination with existing supportive therapies for sickle-cell disease including, for example, transfusion of red blood cells, iron chelation therapy, hydroxyurea therapy, EPO or EPO agonist therapy, and/or pain management therapy. Optionally, ActRII antagonists of the disclosure can be used to reduce the amount, duration, etc. of an existing supportive therapy for sickle-cell disease. For example, while transfusion of red blood cells and iron chelation therapy may help treat certain complications of sickle-cell disease, they sometimes result in adverse side effects. Therefore, in certain aspects, ActRII antagonists as described herein can be used to reduce amount of a second supportive therapy, *e.g.,* reduce blood cell transfusion burden or reduce the dosage of a chelation therapeutic. In certain aspects, the disclosure provides uses of an ActRII antagonist, or combination of ActRII antagonists, (optionally in combination with one or more supportive therapies for sickle-cell disease) for making a medicament for the treatment or prevention of sickle-cell disease, particularly one or more complications of sickle-cell disease as disclosed herein.

In part, the examples of the instant application demonstrate that ActRII antagonists can be used to increase various red blood cell parameters (*e.g*., red blood cell levels, hemoglobin levels, hematocrit levels, *etc*.) and treat anemia resulting from various disorders/conditions. The examples of the disclosure further show that an ActRII antagonist can be used to treat sickle-cell disease. In particular, the disclosure demonstrates that a GDF trap (which comprises a soluble, extracellular domain of an ActRIIB polypeptide having an acidic amino acid at position 79 with respect to SEQ ID NO: 1), when administered *in vivo,* increases red blood cell levels in normal, healthy subjects as well as in an animal model of sickle-cell disease. Accordingly, the data herein indicates that ActRII antagonists can be used to treat or prevent anemia in sickle-cell disease patients. Surprisingly, in addition to directly increasing red blood cell levels, the data of the disclosure indicates that the GDF trap also improves red blood cell morphology. This observed improvement in red blood cell morphology indicates that ActRII antagonist therapy also may be used to treat or prevent various non-anemia complications of sickle-cell disease including, for example, complications arising from vascular occlusion (also referred to as vaso-occlusion). In some instances, these associated complications are of equal or greater importance to health and quality of life in patients with sickle-cell disease than the anemic condition. Together, these data therefore indicate that ActRII antagonists of the present disclosure can be used to treat or prevent various complications of sickle-cell disease including, for example, anemia, anemia crisis, splenomegaly, pain crisis, chest syndrome, acute chest syndrome, blood transfusion requirement, organ damage, pain medicine (management) requirement, splenic sequestration crises, hyperhemolytic crisis, vaso-occlusion, vaso-occlusion crisis, acute myocardial infarction, sickle-cell chronic lung disease, thromboemboli, hepatic failure, hepatomegaly, hepatic sequestration, iron overload, splenic infarction, acute and/or chronic renal failure, pyelonephritis, aneurysm, ischemic stroke, intraparenchymal hemorrhage, subarachnoid hemorrhage, intraventricular hemorrhage, peripheral retinal ischemia, proliferative sickle retinopathy, vitreous hemorrhage, and/or priapism.

In certain instances, preferred ActRII antagonists to be used in accordance with the methods disclosed herein are agents that bind to and/or inhibit GDF11 and/or GDF8 (*e.g.,* an agent that inhibits GDF11- and/or GDF8-mediated activation of ActRIIA and/or ActRIIB signaling transduction, such as SMAD 2/3 signaling). Such agents are referred to collectively as GDF-ActRII antagonists. Optionally, such GDF-ActRII antagonists may further inhibit one or more of activin A, activin B, activin AB, activin C, activin E, GDF11, GDF8, BMP6, BMP7, and Nodal. Therefore, in some instances, the disclosure provides methods of using one or more ActRII antagonists, including, for example, soluble ActRIIA polypeptides, soluble ActRIIB polypeptides, GDF trap polypeptides, anti-ActRIIA antibodies, anti-ActRIIB antibodies, anti-ActRII ligand antibodies (*e.g*, anti-GDF1 antibodies, anti-GDF8 antibodies, anti-activin A antibodies, anti-activin B antibodies, anti-activin AB antibodies, anti-activin C antibodies, anti-activin E antibodies, anti-BMP6 antibodies, anti-BMP7 antibodies, and anti-Nodal antibodies), small-molecule inhibitors of ActRIIA, small-molecule inhibitors of ActRIIB, small-molecule inhibitors of one or more ActRII ligands (*e.g*., activin A, activin B, activin AB, activin C, activin E, GDF11, GDF8, BMP6, BMP7, Nodal, *etc.*), inhibitor nucleotides (nucleotide-based inhibitors) of ActRIIA, inhibitor nucleotides of ActRIIB, inhibitor nucleotides of one or more ActRII ligands (*e.g*., activin A, activin B, activin AB, activin C, activin E, GDF11, GDF8, BMP6, BMP7, Nodal, *etc*.), or combinations thereof, to increase red blood cell levels and/or hemoglobin levels in a subject in need thereof, treat or prevent an anemia in a subject in need thereof, treat sickle-cell disease in a subject in need thereof, or treat one or more complications of sickle-cell disease in a subject in need thereof. In certain instances, ActRII antagonists to be used in accordance with the methods disclosed herein do not substantially bind to and/or inhibit activin A (*e.g*., activin A-mediated activation of ActRIIA and/or ActRIIB signaling transduction, such as SMAD 2/3 signaling).

In part, the present disclosure demonstrates that an ActRII antagonist comprising a variant, extracellular (soluble) ActRIIB domain that binds to and inhibits GDF11 activity (*e.g.,* GDF11-mediated ActRIIA and/or ActRIIB signaling transduction, such as SMAD 2/3 signaling) may be used to increase red blood cell levels *in vivo,* treat anemia resulting from various conditions/disorders, and treat sickle-cell disease (*e*.*g.*, increase red blood cell levels and improve red blood cell morphology in sickle-cell disease patients). Therefore, in certain instances, preferred ActRII antagonists to be used in accordance with the methods disclosed herein (*e.g*., methods of increase red blood cell levels in a subject in need thereof, methods of treating anemia in a subject in need thereof, methods of treating sickle-cell disease, methods of treating or preventing one or more complications of sickle-cell disease in subject in need thereof, *etc.*) are soluble ActRII polypeptides (*e.g.* soluble ActRIIA or ActRIIB polypeptides) that bind to and/or inhibit GDF11 (*e.g.,* GDF11-mediated activation of ActRIIA and/or ActRIIB signaling transduction, such as SMAD 2/3 signaling). While soluble ActRIIA and soluble ActRIIB ActRII antagonists may affect red blood cell formation and/or morphology through a mechanism other than GDF11 antagonism, the disclosure nonetheless demonstrates that desirable therapeutic agents, with respect to the methods disclosed herein, may be selected on the basis of GDF11 antagonism or ActRII antagonism or both. Optionally, such soluble ActRII polypeptide antagonist may further bind to and/or inhibit GDF8 (e.g. inhibit GDF8-mediated activation of ActRIIA and/or ActRIIB signaling transduction, such as SMAD 2/3 signaling). In some instances, soluble ActRIIA and ActRIIB polypeptides of the disclosure that bind to and/or inhibit GDF11 and/or GDF8 may further bind to and/or inhibit one or more additional ActRII ligands selected from: activin A, activin B, activin AB, activin C, activin E, BMP6, BMP7, and Nodal.

In certain aspects, the present disclosure provides GDF traps that are variant ActRII polypeptides (*e.g*., ActRIIA and ActRIIB polypeptides), including ActRII polypeptides having amino- and carboxy-terminal truncations and/or other sequence alterations (one or more amino acid substitutions, additions, deletions, or combinations thereof). Optionally, GDF traps of the disclosure may be designed to preferentially antagonize one or more ligands of ActRII receptors, such as GDF8 (also called myostatin), GDF11, Nodal, BMP6, and BMP7 (also called OP-1). As disclosed herein, examples of GDF traps include a set of variants derived from ActRIIB that have greatly diminished affinity for activin, particularly activin A. These variants exhibit desirable effects on red blood cells while reducing effects on other tissues. Examples of such variants include those having an acidic amino acid [*e.g.,* aspartic acid (D) or glutamic acid (E)] at the position corresponding to position 79 of SEQ ID NO:1. In certain instances, preferred GDF traps to be used in accordance with the methods disclosed herein (*e.g*., methods of increase red blood cell levels in a subject in need thereof, methods of treating anemia in a subject in need thereof, methods of treating sickle-cell disease, methods of treating or preventing one or more complications of sickle-cell disease in subject in need thereof, *etc*.) bind to and/or inhibit GDF11. Optionally, such GDF traps may further bind to and/or inhibit GDF8. In some instances, GDF traps that bind to and/or inhibit GDF11 and/or GDF8 may further bind to and/or inhibit one or more additional ActRII ligands (*e.g*., activin B, activin E, BMP6, BMP7, and Nodal). In some instances, GDF traps to be used in accordance with the methods disclosed herein to not substantially bind to and/or inhibit activin A (*e.g*., activin A-mediated activation of ActRIIA and/or ActRIIB signaling transduction, such as SMAD 2/3 signaling). In certain instances, a GDF trap polypeptide comprises an amino acid sequence that comprises, consists of, or consists essentially of, the amino acid sequence of SEQ ID NOs: 36, 37, 41, 44, 45, 50 or 51, and polypeptides that are at least 80%, 85%, 90%, 95%, 96%, 97%, 98%, or 99% identical to any of the foregoing. In other instances, a GDF trap polypeptide comprises an amino acid sequence that comprises, consists of, or consists essentially of the amino acid sequence of SEQ ID NOs: 2, 3, 4, 5, 6, 10, 11, 22, 26, 28, 29, 31, or 49, and polypeptides that are at least 80%, 85%, 90%, 95%, 96%, 97%, 98%, or 99% identical to any of the foregoing. In still other instances, a GDF trap polypeptide comprises an amino acid sequence that comprises of the amino acid sequence of SEQ ID NOs: 2, 3, 4, 5, 6, 29, 31, or 49, and polypeptides that are at least 80%, 85%, 90%, 95%, 96%, 97%, 98%, or 99% identical to any of the foregoing, wherein the position corresponding to 79 in SEQ ID NO: 1, 4, or 50 is an acidic amino acid. A GDF trap may include a functional fragment of a natural ActRII polypeptide, such as one comprising at least 10, 20, or 30 amino acids of a sequence selected from SEQ ID NOs: 1, 2, 3, 4, 5, 6, 9, 10, 11, or 49 or a sequence of SEQ ID NO: 2, 5, 10, 11, or 49 lacking the C-terminal 1, 2, 3, 4, 5 or 10 to 15 amino acids and lacking 1, 2, 3, 4 or 5 amino acids at the N-terminus. A preferred polypeptide will comprise a truncation relative to SEQ ID NO: 2 or 5 of between 2 and 5 amino acids at the N-terminus and no more than 3 amino acids at the C-terminus. A preferred GDF trap for use in such a preparation consists of, or consists essentially of, the amino acid sequence of SEQ ID NO: 36.

Optionally, a GDF trap comprising an altered ActRII ligand-binding domain has a ratio of K_{d} for activin A binding to K_{d} for GDF11 and/or GDF8 binding that is at least 2-, 5-, 10-, 20, 50-, 100-, or even 1000-fold greater relative to the ratio for the wild-type ligand-binding domain. Optionally, the GDF trap comprising an altered ligand-binding domain has a ratio of IC₅₀ for inhibiting activin A to IC₅₀ for inhibiting GDF11 and/or GDF8 that is at least 2-, 5-, 10-, 20-, 25- 50-, 100-, or even 1000-fold greater relative to the wild-type ActRII ligand-binding domain. Optionally, the GDF trap comprising an altered ligand-binding domain inhibits GDF11 and/or GDF8 with an IC₅₀ at least 2, 5, 10, 20, 50, or even 100 times less than the IC₅₀ for inhibiting activin A. These GDF traps can be fusion proteins that include an immunoglobulin Fc domain (either wild-type or mutant). In certain cases, the subject soluble GDF traps are antagonists (inhibitors) of GDF8 and/or GDF11.

In certain aspects, the disclosure provides GDF traps which are soluble ActRIIB polypeptides comprising an altered ligand-binding (*e.g*., GDF 11-binding) domain. GDF traps with altered ligand-binding domains may comprise, for example, one or more mutations at amino acid residues such as E37, E39, R40, K55, R56, Y60, A64, K74, W78, L79, D80, F82 and F101 of human ActRIIB (numbering is relative to SEQ ID NO: 1). Optionally, the altered ligand-binding domain can have increased selectivity for a ligand such as GDF8/GDF11 relative to a wild-type ligand-binding domain of an ActRIIB receptor. To illustrate, these mutations are demonstrated herein to increase the selectivity of the altered ligand-binding domain for GDF11 (and therefore, presumably, GDF8) over activin: K74Y, K74F, K74I, L79D, L79E, and D80I. The following mutations have the reverse effect, increasing the ratio of activin binding over GDF11: D54A, K55A, L79A and F82A. The overall (GDF11 and activin) binding activity can be increased by inclusion of the "tail" region or, presumably, an unstructured linker region, and also by use of a K74A mutation. Other mutations that caused an overall decrease in ligand binding affinity, include: R40A, E37A, R56A, W78A, D80K, D80R, D80A, D80G, D80F, D80M and D80N. Mutations may be combined to achieve desired effects. For example, many of the mutations that affect the ratio of GDF11:activin binding have an overall negative effect on ligand binding, and therefore, these may be combined with mutations that generally increase ligand binding to produce an improved binding protein with ligand selectivity. In an exemplary instance, a GDF trap is an ActRIIB polypeptide comprising an L79D or L79E mutation, optionally in combination with additional amino acid substitutions, additions, or deletions.

In certain instances, ActRII antagonists to be used in accordance with the methods disclosed herein are ActRIIB polypeptides or ActRIIB-based GDF trap polypeptides. In general such ActRIIB polypeptides and ActRIIB-based GDF trap polypeptides are soluble polypeptides that comprise a portion/domain derived from the ActRIIB sequence of SEQ ID NO:1, 4, or 49, particularly an extracellular, ligand-binding portion/domain derived from the ActRIIB sequence of SEQ ID NO:1, 4, or 49. In some instances, the portion derived from ActRIIB corresponds to a sequence beginning at any one of amino acids 21-29 (*e.g.,* 21, 22, 23, 24, 25, 26, 27, 28, or 29) of SEQ ID NO:1 or 4 [optionally beginning at 22-25 (*e.g.,* 22, 23, 24, or 25) of SEQ ID NO:1 or 4] and ending at any one of amino acids 109-134 (*e.g.,* 109, 110, 111, 112, 113, 114, 115, 116, 117, 118, 119, 120, 121, 122, 123, 124, 125, 126, 127, 128, 129, 130, 131, 132, 133, or 134) of SEQ ID NO: 1 or 4. In some instances, the portion derived from ActRIIB corresponds to a sequence beginning at any one of amino acids 20-29 (*e.g.,* 20, 21, 22, 23, 24, 25, 26, 27, 28, or 29) of SEQ ID NO: 1 or 4 [optionally beginning at 22-25 (*e.g.,* 22, 23, 24, or 25) of SEQ ID NO:1 or 4] and ending at any one of amino acids 109-133 (*e.g.,* 109, 110, 111, 112, 113, 114, 115, 116, 117, 118, 119, 120, 121, 122, 123, 124, 125, 126, 127, 128, 129, 130, 131, 132, or 133) of SEQ ID NO: 1 or 4. In some instances, the portion derived from ActRIIB corresponds to a sequence beginning at any one of amino acids 20-24 (*e.g.,* 20, 21, 22, 23, or 24) of SEQ ID NO: 1 or 4 [optionally beginning at 22-25 (*e.g.,* 22, 23, 24, or 25) of SEQ ID NO:1 or 4] and ending at any one of amino acids 109-133 (*e.g.,* 109, 110, 111, 112, 113, 114, 115, 116, 117, 118, 119, 120, 121, 122, 123, 124, 125, 126, 127, 128, 129, 130, 131, 132, or 133) of SEQ ID NO: 1 or 4. In some instances, the portion derived from ActRIIB corresponds to a sequence beginning at any one of amino acids 21-24 (*e.g.,* 21, 22, 23, or 24) of SEQ ID NO: 1 or 4 and ending at any of amino acids 109-134 (*e.g.,* 109, 110, 111, 112, 113, 114, 115, 116, 117, 118, 119, 120, 121, 122, 123, 124, 125, 126, 127, 128, 129, 130, 131, 132, 133, or 134) of SEQ ID NO: 1 or 4. In some instances, the portion derived from ActRIIB corresponds to a sequence beginning at any one of amino acids 20-24 (*e.g.,* 20, 21, 22, 23, or 24) of SEQ ID NO: 1 or 4 and ending at any one of amino acids 118-133 (*e.g.,* 118, 119, 120, 121, 122, 123, 124, 125, 126, 127, 128, 129, 130, 131, 132, or 133) of SEQ ID NO: 1 or 4 In some embodiments, the portion derived from ActRIIB corresponds to a sequence beginning at any one of amino acids 21-24 (*e.g.,* 21, 22, 23, or 24) of SEQ ID NO: 1 or 4 and ending at any one of amino acids 118-134 (*e.g.,* 118, 119, 120, 121, 122, 123, 124, 125, 126, 127, 128, 129, 130, 131, 132, 133, or 134) of SEQ ID NO: 1 or 4. In some instances, the portion derived from ActRIIB corresponds to a sequence beginning at any one of amino acids 20-24 (*e.g.,* 20, 21, 22, 23, or 24) of SEQ ID NO: 1 or 4 and ending at any one of amino acids 128-133 (*e.g.,* 128, 129, 130, 131, 132, or 133) of SEQ ID NO: 1 or 4. In some instances, the portion derived from ActRIIB corresponds to a sequence beginning at any of amino acids 20-24 (*e.g.,* 20, 21, 22, 23, or 24) of SEQ ID NO: 1 or 39 and ending at any of amino acids 128-133 (*e.g.,* 128, 129, 130, 131, 132, or 133) of SEQ ID NO: 1 or 39. In some instances, the portion derived from ActRIIB corresponds to a sequence beginning at any one of amino acids 21-29 (*e.g.,* 21, 22, 23, 24, 25, 26, 27, 28, or 29) of SEQ ID NO: 1 or 4 and ending at any one of amino acids 118-134 (*e.g.,* 118, 119, 120, 121, 122, 123, 124, 125, 126, 127, 128, 129, 130, 131, 132, 133, or 134) of SEQ ID NO: 1 or 4. In some instances, the portion derived from ActRIIB corresponds to a sequence beginning at any one of amino acids 20-29 (*e.g.,* 20, 21, 22, 23, 24, 25, 26, 27, 28, or 29) of SEQ ID NO: 1 or 4 and ending at any one of amino acids 118-133 (*e.g.,* 118, 119, 120, 121, 122, 123, 124, 125, 126, 127, 128, 129, 130, 131, 132, or 133) of SEQ ID NO: 1 or 4. In some instances, the portion derived from ActRIIB corresponds to a sequence beginning at one any of amino acids 21-29 (*e.g.,* 21, 22, 23, 24, 25, 26, 27, 28, or 29) of SEQ ID NO: 1 or 4 and ending at any one of amino acids 128-134 (*e.g.,* 128, 129, 130, 131, 132, 133, or 134) of SEQ ID NO: 1 or 4. In some instances, the portion derived from ActRIIB corresponds to a sequence beginning at any one of amino acids 20-29 (*e.g.,* 20, 21, 22, 23, 24, 25, 26, 27, 28, or 29) of SEQ ID NO: 1 or 4 and ending at any one of amino acids 128-133 (*e.g.,* 128, 129, 130, 131, 132, or 133) of SEQ ID NO: 1 or 4. Surprisingly, ActRIIB and ActRIIB-based GDF trap constructs beginning at 22-25 (*e.g.,* 22, 23, 24, or 25) of SEQ ID NO: 1 or 4 have activity levels greater than proteins having the full extracellular domain of human ActRIIB. In a preferred instance, the ActRIIB polypeptides and ActRIIB-based GDF trap polypeptides comprise, consist essentially of, or consist of, an amino acid sequence beginning at amino acid position 25 of SEQ ID NO: 1 or 4 and ending at amino acid position 131 of SEQ ID NO: 1 or 4. Any of the foregoing ActRIIB polypeptides or ActRIIB-based GDF trap polypeptides may be produced as a homodimer. Any of the foregoing ActRIIB polypeptides or ActRIIB-based GDF trap polypeptides may further comprise a heterologous portion that comprises a constant region from an IgG heavy chain, such as an Fc domain. Any of the above ActRIIB-based GDF trap polypeptides may comprise an acidic amino acid at the position corresponding to position 79 of SEQ ID NO: 1, optionally in combination with one or more additional amino acid substitutions, deletions, or insertions relative to SEQ ID NO: 1. Any of the above ActRIIB polypeptides or ActRIIB-based GDF trap polypeptides, including homodimer and/or fusion proteins thereof, may bind to and/or inhibit signaling by activin (*e.g*., activin A, activin B, activin C, or activin AB) in a cell-based assay. Any of the above ActRIIB polypeptides or ActRIIB-based GDF trap polypeptides, including homodimer and/or fusion proteins thereof, may bind to and/or inhibit signaling by GDF11 and/or GDF8 in a cell based assay. Optionally, any of the above ActRIIB polypeptides or ActRIIB-based GDF trap polypeptides, including homodimer and/or fusion proteins thereof, may bind to and/or inhibit signaling of one or more of activin B, activin C, activin E, BMP6, BMP7, and Nodal in a cell-based assay.

Other ActRIIB polypeptides and ActRIIB-based GDF Trap polypeptides are contemplated, such as the following. An ActRIIB polypeptide or GDF trap polypeptide comprising an amino acid sequence that is at least 80% (*e.g.,* 85%, 90%, 95%, 96%, 97%, 98%, 99%, or 100%) identical to the sequence of amino acids 29-109 of SEQ ID NO: 1 or 4, wherein the position corresponding to 64 of SEQ ID NO: 1 is an R or K, and wherein the ActRIIB polypeptide or ActRIIB-based GDF trap polypeptide inhibits signaling by activin, GDF8, and/or GDF11 in a cell-based assay. The ActRIIB polypeptide or ActRIIB-based GDF trap polypeptide as above, wherein at least one alteration with respect to the sequence of SEQ ID NO: 1 or 4 is positioned outside of the ligand-binding pocket. The ActRIIB polypeptide or ActRIIB-based GDF trap polypeptide as above, wherein at least one alteration with respect to the sequence of SEQ ID NO: 1 or 4 is a conservative alteration positioned within the ligand-binding pocket. The ActRIIB polypeptide or ActRIIB-based GDF trap polypeptide as above, wherein at least one alteration with respect to the sequence of SEQ ID NO: 1 or 4 is an alteration at one or more positions selected from the group consisting of K74, R40, Q53, K55, F82, and L79.

Other ActRIIB polypeptides and ActRIIB-based GDF trap polypeptides are contemplated, such as the following. An ActRIIB polypeptide or ActRIIB-based GDF trap polypeptide comprising an amino acid sequence that is at least 80% (*e.g.,* 85%, 90%, 95%, 96%, 97%, 98%, 99%, or 100%) identical to the sequence of amino acids 29-109 of SEQ ID NO: 1 or 4, and wherein the protein comprises at least one N-X-S/T sequence at a position other than an endogenous N-X-S/T sequence of ActRIIB, and at a position outside of the ligand binding pocket. The ActRIIB polypeptide or ActRIIB-based GDF trap polypeptide as above, wherein the ActRIIB polypeptide or ActRIIB-based GDF trap polypeptide comprises an N at the position corresponding to position 24 of SEQ ID NO: 1 or 4 and an S or T at the position corresponding to position 26 of SEQ ID NO: 1 or 4, and wherein the ActRIIB polypeptide or ActRIIB-based GDF trap polypeptide inhibits signaling by activin, GDF8, and/or GDF11 in a cell-based assay. The ActRIIB polypeptide or ActRIIB-based GDF trap polypeptide as above, wherein the ActRIIB polypeptide or ActRIIB-based GDF Trap polypeptide comprises an R or K at the position corresponding to position 64 of SEQ ID NO: 1 or 4. The ActRIIB polypeptide or ActRIIB-based GDF trap polypeptide as above, wherein ActRIIB polypeptide or ActRIIB-based GDF trap polypeptide comprises a D or E at the position corresponding to position 79 of SEQ ID NO: 1 or 4, and wherein the ActRIIB polypeptide or ActRIIB-based GDF trap polypeptide inhibits signaling by activin, GDF8, and/or GDF11 in a cell-based assay. The ActRIIB polypeptide or ActRIIB-based GDF trap polypeptide as above, wherein at least one alteration with respect to the sequence of SEQ ID NO: 1 or 4 is a conservative alteration positioned within the ligand-binding pocket. The ActRIIB polypeptide or ActRIIB-based GDF trap polypeptide as above, wherein at least one alteration with respect to the sequence of SEQ ID NO: 1 or 4 is an alteration at one or more positions selected from the group consisting of K74, R40, Q53, K55, F82, and L79. The ActRIIB polypeptide or ActRIIB-based GDF trap polypeptide above, wherein the ActRIIB polypeptide or ActRIIB-based GDF trap polypeptide is a fusion protein further comprising one or more heterologous portion. Any of the above ActRIIB polypeptides or ActRIIB-based GDF trap polypeptides, or fusion proteins thereof, may be produced as a homodimer. Any of the above ActRIIB fusion proteins or ActRIIB-based GDF trap fusion proteins may have a heterologous portion that comprises a constant region from an IgG heavy chain, such as an Fc domain.

In certain instances, a preferred ActRIIB polypeptide, for use in accordance with the methods disclosed herein, comprises an amino acid sequence that comprises, consists of, or consists essentially of, the amino acid sequence of SEQ ID NOs: 2, 3, 5, 6, 29, 31, or 49, and polypeptides that are at least 80%, 85%, 90%, 95%, 96%, 97%, 98%, or 99% identical to any of the foregoing. An ActRIIB polypeptide may include a functional fragment of a natural ActRIIB polypeptide, such as one comprising at least 10, 20 or 30 amino acids of a sequence selected from SEQ ID NOs: 2, 3, 5, 6, 29, 31, or 49 or a sequence of SEQ ID NO: 2 or 5, lacking the C-terminal 1, 2, 3, 4, 5 or 10 to 15 amino acids and lacking 1, 2, 3, 4 or 5 amino acids at the N-terminus. A preferred polypeptide will comprise a truncation relative to SEQ ID NO: 2 or 5 of between 2 and 5 amino acids at the N-terminus and no more than 3 amino acids at the C-terminus. A preferred GDF trap for use in accordance with the methods described herein consists of, or consists essentially of, the amino acid sequence of SEQ ID NO:29.

A general formula for an active (*e.g*., ligand binding) ActRIIA polypeptide is one that comprises a polypeptide that starts at amino acid 30 and ends at amino acid 110 of SEQ ID NO:9. Accordingly, ActRIIA polypeptides and ActRIIA-based GDF traps of the present disclosure may comprise, consist, or consist essentially of a polypeptide that is at least 80%, 85%, 90%, 95%, 96%, 97%, 98%, 99%, or 100% identical to amino acids 30-110 of SEQ ID NO:9. Optionally, ActRIIA polypeptides and ActRIIA-based GDF trap polypeptides of the present disclosure comprise, consists, or consist essentially of a polypeptide that is at least 80%, 85%, 90%, 95%, 96%, 97%, 98%, 99%, or 100% identical to amino acids amino acids 12-82 of SEQ ID NO:9 optionally beginning at a position ranging from 1-5 (*e.g.,* 1, 2, 3, 4, or 5) or 3-5 (*e.g.,* 3, 4, or 5) and ending at a position ranging from 110-116 (*e.g.,* 110, 111, 112, 113, 114, 115, or 116) or 110-115 (*e.g*., 110, 111, 112, 113, 114, or 115) or SEQ ID NO:9, respectively, and comprising no more than 1, 2, 5, 10 or 15 conservative amino acid changes in the ligand binding pocket, and zero, one or more non-conservative alterations at positions 40, 53, 55, 74, 79 and/or 82 in the ligand-binding pocket with respect to SEQ ID NO:9. Any of the foregoing ActRIIA polypeptides or ActRIIA-based GDF trap polypeptides may be produced as a homodimer. Any of the foregoing ActRIIA polypeptides or ActRIIA-based GDF trap polypeptides may further comprise a heterologous portion that comprises a constant region from an IgG heavy chain, such as an Fc domain. Any of the above ActRIIA polypeptides or ActRIIA-based GDF trap polypeptides, including homodimer and/or fusion proteins thereof, may bind to and/or inhibit signaling by activin (*e.g.*, activin A, activin B, activin C, or activin AB) in a cell-based assay. Any of the above ActRIIA polypeptides or ActRIIA-based GDF trap polypeptides, including homodimer and/or fusion proteins thereof, may bind to and/or inhibit signaling by GDF11 and/or GDF8 in a cell-based assay. Optionally, any of the above ActRIIA polypeptides or ActRIIB-based GDF trap polypeptides, including homodimer and/or fusion proteins thereof, may bind to and/or inhibit signaling of one or more of activin B, activin C, activin E, GDF7, and Nodal in a cell-based assay.

In certain instances, preferred ActRIIA polypeptides and ActRIIA-based GDF-trap polypeptides, for use in accordance with the methods disclosed herein, comprise an amino acid sequence that comprises, consists of, or consists essentially of, the amino acid sequence of SEQ ID NOs: 9, 10, 22, 26, or 28, and polypeptides that are at least 80%, 85%, 90%, 95%, 96%, 97%, 98%, or 99% identical to any of the foregoing. An ActRIIA polypeptide or ActRIIA-based GDF-trap polypeptide may include a functional fragment of a natural ActRIIA polypeptide, such as one comprising at least 10, 20 or 30 amino acids of a sequence selected from SEQ ID NOs: 9, 10, 22, 26, or 28 or a sequence of SEQ ID NO:10, lacking the C-terminal 1, 2, 3, 4, 5 or 10 to 15 amino acids and lacking 1, 2, 3, 4 or 5 amino acids at the N-terminus. A preferred polypeptide will comprise a truncation relative to SEQ ID NO:10 of between 2 and 5 amino acids at the N-terminus and no more than 3 amino acids at the C-terminus. A preferred ActRIIA polypeptide for use in the methods described herein consists of, or consists essentially of, the amino acid sequence of SEQ ID NO: 26 or 28.

An ActRII polypeptide (*e.g*. an ActRIIA or ActRIIB polypeptide) or GDF trap polypeptide of the disclosure may include one or more alterations (*e.g*., amino acid additions, deletions, substitutions, or combinations thereof) in the amino acid sequence of an ActRII polypeptide (*e.g*., in the ligand-binding domain) relative to a naturally occurring ActRII polypeptide. The alteration in the amino acid sequence may, for example, alter glycosylation of the polypeptide when produced in a mammalian, insect, or other eukaryotic cell or alter proteolytic cleavage of the polypeptide relative to the naturally occurring ActRII polypeptide.

Optionally, ActRII polypeptides (*e.g*. an ActRIIA or ActRIIB polypeptides) and GDF trap polypeptides of the disclosure comprise one or more modified amino acid residues selected from: a glycosylated amino acid, a PEGylated amino acid, a farnesylated amino acid, an acetylated amino acid, a biotinylated amino acid, an amino acid conjugated to a lipid moiety, and an amino acid conjugated to an organic derivatizing agent.

In some instances, an ActRII polypeptide (*e.g*. an ActRIIA or ActRIIB polypeptide) or GDF trap polypeptide of the disclosure may be a fusion protein that has, as one domain, an ActRII polypeptide or GDF trap polypeptide (*e.g.,* a ligand-binding domain of an ActRII receptor, optionally with one or more sequence variations) and one or more additional heterologous domains that provide a desirable property, such as improved pharmacokinetics, easier purification, targeting to particular tissues, *etc.* For example, a domain of a fusion protein may enhance one or more of *in vivo* stability, *in vivo* half-life, uptake/administration, tissue localization or distribution, formation of protein complexes, multimerization of the fusion protein, and/or purification. ActRII polypeptide and GDF trap fusion proteins may include an immunoglobulin Fc domain (wild-type or mutant) or a serum albumin. In certain instances, an ActRII polypeptide and GDF trap fusion protein comprises a relatively unstructured linker positioned between the Fc domain and the ActRII or GDF trap domain. This unstructured linker may correspond to the roughly 15 amino acid unstructured region at the C-terminal end of the extracellular domain of ActRII or GDF trap (the "tail"), or it may be an artificial sequence of between 3 and 5, 15, 20, 30, 50 or more amino acids that are relatively free of secondary structure. A linker may be rich in glycine and proline residues and may, for example, contain repeating sequences of threonine/serine and glycines [*e.g*., TG₄ (SEQ ID NO:52), TG₃ (SEQ ID NO:53), or SG₄ (SEQ ID NO:54) singlets or repeats] or a series of three glycines. A fusion protein may include a purification subsequence, such as an epitope tag, a FLAG tag, a polyhistidine sequence, and a GST fusion. In certain instances, an ActRII fusion protein or GDF trap fusion comprises a leader sequence. The leader sequence may be a native ActRII leader sequence (*e.g.,* a native ActRIIA or ActRIIB leader sequence) or a heterologous leader sequence. In certain instances, the leader sequence is a tissue plasminogen activator (TPA) leader sequence. In some instance, an ActRII fusion protein or GDF trap fusion protein comprises an amino acid sequence as set forth in the formula A-B-C. The B portion is an N- and C-terminally truncated ActRII or GDF trap polypeptide as described herein. The A and C portions may be independently zero, one, or more than one amino acids, and both A and C portions are heterologous to B. The A and/or C portions may be attached to the B portion via a linker sequence.

Optionally, ActRII polypeptides (*e.g*., ActRIIA and ActRIIB polypeptides) or GDF trap polypeptides, including variants and fusion proteins thereof, to be used in accordance with the methods disclosed herein bind to one or more ActRIIB ligands (*e.g*., activin A, activin B, activin AB, activin C, activin E, GDF11, GDF8, BMP6, BMP7, and/or Nodal) with a Kd less than 10 micromolar, less than 1 micromolar, less than 100 nanomolar, less than 10 nanomolar, or less than 1 nanomolar. Optionally, such ActRII polypeptides or GDF trap polypeptides inhibit ActRII signaling, such as ActRIIA and/or ActRIIB intracellular signal transduction events triggered by an ActRII ligand (*e.g*., SMAD 2/3 and/or SMAD 1/5/8 signaling).

In certain aspects, the disclosure provides pharmaceutical preparations comprising an ActRII antagonist of the present disclosure (*e.g*., an ActRIIA polypeptide, an ActRIIB polypeptide, a GDF trap polypeptide) and a pharmaceutically acceptable carrier. A pharmaceutical preparation may also include one or more additional compounds such as a compound that is used to treat a disorder or condition described herein (*e.g.,* an addition compound that increases red blood cell levels and/or hemoglobin levels in a subject in need thereof, treats or prevents anemia in a subject in need thereof, treats sickle-cell disease is a subject in need thereof, treat or prevents one or more complications of sickle-cell disease is a subject in need thereof). Preferably, a pharmaceutical preparation of the disclosure is substantially pyrogen-free. In general, it is preferable that an ActRIIA polypeptide, an ActRIIB polypeptide, or a GDF trap polypeptide be expressed in a mammalian cell line that mediates suitably natural glycosylation of the polypeptide so as to diminish the likelihood of an unfavorable immune response in a patient. Human and CHO cell lines have been used successfully, and it is expected that other common mammalian expression vectors will be useful. In some instances, preferable ActRIIA polypeptides, ActRIIB polypeptides, and GDF trap polypeptides are glycosylated and have a glycosylation pattern that is obtainable from a mammalian cell, preferably a CHO cell. In certain instances, the disclosure provides packaged pharmaceuticals comprising a pharmaceutical preparation described herein and labeled for use in one or more of increasing red blood cell levels and/or hemoglobin in a mammal (preferably a human), treating or preventing anemia in a mammal (preferably a human), treating sickle-cell disease in a mamamal (preferably a human), and/or treating or preventing one or more complications of sickle-cell disease (*e.g*., anemia, vaso-occlusive crisis, *etc.*) in a mammal (preferably a human).

In certain aspects, the disclosure provides nucleic acids encoding an ActRII polypeptide (*e.g*., an ActRIIA or ActRIIB polypeptide) or GDF trap polypeptide. An isolated polynucleotide may comprise a coding sequence for a soluble ActRII polypeptide or GDF trap polypeptide, such as described herein. For example, an isolated nucleic acid may include a sequence coding for an ActRII polypeptide or GDF trap comprising an extracellular domain (*e.g*., ligand-binding domain) of an ActRII polypeptide having one or more sequence variations and a sequence that would code for part or all of the transmembrane domain and/or the cytoplasmic domain of an ActRII polypeptide, but for a stop codon positioned within the transmembrane domain or the cytoplasmic domain, or positioned between the extracellular domain and the transmembrane domain or cytoplasmic domain. For example, an isolated polynucleotide coding for a GDF trap may comprise a full-length ActRII polynucleotide sequence such as SEQ ID NO: 1, 4, or 9 or having one or more variations, or a partially truncated version, said isolated polynucleotide further comprising a transcription termination codon at least six hundred nucleotides before the 3'-terminus or otherwise positioned such that translation of the polynucleotide gives rise to an extracellular domain optionally fused to a truncated portion of a full-length ActRII. Nucleic acids disclosed herein may be operably linked to a promoter for expression, and the disclosure provides cells transformed with such recombinant polynucleotides. Preferably the cell is a mammalian cell, such as a CHO cell.

In certain aspects, the disclosure provides methods for making an ActRII polypeptide or a GDF trap. Such a method may include expressing any of the nucleic acids disclosed herein (*e.g.,* SEQ ID NO: 8, 13, 27, 32, 39, 42, 46, or 48) in a suitable cell, such as a Chinese hamster ovary (CHO) cell. Such a method may comprise: a) culturing a cell under conditions suitable for expression of the GDF trap polypeptide, wherein said cell is transformed with a GDF trap expression construct; and b) recovering the GDF trap polypeptide so expressed. GDF trap polypeptides may be recovered as crude, partially purified or highly purified fractions using any of the well-known techniques for obtaining protein from cell cultures.

In certain aspects, the present disclosure relates to an antibody, or combination of antibodies, that antagonize ActRII activity (*e.g*., inhibition of ActRIIA and/or ActRIIB signaling transduction, such as SMAD 2/3 and/or SMAD 1/5/8 signaling). In particular, the disclosure provides methods of using an antibody ActRII antagonist, or combination of antibody ActRII antagonists, to, *e.g.,* increase red blood cell levels in a subject in need thereof, treat or prevent an anemia in a subject in need thereof, treat or prevent sickle-cell disease in a subject in need thereof, and/or treat or prevent one or more complication of sickle-cell disease (*e.g*., anemia, anemia crisis, splenomegaly, pain crisis, chest syndrome, acute chest syndrome, blood transfusion requirement, organ damage, pain medicine (management) requirement, splenic sequestration crises, hyperhemolytic crisis, vaso-occlusion, vaso-occlusion crisis, acute myocardial infarction, sickle-cell chronic lung disease, thromboemboli, hepatic failure, hepatomegaly, hepatic sequestration, iron overload, splenic infarction, acute and/or chronic renal failure, pyelonephritis, aneurysm, ischemic stroke, intraparenchymal hemorrhage, subarachnoid hemorrhage, intraventricular hemorrhage, peripheral retinal ischemia, proliferative sickle retinopathy, vitreous hemorrhage, priapism) in a subject in need thereof.

In certain instances, a preferred antibody ActRII antagonist of the disclosure is an antibody, or combination of antibodies, that binds to and/or inhibits activity of at least GDF11 (*e.g.,* GDF11-mediated activation of ActRIIA and/or ActRIIB signaling transduction, such as SMAD 2/3 signaling). Optionally, the antibody, or combination of antibodies, further binds to and/or inhibits activity of GDF8 (*e.g*., GDF8-mediated activation of ActRIIA and/or ActRIIB signaling transduction, such as SMAD 2/3 signaling), particularly in the case of a multispecific antibody that has binding affinity for both GDF11 and GDF8 or in the context of a combination of one or more anti-GDF11 antibody and one or more anti-GDF8 antibody. Optionally, an antibody, or combination of antibodies, of the disclosure does not substantially bind to and/or inhibit activity of activin A (*e.g.,* activin A-mediated activation of ActRIIA or ActRIIB signaling transduction, such as SMAD 2/3 signaling). In some instances, an antibody, or combination of antibodies, of the disclosure that binds to and/or inhibits the activity of GDF11 and/or GDF8 further binds to and/or inhibits activity of one of more of activin A, activin B, activin AB, activin C, activin E, BMP6, BMP7, and Nodal (*e.g.,* activation of ActRIIA or ActRIIB signaling transduction, such as SMAD 2/3 and/or SMAD 1/5/8 signaling), particularly in the case of a multispecific antibody that has binding affinity for multiple ActRII ligands or in the context of a combination multiple antibodies - each having binding affinity for a different ActRII ligand.

In part, the disclosure demonstrates that ActRII antagonists may be used in combination (*e.g*., administered at the same time or different times, but generally in such a manner as to achieve overlapping pharmacological effects) with EPO receptor activators to increase red blood cell levels (erythropoiesis) or treat anemia in patients in need thereof. In part, the disclosure demonstrates that a GDF trap can be administered in combination with an EPO receptor activator to synergistically increase formation of red blood cells in a patient, particularly in sickle-cell patients. Thus, the effect of this combined treatment can be significantly greater than the sum of the effects of the ActRII antagonists and the EPO receptor activator when administered separately at their respective doses. In certain instances, this synergism may be advantageous since it enables target levels of red blood cells to be attained with lower doses of an EPO receptor activator, thereby avoiding potential adverse effects or other problems associated with higher levels of EPO receptor activation. Accordingly, in certain instances, the methods of the present disclosure (*e.g*., compositions for use in increasing red blood cell levels and/or hemoglobin in a subject in need thereof, compositions for use in treating or preventing anemia in a subject in need thereof, compositions for use in treating sickle-cell disease in a subject in need thereof, and/or compositions for use in treating or preventing one or more complications of sickle-cell disease in a subject in need thereof) comprise one or more ActRII antagonists (*e.g*., ActRIIA polypeptides, ActRIIB polypeptides, and/or GDF trap polypeptides). In some instances, the compositions for use of the disclosure are for usein combination with one or more EPO receptor activators.

An EPO receptor activator may stimulate erythropoiesis by directly contacting and activating EPO receptor. In certain instances, the EPO receptor activator is one of a class of compounds based on the 165 amino-acid sequence of native EPO and generally known as erythropoiesis-stimulating agents (ESAs), examples of which are epoetin alfa, epoetin beta (NeoRecormon™), epoetin delta (Dynepo™), and epoetin omega. In other instances, ESAs include synthetic EPO proteins (SEPs) and EPO derivatives with nonpeptidic modifications conferring desirable pharmacokinetic properties (lengthened circulating half-life), examples of which are darbepoetin alfa (Aranesp™) and methoxy-polyethylene-glycol epoetin beta (Micera™). In certain instances, an EPO receptor activator may be an EPO receptor agonist that does not incorporate the EPO polypeptide backbone or is not generally classified as an ESA. Such EPO receptor agonists may include, but are not limited to, peptidic and nonpeptidic mimetics of EPO, agonistic antibodies targeting EPO receptor, fusion proteins comprising an EPO mimetic domain, and erythropoietin receptor extended-duration limited agonists (EREDLA).

In certain instances, an EPO receptor activator may stimulate erythropoiesis indirectly, without contacting EPO receptor itself, by enhancing production of endogenous EPO. For example, hypoxia-inducible transcription factors (HIFs) are endogenous stimulators of EPO gene expression that are suppressed (destabilized) under normoxic conditions by cellular regulatory mechanisms. In part, the disclosure provides increased erythropoiesis in a patient by combined treatment with a GDF trap and an indirect EPO receptor activator with HIF stabilizing properties, such as a prolyl hydroxylase inhibitor.

ActRII antagonists, particularly ActRII polypeptides and GDF trap polypeptides, may also be used for treating or preventing other disorders and conditions such as promoting muscle growth and/or treating or preventing a muscle-related disorder, promoting bone growth and/or treating or preventing a bone-related disorder, treating or preventing cancer (particularly multiple myeloma and/or breast cancer) [see, *e.g.,* U.S. Patent Nos: 7,612,041; 8,173,601; 7,842,663 as well as U.S. Patent Application Publication No. U.S. 2009/0074768]. In certain instances, when administering a GDF trap polypeptide for these other therapeutic indications, it may be desirable to monitor the effects on red blood cells during administration of the ActRII antagonist, or to determine or adjust the dosing of the ActRII antagonist, in order to reduce undesired effects on red blood cells. For example, increases in red blood cell levels, hemoglobin levels, or hematocrit levels may cause increases in blood pressure.

In certain aspects, the disclosure provides a composition for use in treating sickle-cell disease in a subject comprising an ActRII antagonist. In other aspects, the disclosure provides a composition for use in preventing sickle-cell disease in a subject comprising an ActRII antagonist.

In certain aspects, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO: 10. In some instances, the disclosure provides a composition for use in treating sickle-cell disease in a subject comprising a polypeptide comprising the amino acid sequence of SEQ ID NO: 10. In some instances, the disclosure provides a composition for use in treating sickle-cell disease in a subject comprising a polypeptide consisting of the amino acid sequence of SEQ ID NO: 10. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO: 10, wherein the polypeptide binds to activin (*e.g*., activin A and/or activin B). In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:10, wherein the polypeptide binds to GDF11. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:10, wherein the polypeptide binds to GDF11 and activin (*e.g*., activin A and/or activin B). In some instances, the disclosure provides a composition for use in treating sickle-cell disease in a subject comprising a polypeptide comprising an amino acid sequence that is at least 80% *(e.g.,* at least 85%, 90%, 95%, 96%, 97%, 98%, 99%, or 100%) identical to the amino acid sequence of SEQ ID NO:10, wherein the polypeptide binds to GDF8. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:10, wherein the polypeptide binds to GDF8 and GDF11. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:10, wherein the polypeptide binds to GDF8, GDF11, and activin (*e.g*., activin A and/or activin B).

In certain aspects, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:11. In some instances, the disclosure provides a composition for use in treating sickle-cell disease in a subject comprising a polypeptide comprising the amino acid sequence of SEQ ID NO:11. In some instances, the disclosure provides a composition for use in treating sickle-cell disease in a subject comprising a polypeptide consisting of the amino acid sequence of SEQ ID NO:11. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:11, wherein the polypeptide binds to activin (*e.g*., activin A and/or activin B). In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:11, wherein the polypeptide binds to GDF11. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:1 1, wherein the polypeptide binds to GDF11 and activin (*e.g*., activin A and/or activin B). In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:1 1, wherein the polypeptide binds to GDF8. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:11, wherein the polypeptide binds to GDF8 and GDF11. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:11, wherein the polypeptide binds to GDF8, GDF11, and activin (*e.g*., activin A and/or activin B).

In certain aspects, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:22. In some instances, the disclosure provides a composition for use in treating sickle-cell disease in a subject comprising a polypeptide comprising the amino acid sequence of SEQ ID NO:22. In some instances, the disclosure provides a composition for use in treating sickle-cell disease in a subject comprising a polypeptide consisting of the amino acid sequence of SEQ ID NO:22. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:22, wherein the polypeptide binds to activin (*e.g*., activin A and/or activin B). In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:22, wherein the polypeptide binds to GDF11. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:22, wherein the polypeptide binds to GDF11 and activin (*e.g*., activin A and/or activin B). In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:22, wherein the polypeptide binds to GDF8. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:22, wherein the polypeptide binds to GDF8 and GDF11. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:22, wherein the polypeptide binds to GDF8, GDF11, and activin (*e.g*., activin A and/or activin B).

In certain aspects, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:36. In some instances, the disclosure provides a composition for use in treating sickle-cell disease in a subject comprising a polypeptide comprising the amino acid sequence of SEQ ID NO:36. In some instances, the disclosure provides a composition for use in treating sickle-cell disease in a subject comprising a polypeptide consisting of the amino acid sequence of SEQ ID NO:36. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:36, wherein the polypeptide binds to GDF11. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:36, wherein the polypeptide binds to GDF8. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:36, wherein the polypeptide binds to GDF8 and GDF11.

In certain aspects, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:37. In some instances, the disclosure provides a composition for use in treating sickle-cell disease in a subject comprising a polypeptide comprising the amino acid sequence of SEQ ID NO:37. In some instances, the disclosure provides a composition for use in treating sickle-cell disease in a subject comprising a polypeptide consisting of the amino acid sequence of SEQ ID NO:37. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:37, wherein the polypeptide binds to GDF11. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:37, wherein the polypeptide binds to GDF8. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:37, wherein the polypeptide binds to GDF8 and GDF11.

In certain aspects, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:44. In some instances, the disclosure provides a composition for use in treating sickle-cell disease in a subject comprising a polypeptide comprising the amino acid sequence of SEQ ID NO:44. In some instances, the disclosure provides a composition for use in treating sickle-cell disease in a subject comprising a polypeptide consisting of the amino acid sequence of SEQ ID NO:44. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:44, wherein the polypeptide binds to GDF11. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:44, wherein the polypeptide binds to GDF8. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:44, wherein the polypeptide binds to GDF8 and GDF11.

In certain aspects, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1. In some instances, the disclosure provides a composition for use in treating sickle-cell disease in a subject comprising a polypeptide comprising the sequence of amino acids 29-109 of SEQ ID NO: 1. In some instances, the disclosure provides a composition for use in treating sickle-cell disease in a subject comprising a polypeptide consisting of the sequence of amino acids 29-109 of SEQ ID NO: 1. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide binds to activin (*e.g*., activin A and/or activin B). In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide binds to GDF11. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide binds to GDF11 and activin (*e.g*., activin A and/or activin B). In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide binds to GDF8. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide binds to GDF8 and GDF11. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide binds to GDF8, GDF11, and activin (*e.g*., activin A and/or activin B).

In certain aspects, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1. In some instances, the disclosure provides a composition for use in treating sickle-cell disease in a subject comprising a polypeptide comprising the sequence of amino acids 25-131 of SEQ ID NO: 1. In some instances, the disclosure provides a composition for use in treating sickle-cell disease in a subject comprising a polypeptide consisting of the sequence of amino acids 25-131 of SEQ ID NO:1. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide binds to activin (*e.g*., activin A and/or activin B). In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide binds to GDF11. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide binds to GDF11 and activin (*e.g*., activin A and/or activin B). In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide binds to GDF8. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide binds to GDF8 and GDF11. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide binds to GDF8, GDF11, and activin (*e.g*., activin A and/or activin B).

In certain aspects, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide comprises an acidic amino acid at position 79 with respect to SEQ ID NO:1. In some instances, the disclosure provides a composition for use in treating sickle-cell disease in a subject comprising a polypeptide comprising the sequence of amino acids 29-109 of SEQ ID NO: 1, wherein the polypeptide comprises an acidic amino acid at position 79 with respect to SEQ ID NO: 1. In some instances, the disclosure provides a composition for use in treating sickle-cell disease in a subject comprising a polypeptide consisting of the sequence of amino acids 29-109 of SEQ ID NO:1, wherein the polypeptide comprises an acidic amino acid at position 79 with respect to SEQ ID NO:1. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide comprises an acidic amino acid at position 79 with respect to SEQ ID NO:1, and wherein the polypeptide binds to GDF11. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide comprises an acidic amino acid at position 79 with respect to SEQ ID NO:1, wherein the polypeptide binds to GDF8. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide comprises an acidic amino acid at position 79 with respect to SEQ ID NO:1, and wherein the polypeptide binds to GDF8 and GDF11.

In certain aspects, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide comprises an acidic amino acid at position 79 with respect to SEQ ID NO: 1. In some instances, the disclosure provides a composition for use in treating sickle-cell disease in a subject comprising a polypeptide comprising the sequence of amino acids 25-131 of SEQ ID NO: 1, wherein the polypeptide comprises an acidic amino acid at position 79 with respect to SEQ ID NO:1. In some instances, the disclosure provides a composition for use in treating sickle-cell disease in a subject comprising a polypeptide consisting of the sequence of amino acids 25-131 of SEQ ID NO:1, wherein the polypeptide comprises an acidic amino acid at position 79 with respect to SEQ ID NO:1. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide comprises an acidic amino acid at position 79 with respect to SEQ ID NO:1, and wherein the polypeptide binds to GDF11. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide comprises an acidic amino acid at position 79 with respect to SEQ ID NO:1, and wherein the polypeptide binds to GDF8. In some instances, the disclosure provides a composition for use in treating sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide comprises an acidic amino acid at position 79 with respect to SEQ ID NO:1, and wherein the polypeptide binds to GDF8 and GDF11.

In certain aspects, the disclosure provides a composition for use in treating a complication of sickle-cell disease (*e.g*., anemia, anemia crisis, splenomegaly, pain crisis, chest syndrome, acute chest syndrome, blood transfusion requirement, organ damage, pain medicine requirement, splenic sequestration crises, hyperhemolytic crisis, vaso-occlusion, vaso-occlusion crisis, acute myocardial infarction, sickle-cell chronic lung disease, thromboemboli, hepatic failure, hepatomegaly, hepatic sequestration, iron overload, splenic infarction, acute or chronic renal failure, pyelonephritis, aneurysm, ischemic stroke, intraparenchymal hemorrhage, subarachnoid hemorrhage, intraventricular hemorrhage, peripheral retinal ischemia, proliferative sickle retinopathy, vitreous hemorrhage, and/or priapism) in a subject comprising an ActRII antagonist. In some instances, the disclosure provides a composition for use in treating a complication of sickle-cell disease in a subject comprising an ActRII antagonist, wherein the complication of sickle-cell disease is anemia. In some instances, the disclosure provides a composition for use in treating a complication of sickle-cell disease in a subject comprising an ActRII antagonist, wherein the complication of sickle-cell disease is anemia crisis. In some instances, the disclosure provides a composition for use in treating a complication of sickle-cell disease in a subject comprising an ActRII antagonist, wherein the complication of sickle-cell disease is splenomegaly. In some instances, the disclosure provides a composition for use in treating a complication of sickle-cell disease in a subject comprising an ActRII antagonist, wherein the complication of sickle-cell disease is pain crisis. In some instances, the disclosure provides a composition for use in treating a complication of sickle-cell disease in a subject comprising an ActRII antagonist, wherein the complication of sickle-cell disease is chest syndrome. In some instances, the disclosure provides a composition for use in treating a complication of sickle-cell disease in a subject comprising an ActRII antagonist, wherein the complication of sickle-cell disease is acute chest syndrome. In some instances, the disclosure provides a composition for use in treating a complication of sickle-cell disease in a subject comprising an ActRII antagonist, wherein the complication of sickle-cell disease is blood transfusion requirement. In some instances, the disclosure provides a composition for use in treating a complication of sickle-cell disease in a subject comprising an ActRII antagonist, wherein the complication of sickle-cell disease is organ damage. In some instances, the disclosure provides a composition for use in treating a complication of sickle-cell disease in a subject comprising an ActRII antagonist, wherein the complication of sickle-cell disease is pain medicine requirement. In some instances, the disclosure provides a composition for use in treating a complication of sickle-cell disease in a subject comprising an ActRII antagonist, wherein the complication of sickle-cell disease is splenic sequestration crises. In some instances, the disclosure provides a composition for use in treating a complication of sickle-cell disease in a subject comprising an ActRII antagonist, wherein the complication of sickle-cell disease is hyperhemolytic crisis. In some instances, the disclosure provides a composition for use in treating a complication of sickle-cell disease in a subject comprising an ActRII antagonist, wherein the complication of sickle-cell disease is vaso-occlusion. In some instances, the disclosure provides a composition for use in treating a complication of sickle-cell disease in a subject comprising an ActRII antagonist, wherein the complication of sickle-cell disease is vaso-occlusion crisis. In some instances, the disclosure provides a composition for use in treating a complication of sickle-cell disease in a subject comprising an ActRII antagonist, wherein the complication of sickle-cell disease is acute myocardial infarction. In some instances, the disclosure provides a composition for use in treating a complication of sickle-cell disease in a subject comprising an ActRII antagonist, wherein the complication of sickle-cell disease is sickle-cell chronic lung disease. In some instances, the disclosure provides a composition for use in treating a complication of sickle-cell disease in a subject comprising an ActRII antagonist, wherein the complication of sickle-cell disease is thromboemboli. In some instances, the disclosure provides a composition for use in treating a complication of sickle-cell disease in a subject comprising an ActRII antagonist, wherein the complication of sickle-cell disease is hepatic failure. In some instances, the disclosure provides a composition for use in treating a complication of sickle-cell disease in a subject comprising an ActRII antagonist, wherein the complication of sickle-cell disease is hepatomegaly. In some instances, the disclosure provides a composition for use in treating a complication of sickle-cell disease in a subject comprising an ActRII antagonist, wherein the complication of sickle-cell disease is hepatic sequestration. In some instances, the disclosure provides a composition for use in treating a complication of sickle-cell disease in a subject comprising an ActRII antagonist, wherein the complication of sickle-cell disease is iron overload. In some instances, the disclosure provides a composition for use in treating a complication of sickle-cell disease in a subject comprising an ActRII antagonist, wherein the complication of sickle-cell disease is splenic infarction. In some instances, the disclosure provides a composition for use in treating a complication of sickle-cell disease in a subject comprising an ActRII antagonist, wherein the complication of sickle-cell disease is acute or chronic renal failure. In some instances, the disclosure provides a composition for use in treating a complication of sickle-cell disease in a subject comprising an ActRII antagonist, wherein the complication of sickle-cell disease is pyelonephritis. In some instances, the disclosure provides a composition for use in treating a complication of sickle-cell disease in a subject comprising an ActRII antagonist, wherein the complication of sickle-cell disease is aneurysm. In some instances, the disclosure provides a composition for use in treating a complication of sickle-cell disease in a subject comprising an ActRII antagonist, wherein the complication of sickle-cell disease is ischemic stroke. In some instances, the disclosure provides a composition for use in treating a complication of sickle-cell disease in a subject comprising an ActRII antagonist, wherein the complication of sickle-cell disease is intraparenchymal hemorrhage. In some instances, the disclosure provides a composition for use in treating a complication of sickle-cell disease in a subject comprising an ActRII antagonist, wherein the complication of sickle-cell disease is subarachnoid hemorrhage. In some instances, the disclosure provides a composition for use in treating a complication of sickle-cell disease in a subject comprising an ActRII antagonist, wherein the complication of sickle-cell disease is intraventricular hemorrhage. In some instances, the disclosure provides a composition for use in treating a complication of sickle-cell disease in a subject comprising an ActRII antagonist, wherein the complication of sickle-cell disease is peripheral retinal ischemia. In some instances, the disclosure provides a composition for use in treating a complication of sickle-cell disease in a subject comprising an ActRII antagonist, wherein the complication of sickle-cell disease is proliferative sickle retinopathy. In some instances, the disclosure provides a composition for use in treating a complication of sickle-cell disease in a subject comprising an ActRII antagonist, wherein the complication of sickle-cell disease is vitreous hemorrhage. In some instances, the disclosure provides a composition for use in treating a complication of sickle-cell disease in a subject comprising an ActRII antagonist, wherein the complication of sickle-cell disease is priapism.

In certain aspects, the disclosure provides a composition for use in preventing a complication of sickle-cell disease (*e.g*., anemia, anemia crisis, splenomegaly, pain crisis, chest syndrome, acute chest syndrome, blood transfusion requirement, organ damage, pain medicine requirement, splenic sequestration crises, hyperhemolytic crisis, vaso-occlusion, vaso-occlusion crisis, acute myocardial infarction, sickle-cell chronic lung disease, thromboemboli, hepatic failure, hepatomegaly, hepatic sequestration, iron overload, splenic infarction, acute and/or chronic renal failure, pyelonephritis, aneurysm, ischemic stroke, intraparenchymal hemorrhage, subarachnoid hemorrhage, intraventricular hemorrhage, peripheral retinal ischemia, proliferative sickle retinopathy, vitreous hemorrhage, and/or priapism) in a subject comprising an ActRII antagonist.

In certain aspects, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:10. In some instances, the disclosure provides a composition for use in treating a complication of sickle-cell disease in a subject comprising a polypeptide comprising the amino acid sequence of SEQ ID NO:10. In some instances, the disclosure provides a composition for use in treating a complication of sickle-cell disease in a subject comprising a polypeptide consisting of the amino acid sequence of SEQ ID NO:10. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:10, wherein the polypeptide binds to activin (*e.g*., activin A and/or activin B). In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:10, wherein the polypeptide binds to GDF11. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:10, wherein the polypeptide binds to GDF11 and activin (*e.g.*, activin A and/or activin B). In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:10, wherein the polypeptide binds to GDF8. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:10, wherein the polypeptide binds to GDF8 and GDF11. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:10, wherein the polypeptide binds to GDF8, GDF11, and activin (*e.g.*, activin A and/or activin B).

In certain aspects, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:11. In some instances, the disclosure provides a composition for use in treating a complication of sickle-cell disease in a subject comprising a polypeptide comprising the amino acid sequence of SEQ ID NO:11. In some instances, the disclosure provides a composition for use in treating a complication of sickle-cell disease in a subject comprising a polypeptide consisting of the amino acid sequence of SEQ ID NO:11. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:1 1, wherein the polypeptide binds to activin (*e.g*., activin A and/or activin B). In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:11, wherein the polypeptide binds to GDF11. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:1 1, wherein the polypeptide binds to GDF11 and activin (*e.g*., activin A and/or activin B). In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:11, wherein the polypeptide binds to GDF8. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:11, wherein the polypeptide binds to GDF8 and GDF11. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:11, wherein the polypeptide binds to GDF8, GDF11, and activin (*e.g*., activin A and/or activin B).

In certain aspects, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:22. In some instances, the disclosure provides a composition for use in treating a complication of sickle-cell disease in a subject comprising a polypeptide comprising the amino acid sequence of SEQ ID NO:22. In some instances, the disclosure provides a composition for use in treating a complication of sickle-cell disease in a subject comprising a polypeptide consisting of the amino acid sequence of SEQ ID NO:22. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:22, wherein the polypeptide binds to activin (*e.g*., activin A and/or activin B). In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:22, wherein the polypeptide binds to GDF11. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:22, wherein the polypeptide binds to GDF11 and activin (*e.g*., activin A and/or activin B). In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:22, wherein the polypeptide binds to GDF8. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:22, wherein the polypeptide binds to GDF8 and GDF11. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:22, wherein the polypeptide binds to GDF8, GDF11, and activin (*e.g*., activin A and/or activin B).

In certain aspects, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:36. In some instances, the disclosure provides a composition for use in treating a complication of sickle-cell disease in a subject comprising a polypeptide comprising the amino acid sequence of SEQ ID NO:36. In some instances, the disclosure provides a composition for use in treating a complication of sickle-cell disease in a subject comprising a polypeptide consisting of the amino acid sequence of SEQ ID NO:36. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:36, wherein the polypeptide binds to GDF11. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:36, wherein the polypeptide binds to GDF8. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:36, wherein the polypeptide binds to GDF8 and GDF11.

In certain aspects, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:37. In some instances, the disclosure provides a composition for use in treating a complication of sickle-cell disease in a subject comprising a polypeptide comprising the amino acid sequence of SEQ ID NO:37. In some instances, the disclosure provides a composition for use in treating a complication of sickle-cell disease in a subject comprising a polypeptide consisting of the amino acid sequence of SEQ ID NO:37. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:37, wherein the polypeptide binds to GDF11. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:37, wherein the polypeptide binds to GDF8. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:37, wherein the polypeptide binds to GDF8 and GDF11.

In certain aspects, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:44. In some instances, the disclosure provides a composition for use in treating a complication of sickle-cell disease in a subject comprising a polypeptide comprising the amino acid sequence of SEQ ID NO:44. In some instances, the disclosure provides a composition for use in treating a complication of sickle-cell disease in a subject comprising a polypeptide consisting of the amino acid sequence of SEQ ID NO:44. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:44, wherein the polypeptide binds to GDF11. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:44, wherein the polypeptide binds to GDF8. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the amino acid sequence of SEQ ID NO:44, wherein the polypeptide binds to GDF8 and GDF11.

In certain aspects, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1. In some instances, the disclosure provides a composition for use in treating a complication of sickle-cell disease in a subject comprising a polypeptide comprising the sequence of amino acids 29-109 of SEQ ID NO:1. In some instances, the disclosure provides a composition for use in treating a complication of sickle-cell disease in a subject comprising a polypeptide consisting of the sequence of amino acids 29-109 of SEQ ID NO:1. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide binds to activin (*e.g*., activin A and/or activin B). In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide binds to GDF11. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide binds to GDF11 and activin (*e.g*., activin A and/or activin B). In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide binds to GDF8. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide binds to GDF8 and GDF11. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide binds to GDF8, GDF11, and activin (*e.g*., activin A and/or activin B).

In certain aspects, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1. In some instances, the disclosure provides a composition for use in treating a complication of sickle-cell disease in a subject comprising a polypeptide comprising the sequence of amino acids 25-131 of SEQ ID NO:1. In some instances, the disclosure provides a composition for use in treating a complication of sickle-cell disease in a subject comprising a polypeptide consisting of the sequence of amino acids 25-131 of SEQ ID NO:1. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide binds to activin (*e.g.*, activin A and/or activin B). In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide binds to GDF11. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide binds to GDF11 and activin (*e.g*., activin A and/or activin B). In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide binds to GDF8. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide binds to GDF8 and GDF11. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide binds to GDF8, GDF11, and activin (*e.g*., activin A and/or activin B).

In certain aspects, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide comprises an acidic amino acid at position 79 with respect to SEQ ID NO:1. In some instances, the disclosure provides a composition for use in treating a complication of sickle-cell disease in a subject comprising a polypeptide comprising the sequence of amino acids 29-109 of SEQ ID NO:1, wherein the polypeptide comprises an acidic amino acid at position 79 with respect to SEQ ID NO: 1. In some instances, the disclosure provides a composition for use in treating a complication of sickle-cell disease in a subject comprising a polypeptide consisting of the sequence of amino acids 29-109 of SEQ ID NO:1, wherein the polypeptide comprises an acidic amino acid at position 79 with respect to SEQ ID NO:1. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide comprises an acidic amino acid at position 79 with respect to SEQ ID NO:1, and wherein the polypeptide binds to GDF11. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide comprises an acidic amino acid at position 79 with respect to SEQ ID NO:1, wherein the polypeptide binds to GDF8. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 29-109 of SEQ ID NO:1, wherein the polypeptide comprises an acidic amino acid at position 79 with respect to SEQ ID NO:1, and wherein the polypeptide binds to GDF8 and GDF11.

In certain aspects, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide comprises an acidic amino acid at position 79 with respect to SEQ ID NO:1. In some instances, the disclosure provides a composition for use in treating a complication of sickle-cell disease in a subject comprising a polypeptide comprising the sequence of amino acids 25-131 of SEQ ID NO:1, wherein the polypeptide comprises an acidic amino acid at position 79 with respect to SEQ ID NO:1. In some instances, the disclosure provides a composition for use in treating a complication of sickle-cell disease in a subject comprising a polypeptide consisting of the sequence of amino acids 25-131 of SEQ ID NO:1, wherein the polypeptide comprises an acidic amino acid at position 79 with respect to SEQ ID NO:1. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide comprises an acidic amino acid at position 79 with respect to SEQ ID NO:1, and wherein the polypeptide binds to GDF11. In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide comprises an acidic amino acid at position 79 with respect to SEQ ID NO:1, and wherein the polypeptide binds to GDF8.

In some instances, the disclosure provides a composition for use in treating a complication of sickle-cell disease in a subject comprising a polypeptide comprising an amino acid sequence that is at least 80% (*e.g.,* at least 85%, 90%, 95%, 96%, 97%, 98%, 99%, or 100%) identical to the sequence of amino acids 25-131 of SEQ ID NO:1, wherein the polypeptide comprises an acidic amino acid at position 79 with respect to SEQ ID NO:1, and wherein the
polypeptide binds to GDF8 and GDF11.

### BRIEF DESCRIPTION OF THE DRAWINGS

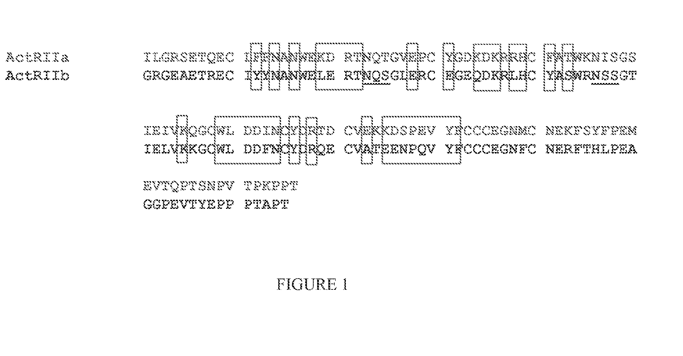

Figure 1 shows an alignment of extracellular domains of human ActRIIA (SEQ ID NO: 56) and human ActRIIB (SEQ ID NO: 2) with the residues that are deduced herein, based on composite analysis of multiple ActRIIB and ActRIIA crystal structures, to directly contact ligand indicated with boxes.

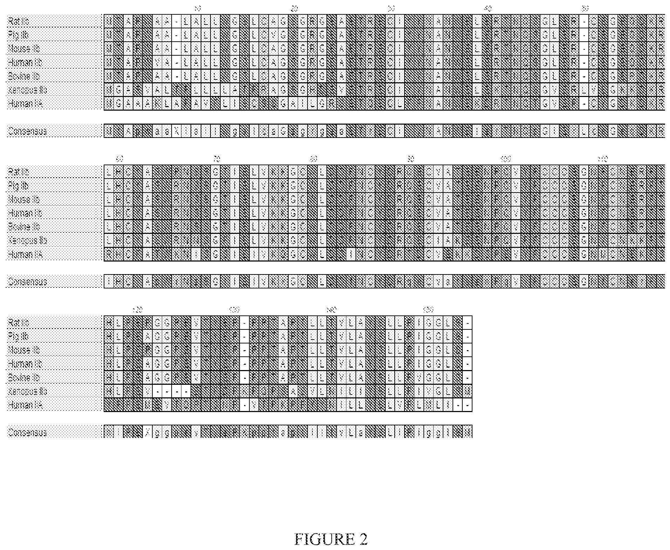

Figure 2 shows a multiple sequence alignment of various vertebrate ActRIIB proteins and human ActRIIA (SEQ ID NOs: 57-64).

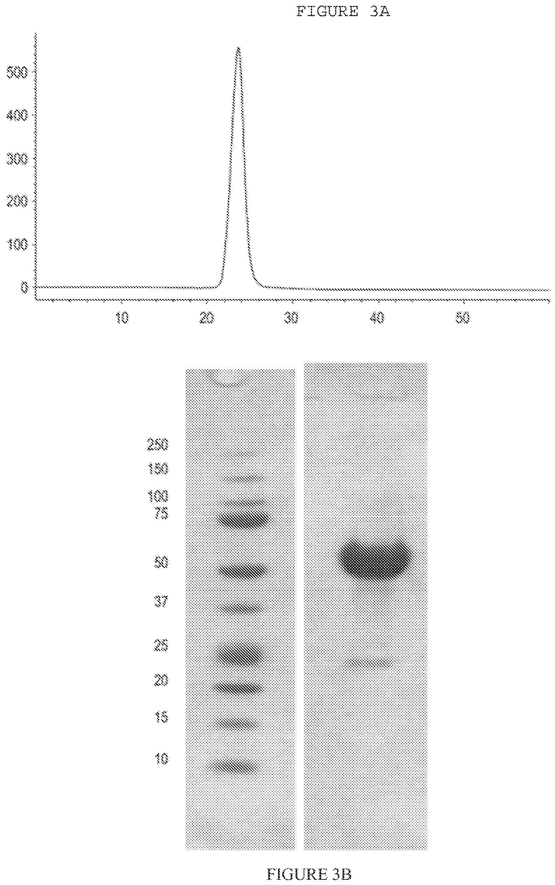

Figures 3A and 3B show the purification of ActRIIA-hFc expressed in CHO cells. The protein purifies as a single, well-defined peak as visualized by sizing column (3A) and Coomassie stained SDS-PAGE (3B) (left lane: molecular weight standards; right lane: ActRIIA-hFc).

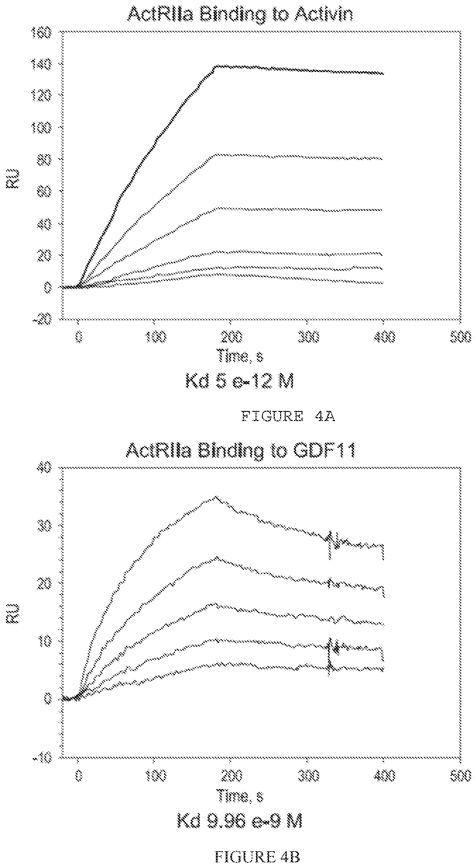

Figures 4A and 4B show the binding of ActRIIA-hFc to activin and GDF-11, as measured by Biacore™ assay.

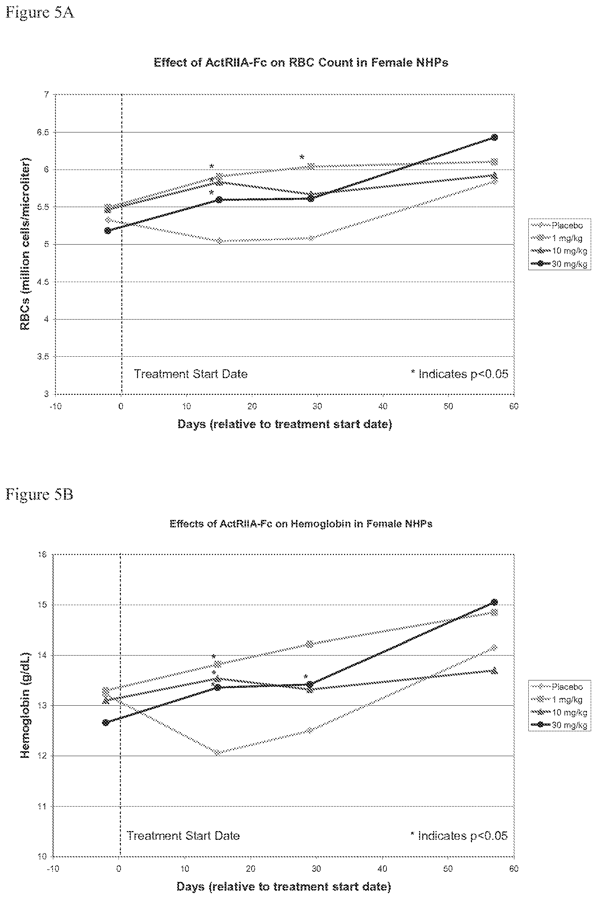

Figures 5A and 5B show the effects of ActRIIA-hFc on red blood cell counts in female non-human primates (NHPs). Female cynomolgus monkeys (four groups of five monkeys each) were treated with placebo or 1 mg/kg, 10 mg/kg or 30 mg/kg of ActRIIA-hFc on day 0, day 7, day 14, and day 21. Figure 5A shows red blood cell (RBC) counts. Figure 5B shows hemoglobin levels. Statistical significance is relative to baseline for each treatment group. At day 57, two monkeys remained in each group.

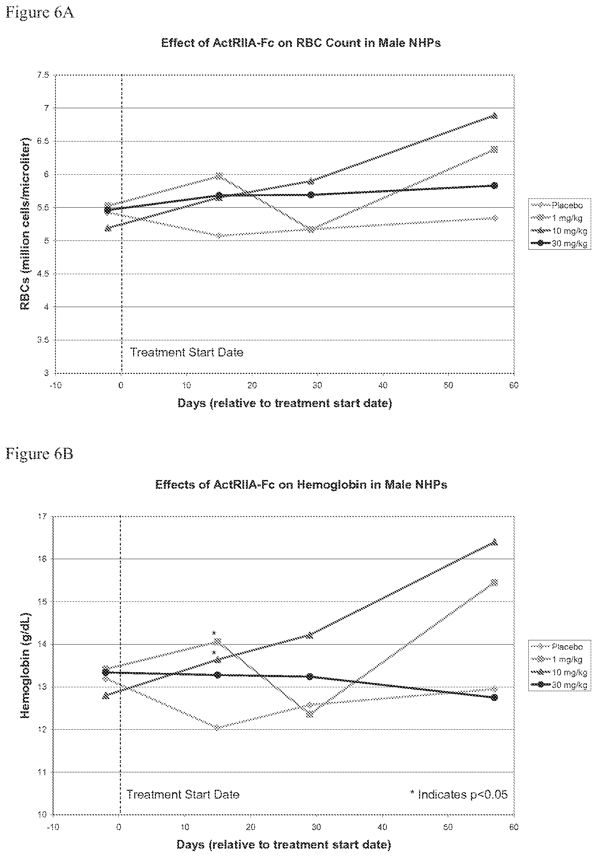

Figures 6A and 6B show the effects of ActRIIA-hFc on red blood cell counts in male non-human primates. Male cynomolgus monkeys (four groups of five monkeys each) were treated with placebo or 1 mg/kg, 10 mg/kg, or 30 mg/kg of ActRIIA-hFc on day 0, day 7, day 14, and day 21. Figure 6A shows red blood cell (RBC) counts. Figure 6B shows hemoglobin levels. Statistical significance is relative to baseline for each treatment group. At day 57, two monkeys remained in each group.

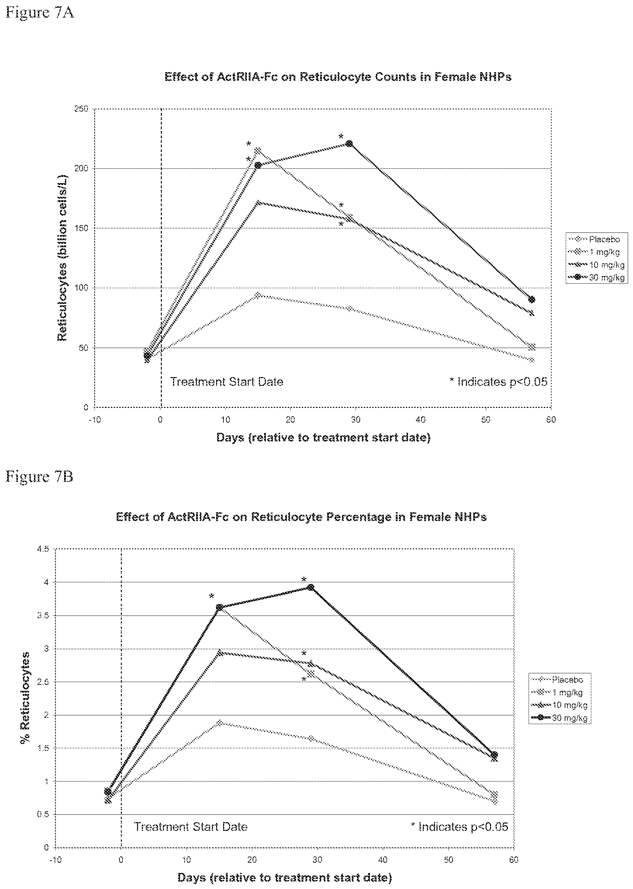

Figures 7A and 7B show the effects of ActRIIA-hFc on reticulocyte counts in female non-human primates. Cynomolgus monkeys (four groups of five monkeys each) were treated with placebo or 1 mg/kg, 10 mg/kg, or 30 mg/kg of ActRIIA-hFc on day 0, day 7, day 14, and day 21. Figure 7A shows absolute reticulocyte counts. Figure 7B shows the percentage of reticulocytes relative to RBCs. Statistical significance is relative to baseline for each group. At day 57, two monkeys remained in each group.

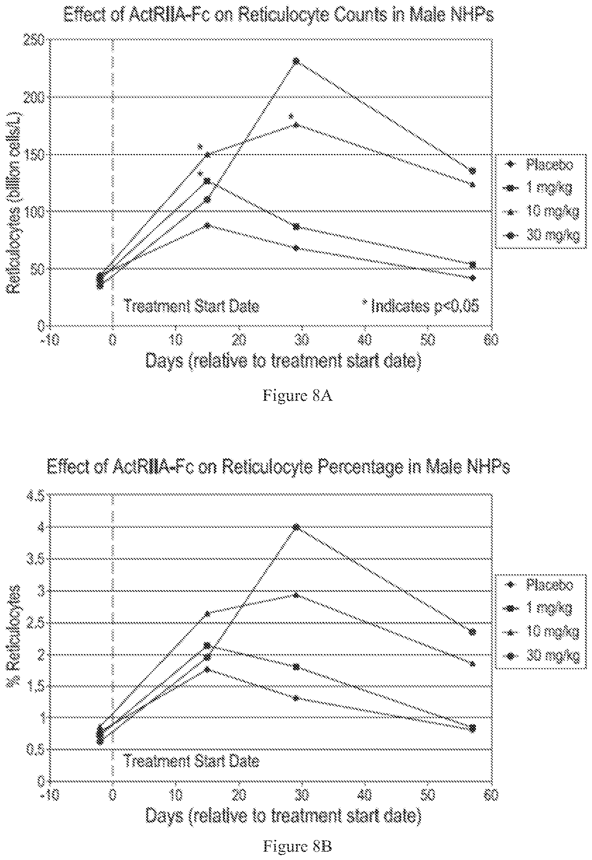

Figures 8A and 8B show the effects of ActRIIA-hFc on reticulocyte counts in male non-human primates. Cynomolgus monkeys (four groups of five monkeys each) were treated with placebo or 1 mg/kg, 10 mg/kg, or 30 mg/kg of ActRIIA-hFc on day 0, day 7, day 14, and day 21. Figure 8A shows absolute reticulocyte counts. Figure 8B shows the percentage of reticulocytes relative to RBCs. Statistical significance is relative to baseline for each group. At day 57, two monkeys remained in each group.

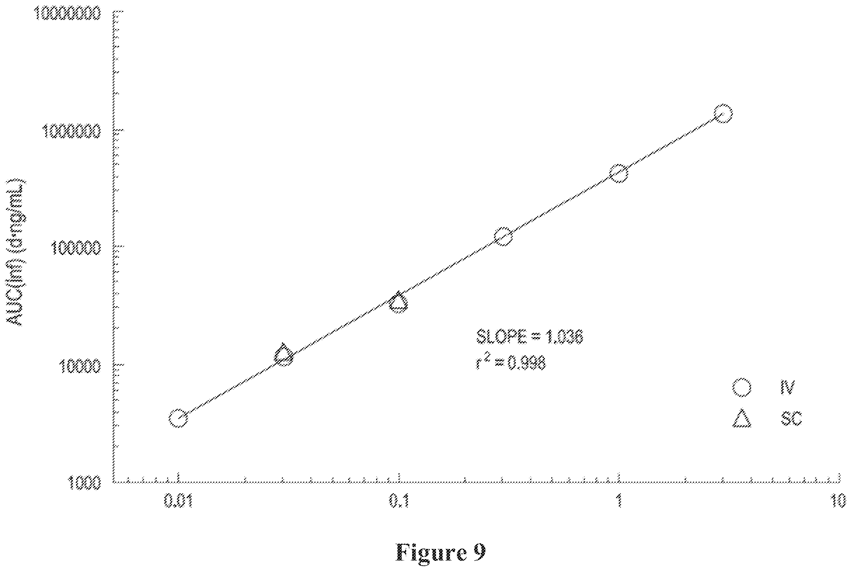

Figure 9 shows results from the human clinical trial described in Example 4, where the area-under-curve (AUC) and administered dose of ActRIIA-hFc have a linear correlation, regardless of whether ActRIIA-hFc was administered intravenously (IV) or subcutaneously (SC).

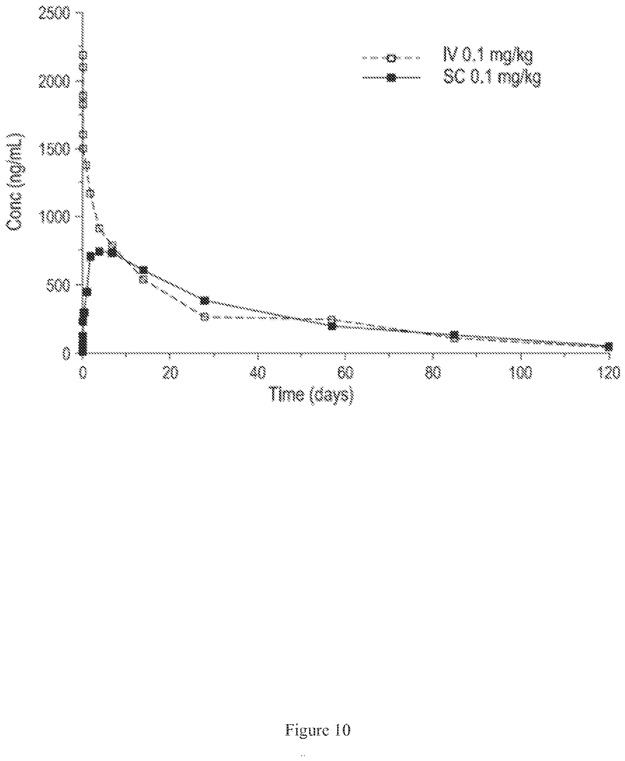

Figure 10 shows a comparison of serum levels of ActRIIA-hFc in patients administered IV or SC.

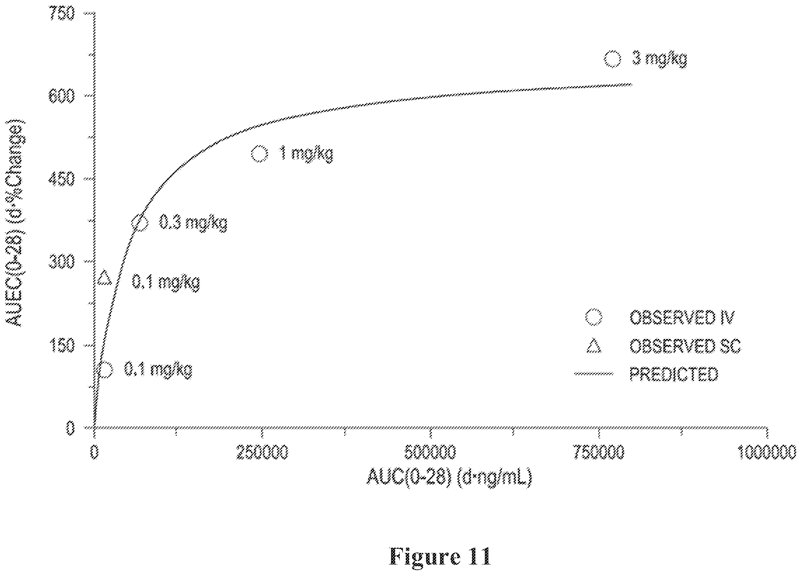

Figure 11 shows bone alkaline phosphatase (BAP) levels in response to different dose levels of ActRIIA-hFc. BAP is a marker for anabolic bone growth.

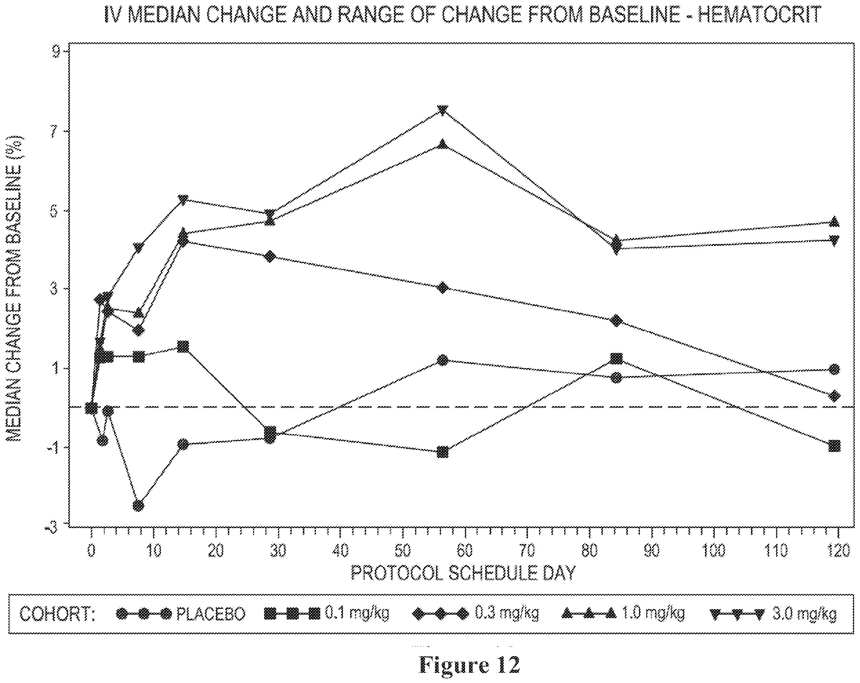

Figure 12 depicts the median change from baseline of hematocrit levels from the human clinical trial described in Example 4. ActRIIA-hFc was administered intravenously (IV) at the indicated dosage.

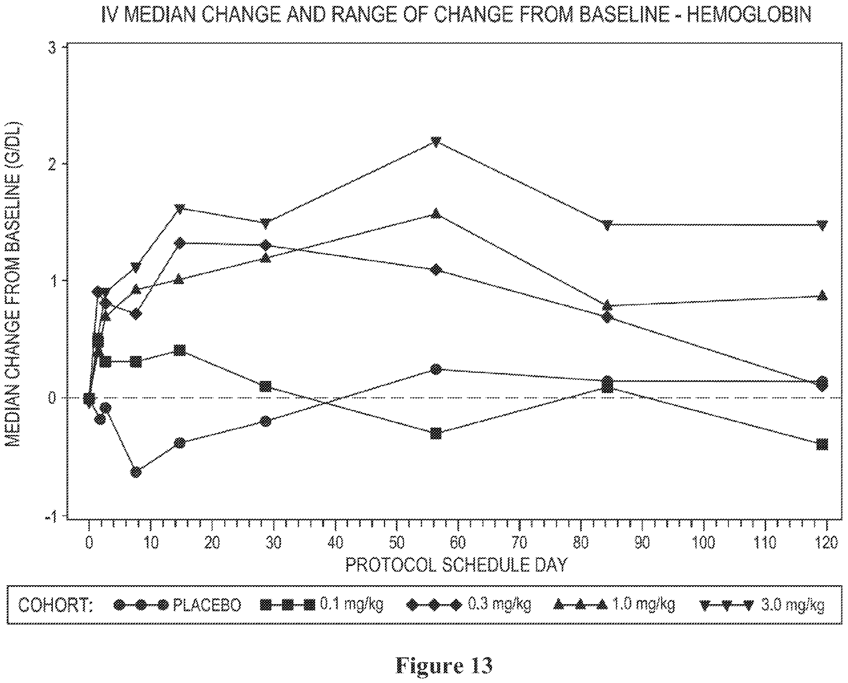

Figure 13 depicts the median change from baseline of hemoglobin levels from the human clinical trial described in Example 4. ActRIIA-hFc was administered intravenously (IV) at the indicated dosage.

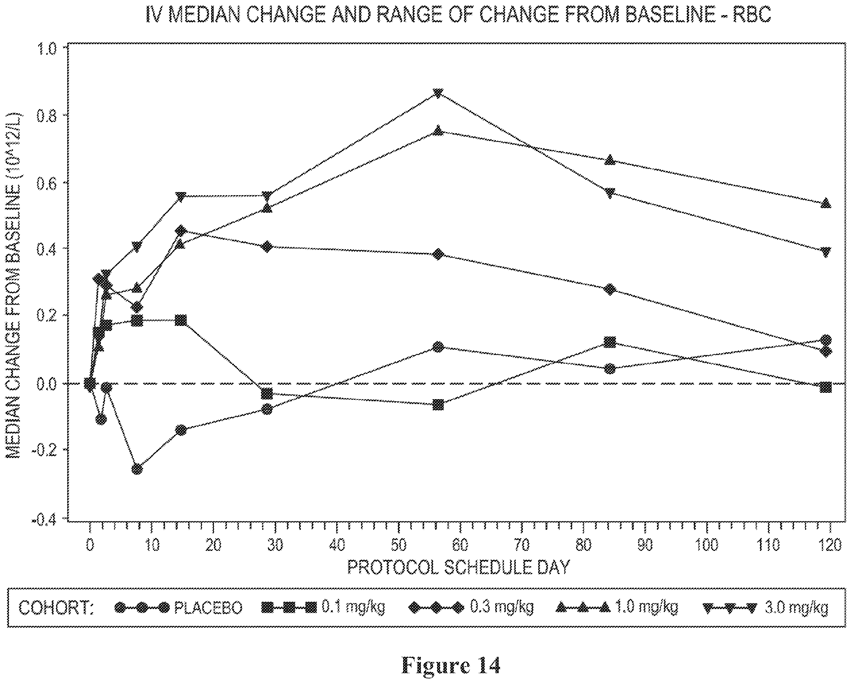

Figure 14 depicts the median change from baseline of RBC (red blood cell) count from the human clinical trial described in Example 4. ActRIIA-hFc was administered intravenously (IV) at the indicated dosage.

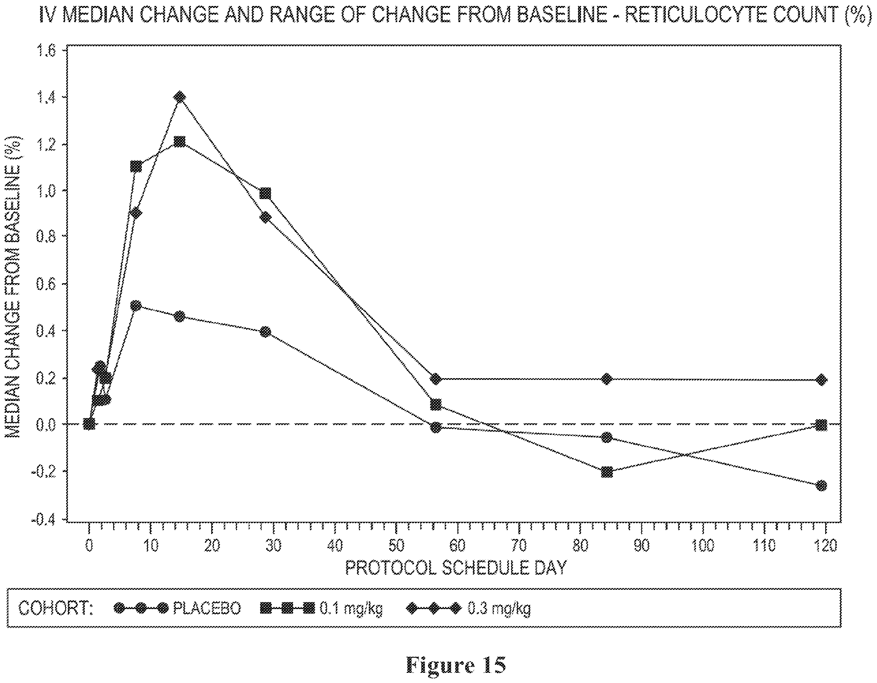

Figure 15 depicts the median change from baseline of reticulocyte count from the human clinical trial described in Example 4. ActRIIA-hFc was administered intravenously (IV) at the indicated dosage.

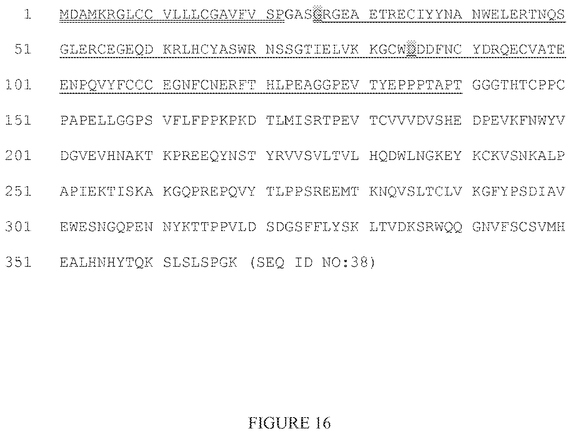

Figure 16 shows the full amino acid sequence for the GDF trap ActRIIB(L79D 20-134)-hFc (SEQ ID NO:38), including the TPA leader sequence (double underlined), ActRIIB extracellular domain (residues 20-134 in SEQ ID NO: 1; underlined), and hFc domain. The aspartate substituted at position 79 in the native sequence is double underlined and highlighted, as is the glycine revealed by sequencing to be the N-terminal residue in the mature fusion protein.

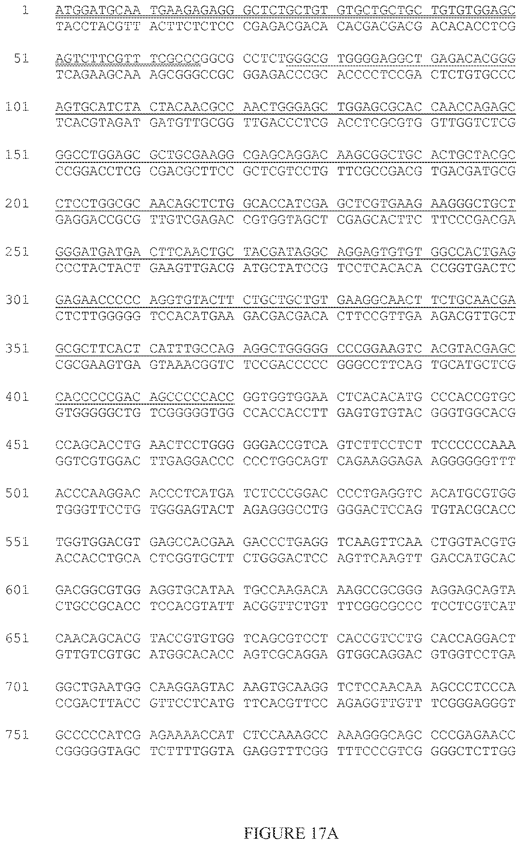

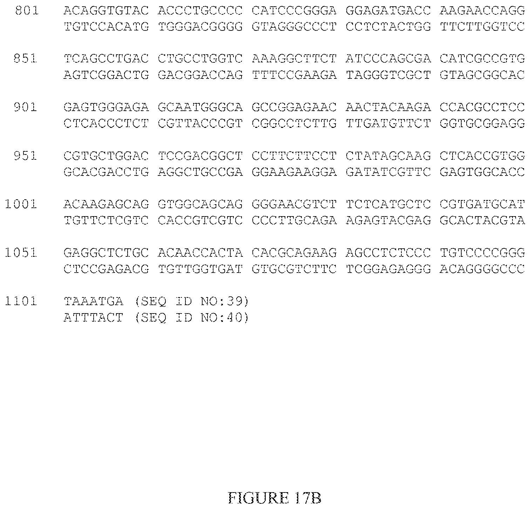

Figures 17A and 17B show a nucleotide sequence encoding ActRIIB(L79D 20-134)-hFc. SEQ ID NO:39 corresponds to the sense strand, and SEQ ID NO:40 corresponds to the antisense strand. The TPA leader (nucleotides 1-66) is double underlined, and the ActRIIB extracellular domain (nucleotides 76-420) is underlined.

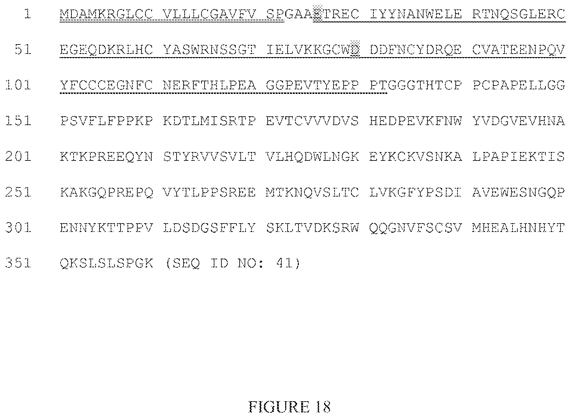

Figure 18 shows the full amino acid sequence for the truncated GDF trap ActRIIB(L79D 25-131)-hFc (SEQ ID NO:41), including the TPA leader (double underlined), truncated ActRIIB extracellular domain (residues 25-131 in SEQ ID NO:1; underlined), and hFc domain. The aspartate substituted at position 79 in the native sequence is double underlined and highlighted, as is the glutamate revealed by sequencing to be the N-terminal residue in the mature fusion protein.

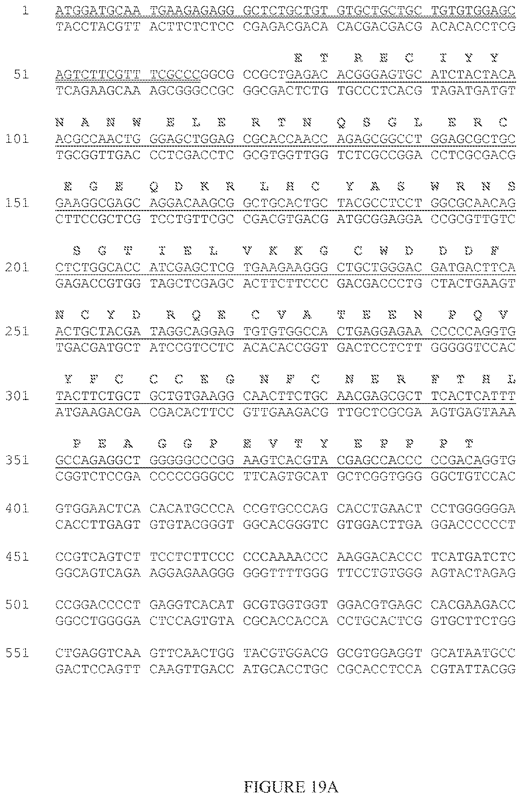

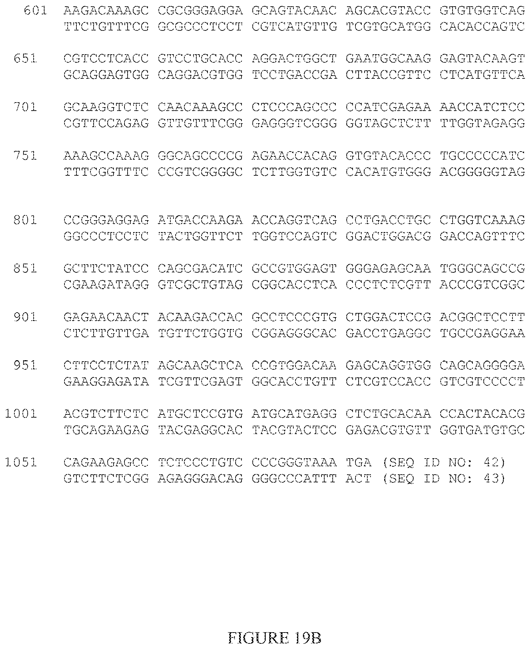

Figures 19A and 19B show a nucleotide sequence encoding ActRIIB(L79D 25-131)-hFc. SEQ ID NO:42 corresponds to the sense strand, and SEQ ID NO:43 corresponds to the antisense strand. The TPA leader (nucleotides 1-66) is double underlined, and the truncated ActRIIB extracellular domain (nucleotides 76-396) is underlined. The amino acid sequence for the ActRIIB extracellular domain (residues 25-131 in SEQ ID NO: 1) is also shown.

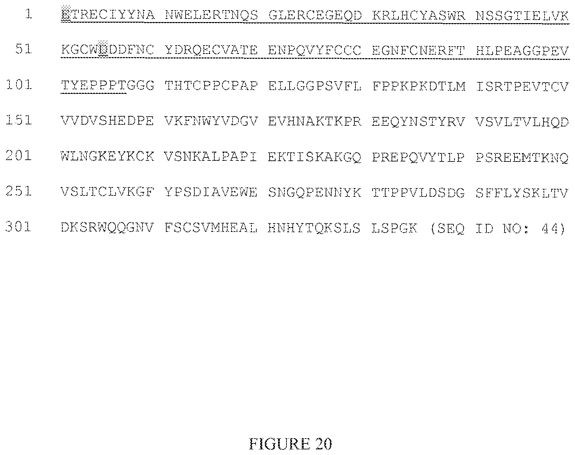

Figure 20 shows the amino acid sequence for the truncated GDF trap ActRIIB(L79D 25-131)-hFc without a leader (SEQ ID NO:44). The truncated ActRIIB extracellular domain (residues 25-131 in SEQ ID NO:1) is underlined. The aspartate substituted at position 79 in the native sequence is double underlined and highlighted, as is the glutamate revealed by sequencing to be the N-terminal residue in the mature fusion protein.

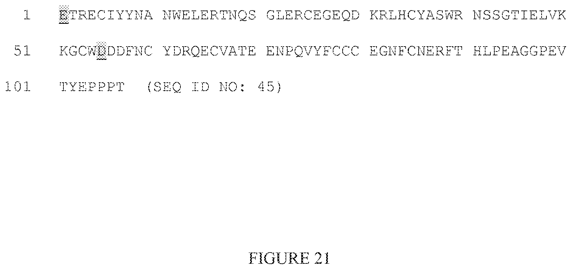

Figure 21 shows the amino acid sequence for the truncated GDF trap ActRIIB(L79D 25-131) without the leader, hFc domain, and linker (SEQ ID NO:45). The aspartate substituted at position 79 in the native sequence is underlined and highlighted, as is the glutamate revealed by sequencing to be the N-terminal residue in the mature fusion protein.

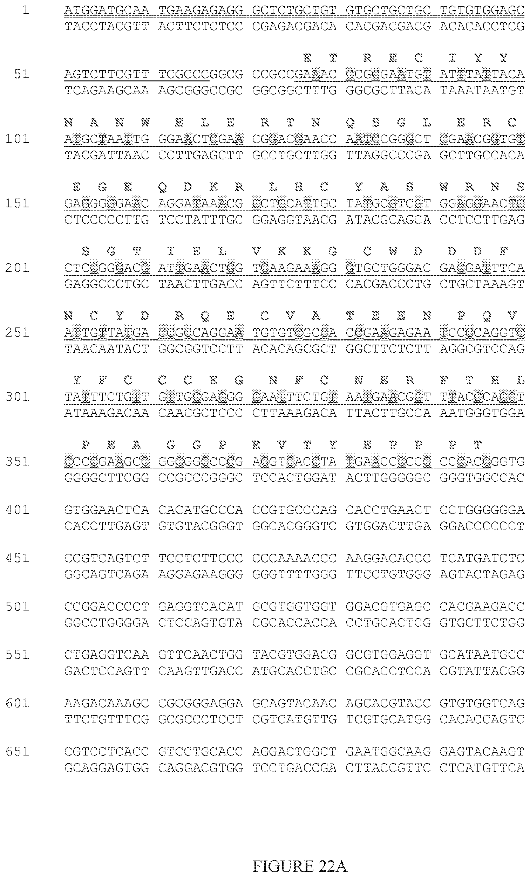

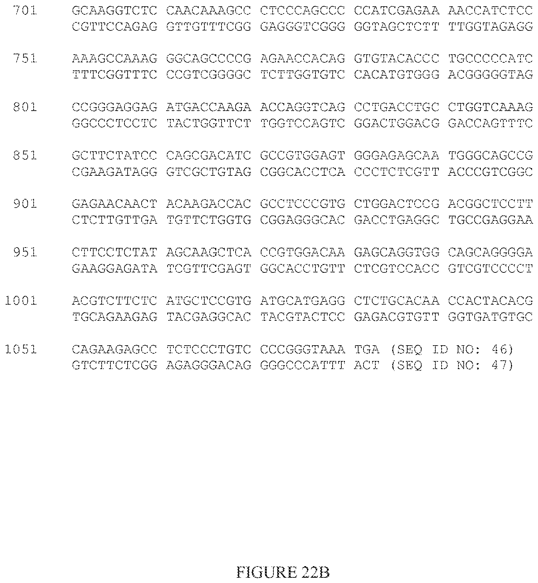

Figures 22A and 22B show an alternative nucleotide sequence encoding ActRIIB(L79D 25-131)-hFc. SEQ ID NO:46 corresponds to the sense strand, and SEQ ID NO:47 corresponds to the antisense strand. The TPA leader (nucleotides 1-66) is double underlined, the truncated ActRIIB extracellular domain (nucleotides 76-396) is underlined, and substitutions in the wild-type nucleotide sequence of the extracellular domain are double underlined and highlighted (compare with SEQ ID NO:42, Figure 19). The amino acid sequence for the ActRIIB extracellular domain (residues 25-131 in SEQ ID NO:1) is also shown.

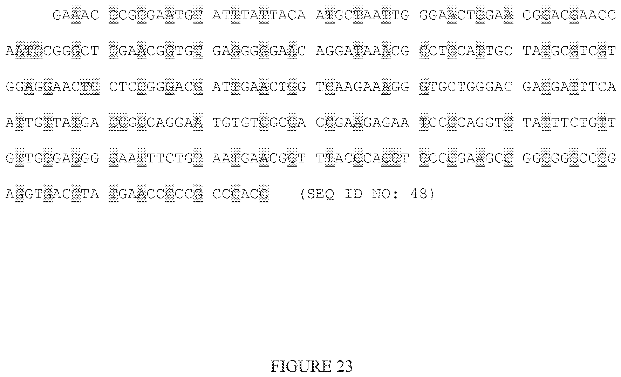

Figure 23 shows nucleotides 76-396 (SEQ ID NO:48) of the alternative nucleotide sequence shown in Figure 22 (SEQ ID NO:46). The same nucleotide substitutions indicated in Figure 22 are also underlined and highlighted here. SEQ ID NO:48 encodes only the truncated ActRIIB extracellular domain (corresponding to residues 25-131 in SEQ ID NO:1) with a L79D substitution, *e.g.,* ActRIIB(L79D 25-131).

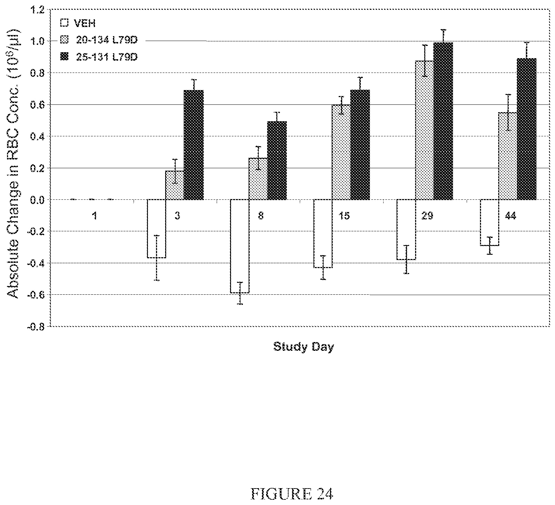

Figure 24 shows the effect of treatment with ActRIIB(L79D 20-134)-hFc (gray) or ActRIIB(L79D 25-131)-hFc (black) on the absolute change in red blood cell concentration from baseline in cynomolgus monkey. VEH = vehicle. Data are means ± SEM. n = 4-8 per group.

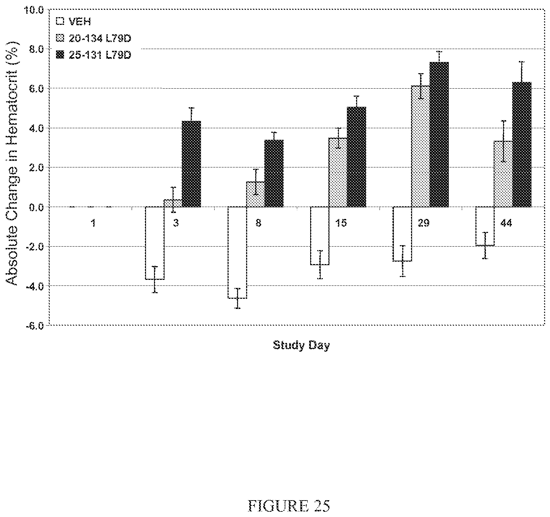

Figure 25 shows the effect of treatment with ActRIIB(L79D 20-134)-hFc (gray) or ActRIIB(L79D 25-131)-hFc (black) on the absolute change in hematocrit from baseline in cynomolgus monkey. VEH = vehicle. Data are means ± SEM. n = 4-8 per group.

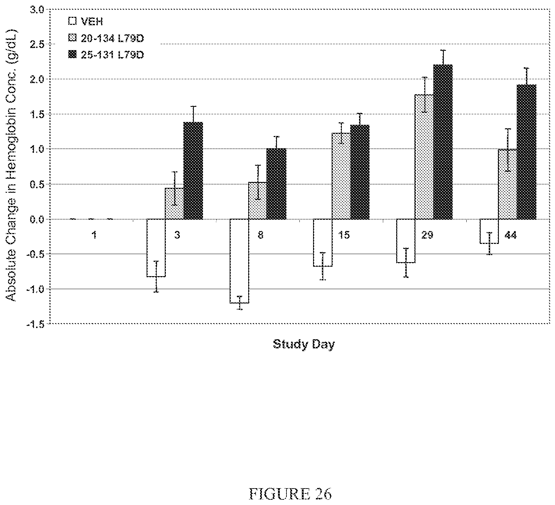

Figure 26 shows the effect of treatment with ActRIIB(L79D 20-134)-hFc (gray) or ActRIIB(L79D 25-131)-hFc (black) on the absolute change in hemoglobin concentration from baseline in cynomolgus monkey. VEH = vehicle. Data are means ± SEM. n = 4-8 per group.

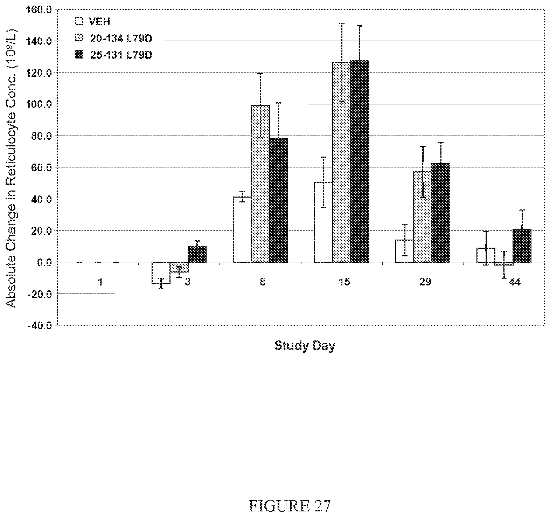

Figure 27 shows the effect of treatment with ActRIIB(L79D 20-134)-hFc (gray) or ActRIIB(L79D 25-131)-hFc (black) on the absolute change in circulating reticulocyte concentration from baseline in cynomolgus monkey. VEH = vehicle. Data are means ± SEM. n = 4-8 per group.

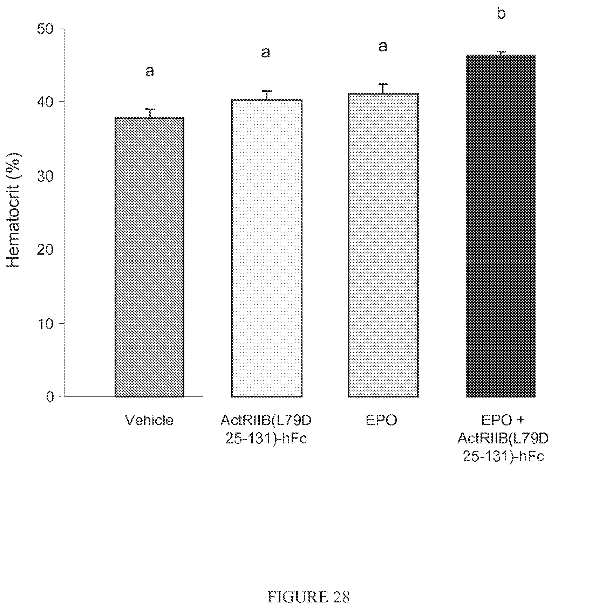

Figure 28 shows the effect of combined treatment with erythropoietin (EPO) and ActRIIB(L79D 25-131)-hFc for 72 hours on hematocrit in mice. Data are means ± SEM (n = 4 per group), and means that are significantly different from each other (p < 0.05, unpaired t-test) are designated by different letters. Combined treatment increased hematocrit by 23% compared to vehicle, a synergistic increase greater than the sum of the separate effects of EPO and ActRIIB(L79D 25-131)-hFc.

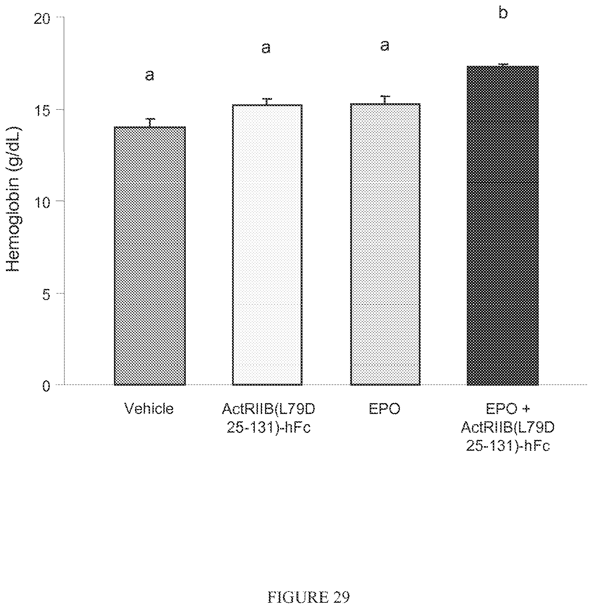

Figure 29 shows the effect of combined treatment with EPO and ActRIIB(L79D 25-131)-hFc for 72 hours on hemoglobin concentrations in mice. Data are means ± SEM (n = 4 per group), and means that are significantly different from each other (p < 0.05) are designated by different letters. Combined treatment increased hemoglobin concentrations by 23% compared to vehicle, which was also a synergistic effect.

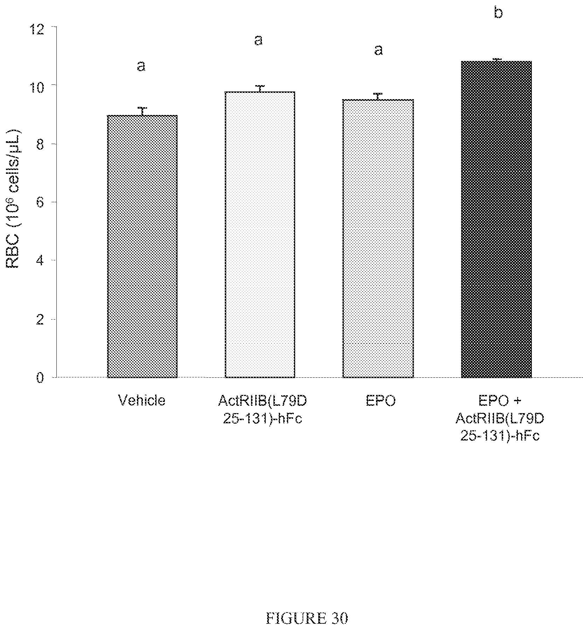

Figure 30 shows the effect of combined treatment with EPO and ActRIIB(L79D 25-131)-hFc for 72 hours on red blood cell (RBC) concentrations in mice. Data are means ± SEM (n = 4 per group), and means that are significantly different from each other (p < 0.05) are designated by different letters. Combined treatment increased red blood cell concentrations by 20% compared to vehicle, which was also a synergistic effect.

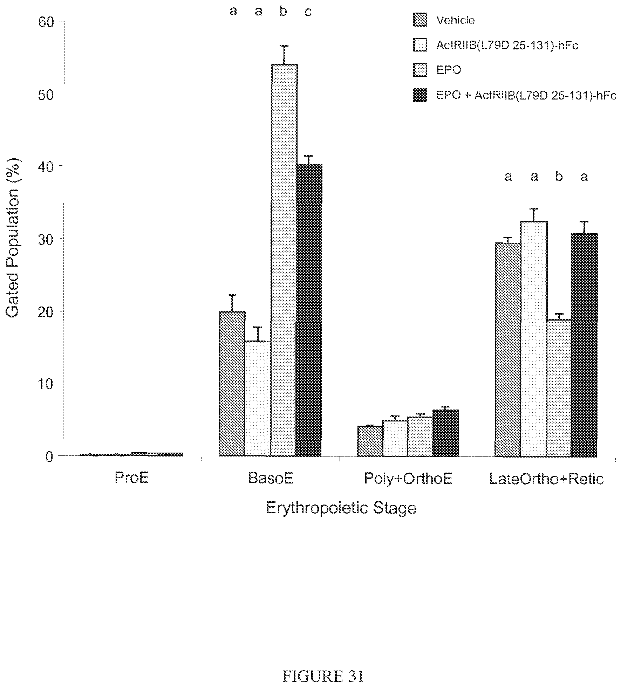

Figure 31 shows the effect of combined treatment with EPO and ActRIIB(L79D 25-131)-hFc for 72 hours on numbers of erythropoietic precursor cells in mouse spleen. Data are means ± SEM (n = 4 per group), and means that are significantly different from each other (p < 0.01) are designated by different letters. Whereas EPO alone increased the number of basophilic erythroblasts (BasoE) dramatically at the expense of late-stage precursor maturation, combined treatment increased BasoE numbers to a lesser but still significant extent while supporting undiminished maturation of late-stage precursors.

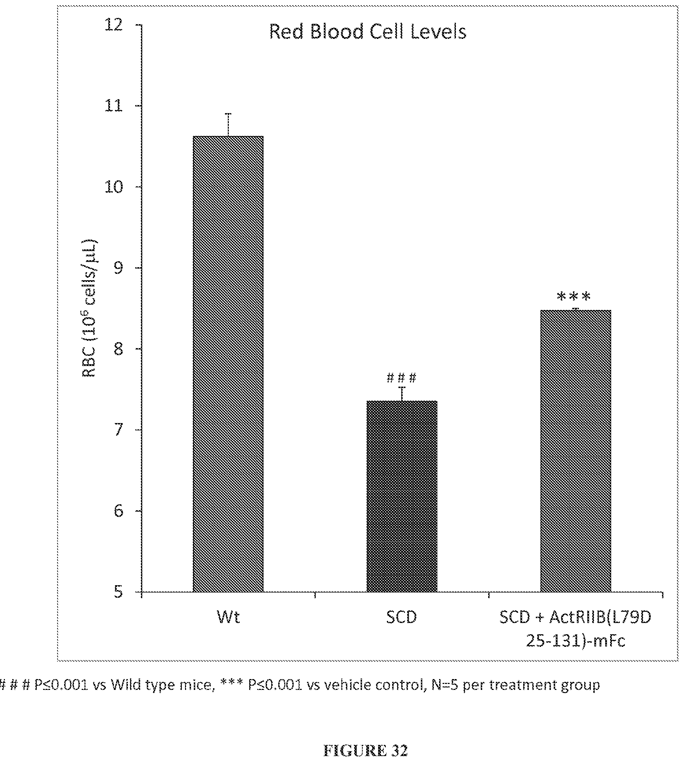

Figure 32 shows the effect of ActRIIB(L79D 25-131)-mFc on the absolute change in red blood cell (RBC) concentration in sickle-cell disease (SCD) mice. Data are means ± SEM (n = 5 per group). Wt = wild-type mice, which were non-symptomatic compound heterozygote (β/β^{S}) mice. ActRIIB(L79D 25-131)-mFc treatment resulted in a significant increase in red blood cell levels in sickle-cell mice (P ≤ 0.001) in comparision to control mice (sickle-cell mice administered vehicle alone).

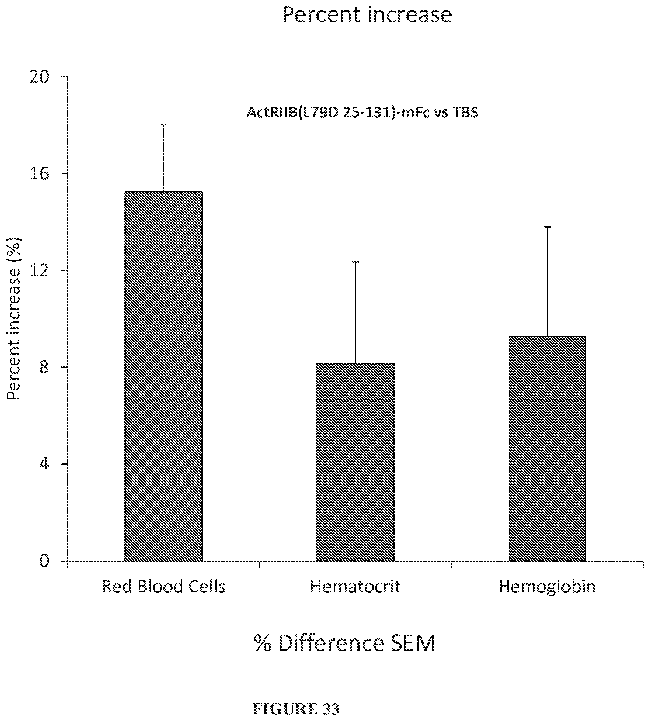

Figure 33 shows the effect of ActRIIB(L79D 25-131)-mFc on red blood cell levels, hematocrit levels, and hemoglobin levels in sickle-cell mice. Data are mean changes from baseline over 4 weeks (± SEM) vs. vehicle-treated sickle-cell control mice. ActRIIB(L79D 25-13 1)-mFc treatment resulted in a significant increase in red blood cell levels, hematocrit levels, and hemoglobin levels in sickle-cell mice in comparision to vehicle-treated sickle-cell mice (control).

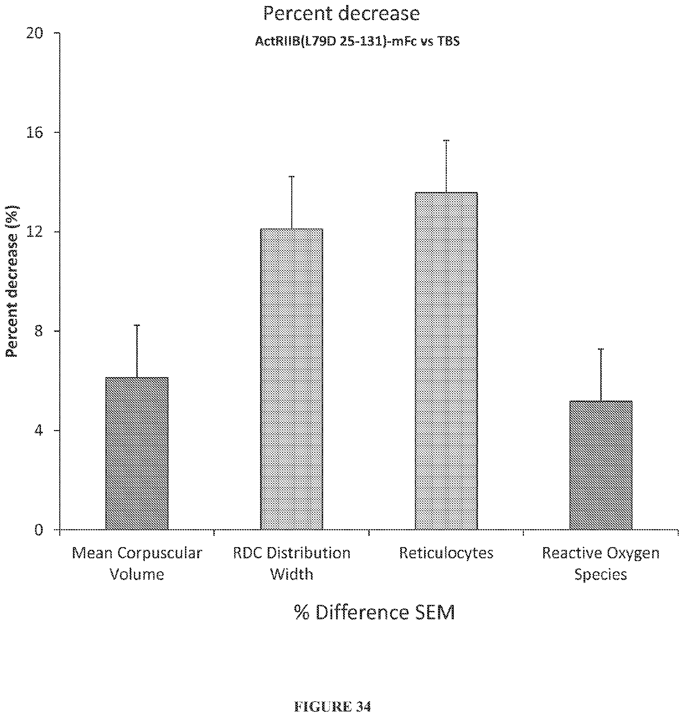

Figure 34 shows the effect of ActRIIB(L79D 25-13 1)-mFc on various blood parameters (*i.e.,* mean corpuscular volume, red blood cell (RDC) distribution width, reticulocytes, and reactive oxygen species) in sickle-cell mice. Data are mean changes from baseline over 4 weeks (± SEM) vs. vehicle-treated sickle-cell mice (control). ActRIIB(L79D 25-13 1)-mFc treatment resulted in a significant increase in mean corpuscular volume, red blood cell (RDC) distribution width, reticulocytes, and reactive oxygen species in sickle-cell mice in comparison to control mice.

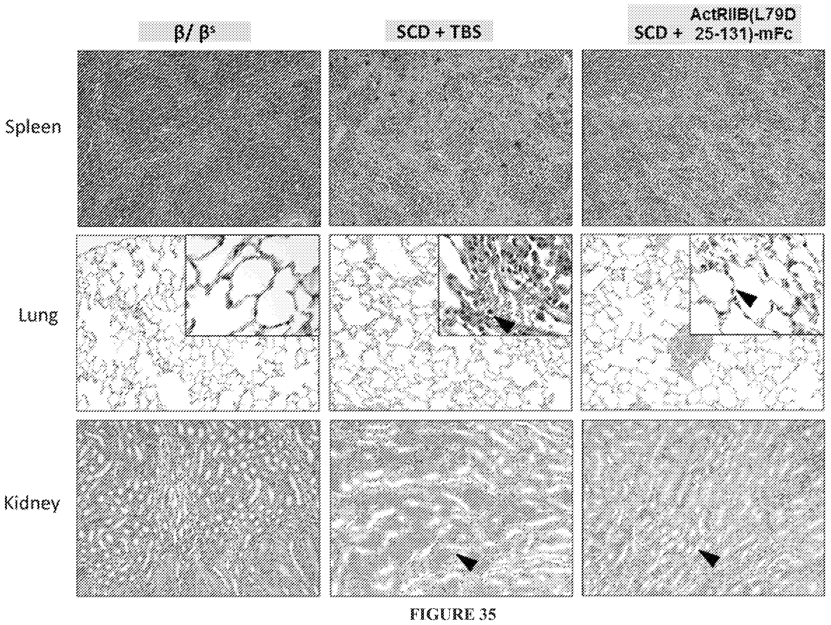

Figure 35 shows the effect of ActRIIB(L79D 25-13 1)-mFc on end organ damage in SCD mice. Histological samples (spleen, lung, and kidney) from β/β^{S} mice, SCD mice treated with vehicle (TBS) alone (s.c., twice weekly for six weeks), and SCD mice treated with ActRIIB(L79D 25-13 1)-mFc (s.c., twice weekly at 10 mg/kg for six weeks) are depicted. Histological analysis of ActRIIB(L79D 25-13 1)-mFc treated mice shows a substantial reduction in vascular congestion and damage in the spleen and kidneys (corticomedullary junction shown) compared to vehicle-treated SCD mice. In addition, ActRIIB(L79D 25-13 1)-mFc therapy reduced thickening of the alveolar wall in the lungs (see black arrows) to a greater extent than vehicle treatment. Objective magnification - 20X; inserts - 100X.

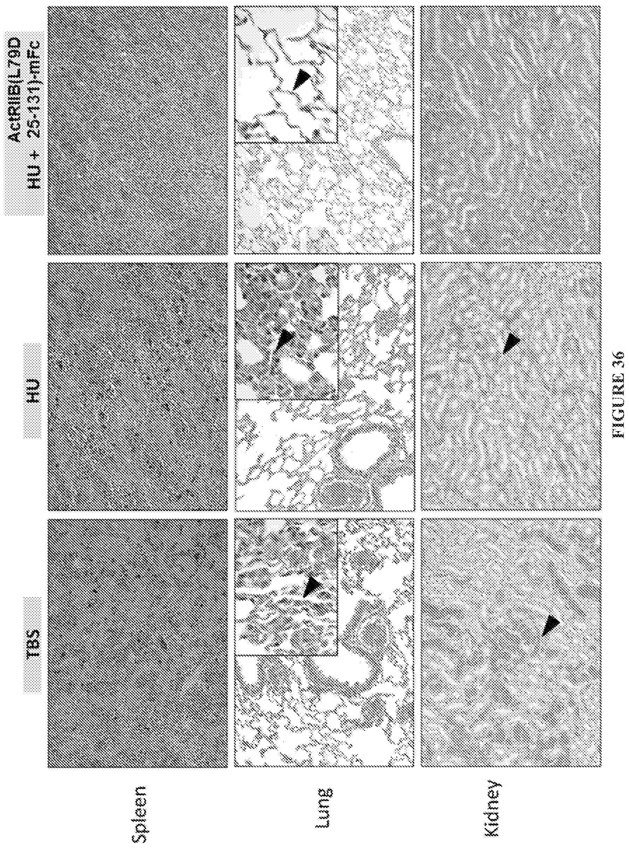

Figure 36 shows the effect of ActRIIB(L79D 25-131)-mFc and hydroxyurea (HU) combination therapy on end organ damage in SCD mice. Histological samples (spleen, lung, and kidney) from SCD mice treated with vehicle (TBS) alone (s.c., twice weekly), SCD mice treated with HU alone (i.p., twice weekly at 100 mg/kg), and SCD mice treated with ActRIIB(L79D 25-13 1)-mFc (s.c. injection, twice weekly at 10 mg/kg) and HU (i.p., twice weekly at 100 mg/kg) are depicted. Histological analysis of the ActRIIB(L79D 25-13 1)-mFc and HU combination therapy treated SCD mice shows a greater reduction in vascular congestion and damage in the spleen and kidneys compared to vehicle-treated or HU monotherapy treated SCD mice. In addition, ActRIIB(L79D 25-13 1)-mFc and HU combination therapy reduced alveolar thickening in the lung (see black arrows) to a greater extent than HU monotherapy treatment. Objective magnification - 20X; inserts - 100X.

### DETAILED DESCRIPTION OF THE DISCLOSURE

### 1. Overview

The transforming growth factor-beta (TGF-beta) superfamily contains a variety of growth factors that share common sequence elements and structural motifs. These proteins are known to exert biological effects on a large variety of cell types in both vertebrates and invertebrates. Members of the superfamily perform important functions during embryonic development in pattern formation and tissue specification and can influence a variety of differentiation processes, including adipogenesis, myogenesis, chondrogenesis, cardiogenesis, hematopoiesis, neurogenesis, and epithelial cell differentiation. By manipulating the activity of a member of the TGF-beta family, it is often possible to cause significant physiological changes in an organism. For example, the Piedmontese and Belgian Blue cattle breeds carry a loss-of-function mutation in the GDF8 (also called myostatin) gene that causes a marked increase in muscle mass [see, *e.g.,* Grobet et al. (1997) Nat Genet. 17(1):71-4]. Furthermore, in humans, inactive alleles of GDF8 are associated with increased muscle mass and, reportedly, exceptional strength [see, *e.g.,* Schuelke et al. (2004) N Engl J Med, 350:2682-8].

TGF-β signals are mediated by heteromeric complexes of type I and type II serine/threonine kinase receptors, which phosphorylate and activate downstream SMAD proteins (*e.g.,* SMAD proteins 1, 2, 3, 5, and 8) upon ligand stimulation [see, *e.g.,* Massagué (2000) Nat. Rev. Mol. Cell Biol. 1:169-178]. These type I and type II receptors are transmembrane proteins, composed of a ligand-binding extracellular domain with cysteine-rich region, a transmembrane domain, and a cytoplasmic domain with predicted serine/threonine specificity. Type I receptors are essential for signaling. Type II receptors are required for binding ligands and for activation of type I receptors. Type I and II activin receptors form a stable complex after ligand binding, resulting in phosphorylation of type I receptors by type II receptors.

Two related type II receptors (ActRII), ActRIIA and ActRIIB, have been identified as the type II receptors for activins [see, *e.g*., Mathews and Vale (1991) Cell 65:973-982; and Attisano et al. (1992) Cell 68: 97-108]. Besides activins, ActRIIA and ActRIIB can biochemically interact with several other TGF-β family proteins including, for example, BMP6, BMP7, Nodal, GDF8, and GDF11 [see, *e.g.,* Yamashita et al. (1995) J. Cell Biol. 130:217-226; Lee and McPherron (2001) Proc. Natl. Acad. Sci. USA 98:9306-9311; Yeo and Whitman (2001) Mol. Cell 7: 949-957; and Oh et al. (2002) Genes Dev. 16:2749-54]. ALK4 is the primary type I receptor for activins, particularly for activin A, and ALK-7 may serve as a receptor for other activins as well, particularly for activin B. In certain instances, the present disclosure relates to antagonizing a ligand of an ActRII receptor (also referred to as an ActRII ligand) with one or more inhibitor agents disclosed herein, particularly inhibitor agents that can antagonize one or more of activin A, activin B, activin C, activin E, GDF11, and/or GDF8.

Activins are dimeric polypeptide growth factors that belong to the TGF-beta superfamily. There are three principal activin forms (A, B, and AB) that are homo/heterodimers of two closely related β subunits (β_{A}β_{A}, β_{B}β_{B}, and β_{A}β_{B}, respectively). The human genome also encodes an activin C and an activin E, which are primarily expressed in the liver, and heterodimeric forms containing β_{C} or β_{E} are also known.

In the TGF-beta superfamily, activins are unique and multifunctional factors that can stimulate hormone production in ovarian and placental cells, support neuronal cell survival, influence cell-cycle progress positively or negatively depending on cell type, and induce mesodermal differentiation at least in amphibian embryos [DePaolo et al. (1991) Proc Soc Ep Biol Med. 198:500-512; Dyson et al. (1997) Curr Biol. 7:81-84; and Woodruff (1998) Biochem Pharmacol. 55:953-963]. Moreover, erythroid differentiation factor (EDF) isolated from the stimulated human monocytic leukemic cells was found to be identical to activin A [Murata et al. (1988) PNAS, 85:2434]. It has been suggested that activin A promotes erythropoiesis in the bone marrow. In several tissues, activin signaling is antagonized by its related heterodimer, inhibin. For example, during the release of follicle-stimulating hormone (FSH) from the pituitary, activin promotes FSH secretion and synthesis, while inhibin prevents FSH secretion and synthesis. Other proteins that may regulate activin bioactivity and/or bind to activin include follistatin (FS), follistatin-related protein (FSRP, also known as FLRG or FSTL3), and α₂-macroglobulin.

As described herein, agents that bind to "activin A" are agents that specifically bind to the β_{A} subunit, whether in the context of an isolated β_{A} subunit or as a dimeric complex (*e.g.,* a β_{A}β_{A} homodimer or a β_{A}β_{β} heterodimer). In the case of a heterodimer complex (*e.g.,* a β_{A}β_{β} heterodimer), agents that bind to "activin A" are specific for epitopes present within the β_{A} subunit, but do not bind to epitopes present within the non-β_{A} subunit of the complex (*e.g.,* the β_{B} subunit of the complex). Similarly, agents disclosed herein that antagonize (inhibit) "activin A" are agents that inhibit one or more activities as mediated by a β_{A} subunit, whether in the context of an isolated β_{A} subunit or as a dimeric complex (*e.g.,* a β_{A}β_{A} homodimer or a β_{A}β_{β} heterodimer). In the case of β_{A}β_{β} heterodimers, agents that inhibit "activin A" are agents that specifically inhibit one or more activities of the β_{A} subunit, but do not inhibit the activity of the non-β_{A} subunit of the complex (*e.g*., the β_{B} subunit of the complex). This principle applies also to agents that *bind to* and/or *inhibit* "activin B", "activin C", and "activin E". Agents disclosed herein that antagonize "activin AB" are agents that inhibit one or more activities as mediated by the β_{A} subunit and one or more activities as mediated by the β_{B} subunit.

Nodal proteins have functions in mesoderm and endoderm induction and formation, as well as subsequent organization of axial structures such as heart and stomach in early embryogenesis. It has been demonstrated that dorsal tissue in a developing vertebrate embryo contributes predominantly to the axial structures of the notochord and pre-chordal plate while it recruits surrounding cells to form non-axial embryonic structures. Nodal appears to signal through both type I and type II receptors and intracellular effectors known as SMAD proteins. Studies support the idea that ActRIIA and ActRIIB serve as type II receptors for Nodal [see, *e.g.,* Sakuma et al. (2002) Genes Cells. 2002, 7:401-12]. It is suggested that Nodal ligands interact with their co-factors (*e.g*., cripto) to activate activin type I and type II receptors, which phosphorylate SMAD2. Nodal proteins are implicated in many events critical to the early vertebrate embryo, including mesoderm formation, anterior patterning, and left-right axis specification. Experimental evidence has demonstrated that Nodal signaling activates pAR3-Lux, a luciferase reporter previously shown to respond specifically to activin and TGF-beta. However, Nodal is unable to induce pTlx2-Lux, a reporter specifically responsive to bone morphogenetic proteins. Recent results provide direct biochemical evidence that Nodal signaling is mediated by both activin-TGF-beta pathway SMADs, SMAD2 and SMAD3. Further evidence has shown that the extracellular cripto protein is required for Nodal signaling, making it distinct from activin or TGF-beta signaling.

Growth and differentiation factor-8 (GDF8) is also known as myostatin. GDF8 is a negative regulator of skeletal muscle mass. GDF8 is highly expressed in the developing and adult skeletal muscle. The GDF8 null mutation in transgenic mice is characterized by a marked hypertrophy and hyperplasia of the skeletal muscle [McPherron et al., Nature (1997) 387:83-90]. Similar increases in skeletal muscle mass are evident in naturally occurring mutations of GDF8 in cattle [see, *e.g.,* Ashmore et al. (1974) Growth, 38:501-507; Swatland and Kieffer (1994) J. Anim. Sci. 38:752-757; McPherron and Lee (1997) Proc. Natl. Acad. Sci. USA 94:12457-12461; and Kambadur et al. (1997) Genome Res. 7:910-915] and, strikingly, in humans [see, *e.g.,* Schuelke et al. (2004) N Engl J Med 350:2682-8]. Studies have also shown that muscle wasting associated with HIV-infection in humans is accompanied by increases in GDF8 protein expression [see, *e.g.,* Gonzalez-Cadavid et al. (1998) PNAS 95:14938-43]. In addition, GDF8 can modulate the production of muscle-specific enzymes *(e.g.,* creatine kinase) and modulate myoblast cell proliferation [see, *e.g.* international patent application publication no. WO 00/43781]. The GDF8 propeptide can noncovalently bind to the mature GDF8 domain dimer, inactivating its biological activity [see, *e.g.,* Miyazono et al. (1988) J. Biol. Chem., 263: 6407-6415; Wakefield et al. (1988) J. Biol. Chem., 263: 7646-7654; and Brown et al. (1990) Growth Factors, 3: 35-43]. Other proteins which bind to GDF8 or structurally related proteins and inhibit their biological activity include follistatin, and potentially, follistatin-related proteins [see, *e.g.,* Gamer et al. (1999) Dev. Biol., 208: 222-232].

Growth and differentiation factor-11 (GDF11), also known as BMP11, is a secreted protein [McPherron et al. (1999) Nat. Genet. 22: 260-264]. GDF11 is expressed in the tail bud, limb bud, maxillary and mandibular arches, and dorsal root ganglia during mouse development [see, *e.g.,* Nakashima et al. (1999) Mech. Dev. 80: 185-189]. GDF11 plays a unique role in patterning both mesodermal and neural tissues [see, *e.g.,* Gamer et al. (1999) Dev Biol., 208:222-32]. GDF11 was shown to be a negative regulator of chondrogenesis and myogenesis in developing chick limb [see, *e.g.,* Gamer et al. (2001) Dev Biol. 229:407-20]. The expression of GDF11 in muscle also suggests its role in regulating muscle growth in a similar way to GDF8. In addition, the expression of GDF11 in brain suggests that GDF11 may also possess activities that relate to the function of the nervous system. Interestingly, GDF11 was found to inhibit neurogenesis in the olfactory epithelium [see, *e.g.,* Wu et al. (2003) Neuron. 37:197-207].

Bone morphogenetic protein (BMP7), also called osteogenic protein-1 (OP-1), is well known to induce cartilage and bone formation. In addition, BMP7 regulates a wide array of physiological processes. For example, BMP7 may be the osteoinductive factor responsible for the phenomenon of epithelial osteogenesis. It is also found that BMP7 plays a role in calcium regulation and bone homeostasis. Like activin, BMP7 binds to type II receptors, ActRIIA and ActRIIB. However, BMP7 and activin recruit distinct type I receptors into heteromeric receptor complexes. The major BMP7 type I receptor observed was ALK2, while activin bound exclusively to ALK4 (ActRIIB). BMP7 and activin elicited distinct biological responses and activated different SMAD pathways [see, *e.g.,* Macias-Silva et al. (1998) J Biol Chem. 273:25628-36].

As demonstrated herein, ActRII polypeptides (*e.g.,* ActRIIA and ActRIIB polypeptides) can be used to increase red blood cell levels *in vivo.* In certain examples, it is shown that a GDF trap polypeptide (specifically a variant ActRIIB polypeptide) is characterized by unique biological properties in comparison to a corresponding sample of a wild-type (unmodified) ActRII polypeptide. This GDF trap is characterized, in part, by substantial loss of binding affinity for activin A, and therefore significantly diminished capacity to antagonize activin A activity, but retains near wild-type levels of binding and inhibition of GDF11. The GDF trap is more effective at increasing red blood cell levels compared to the wild-type ActRII polypeptide and has beneficial effects in a variety of models for anemia and sickle-cell disease. In particular, it is shown that a GDF trap polypeptide can be used to improve red blood cell morphology, increase red blood cell levels, increase the oxygen carrying capacity of hemoglobin, and prevent tissue damage due to vascular congestion and hypoxia in sickle-cell patients, which indicates a much broader use for ActRII antagonists in the treatment of sickle-cell disease beyond the alleviation of anemia. It should be noted that hematopoiesis is a complex process, regulated by a variety of factors, including erythropoietin, G-CSF, and iron homeostasis. The terms "increase red blood cell levels" and "promote red blood cell formation" refer to clinically observable metrics, such as hematocrit, red blood cell counts, and hemoglobin measurements, and are intended to be neutral as to the mechanism by which such changes occur.

The data of the present disclosure therefore indicate that the observed biological activity of an ActRII polypeptide, with respect to red blood cell levels, is not dependent on activin A inhibition. However, it is to be noted that the unmodified ActRIIB polypeptide, which retains activin A binding, still demonstrates the capacity to increase red blood cells *in vivo.* Furthermore, an ActRIIB or ActRIIA polypeptide that retains activin A inhibition may be more desirable in some applications, in comparison to a GDF trap having diminished binding affinity for activin A, where more modest gains in red blood cell levels are desirable and/or where some level of off-target activity is acceptable (or even desirable).

Accordingly, the methods of the present disclosure, in general, are directed to the use of one or more ActRII antagonist agents described herein, optionally in combination with one or more supportive therapies, to increase red blood cell formation in a subject in need thereof, treat or prevent an anemia in a subject in need thereof, to treat sickle-cell disease in a subject in need thereof, and to treat or prevent one or more complications of sickle-cell disease (*e.g*., anemia, anemia crisis, splenomegaly, pain crisis, chest syndrome, acute chest syndrome, blood transfusion requirement, organ damage, pain medicine requirement, splenic sequestration crises, hyperhemolytic crisis, vaso-occlusion, vaso-occlusion crisis, acute myocardial infarction, sickle-cell chronic lung disease, thromboemboli, hepatic failure, hepatomegaly, hepatic sequestration, iron overload, splenic infarction, acute and/or chronic renal failure, pyelonephritis, aneurysm, ischemic stroke, intraparenchymal hemorrhage, subarachnoid hemorrhage, intraventricular hemorrhage, peripheral retinal ischemia, proliferative sickle retinopathy, vitreous hemorrhage, priapism).

In some instances, the ActRII antagonist agents described herein may be used in combination with an EPO receptor activator or in patients that have failed treatment with an EPO receptor activator.

EPO is a glycoprotein hormone involved in the growth and maturation of erythroid progenitor cells into erythrocytes. EPO is produced by the liver during fetal life and by the kidney in adults. Decreased production of EPO, which commonly occurs in adults as a consequence of renal failure, leads to anemia. EPO has been produced by genetic engineering techniques based on expression and secretion of the protein from a host cell transfected with the EPO gene. Administration of such recombinant EPO has been effective in the treatment of anemia. For example, Eschbach et al. (1987, N Engl J Med 316:73) describe the use of EPO to correct anemia caused by chronic renal failure.

Effects of EPO are mediated through its binding to, and activation of, a cell surface receptor belonging to the cytokine receptor superfamily and designated the EPO receptor. The human and murine EPO receptors have been cloned and expressed [see, *e.g.,* D'Andrea et al. (1989) Cell 57:277; Jones et al. (1990) Blood 76:31; Winkelman et al. (1990) Blood 76:24; and U.S. Pat. No. 5,278,065]. The human EPO receptor gene encodes a 483-aminoacid transmembrane protein comprising an extracellular domain of approximately 224 amino acids and exhibits approximately 82% amino acid sequence identity with the murine EPO receptor (see, *e.g.,* U.S. Pat. No. 6,319,499). The cloned, full-length EPO receptor expressed in mammalian cells (66-72 kDa) binds EPO with an affinity (K_{D} = 100-300 nM) similar to that of the native receptor on erythroid progenitor cells. Thus, this form is thought to contain the main EPO binding determinant and is referred to as the EPO receptor. By analogy with other closely related cytokine receptors, the EPO receptor is thought to dimerize upon agonist binding. Nevertheless, the detailed structure of the EPO receptor, which may be a multimeric complex, and its specific mechanism of activation are not completely understood (see, *e.g.,* U.S. Pat. No. 6,319,499).

Activation of the EPO receptor results in several biological effects. These include increased proliferation of immature erythroblasts, increased differentiation of immature erythroblasts, and decreased apoptosis in erythroid progenitor cells [see, *e.g.,* Liboi et al. (1993) Proc Natl Acad Sci USA 90:11351-11355; Koury et al. (1990) Science 248:378-381]. The EPO receptor signal transduction pathways mediating proliferation and differentiation appear to be distinct [see, *e.g.,* Noguchi et al. (1988) Mol Cell Biol 8:2604; Patel et al. (1992) J Biol Chem, 267:21300; and Liboi et al. (1993) Proc Natl Acad Sci USA 90:11351-11355]. Some results suggest that an accessory protein may be required for mediation of the differentiation signal [see, *e.g.,* Chiba et al. (1993) Nature 362:646; and Chiba et al. (1993) Proc Natl Acad Sci USA 90:11593]. However, there is controversy regarding the role of accessory proteins in differentiation since a constitutively activated form of the receptor can stimulate both proliferation and differentiation [see, *e.g.,* Pharr et al. (1993) Proc Natl Acad Sci USA 90:938].

EPO receptor activators include small molecule erythropoiesis-stimulating agents (ESAs) as well as EPO-based compounds. An example of the former is a dimeric peptide-based agonist covalently linked to polyethylene glycol (proprietary names Hematide™ and Omontys®), which has shown erythropoiesis-stimulating properties in healthy volunteers and in patients with both chronic kidney disease and endogenous anti-EPO antibodies [see, *e.g.,* Stead et al. (2006) Blood 108:1830-1834; and Macdougall et al. (2009) N Engl J Med 361:1848-1855]. Other examples include nonpeptide-based ESAs [see, *e.g.,* Qureshi et al. (1999) Proc Natl Acad Sci USA 96:12156-12161].

EPO receptor activators also include compounds that stimulate erythropoiesis indirectly, without contacting EPO receptor itself, by enhancing production of endogenous EPO. For example, hypoxia-inducible transcription factors (HIFs) are endogenous stimulators of EPO gene expression that are suppressed (destabilized) under normoxic conditions by cellular regulatory mechanisms. Therefore, inhibitors of HIF prolyl hydroxylase enzymes are being investigated for EPO-inducing activity *in vivo.* Other indirect activators of EPO receptor include inhibitors of GATA-2 transcription factor [see, *e.g.,* Nakano et al. (2004) Blood 104:4300-4307], which tonically inhibits EPO gene expression, and inhibitors of hemopoietic cell phosphatase (HCP or SHP-1), which functions as a negative regulator of EPO receptor signal transduction [see, *e.g.,* Klingmuller et al. (1995) Cell 80:729-738].

The terms used in this specification generally have their ordinary meanings in the art, within the context of this disclosure and in the specific context where each term is used. Certain terms are discussed below or elsewhere in the specification, to provide additional guidance to the practitioner in describing the compositions and methods of the disclosure and how to make and use them. The scope or meaning of any use of a term will be apparent from the specific context in which they are used.

"Homologous," in all its grammatical forms and spelling variations, refers to the relationship between two proteins that possess a "common evolutionary origin," including proteins from superfamilies in the same species of organism, as well as homologous proteins from different species of organism. Such proteins (and their encoding nucleic acids) have sequence homology, as reflected by their sequence similarity, whether in terms of percent identity or by the presence of specific residues or motifs and conserved positions.

The term "sequence similarity," in all its grammatical forms, refers to the degree of identity or correspondence between nucleic acid or amino acid sequences that may or may not share a common evolutionary origin.

However, in common usage and in the instant application, the term "homologous," when modified with an adverb such as "highly," may refer to sequence similarity and may or may not relate to a common evolutionary origin.

"Percent (%) sequence identity" with respect to a reference polypeptide (or nucleotide) sequence is defined as the percentage of amino acid residues (or nucleic acids) in a candidate sequence that are identical to the amino acid residues (or nucleic acids) in the reference polypeptide (nucleotide) sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid (nucleic acid) sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, Calif., or may be compiled from the source code. The ALIGN-2 program should be compiled for use on a UNIX operating system, including digital UNIX V4.0D. All sequence comparison parameters are set by the ALIGN-2 program and do not vary.

As used herein "does not substantially bind to *X"* is intended to mean that an agent has a K_{D} that is greater than about 10⁻⁷, 10⁻⁶, 10⁻⁵, 10⁻⁴ or greater (*e.g.,* no detectable binding by the assay used to determine the K_{D}) for "X".

### 2. ActRII Antagonists

The data presented herein demonstrates that antagonists (inhibitors) of ActRII (*e.g.,* antagonist of ActRIIA and/or ActRIIB SMAD 2/3 and/or SMAD 1/5/8 signaling) can be used to increase red blood cell levels *in vivo* and provide other benefits to subject (patients) in need thereof. In particular, such ActRII antagonists are shown herein to be effective in treating various anemias as well as various complications (*e.g*., disorders/conditions) of sickle-cell disease. Accordingly, the present disclosure provides, in part, various ActRII antagonist agents that can be used, alone or in combination with one or more erythropoiesis stimulating agents (*e.g.,* EPO) or other supportive therapies [*e.g.,* treatment with hydroxyurea, blood transfusion, iron chelation therapy, and/or pain management (*e.g*., treatment with one or more of opioid analgesic agents, non-steroidal anti-inflammatory drugs, and/or corticosteroids)], to increase red blood cell levels in a subject in need thereof, treat or prevent an anemia in a subject in need thereof, treat sickle cell disease in a subject in need thereof, and/or treat or prevent one or more complication of sickle-cell disease (*e.g*., anemia, anemia crisis, splenomegaly, pain crisis, chest syndrome, acute chest syndrome, blood transfusion requirement, organ damage, pain medicine requirement, splenic sequestration crises, hyperhemolytic crisis, vaso-occlusion, vaso-occlusion crisis, acute myocardial infarction, sickle-cell chronic lung disease, thromboemboli, hepatic failure, hepatomegaly, hepatic sequestration, iron overload, splenic infarction, acute and/or chronic renal failure, pyelonephritis, aneurysm, ischemic stroke, intraparenchymal hemorrhage, subarachnoid hemorrhage, intraventricular hemorrhage, peripheral retinal ischemia, proliferative sickle retinopathy, vitreous hemorrhage, and/or priapism) in a subject in need thereof.

In certain instances, preferred ActRII antagonists to be used in accordance with the methods disclosed herein are GDF-ActRII antagonists (*e.g*., antagonists of GDF-mediated ActRIIA and/or ActRIIB signaling transduction, such as SMAD 2/3 signaling), particularly GDF11- and/or GDF8-mediated ActRII signaling. In some instances, preferred ActRII antagonists of the present disclosure are soluble ActRII polypeptides (*e.g*., soluble ActRIIA and ActRIIB polypeptides) and GDF trap polypeptides, such as ActRIIA-Fc fusion proteins, ActRIIB-Fc fusion proteins, and GDF trap-Fc fusion proteins.

Although soluble ActRII polypeptides and GDF trap polypeptides of the disclosure may affect red blood cell levels and/or various complications of sickle-cell disease through a mechanism other than GDF (*e.g.* GDF11 and/or GDF8) antagonism [*e.g.,* GDF11 and/or GDF8 inhibition may be an indicator of the tendency of an agent to inhibit the activities of a spectrum of additional agents, including, perhaps, other members of the TGF-beta superfamily (*e.g*., activin B, activin C, activin E, BMP6, BMP7, and/or Nodal) and such collective inhibition may lead to the desired effect on, *e.g.,* hematopoiesis], other types of GDF-ActRII antagonist are expected to be useful including, for example, anti-GDFl 1 antibodies; anti-GDF8 antibodies; anti-activin A, B, C, and/or E antibodies, anti-ActRIIA antibodies; anti-ActRIIB antibodies; anti-ActRIIA/IIB antibodies, antisense, RNAi, or ribozyme nucleic acids that inhibit the production of one or more of GDF11, GDF8, ActRIIA, and/or ActRIIB; and other inhibitors (*e.g*., small-molecule inhibitors) of one or more of GDF11, GDF8, ActRIIA, and/or ActRIIB, particularly agents that disrupt GDF11- and/or GDF8-ActRIIA binding and/or GDF11- and/or GDF8-ActRIIB binding as well as agents that inhibit expression of one or more of GDF11, GDF8, ActRIIA, and/or ActRIIB. Optionally, GDF-ActRII antagonists of the present disclosure may bind to and/or inhibit the activity (or expression) of other ActRII ligands including, for example, activin A, activin AB, activin B, activin C, activin E, BMP6, BMP7, and/or Nodal. Optionally, a GDF-ActRII antagonist of the present disclosure may be used in combination with at least one additional ActRII antagonist agent that binds to and/or inhibits the activity (or expression) of one or more additional ActRII ligands including, for example, activin A, activin AB, activin B, activin C, activin E, BMP6, BMP7, and/or Nodal. In some instances, ActRII antagonists to be used in accordance with the methods disclosed herein do not substantially bind to and/or inhibit activin A (*e.g.,* activin A-mediated activation of ActRIIA and/or ActRIIB signaling transduction, such as SMAD 2/3 signaling).

### A. ActRII polypeptides and GDF traps

In certain aspects, the present disclosure relates to ActRII polypeptides. In particular, the disclosure provides methods of using one or more ActRII polypeptides, alone or in combination with one or more erythropoiesis stimulating agents (*e.g.,* EPO) or other supportive therapies (*e.g*., transfusion of red blood cells or whole blood, iron chelation therapy, treatment with hydroxyurea *etc.*), to, *e.g.,* increase red blood cell levels in a subject in need thereof, treat or prevent an anemia in a subject in need thereof, treat sickle-cell disease in a subject in need thereof, treat or prevent one or more complications of sickle-cell disease (*e.g*., anemia, anemia crisis, splenomegaly, pain crisis, chest syndrome, acute chest syndrome, blood transfusion requirement, organ damage, pain medicine (management) requirement, splenic sequestration crises, hyperhemolytic crisis, vaso-occlusion, vaso-occlusion crisis, acute myocardial infarction, sickle-cell chronic lung disease, thromboemboli, hepatic failure, hepatomegaly, hepatic sequestration, iron overload, splenic infarction, acute and/or chronic renal failure, pyelonephritis, aneurysm, ischemic stroke, intraparenchymal hemorrhage, subarachnoid hemorrhage, intraventricular hemorrhage, peripheral retinal ischemia, proliferative sickle retinopathy, vitreous hemorrhage, and/or priapism), and/or reduce blood transfusion burden in a subject in need thereof. As used herein the term "ActRII" refers to the family of type II activin receptors. This family includes both the activin receptor type IIA and the activin receptor type IIB.

As used herein, the term "ActRIIB" refers to a family of activin receptor type IIB (ActRIIB) proteins from any species and variants derived from such ActRIIB proteins by mutagenesis or other modification. Reference to ActRIIB herein is understood to be a reference to any one of the currently identified forms. Members of the ActRIIB family are generally transmembrane proteins, composed of a ligand-binding extracellular domain comprising a cysteine-rich region, a transmembrane domain, and a cytoplasmic domain with predicted serine/threonine kinase activity.

The term "ActRIIB polypeptide" includes polypeptides comprising any naturally occurring polypeptide of an ActRIIB family member as well as any variants thereof (including mutants, fragments, fusions, and peptidomimetic forms) that retain a useful activity. Examples of such variant ActRIIA polypeptides are provided throughout the present disclosure as well as in International Patent Application Publication No. WO 2006/012627. Optionally, ActRIIB polypeptides of the present disclosure can be used to increase red blood cell levels in a subject. Numbering of amino acids for all ActRIIB-related polypeptides described herein is based on the numbering of the human ActRIIB precursor protein sequence provided below (SEQ ID NO:1), unless specifically designated otherwise.

The human ActRIIB precursor protein sequence is as follows:

1

51

101

151

201

251

301

351

401

451

501 (SEQ ID NO:1)

The signal peptide is indicated with single underline; the extracellular domain is indicated in **bold** font; and the potential, endogenous N-linked glycosylation sites are indicated with double underline.

The processed soluble (extracellular) human ActRIIB polypeptide sequence is as follows:

GRGEAETRECIYYNANWELERTNQSGLERCEGEQDKRLHCYASWRNSSGTIELVKK
GCWLDDFNCYDRQECVATEENPQVYFCCCEGNFCNERFTHLPEAGGPEVTYEPPPT
APT (SEQ ID NO:2).

In some instances, the protein may be produced with an "SGR..." sequence at the N-terminus. The C-terminal "tail" of the extracellular domain is indicated by single underline. The sequence with the "tail" deleted (a Δ15 sequence) is as follows:

GRGEAETRECIYYNANWELERTNQSGLERCEGEQDKRLHCYASWRNSSGTIELVKK
GCWLDDFNCYDRQECVATEENPQVYFCCCEGNFCNERFTHLPEA (SEQ ID NO:3).

A form of ActRIIB with an alanine at position 64 of SEQ ID NO:1 (A64) is also reported in the literature [see, *e.g.,* Hilden et al. (1994) Blood, 83(8): 2163-2170]. Applicants have ascertained that an ActRIIB-Fc fusion protein comprising an extracellular domain of ActRIIB with the A64 substitution has a relatively low affinity for activin and GDF11. By contrast, the same ActRIIB-Fc fusion protein with an arginine at position 64 (R64) has an affinity for activin and GDF11 in the low nanomolar to high picomolar range. Therefore, sequences with an R64 are used as the "wild-type" reference sequence for human ActRIIB in this disclosure.

The form of ActRIIB with an alanine at position 64 is as follows:

1 MTAPWVALAL LWGSLCAGSG RGEAETRECI YYNANWELER TNQSGLERCE

51GEQDKRLHCY ASWANSSGTI ELVKKGCWLD DFNCYDRQEC VATEENPQVY
101FCCCEGNFCN ERFTHLPEAG GPEVTYEPPP TAPTLLTVLA YSLLPIGGLS
151LIVLLAFWMY RHRKPPYGHV DIHEDPGPPP PSPLVGLKPL QLLEIKARGR
201FGCVWKAQLM NDFVAVKIFP LQDKQSWQSE REIFSTPGMK HENLLQFIAA
251EKRGSNLEVE LWLITAFHDK GSLTDYLKGN IITWNELCHV AETMSRGLSY
301LHEDVPWCRG EGHKPSIAHR DFKSKNVLLK SDLTAVLADF GLAVRFEPGK
351PPGDTHGQVG TRRYMAPEVL EGAINFQRDA FLRIDMYAMG LVLWELVSRC
401KAADGPVDEY MLPFEEEIGQ HPSLEELQEV VVHKKMRPTI KDHWLKHPGL
451AQLCVTIEEC WDHDAEARLS AGCVEERVSL IRRSVNGTTS DCLVSLVTSV
501TNVDLPPKES SI (SEQ ID NO:4).

The signal peptide is indicated by single underline and the extracellular domain is indicated by **bold** font.

The processed soluble (extracellular) ActRIIB polypeptide sequence of the alternative A64 form is as follows:

GRGEAETRECIYYNANWELERTNQSGLERCEGEQDKRLHCYASWANSSGTIELVKK
GCWLDDFNCYDRQECVATEENPQVYFCCCEGNFCNERFTHLPEAGGPEVTYEPPPT
APT (SEQ ID NO:5).

In some instances, the protein may be produced with an "SGR..." sequence at the N-terminus. The C-terminal "tail" of the extracellular domain is indicated by single underline. The sequence with the "tail" deleted (a Δ15 sequence) is as follows:

GRGEAETRECIYYNANWELERTNQSGLERCEGEQDKRLHCYASWANSSGTIELVKK
GCWLDDFNCYDRQECVATEENPQVYFCCCEGNFCNERFTHLPEA (SEQ ID NO:6).

The nucleic acid sequence encoding human ActRIIB precursor protein is shown below (SEQ ID NO: 7), consisting of nucleotides 25-1560 of Genbank Reference Sequence NM_001106.3, which encode amino acids 1-513 of the ActRIIB precursor. The sequence as shown provides an arginine at position 64 and may be modified to provide an alanine instead. The signal sequence is underlined.

1ATGACGGCGC CCTGGGTGGC CCTCGCCCTC CTCTGGGGAT CGCTGTGCGC
51CGGCTCTGGG CGTGGGGAGG CTGAGACACG GGAGTGCATC TACTACAACG
101CCAACTGGGA GCTGGAGCGC ACCAACCAGA GCGGCCTGGA GCGCTGCGAA

151GGCGAGCAGG ACAAGCGGCT GCACTGCTAC GCCTCCTGGC GCAACAGCTC
201TGGCACCATC GAGCTCGTGA AGAAGGGCTG CTGGCTAGAT GACTTCAACT
251GCTACGATAG GCAGGAGTGT GTGGCCACTG AGGAGAACCC CCAGGTGTAC
301TTCTGCTGCT GTGAAGGCAA CTTCTGCAAC GAACGCTTCA CTCATTTGCC
351AGAGGCTGGG GGCCCGGAAG TCACGTACGA GCCACCCCCG ACAGCCCCCA
401CCCTGCTCAC GGTGCTGGCC TACTCACTGC TGCCCATCGG GGGCCTTTCC
451CTCATCGTCC TGCTGGCCTT TTGGATGTAC CGGCATCGCA AGCCCCCCTA
501CGGTCATGTG GACATCCATG AGGACCCTGG GCCTCCACCA CCATCCCCTC
551TGGTGGGCCT GAAGCCACTG CAGCTGCTGG AGATCAAGGC TCGGGGGCGC
601TTTGGCTGTG TCTGGAAGGC CCAGCTCATG AATGACTTTG TAGCTGTCAA
651GATCTTCCCA CTCCAGGACA AGCAGTCGTG GCAGAGTGAA CGGGAGATCT
701TCAGCACACC TGGCATGAAG CACGAGAACC TGCTACAGTT CATTGCTGCC
751GAGAAGCGAG GCTCCAACCT CGAAGTAGAG CTGTGGCTCA TCACGGCCTT
801CCATGACAAG GGCTCCCTCA CGGATTACCT CAAGGGGAAC ATCATCACAT
851GGAACGAACT GTGTCATGTA GCAGAGACGA TGTCACGAGG CCTCTCATAC
901CTGCATGAGG ATGTGCCCTG GTGCCGTGGC GAGGGCCACA AGCCGTCTAT
951TGCCCACAGG GACTTTAAAA GTAAGAATGT ATTGCTGAAG AGCGACCTCA
1001CAGCCGTGCT GGCTGACTTT GGCTTGGCTG TTCGATTTGA GCCAGGGAAA
1051CCTCCAGGGG ACACCCACGG ACAGGTAGGC ACGAGACGGT ACATGGCTCC
1101TGAGGTGCTC GAGGGAGCCA TCAACTTCCA GAGAGATGCC TTCCTGCGCA
1151TTGACATGTA TGCCATGGGG TTGGTGCTGT GGGAGCTTGT GTCTCGCTGC
1201AAGGCTGCAG ACGGACCCGT GGATGAGTAC ATGCTGCCCT TTGAGGAAGA
1251GATTGGCCAG CACCCTTCGT TGGAGGAGCT GCAGGAGGTG GTGGTGCACA
1301AGAAGATGAG GCCCACCATT AAAGATCACT GGTTGAAACA CCCGGGCCTG
1351GCCCAGCTTT GTGTGACCAT CGAGGAGTGC TGGGACCATG ATGCAGAGGC
1401TCGCTTGTCC GCGGGCTGTG TGGAGGAGCG GGTGTCCCTG ATTCGGAGGT
1451CGGTCAACGG CACTACCTCG GACTGTCTCG TTTCCCTGGT GACCTCTGTC
1501ACCAATGTGG ACCTGCCCCC TAAAGAGTCA AGCATC

(SEQ ID NO: 7).

A nucleic acid sequence encoding processed soluble (extracellular) human ActRIIB polypeptide is as follows (SEQ ID NO: 8). The sequence as shown provides an arginine at position 64, and may be modified to provide an alanine instead.

1GGGCGTGGGG AGGCTGAGAC ACGGGAGTGC ATCTACTACA ACGCCAACTG

51GGAGCTGGAG CGCACCAACC AGAGCGGCCT GGAGCGCTGC GAAGGCGAGC
101AGGACAAGCG GCTGCACTGC TACGCCTCCT GGCGCAACAG CTCTGGCACC
151ATCGAGCTCG TGAAGAAGGG CTGCTGGCTA GATGACTTCA ACTGCTACGA
201TAGGCAGGAG TGTGTGGCCA CTGAGGAGAA CCCCCAGGTG TACTTCTGCT
251GCTGTGAAGG CAACTTCTGC AACGAACGCT TCACTCATTT GCCAGAGGCT
301GGGGGCCCGG AAGTCACGTA CGAGCCACCC CCGACAGCCC CCACC
(SEQ ID NO:8).

In certain instances, the present disclosure relates to ActRIIA polypeptides. As used herein, the term "ActRIIA" refers to a family of activin receptor type IIA (ActRIIA) proteins from any species and variants derived from such ActRIIA proteins by mutagenesis or other modification. Reference to ActRIIA herein is understood to be a reference to any one of the currently identified forms. Members of the ActRIIA family are generally transmembrane proteins, composed of a ligand-binding extracellular domain comprising a cysteine-rich region, a transmembrane domain, and a cytoplasmic domain with predicted serine/threonine kinase activity.

The term "ActRIIA polypeptide" includes polypeptides comprising any naturally occurring polypeptide of an ActRIIA family member as well as any variants thereof (including mutants, fragments, fusions, and peptidomimetic forms) that retain a useful activity. Examples of such variant ActRIIA polypeptides are provided throughout the present disclosure as well as in International Patent Application Publication No. WO 2006/012627. Optionally, ActRIIA polypeptides of the present disclosure can be used to increase red blood cell levels in a subject. Numbering of amino acids for all ActRIIA-related polypeptides described herein is based on the numbering of the human ActRIIA precursor protein sequence provided below (SEQ ID NO:9), unless specifically designated otherwise.

The human ActRIIA precursor protein sequence is as follows:

1MGAAAKLAFA VFLISCSSGA ILGRSETQEC LFFNANWEKD RTNQTGVEPC
51YGDKDKRRHC FATWKNISGS IEIVKQGCWL DDINCYDRTD CVEKKDSPEV
101YFCCCEGNMC NEKFSYFPEM EVTQPTSNPV TPKPPYYNIL LYSLVPLMLI
151AGIVICAFWV YRHHKMAYPP VLVPTQDPGP PPPSPLLGLK PLQLLEVKAR
201GRFGCVWKAQ LLNEYVAVKI FPIQDKQSWQ NEYEVYSLPG MKHENILQFI
251GAEKRGTSVD VDLWLITAFH EKGSLSDFLK ANVVSWNELC HIAETMARGL
301AYLHEDIPGL KDGHKPAISH RDIKSKNVLL KNNLTACIAD FGLALKFEAG
351KSAGDTHGQV GTRRYMAPEV LEGAINFQRD AFLRIDMYAM GLVLWELASR

401CTAADGPVDE YMLPFEEEIG QHPSLEDMQE VVVHKKKRPV LRDYWQKHAG

451MAMLCETIEE CWDHDAEARL SAGCVGERIT QMQRLTNIIT TEDIVTVVTM

501VTNVDFPPKE SSL (SEQ ID NO:9)

The signal peptide is indicated by single underline; the extracellular domain is indicated in **bold** font; and the potential, endogenous N-linked glycosylation sites are indicated by double underline.

The processed soluble (extracellular) human ActRIIA polypeptide sequence is as follows:

ILGRSETQECLFFNANWEKDRTNQTGVEPCYGDKDKRRHCFATWKNISGSIEIVKQG
CWLDDINCYDRTDCVEKKDSPEVYFCCCEGNMCNEKFSYFPEMEVTQPTSNPVTPK
PP (SEQ ID NO:10)

The C-terminal "tail" of the extracellular domain is indicated by single underline. The sequence with the "tail" deleted (a Δ15 sequence) is as follows:

ILGRSETQECLFFNANWEKDRTNQTGVEPCYGDKDKRRHCFATWKNISGSIEIVKQG
CWLDDINCYDRTDCVEKKDSPEVYFCCCEGNMCNEKFSYFPEM (SEQ ID NO:11)

The nucleic acid sequence encoding human ActRIIA precursor protein is shown below (SEQ ID NO: 12), as follows nucleotides 159-1700 of Genbank Reference Sequence NM_001616.4. The signal sequence is underlined.

1atggagctg ctgcaaagtt ggcgtttgcc gtctttctta tctcctgttc
51ttcaggtgct atacttggta gatcagaaac tcaggagtgt cttttcttta
101atgctaattg ggaaaaagac agaaccaatc aaactggtgt tgaaccgtgt
151tatggtgaca aagataaacg gcggcattgt tttgctacct ggaagaatat
201ttctggttcc attgaaatag tgaaacaagg ttgttggctg gatgatatca
251actgctatga caggactgat tgtgtagaaa aaaaagacag ccctgaagta
301tatttttgtt gctgtgaggg caatatgtgt aatgaaaagt tttcttattt
351tccggagatg gaagtcacac agcccacttc aaatccagtt acacctaagc
401caccctatta caacatcctg ctctattcct tggtgccact tatgttaatt
451gcggggattg tcatttgtgc attttgggtg tacaggcatc acaagatggc
501ctaccctcct gtacttgttc caactcaaga cccaggacca cccccacctt
551ctccattact aggtttgaaa ccactgcagt tattagaagt gaaagcaagg
601ggaagatttg gttgtgtctg gaaagcccag ttgcttaacg aatatgtggc
651tgtcaaaata tttccaatac aggacaaaca gtcatggcaa aatgaatacg
701aagtctacag tttgcctgga atgaagcatg agaacatatt acagttcatt

751ggtgcagaaa aacgaggcac cagtgttgat gtggatcttt ggctgatcac
801agcatttcat gaaaagggtt cactatcaga ctttcttaag gctaatgtgg
851tctcttggaa tgaactgtgt catattgcag aaaccatggc tagaggattg
901gcatatttac atgaggatat acctggccta aaagatggcc acaaacctgc
951catatctcac agggacatca aaagtaaaaa tgtgctgttg aaaaacaacc
1001tgacagcttg cattgctgac tttgggttgg ccttaaaatt tgaggctggc
1051aagtctgcag gcgataccca tggacaggtt ggtacccgga ggtacatggc
1101tccagaggta ttagagggtg ctataaactt ccaaagggat gcatttttga
1151ggatagatat gtatgccatg ggattagtcc tatgggaact ggcttctcgc
1201tgtactgctg cagatggacc tgtagatgaa tacatgttgc catttgagga
1251ggaaattggc cagcatccat ctcttgaaga catgcaggaa gttgttgtgc
1301ataaaaaaaa gaggcctgtt ttaagagatt attggcagaa acatgctgga
1351atggcaatgc tctgtgaaac cattgaagaa tgttgggatc acgacgcaga
1401agccaggtta tcagctggat gtgtaggtga aagaattacc cagatgcaga
1451gactaacaaa tattattacc acagaggaca ttgtaacagt ggtcacaatg
1501gtgacaaatg ttgactttcc tcccaaagaa tctagtcta
(SEQ ID NO:12)

The nucleic acid sequence encoding processed soluble (extracellular) human ActRIIA polypeptide is as follows:

1atacttggta gatcagaaac tcaggagtgt cttttcttta atgctaattg
51ggaaaaagac agaaccaatc aaactggtgt tgaaccgtgt tatggtgaca
101aagataaacg gcggcattgt tttgctacct ggaagaatat ttctggttcc
151attgaaatag tgaaacaagg ttgttggctg gatgatatca actgctatga
201caggactgat tgtgtagaaa aaaaagacag ccctgaagta tatttttgtt
251gctgtgaggg caatatgtgt aatgaaaagt tttcttattt tccggagatg
301gaagtcacac agcccacttc aaatccagtt acacctaagc caccc
(SEQ ID NO:13).

An alignment of the amino acid sequences of human ActRIIB soluble extracellular domain and human ActRIIA soluble extracellular domain are illustrated in Figure 1. This alignment indicates amino acid residues within both receptors that are believed to directly contact ActRII ligands. Figure 2 depicts a multiple-sequence alignment of various vertebrate ActRIIB proteins and human ActRIIA. From these alignments it is possible to predict key amino acid positions within the ligand-binding domain that are important for normal ActRII-ligand binding activities as well as to predict amino acid positions that are likely to be tolerant to substitution without significantly altering normal ActRII-ligand binding activities.

In other aspects, the present disclosure relates to GDF trap polypeptides (also referred to as "GDF traps"), which may be used, for example, alone or in combination with one or more erythropoiesis stimulating agents (*e.g.,* EPO) or other supportive therapies (*e.g.,* transfusion of red blood cells, whole blood, iron chelation therapy, treatment with hydroxyurea, *etc*.), to, *e.g.,* increase red blood cell levels in a subject in need thereof, treat or prevent an anemia in a subject in need thereof, treat sickle cell disease in a subject in need thereof, treat or prevent one or more complication of sickle-cell disease (*e.g*., anemia, anemia crisis, splenomegaly, pain crisis, chest syndrome, acute chest syndrome, blood transfusion requirement, organ damage, pain medicine (management) requirement, splenic sequestration crises, hyperhemolytic crisis, vaso-occlusion, vaso-occlusion crisis, acute myocardial infarction, sickle-cell chronic lung disease, thromboemboli, hepatic failure, hepatomegaly, hepatic sequestration, iron overload, splenic infarction, acute and/or chronic renal failure, pyelonephritis, aneurysm, ischemic stroke, intraparenchymal hemorrhage, subarachnoid hemorrhage, intraventricular hemorrhage, peripheral retinal ischemia, proliferative sickle retinopathy, vitreous hemorrhage, and/or priapism), reduce blood transfusion burden in a subject in need thereof.

In some instances, GDF traps of the present disclosure are soluble, variant ActRII polypeptides (*e.g*., ActRIIA and ActRIIB polypeptides) that comprise one or more mutations (*e.g*., amino acid additions, deletions, substitutions, and combinations thereof) in the extracellular domain (also referred to as the ligand-binding domain) of an ActRII polypeptide (*e.g.,* a "wild-type" ActRII polypeptide) such that the variant ActRII polypeptide has one or more altered ligand-binding activities than the corresponding wild-type ActRII polypeptide. In preferred instances, GDF trap polypeptides of the present disclosure retain at least one similar activity as a corresponding wild-type ActRII polypeptide (*e.g*., an ActRIIA or ActRIIB polypeptide). For example, a GDF trap may bind to and/or inhibit (*e.g*. antagonize) the function of one or more ActRII ligands (*e.g*., inhibit ActRII ligand-mediated activation of the ActRIIA and/or ActRIIB signaling transduction, such as SMAD 2/3 and/or SMAD 1/5/8 signaling pathway). In some instances, GDF traps of the present disclosure bind to and/or inhibit one or more of activin A, activin B, activin AB, activin C, activin E, Nodal, GDF8, GDF11, BMP6 and/or BMP7).

In certain instances, GDF trap polypeptides of the disclosure have elevated binding affinity for one or more specific ActRII ligands (*e.g*., GDF8, GDF11, BMP6, Nodal, and/or BMP7). In other instances, GDF trap polypeptides of the disclosure have decreased binding affinity for one or more specific ActRII ligands (*e.g*., activin A, activin B, activin AB, activin C, and/or activin E). In still other instances, GDF trap polypeptides of the disclosure have elevated binding affinity for one or more specific ActRII ligands and decreased binding affinity for one or more different/other ActRII ligands. Accordingly, the present disclosure provides GDF trap polypeptides that have an altered binding specificity for one or more ActRII ligands.

In certain preferred instances, GDF traps of the present disclosure are designed to preferentially bind to and antagonize GDF11 and/or GDF8 (also known as myostatin), *e.g.,* in comparision to a wild-type ActRII polypeptide. Optionally, such GDF11 and/or GDF8-binding traps may further bind to and/or antagonize one or more of Nodal, GDF8, GDF11, BMP6 and/or BMP7. Optionally, such GDF11 and/or GDF8-binding traps may further bind to and/or antagonize one or more of activin B, activin C, activin E, Nodal, GDF8, GDF11, BMP6 and/or BMP7. Optionally, such GDF11 and/or GDF8-binding traps may further bind to and/or antagonize one or more of activin A, activin A/B, activin B, activin C, activin E, Nodal, GDF8, GDF11, BMP6 and/or BMP7. In certain instances, GDF traps of the present disclosure have diminished binding affinity for activins (*e.g*., activin A, activin A/B, activin B, activin C, activin E), *e.g.,* in comparision to a wild-type ActRII polypeptide. In certain preferred instances, a GDF trap polypeptide of the present disclosure has diminished binding affinity for activin A.

For example, the disclosure provides GDF trap polypeptides that preferentially bind to and/or antagonize GDF8/GDF11 relative to activin A. As demonstrated by the Examples of the disclosure, such GDF trap polypeptides are more potent activators of erythropoiesis *in vivo* in comparision to ActRII polypeptides that retain high binding affinity for activin A. Furthermore, these non-activin-A-binding GDF trap polypeptides demonstrate decreased effects on other tissues. Therefore, such GDF traps may be useful for increasing red blood cell levels in a subject while reducing potential off-target effects associated with binding/antagonizing activin A. However, such selective GDF trap polypeptides may be less desirable in some applications wherein more modest gains in red blood cell levels may be needed for therapeutic effect and wherein some level of off-target effect is acceptable (or even desirable).

Amino acid residues of the ActRIIB proteins (*e.g*., E39, K55, Y60, K74, W78, L79, D80, and F101) are in the ActRIIB ligand-binding pocket and help mediated binding to its ligands including, for example, activin A, GDF11, and GDF8. Thus the present disclosure provides GDF trap polypeptides comprising an altered-ligand binding domain (*e.g.,* a GDF8/GDF11-binding domain) of an ActRIIB receptor which comprises one or more mutations at those amino acid residues.

Optionally, the altered ligand-binding domain can have increased selectivity for a ligand such as GDF11 and/or GDF8 relative to a wild-type ligand-binding domain of an ActRIIB receptor. To illustrate, one or more mutations may be selected that increase the selectivity of the altered ligand-binding domain for GDF11 and/or GDF8 over one or more activins (activin A, activin B, activin AB, activin C, and/or activin A), particularly activin A. Optionally, the altered ligand-binding domain has a ratio of K_{d} for activin binding to K_{d} for GDF11 and/or GDF8 binding that is at least 2-, 5-, 10-, 20-, 50-, 100- or even 1000-fold greater relative to the ratio for the wild-type ligand-binding domain. Optionally, the altered ligand-binding domain has a ratio of IC₅₀ for inhibiting activin to IC₅₀ for inhibiting GDF11 and/or GDF8 that is at least 2-, 5-, 10-, 20-, 50-, 100- or even 1000-fold greater relative to the wild-type ligand-binding domain. Optionally, the altered ligand-binding domain inhibits GDF11 and/or GDF8 with an IC₅₀ at least 2-, 5-, 10-, 20-, 50-, 100- or even 1000-times less than the IC₅₀ for inhibiting activin.

As a specific example, the positively-charged amino acid residue Asp (D80) of the ligand-binding domain of ActRIIB can be mutated to a different amino acid residue to produce a GDF trap polypeptide that preferentially binds to GDF8, but not activin. Preferably, the D80 residue with respect to SEQ ID NO:1 is changed to an amino acid residue selected from the group consisting of: an uncharged amino acid residue, a negative amino acid residue, and a hydrophobic amino acid residue. As a further specific example, the hydrophobic residue L79 of SEQ ID NO:1 can be altered to confer altered activin-GDF11/GDF8 binding properties. For example, an L79P substitution reduces GDF11 binding to a greater extent than activin binding. In contrast, replacement of L79 with an acidic amino acid [an aspartic acid or glutamic acid; an L79D or an L79E substitution] greatly reduces activin A binding affinity while retaining GDF11 binding affinity. In exemplary instances, the methods described herein utilize a GDF trap polypeptide which is a variant ActRIIB polypeptide comprising an acidic amino acid (*e.g.,* D or E) at the position corresponding to position 79 of SEQ ID NO: 1, optionally in combination with one or more additional amino acid substitutions, additions, or deletions.

As will be recognized by one of skill in the art, most of the described mutations, variants or modifications described herein may be made at the nucleic acid level or, in some cases, by post-translational modification or chemical synthesis. Such techniques are well known in the art and some of which are described herein.

In certain instances, the present disclosure relates to ActRII polypeptides (ActRIIA and ActRIIB polypeptides) which are soluble ActRII polypeptides. As described herein, the term "soluble ActRII polypeptide" generally refers to polypeptides comprising an extracellular domain of an ActRII protein. The term "soluble ActRII polypeptide," as used herein, includes any naturally occurring extracellular domain of an ActRII protein as well as any variants thereof (including mutants, fragments, and peptidomimetic forms) that retain a useful activity (*e.g*., a GDF trap polypeptide as described herein). Other examples of soluble ActRII polypeptides comprise a signal sequence in addition to the extracellular domain of an ActRII or GDF trap protein. For example, the signal sequence can be a native signal sequence of an ActRIIA or ActRIIB protein, or a signal sequence from another protein including, for example, a tissue plasminogen activator (TPA) signal sequence or a honey bee melittin (HBM) signal sequence.

In part, the present disclosure identifies functionally active portions and variants of ActRII polypeptides that can be used as guidance for generating and using ActRIIA polypeptides, ActRIIB polypeptides, and GDF trap polypeptides within the scope of the methods described herein.

ActRII proteins have been characterized in the art in terms of structural and functional characteristics, particularly with respect to ligand binding [see, *e.g.,* Attisano et al. (1992) Cell 68(1):97-108; Greenwald et al. (1999) Nature Structural Biology 6(1): 18-22; Allendorph et al. (2006) PNAS 103(20: 7643-7648; Thompson et al. (2003) The EMBO Journal 22(7): 1555-1566; and U.S. Patent Nos: 7,709,605, 7,612,041, and 7,842,663].

For example, Attisano *et al.* showed that a deletion of the proline knot at the C-terminus of the extracellular domain of ActRIIB reduced the affinity of the receptor for activin. An ActRIIB-Fc fusion protein containing amino acids 20-119 of present SEQ ID NO:1, "ActRIIB(20-119)-Fc", has reduced binding to GDF-11 and activin relative to an ActRIIB(20-134)-Fc, which includes the proline knot region and the complete juxtamembrane domain (see, *e.g.,* U.S. Patent No. 7,842,663). However, an ActRIIB(20-129)-Fc protein retains similar but somewhat reduced activity relative to the wild-type, even though the proline knot region is disrupted. Thus, ActRIIB extracellular domains that stop at amino acid 134, 133, 132, 131, 130 and 129 (with respect to present SEQ ID NO:1) are all expected to be active, but constructs stopping at 134 or 133 may be most active. Similarly, mutations at any of residues 129-134 (with respect to SEQ ID NO:1) are not expected to alter ligand-binding affinity by large margins. In support of this, mutations of P129 and P130 (with respect to SEQ ID NO:1) do not substantially decrease ligand binding. Therefore, an ActRIIB polypeptide or an ActRIIB-based GDF trap polypeptide of the present disclosure may end as early as amino acid 109 (the final cysteine), however, forms ending at or between 109 and 119 (*e.g.,* 109, 110, 111, 112, 113, 114, 115, 116, 117, 118, or 119) are expected to have reduced ligand binding. Amino acid 119 (with respect to present SEQ ID NO:1) is poorly conserved and so is readily altered or truncated. ActRIIB polypeptides and ActRIIB-based GDF traps ending at 128 (with respect to present SEQ ID NO:1) or later should retain ligand binding activity. ActRIIB polypeptides and ActRIIB-based GDF traps ending at or between 119 and 127 (*e.g.,* 119, 120, 121, 122, 123, 124, 125, 126, or 127),with respect to SEQ ID NO:1, will have an intermediate binding ability. Any of these forms may be desirable to use, depending on the clinical or experimental setting.

At the N-terminus of ActRIIB, it is expected that a protein beginning at amino acid 29 or before (with respect to present SEQ ID NO:1) will retain ligand-binding activity. Amino acid 29 represents the initial cysteine. An alanine-to-asparagine mutation at position 24 (with respect to present SEQ ID NO:1) introduces an N-linked glycosylation sequence without substantially affecting ligand binding (see, *e.g.,* U.S. Patent No. 7,842,663). This confirms that mutations in the region between the signal cleavage peptide and the cysteine cross-linked region, corresponding to amino acids 20-29, are well tolerated. In particular, ActRIIB polypeptides and ActRIIB-based GDF traps beginning at position 20, 21, 22, 23, and 24 (with respect to present SEQ ID NO:1) should retain general ligand-biding activity, and ActRIIB polypeptides and ActRIIB-based GDF traps beginning at positions 25, 26, 27, 28, and 29 (with respect to present SEQ ID NO:1) are also expected to retain ligand-biding activity. Data shown herein as well as in, *e.g.,* U.S. Patent No. 7,842,663 demonstrates that, surprisingly, an ActRIIB construct beginning at 22, 23, 24, or 25 will have the most activity.

Taken together, an active portion (*e.g*., ligand-binding activity) of ActRIIB comprises amino acids 29-109 of SEQ ID NO:1. Therefore ActRIIB polypeptides and ActRIIB-based GDF traps of the present disclosure may, for example, comprise an amino acid sequence that is at least 80%, 85%, 90%, 95%, 96%, 97%, 98%, 99%, or 100% identical to a portion of ActRIIB beginning at a residue corresponding to amino acids 20-29 (*e.g.,* beginning at amino acid 20, 21, 22, 23, 24, 25, 26, 27, 28, or 29) of SEQ ID NO: 1 and ending at a position corresponding to amino acids 109-134 (*e.g.,* ending at amino acid 109, 110, 111, 112, 113, 114, 115, 116, 117, 118, 119, 120, 121, 122, 123, 124, 125, 126, 127, 128, 129, 130, 131, 132, 133, or 134) of SEQ ID NO: 1. In some instances, ActRIIB-based GDF trap polypeptides of the present disclosure do not comprise or consist of amino acids 29-109 of SEQ ID NO:1. Other examples include polypeptides that begin at a position from 20-29 (*e.g.,* position 20, 21, 22, 23, 24, 25, 26, 27, 28, or 29) or 21-29 (*e.g.,* position 21, 22, 23, 24, 25, 26, 27, 28, or 29) and end at a position from 119-134 (*e.g.,* 119, 120, 121, 122, 123, 124, 125, 126, 127, 128, 129, 130, 131, 132, 133, or 134), 119-133 (*e.g.,* 119, 120, 121, 122, 123, 124, 125, 126, 127, 128, 129, 130, 131, 132, or 133), 129-134 (*e.g.,* 129, 130, 131, 132, 133, or 134), or 129-133 (*e.g.,* 129, 130, 131, 132, or 133) of SEQ ID NO: 1. Other examples include constructs that begin at a position from 20-24 (*e.g.,* 20, 21, 22, 23, or 24), 21-24 (*e.g.,* 21, 22, 23, or 24), or 22-25 (*e.g.,* 22, 22, 23, or 25) and end at a position from 109-134 (*e.g.,* 109, 110, 111, 112, 113, 114, 115, 116, 117, 118, 119, 120, 121, 122, 123, 124, 125, 126, 127, 128, 129, 130, 131, 132, 133, or 134), 119-134 (*e.g.,* 119, 120, 121, 122, 123, 124, 125, 126, 127, 128, 129, 130, 131, 132, 133, or 134) or 129-134 *(e.g.,* 129, 130, 131, 132, 133, or 134) of SEQ ID NO: 1. Variants within these ranges are also contemplated, particularly those having at least 80%, 85%, 90%, 95%, 96%, 97%, 98%, 99%, or 100% identity to the corresponding portion of SEQ ID NO: 1. In some instances, the ActRIIB polypeptides and ActRIIB-based GDF traps comprise a polypeptide having an amino acid sequence that is at least 80%, 85%, 90%, 95%, 96%, 97%, 98%, 99%, or 100% identical to amino acid residues 25-131 of SEQ ID NO: 1. In certain instances, ActRIIB-based GDF trap polypeptides do not comprise or consist of amino acids 25-131 of SEQ ID NO: 1.

The disclosure includes the results of an analysis of composite ActRIIB structures, shown in Figure 1, demonstrating that the ligand-binding pocket is defined, in part, by residues Y31, N33, N35, L38 through T41, E47, E50, Q53 through K55, L57, H58, Y60, S62, K74, W78 through N83, Y85, R87, A92, and E94 through F101. At these positions, it is expected that conservative mutations will be tolerated, although a K74A mutation is well-tolerated, as are R40A, K55A, F82A and mutations at position L79. R40 is a K in Xenopus, indicating that basic amino acids at this position will be tolerated. Q53 is R in bovine ActRIIB and K in Xenopus ActRIIB, and therefore amino acids including R, K, Q, N and H will be tolerated at this position. Thus, a general formula for an ActRIIB polypeptide and ActRIIB-based GDF trap polypeptide of the disclosure is one that comprises an amino acid sequence that is at least 80%, 85%, 90%, 95%, 96%, 97%, 98%, 99%, or 100% identical to amino acids 29-109 of SEQ ID NO: 1, optionally beginning at a position ranging from 20-24 (*e.g.,* 20, 21, 22, 23, or 24) or 22-25(*e.g.,* 22, 23, 24, or 25) and ending at a position ranging from 129-134 (*e.g.,* 129, 130, 131, 132, 133, or 134), and comprising no more than 1, 2, 5, 10 or 15 conservative amino acid changes in the ligand-binding pocket, and zero, one or more non-conservative alterations at positions 40, 53, 55, 74, 79 and/or 82 in the ligand-binding pocket. Sites outside the binding pocket, at which variability may be particularly well tolerated, include the amino and carboxy termini of the extracellular domain (as noted above), and positions 42-46 and 65-73 (with respect to SEQ ID NO:1). An asparagine-to-alanine alteration at position 65 (N65A) actually improves ligand binding in the A64 background, and is thus expected to have no detrimental effect on ligand binding in the R64 background (see, *e.g.,* U.S. Patent No. 7,842,663). This change probably eliminates glycosylation at N65 in the A64 background, thus demonstrating that a significant change in this region is likely to be tolerated. While an R64A change is poorly tolerated, R64K is well-tolerated, and thus another basic residue, such as H may be tolerated at position 64 (see, *e.g.,* U.S. Patent No. 7,842,663).

ActRIIB is well-conserved across nearly all vertebrates, with large stretches of the extracellular domain conserved completely. Many of the ligands that bind to ActRIIB are also highly conserved. Accordingly, comparisons of ActRIIB sequences from various vertebrate organisms provide insights into residues that may be altered. Therefore, an active, human ActRIIB variant polypeptide and ActRIIB-based GDF trap useful in accordance with the presently disclosed methods may include one or more amino acids at corresponding positions from the sequence of another vertebrate ActRIIB, or may include a residue that is similar to that in the human or other vertebrate sequence. The following examples illustrate this approach to defining an active ActRIIB variant. L46 is a valine in Xenopus ActRIIB, and so this position may be altered, and optionally may be altered to another hydrophobic residue, such as V, I or F, or a non-polar residue such as A. E52 is a K in Xenopus, indicating that this site may be tolerant of a wide variety of changes, including polar residues, such as E, D, K, R, H, S, T, P, G, Y and probably A. T93 is a K in Xenopus, indicating that a wide structural variation is tolerated at this position, with polar residues favored, such as S, K, R, E, D, H, G, P, G and Y. F108 is a Y in Xenopus, and therefore Y or other hydrophobic group, such as I, V or L should be tolerated. E111 is K in Xenopus, indicating that charged residues will be tolerated at this position, including D, R, K and H, as well as Q and N. R112 is K in Xenopus, indicating that basic residues are tolerated at this position, including R and H. A at position 119 is relatively poorly conserved, and appears as P in rodents and V in Xenopus, thus essentially any amino acid should be tolerated at this position.

It has been previously demonstrated that the addition of a further N-linked glycosylation site (N-X-S/T) is well-tolerated relative to the ActRIIB(R64)-Fc form (see, *e.g.,* U.S. Patent No. 7,842,663). Therefore, N-X-S/T sequences may be generally introduced at positions outside the ligand binding pocket defined in Figure 1 in ActRIIB polypeptide and ActRIIB-based GDF traps of the present disclosure. Particularly suitable sites for the introduction of non-endogenous N-X-S/T sequences include amino acids 20-29, 20-24, 22-25, 109-134, 120-134 or 129-134 (with respect to SEQ ID NO:1). N-X-S/T sequences may also be introduced into the linker between the ActRIIB sequence and an Fc domain or other fusion component. Such a site may be introduced with minimal effort by introducing an N in the correct position with respect to a pre-existing S or T, or by introducing an S or T at a position corresponding to a pre-existing N. Thus, desirable alterations that would create an N-linked glycosylation site are: A24N, R64N, S67N (possibly combined with an N65A alteration), E105N, R112N, G120N, E123N, P129N, A132N, R112S and R112T (with respect to SEQ ID NO:1). Any S that is predicted to be glycosylated may be altered to a T without creating an immunogenic site, because of the protection afforded by the glycosylation. Likewise, any T that is predicted to be glycosylated may be altered to an S. Thus the alterations S67T and S44T (with respect to SEQ ID NO:1) are contemplated. Likewise, in an A24N variant, an S26T alteration may be used. Accordingly, an ActRIIB polypeptide and ActRIIB-based GDF trap polypeptide of the present disclosure may be an variant having one or more additional, non-endogenous N-linked glycosylation consensus sequences as described above.

The variations described herein may be combined in various ways. Additionally, the results of the mutagenesis program described herein indicate that there are amino acid positions in ActRIIB that are often beneficial to conserve. With respect to SEQ ID NO:1, these include position 64 (basic amino acid), position 80 (acidic or hydrophobic amino acid), position 78 (hydrophobic, and particularly tryptophan), position 37 (acidic, and particularly aspartic or glutamic acid), position 56 (basic amino acid), position 60 (hydrophobic amino acid, particularly phenylalanine or tyrosine). Thus, in the ActRIIB polypeptides and ActRIIB-based GDF traps disclosed herein, the disclosure provides a framework of amino acids that may be conserved. Other positions that may be desirable to conserve are as follows: position 52 (acidic amino acid), position 55 (basic amino acid), position 81 (acidic), 98 (polar or charged, particularly E, D, R or K), all with respect to SEQ ID NO:1.

A general formula for an active (*e.g*., ligand binding) ActRIIA polypeptide is one that comprises a polypeptide that starts at amino acid 30 and ends at amino acid 110 of SEQ ID NO:9. Accordingly, ActRIIA polypeptides and ActRIIA-based GDF traps of the present disclosure may comprise a polypeptide that is at least 80%, 85%, 90%, 95%, 96%, 97%, 98%, 99%, or 100% identical to amino acids 30-110 of SEQ ID NO:9. In some instances, ActRIIA-based GDF traps of the present disclosure do not comprise or consist of amino acids 30-110 of SEQ ID NO:9. Optionally, ActRIIA polypeptides and ActRIIA-based GDF trap polypeptides of the present disclosure comprise a polypeptide that is at least 80%, 85%, 90%, 95%, 96%, 97%, 98%, 99%, or 100% identical to amino acids amino acids 12-82 of SEQ ID NO:9 optionally beginning at a position ranging from 1-5 (*e.g.,* 1, 2, 3, 4, or 5) or 3-5 (*e.g., 3,* 4, or 5) and ending at a position ranging from 110-116 (*e.g*., 110, 111, 112, 113, 114, 115, or 116) or 110-115 (*e.g.,* 110, 111, 112, 113, 114, or 115), respectively, and comprising no more than 1, 2, 5, 10 or 15 conservative amino acid changes in the ligand binding pocket, and zero, one or more non-conservative alterations at positions 40, 53, 55, 74, 79 and/or 82 in the ligand-binding pocket with respect to SEQ ID NO:9.

In certain instances, functionally active fragments of ActRII polypeptides (*e.g*. ActRIIA and ActRIIB polypeptides) and GDF trap polypeptides of the present disclosure can be obtained by screening polypeptides recombinantly produced from the corresponding fragment of the nucleic acid encoding an ActRII polypeptide or GDF trap polypeptide (*e.g.*, SEQ ID NOs: 7, 8, 12, 13, 27, 32, 39, 40, 42, 46, and 48). In addition, fragments can be chemically synthesized using techniques known in the art such as conventional Merrifield solid phase f-Moc or t-Boc chemistry. The fragments can be produced (recombinantly or by chemical synthesis) and tested to identify those peptidyl fragments that can function as antagonists (inhibitors) of ActRII receptors and/or one or more ActRII ligands (*e.g*., GDF11, GDF8, activin A, activin B, activin AB, activin C, activin E, BMP6, BMP7, and/or Nodal).

In some instances, an ActRIIA polypeptide of the present disclosure is a polypeptide comprising an amino acid sequence that is at least 75% identical to an amino acid sequence selected from SEQ ID NOs: 9, 10, 11, 22, 26, and 28. In certain instances, the ActRIIA polypeptide comprises an amino acid sequence that is at least 80%, 85%, 90%, 95%, 97%, 98%, 99% or 100% identical to an amino acid sequence selected from SEQ ID NOs: 9, 10, 11, 22, 26, and 28. In certain instances, the ActRIIA polypeptide consists essentially of, or consists of, an amino acid sequence that is at least 80%, 85%, 90%, 95%, 97%, 98%, 99% or 100% identical to an amino acid sequence selected from SEQ ID NOs: 9, 10, 11, 22, 26, and 28.

In some instances, an ActRIIB polypeptide of the present disclosure is a polypeptide comprising an amino acid sequence that is at least 75% identical to an amino acid sequence selected from SEQ ID NOs: 1, 2, 3, 4, 5, 6, 29, 31, and 49. In certain instances, the ActRIIB polypeptide comprises an amino acid sequence that is at least 80%, 85%, 90%, 95%, 97%, 98%, 99% or 100% identical to an amino acid sequence selected from SEQ ID NOs: 1, 2, 3, 4, 5, 6, 29, 31, and 49. In certain instances, the ActRIIB polypeptide consists essentially of, or consists of, an amino acid sequence that is at least 80%, 85%, 90%, 95%, 97%, 98%, 99% or 100% identical to an amino acid sequence selected from SEQ ID NOs: 1, 2, 3, 4, 5, 6, 29, 31, and 49.

In some instances, a GDF trap polypeptide of the present disclosure is a variant ActRIIB polypeptide comprising an amino acid sequence that is at least 75% identical to an amino acid sequence selected from SEQ ID NOs: 1, 2, 3, 4, 5, 6, 29, 30, 31, 36, 37, 38, 41, 44, 45, 49, 50, and 51. In certain instances, the GDF trap comprises an amino acid sequence that is at least 80%, 85%, 90%, 95%, 97%, 98%, 99% or 100% identical to an amino acid sequence selected from SEQ ID NOs: 1, 2, 3, 4, 5, 6, 29, 30, 31, 36, 37, 38, 41, 44, 45, 49, 50, and 51. In certain instances, the GDF trap comprises an amino acid sequence that is at least 80%, 85%, 90%, 95%, 97%, 98%, 99% or 100% identical to an amino acid sequence selected from SEQ ID NOs: 1, 2, 3, 4, 5, 6, 29, 30, 31, 36, 37, 38, 41, 44, 45, 49, 50, and 51, wherein the position corresponding to L79 of SEQ ID NO:1, 4, or 49 is an acidic amino acids (a D or E amino acid residue). In certain instances, the GDF trap consists essentially of, or consists of, an amino acid sequence that at least 80%, 85%, 90%, 95%, 97%, 98%, 99% or 100% identical to an amino acid sequence selected from SEQ ID NOs: 36, 37, 38, 41, 44, 45, 50, and 51. In certain instances, the GDF trap does not comprise or consists of an amino acid sequence selected from SEQ ID NOs: 1, 2, 3, 4, 5, 6, 29, and 31.

In some instances, a GDF trap polypeptide of the present disclosure is a variant ActRIIA polypeptide comprising an amino acid sequence that is at least 75% identical to an amino acid sequence selected from SEQ ID NOs: 9, 10, 11, 22, 26, 28, 29, and 31. In certain instances, the GDF trap comprises an amino acid sequence that is at least 80%, 85%, 90%, 95%, 97%, 98%, 99% or 100% identical to an amino acid sequence selected from SEQ ID NOs: 9, 10, 11, 22, 26, 28, 29, and 31. In certain instances, the GDF trap consists essentially of, or consists of, an amino acid sequence that at least 80%, 85%, 90%, 95%, 97%, 98%, 99% or 100% identical to an amino acid sequence selected from SEQ ID NOs: 9, 10, 11, 22, 26, 28, 29, and 31. In certain instances, the GDF trap does not comprise or consists of an amino acid sequence selected from SEQ ID NOs: 9, 10, 11, 22, 26, 28, 29, and 31.

In some instances, the present disclosure contemplates making functional variants by modifying the structure of an ActRII polypeptide (*e.g*. and ActRIIA or ActRIIB polypeptide) or a GDF trap for such purposes as enhancing therapeutic efficacy, or stability (*e.g.,* shelf-life and resistance to proteolytic degradation *in vivo*). Variants can be produced by amino acid substitution, deletion, addition, or combinations thereof. For instance, it is reasonable to expect that an isolated replacement of a leucine with an isoleucine or valine, an aspartate with a glutamate, a threonine with a serine, or a similar replacement of an amino acid with a structurally related amino acid (*e.g*., conservative mutations) will not have a major effect on the biological activity of the resulting molecule. Conservative replacements are those that take place within a family of amino acids that are related in their side chains. Whether a change in the amino acid sequence of a polypeptide of the disclosure results in a functional homolog can be readily determined by assessing the ability of the variant polypeptide to produce a response in cells in a fashion similar to the wild-type polypeptide, or to bind to one or more ligands, such as GDF11, activin A, activin B, activin AB, activin C, activin E, GDF8, BMP6, and BMP7, as compared to the unmodified or a wild-type polypeptide.

In certain instances, the present disclosure contemplates specific mutations of ActRII polypeptides and GDF trap polypeptides of the present disclosure so as to alter the glycosylation of the polypeptide. Such mutations may be selected so as to introduce or eliminate one or more glycosylation sites, such as O-linked or N-linked glycosylation sites. Asparagine-linked glycosylation recognition sites generally comprise a tripeptide sequence, asparagine-X-threonine or asparagine-X-serine (where "X" is any amino acid) which is specifically recognized by appropriate cellular glycosylation enzymes. The alteration may also be made by the addition of, or substitution by, one or more serine or threonine residues to the sequence of the polypeptide (for O-linked glycosylation sites). A variety of amino acid substitutions or deletions at one or both of the first or third amino acid positions of a glycosylation recognition site (and/or amino acid deletion at the second position) results in non-glycosylation at the modified tripeptide sequence. Another means of increasing the number of carbohydrate moieties on a polypeptide is by chemical or enzymatic coupling of glycosides to the polypeptide. Depending on the coupling mode used, the sugar(s) may be attached to (a) arginine and histidine; (b) free carboxyl groups; (c) free sulfhydryl groups such as those of cysteine; (d) free hydroxyl groups such as those of serine, threonine, or hydroxyproline; (e) aromatic residues such as those of phenylalanine, tyrosine, or tryptophan; or (f) the amide group of glutamine. Removal of one or more carbohydrate moieties present on a polypeptide may be accomplished chemically and/or enzymatically. Chemical deglycosylation may involve, for example, exposure of a polypeptide to the compound trifluoromethanesulfonic acid, or an equivalent compound. This treatment results in the cleavage of most or all sugars except the linking sugar (N-acetylglucosamine or N-acetylgalactosamine), while leaving the amino acid sequence intact. Enzymatic cleavage of carbohydrate moieties on polypeptides can be achieved by the use of a variety of endo- and exo-glycosidases as described by Thotakura et al. [Meth. Enzymol. (1987) 138:350]. The sequence of a polypeptide may be adjusted, as appropriate, depending on the type of expression system used, as mammalian, yeast, insect, and plant cells may all introduce differing glycosylation patterns that can be affected by the amino acid sequence of the peptide. In general, ActRII polypeptides and GDF trap polypeptides of the present disclosure for use in humans may be expressed in a mammalian cell line that provides proper glycosylation, such as HEK293 or CHO cell lines, although other mammalian expression cell lines are expected to be useful as well.

This disclosure further contemplates a method of generating mutants, particularly sets of combinatorial mutants of ActRII polypeptides and GDF trap polypeptides of the present disclosure, as well as truncation mutants. Pools of combinatorial mutants are especially useful for identifying ActRII and GDF trap sequences. The purpose of screening such combinatorial libraries may be to generate, for example, polypeptides variants which have altered properties, such as altered pharmacokinetic or altered ligand binding. A variety of screening assays are provided below, and such assays may be used to evaluate variants. For example, ActRII polypeptides and GDF trap polypeptides may be screened for ability to bind to an ActRII receptor, to prevent binding of an ActRII ligand (*e.g.,* GDF11, GDF8, activin A, activin B, activin AB, activin C, activin E, BMP7, BMP6, and/or Nodal) to an ActRII polypeptide, or to interfere with signaling caused by an ActRII ligand.

The activity of an ActRII polypeptides or GDF trap polypeptides may also be tested in a cell-based or *in vivo* assay. For example, the effect of an ActRII polypeptide or GDF trap polypeptide on the expression of genes involved in hematopoiesis may be assessed. This may, as needed, be performed in the presence of one or more recombinant ActRII ligand proteins (*e.g*., GDF11, GDF8, activin A, activin B, activin AB, activin C, activin E, BMP7, BMP6, and/or Nodal), and cells may be transfected so as to produce an ActRII polypeptide or GDF trap polypeptide, and optionally, an ActRII ligand. Likewise, an ActRII polypeptide or GDF trap polypeptide may be administered to a mouse or other animal, and one or more blood measurements, such as an RBC count, hemoglobin, or reticulocyte count may be assessed using art-recognized methods.

Combinatorial-derived variants can be generated which have a selective or generally increased potency relative to a reference ActRII polypeptide or GDF trap polypeptide. Such variants, when expressed from recombinant DNA constructs, can be used in gene therapy protocols. Likewise, mutagenesis can give rise to variants which have intracellular half-lives dramatically different than the corresponding unmodified ActRII polypeptide or GDF trap polypeptide. For example, the altered protein can be rendered either more stable or less stable to proteolytic degradation or other cellular processes which result in destruction,or otherwise inactivation, of an unmodified polypeptide. Such variants, and the genes which encode them, can be utilized to alter ActRII polypeptide or GDF trap polypeptide levels by modulating the half-life of the polypeptide. For instance, a short half-life can give rise to more transient biological effects and, when part of an inducible expression system, can allow tighter control of recombinant ActRII polypeptide or GDF trap polypeptide levels within the cell. In an Fc fusion protein, mutations may be made in the linker (if any) and/or the Fc portion to alter the half-life of the protein.

A combinatorial library may be produced by way of a degenerate library of genes encoding a library of polypeptides which each include at least a portion of potential ActRII or GDF trap sequences. For instance, a mixture of synthetic oligonucleotides can be enzymatically ligated into gene sequences such that the degenerate set of potential ActRII or GDF trap polypeptide encoding nucleotide sequences are expressible as individual polypeptides, or alternatively, as a set of larger fusion proteins (*e.g.,* for phage display).

There are many ways by which the library of potential homologs can be generated from a degenerate oligonucleotide sequence. Chemical synthesis of a degenerate gene sequence can be carried out in an automatic DNA synthesizer, and the synthetic genes can then be ligated into an appropriate vector for expression. The synthesis of degenerate oligonucleotides is well known in the art. See, *e.g.,* Narang, SA (1983) Tetrahedron 39:3; Itakura et al. (1981) Recombinant DNA, Proc. 3rd Cleveland Sympos. Macromolecules, ed. AG Walton, Amsterdam: Elsevier pp273-289; Itakura et al. (1984) Annu. Rev. Biochem. 53:323; Itakura et al. (1984) Science 198:1056; Ike et al. (1983) Nucleic Acid Res. 11:477. Such techniques have been employed in the directed evolution of other proteins. See, *e.g.,* Scott et al., (1990) Science 249:386-390; Roberts et al. (1992) PNAS USA 89:2429-2433; Devlin et al. (1990) Science 249: 404-406; Cwirla et al., (1990) PNAS USA 87: 6378-6382; as well as U.S. Patent Nos: 5,223,409, 5,198,346, and 5,096,815.

Alternatively, other forms of mutagenesis can be utilized to generate a combinatorial library. For example, ActRII polypeptides or GDF trap polypeptides of the present disclosure can be generated and isolated from a library by screening using, for example, alanine scanning mutagenesis [see, *e.g.,* Ruf et al. (1994) Biochemistry 33:1565-1572; Wang et al. (1994) J. Biol. Chem. 269:3095-3099; Balint et al. (1993) Gene 137:109-118; Grodberg et al. (1993) Eur. J. Biochem. 218:597-601; Nagashima et al. (1993) J. Biol. Chem. 268:2888-2892; Lowman et al. (1991) Biochemistry 30:10832-10838; and Cunningham et al. (1989) Science 244:1081-1085], by linker scanning mutagenesis [see, *e.g.,* Gustin et al. (1993) Virology 193:653-660; and Brown et al. (1992) Mol. Cell Biol. 12:2644-2652; McKnight et al. (1982) Science 232:316], by saturation mutagenesis [see, *e.g.,* Meyers et al., (1986) Science 232:613]; by PCR mutagenesis [see, *e.g.,* Leung et al. (1989) Method Cell Mol Biol 1:11-19]; or by random mutagenesis, including chemical mutagenesis [*see, e.g.,* Miller et al. (1992) A Short Course in Bacterial Genetics, CSHL Press, Cold Spring Harbor, NY; and Greener et al. (1994) Strategies in Mol Biol 7:32-34]. Linker scanning mutagenesis, particularly in a combinatorial setting, is an attractive method for identifying truncated (bioactive) forms of ActRII polypeptides.

A wide range of techniques are known in the art for screening gene products of combinatorial libraries made by point mutations and truncations, and, for that matter, for screening cDNA libraries for gene products having a certain property. Such techniques will be generally adaptable for rapid screening of the gene libraries generated by the combinatorial mutagenesis of ActRII polypeptides or GDF trap polypeptides of the disclosure. The most widely used techniques for screening large gene libraries typically comprises cloning the gene library into replicable expression vectors, transforming appropriate cells with the resulting library of vectors, and expressing the combinatorial genes under conditions in which detection of a desired activity facilitates relatively easy isolation of the vector encoding the gene whose product was detected. Preferred assays include ActRII ligand (*e.g.,* GDF11, GDF8, activin A, activin B, activin AB, activin C, activin E, BMP7, BMP6, and/or Nodal) binding assays and/or ActRII ligand-mediated cell signaling assays.

In certain instances, ActRII polypeptides or GDF trap polypeptides of the present disclosure may further comprise post-translational modifications in addition to any that are naturally present in the ActRII (*e.g.,* an ActRIIA or ActRIIB polypeptide) or GDF trap polypeptide. Such modifications include, but are not limited to, acetylation, carboxylation, glycosylation, phosphorylation, lipidation, and acylation. As a result, the ActRII polypeptide or GDF trap polypeptide may contain non-amino acid elements, such as polyethylene glycols, lipids, polysaccharide or monosaccharide, and phosphates. Effects of such non-amino acid elements on the functionality of a ligand trap polypeptide may be tested as described herein for other ActRII or GDF trap variants. When a polypeptide of the disclosure is produced in cells by cleaving a nascent form of the polypeptide, post-translational processing may also be important for correct folding and/or function of the protein. Different cells (*e.g.,* CHO, HeLa, MDCK, 293, WI38, NIH-3T3 or HEK293) have specific cellular machinery and characteristic mechanisms for such post-translational activities and may be chosen to ensure the correct modification and processing of the ActRII polypeptides or GDF trap polypeptides.

In certain aspects, ActRII polypeptides or GDF trap polypeptides of the present disclosure include fusion proteins having at least a portion (domain) of an ActRII polypeptide (*e.g.,* an ActRIIA or ActRIIB polypeptide) or GDF trap polypeptide and one or more heterologous portions (domains). Well-known examples of such fusion domains include, but are not limited to, polyhistidine, Glu-Glu, glutathione S-transferase (GST), thioredoxin, protein A, protein G, an immunoglobulin heavy-chain constant region (Fc), maltose binding protein (MBP), or human serum albumin. A fusion domain may be selected so as to confer a desired property. For example, some fusion domains are particularly useful for isolation of the fusion proteins by affinity chromatography. For the purpose of affinity purification, relevant matrices for affinity chromatography, such as glutathione-, amylase-, and nickel- or cobalt- conjugated resins are used. Many of such matrices are available in "kit" form, such as the Pharmacia GST purification system and the QIAexpress™ system (Qiagen) useful with (HIS₆) fusion partners. As another example, a fusion domain may be selected so as to facilitate detection of the ligand trap polypeptides. Examples of such detection domains include the various fluorescent proteins (*e.g*., GFP) as well as "epitope tags," which are usually short peptide sequences for which a specific antibody is available. Well-known epitope tags for which specific monoclonal antibodies are readily available include FLAG, influenza virus haemagglutinin (HA), and c-myc tags. In some cases, the fusion domains have a protease cleavage site, such as for Factor Xa or thrombin, which allows the relevant protease to partially digest the fusion proteins and thereby liberate the recombinant proteins therefrom. The liberated proteins can then be isolated from the fusion domain by subsequent chromatographic separation. In certain preferred instances, an ActRII polypeptide or a GDF trap polypeptide is fused with a domain that stabilizes the polypeptide *in vivo* (a "stabilizer" domain). By "stabilizing" is meant anything that increases serum half-life, regardless of whether this is because of decreased destruction, decreased clearance by the kidney, or other pharmacokinetic effect. Fusions with the Fc portion of an immunoglobulin are known to confer desirable pharmacokinetic properties on a wide range of proteins. Likewise, fusions to human serum albumin can confer desirable properties. Other types of fusion domains that may be selected include multimerizing (*e.g*., dimerizing, tetramerizing) domains and functional domains (that confer an additional biological function, such as further stimulation of muscle growth).

In certain instances, the present disclosure provides ActRII or GDF trap fusion proteins comprising the following IgG1 Fc domain sequence:

1THTCPPCPAP ELLGGPSVFL FPPKPKDTLM ISRTPEVTCV VVDVSHEDPE

51VKFNWYVDGV EVHNAKTKPR EEQYNSTYRV VSVLTVLHQD WLNGKEYKCK

101VSNKALPVPI EKTISKAKGQ PREPQVYTLP PSREEMTKNQ VSLTCLVKGF

151YPSDIAVEWE SNGQPENNYK TTPPVLDSDG SFFLYSKLTV DKSRWQQGNV

201FSCSVMHEAL HNHYTQKSLS LSPGK (SEQ ID NO:14).

In other instances, the present disclosure provides ActRII or GDF trap fusion proteins comprising the following variant of the IgG1 Fc domain:

1THTCPPCPAP ELLGGPSVFL FPPKPKDTLM ISRTPEVTCV VVDVSHEDPE

51VKFNWYVDGV EVHNAKTKPR EEQYNSTYRV VSVLTVLHQD WLNGKEYKCK

101VSNKALPAPI EKTISKAKGQ PREPQVYTLP PSREEMTKNQ VSLTCLVKGF

151YPSDIAVEWE SNGQPENNYK TTPPVLDSDG SFFLYSKLTV DKSRWQQGNV

201FSCSVMHEAL HNHYTQKSLS LSPGK (SEQ ID NO:64)

In still other instances, the present disclosure provides ActRII or GDF trap fusion proteins comprising the following variant of the IgG1 Fc domain:

1THTCPPCPAP ELLGGPSVFL FPPKPKDTLM ISRTPEVTCV VVD(A)VSHEDPE

51VKFNWYVDGV EVHNAKTKPR EEQYNSTYRV VSVLTVLHQD WLNGKEYKCK(A)

101VSNKALPVPI EKTISKAKGQ PREPQVYTLP PSREEMTKNQ VSLTCLVKGF

151YPSDIAVEWE SNGQPENNYK TTPPVLDSDG PFFLYSKLTV DKSRWQQGNV

201FSCSVMHEAL HN(A)HYTQKSLS LSPGK (SEQ ID NO:15).

Optionally, the IgG1 Fc domain has one or more mutations at residues such as Asp-265, lysine 322, and Asn-434. In certain cases, the mutant IgG1 Fc domain having one or more of these mutations (*e.g*., Asp-265 mutation) has reduced ability of binding to the Fcγ receptor relative to a wild-type Fc domain. In other cases, the mutant Fc domain having one or more of these mutations (*e.g*., Asn-434 mutation) has increased ability of binding to the MHC class I-related Fc-receptor (FcRN) relative to a wild-type IgG1 Fc domain.

In certain other instances, the present disclosure provides ActRII or GDF trap fusion proteins comprising variants of the IgG2 Fc domain, including the following:

1VECPPCPAPP VAGPSVFLFP PKPKDTLMIS RTPEVTCVVV DVSHEDPEVQ

51 FNWYVDGVEV HNAKTKPREE QFNSTFRVVS VLTVVHQDWL NGKEYKCKVS

101 NKGLPAPIEK TISKTKGQPR EPQVYTLPPS REEMTKNQVS LTCLVKGFYP

151 SDIAVEWESN GQPENNYKTT PPMLDSDGSF FLYSKLTVDK SRWQQGNVFS

201 CSVMHEALHN HYTQKSLSLS PGK (SEQ ID NO:65)

It is understood that different elements of the fusion proteins may be arranged in any manner that is consistent with the desired functionality. For example, an ActRII polypeptide domain or GDF trap polypeptide domain may be placed C-terminal to a heterologous domain, or alternatively, a heterologous domain may be placed C-terminal to an ActRII polypeptide domain or GDF trap polypeptide domain. The ActRII polypeptide domain or GDF trap polypeptide domain and the heterologous domain need not be adjacent in a fusion protein, and additional domains or amino acid sequences may be included C- or N-terminal to either domain or between the domains.

For example, an ActRII or GDF trap fusion protein may comprise an amino acid sequence as set forth in the formula A-B-C. The B portion corresponds to an ActRII polypeptide domain or a GDF trap polypeptide domain. The A and C portions may be independently zero, one, or more than one amino acid, and both the A and C portions when present are heterologous to B. The A and/or C portions may be attached to the B portion via a linker sequence. Exemplary linkers include short polypeptide linkers such as 2-10, 2-5, 2-4, 2-3 glycine residues, such as, for example, a Gly-Gly-Gly linker. Other suitable linkers are described herein above [*e.g.,* a TGGG linker (SEQ ID NO: 53)]. In certain instances, an ActRII or GDF trap fusion protein comprises an amino acid sequence as set forth in the formula A-B-C, wherein A is a leader (signal) sequence, B consists of an ActRII or GDF polypeptide domain, and C is a polypeptide portion that enhances one or more of *in vivo* stability, *in vivo* half-life, uptake/administration, tissue localization or distribution, formation of protein complexes, and/or purification. In certain instances, an ActRII or GDF trap fusion protein comprises an amino acid sequence as set forth in the formula A-B-C, wherein A is a TPA leader sequence, B consists of an ActRII or GDF polypeptide domain, and C is an immunoglobulin Fc domain. Preferred fusion proteins comprises the amino acid sequence set forth in any one of SEQ ID NOs: 22, 26, 29, 31, 36, 38, 41, 44, and 51.

In certain instances, ActRII polypeptides or GDF trap polypeptides of the present disclosure contain one or more modifications that are capable of stabilizing the polypeptides. For example, such modifications enhance the *in vitro* half-life of the polypeptides, enhance circulatory half-life of the polypeptides, and/or reduce proteolytic degradation of the polypeptides. Such stabilizing modifications include, but are not limited to, fusion proteins (including, for example, fusion proteins comprising an ActRII polypeptide domain or a GDF trap polypeptide domain and a stabilizer domain), modifications of a glycosylation site (including, for example, addition of a glycosylation site to a polypeptide of the disclosure), and modifications of carbohydrate moiety (including, for example, removal of carbohydrate moieties from a polypeptide of the disclosure). As used herein, the term "stabilizer domain" not only refers to a fusion domain (*e.g*., an immunoglobulin Fc domain) as in the case of fusion proteins, but also includes nonproteinaceous modifications such as a carbohydrate moiety, or nonproteinaceous moiety, such as polyethylene glycol.

In preferred instances, ActRII polypeptides and GDF traps to be used in accordance with the methods described herein are isolated polypeptides. As used herein, an isolated protein or polypeptide is one which has been separated from a component of its natural environment. In some instances, a polypeptide of the disclosure is purified to greater than 95%, 96%, 97%, 98%, or 99% purity as determined by, for example, electrophoretic (*e.g*., SDS-PAGE, isoelectric focusing (IEF), capillary electrophoresis) or chromatographic (*e.g*., ion exchange or reverse phase HPLC). Methods for assessment of antibody purity are well known in the art [see, *e.g.,* Flatman et al., (2007) J. Chromatogr. B 848:79-87].

In certain instances, ActRII polypeptides and GDF traps of the disclosure can be produced by a variety of art-known techniques. For example, polypeptides of the disclosure can be synthesized using standard protein chemistry techniques such as those described in Bodansky, M. Principles of Peptide Synthesis, Springer Verlag, Berlin (1993) and Grant G. A. (ed.), Synthetic Peptides: A User's Guide, W. H. Freeman and Company, New York (1992). In addition, automated peptide synthesizers are commercially available (see, *e.g.,* Advanced ChemTech Model 396; Milligen/Biosearch 9600). Alternatively, the polypeptides of the disclosure, including fragments or variants thereof, may be recombinantly produced using various expression systems [*e.g*., E. coli, Chinese Hamster Ovary (CHO) cells, COS cells, baculovirus] as is well known in the art. In a further instance, the modified or unmodified polypeptides of the disclosure may be produced by digestion of recombinantly produced full-length ActRII or GDF trap polypeptides by using, for example, a protease, *e.g.,* trypsin, thermolysin, chymotrypsin, pepsin, or paired basic amino acid converting enzyme (PACE). Computer analysis (using a commercially available software, *e.g.,* MacVector, Omega, PCGene, Molecular Simulation, Inc.) can be used to identify proteolytic cleavage sites. Alternatively, such polypeptides may be produced from recombinantly produced full-length ActRII or GDF trap polypeptides using chemical cleavage (*e.g*., cyanogen bromide, hydroxylamine, *etc.).*

Any of the ActRII polypeptides disclosed herein (*e.g*., ActRIIA or ActRIIB polypeptides) can be combined with one or more additional ActRII antagonist agents of the disclosure to achieve the desired effect (*e.g*., increase red blood cell levels and/or hemoglobin in a subject in need thereof, treat or prevent an anemia, treat sickle-cell disease, treat or prevent one or more complications of sickle-cell disease). For example, an ActRII polypeptide disclosed herein can be used in combination with i) one or more additional ActRII polypeptides disclosed herein, ii) one or more GDF traps disclosed herein; iii) one or more ActRII antagonist antibodies disclosed herein (*e.g.,* an anti-activin A antibody, an anti-activin B antibody, an anti-activin C antibody, an anti-activin E antibody, an anti-GDFl 1 antibody, an anti-GDF8 antibody, an anti-BMP6 antibody, an anti-BMP7 antibody, an anti-ActRIIA antibody, and/or or an anti-ActRIIB antibody); iv) one or more small-molecule ActRII antagonists disclosed herein (*e.g.,* a small-molecule antagonist of one or more of GDF11, GDF8, activin A, activin B, activin AB, activin C, activin E, BMP6, BMP7, Nodal, ActRIIA, and/or ActRIIB); v) one or more of the polynucleotide ActRII antagonists disclosed herein (*e.g.,* a polynucleotide antagonist of one or more of GDF11, GDF8, activin A, activin B, activin AB, activin C, activin E, BMP6, BMP7, Nodal, ActRIIA, and/or ActRIIB); vi) one or more follistatin polypeptides disclosed herein; and/or vii) one or more FLRG polypeptides disclosed herein.

Similarly, any of the GDF traps disclosed herein can be combined with one or more additional ActRII antagonist agents of the disclosure to achieve the desired effect (*e.g.,* increase red blood cell levels and/or hemoglobin in a patient in need thereof, treat or prevent an anemia, treat sickle-cell disease, treat or prevent one or more complications of sickle-cell disease). For example, a GDF trap disclosed herein can be used in combination with i) one or more additional GDF traps disclosed herein, ii) one or more ActRII polypeptides disclosed herein (*e.g*., ActRIIA or ActRIIB polypeptides) disclosed herein; iii) one or more ActRII antagonist antibodies disclosed herein (*e.g.,* an anti-activin A antibody, an anti-activin B antibody, an anti-activin C antibody, an anti-activin E antibody, an anti-GDFl 1 antibody, an anti-GDF8 antibody, an anti-BMP6 antibody, an anti-BMP7 antibody, an anti-ActRIIA antibody, and/or or an anti-ActRIIB antibody); iv) one or more small-molecule ActRII antagonists disclosed herein (*e.g.,* a small-molecule antagonist of one or more of GDF11, GDF8, activin A, activin B, activin AB, activin C, activin E, BMP6, BMP7, Nodal, ActRIIA, and/or ActRIIB); v) one or more of the polynucleotide ActRII antagonists disclosed herein (*e.g.,* a polynucleotide antagonist of one or more of GDF11, GDF8, activin A, activin B, activin AB, activin C, activin E, BMP6, BMP7, Nodal, ActRIIA, and/or ActRIIB); vi) one or more follistatin polypeptides disclosed herein; and/or vii) one or more FLRG polypeptides disclosed herein.

### B. Nucleic Acids Encoding ActRII Polypeptides and GDF Traps

In certain instances, the present disclosure provides isolated and/or recombinant nucleic acids encoding the ActRII polypeptides and GDF trap polypeptides (including fragments, functional variants, and fusion proteins thereof) disclosed herein. For example, SEQ ID NO:12 encodes the naturally occurring human ActRIIA precursor polypeptide, while SEQ ID NO:13 encodes the processed extracellular domain of ActRIIA. In addition, SEQ ID NO:7 encodes a naturally occurring human ActRIIB precursor polypeptide (the R64 variant described above), while SEQ ID NO:8 encodes the processed extracellular domain of ActRIIB (the R64 variant described above). The subject nucleic acids may be single-stranded or double stranded. Such nucleic acids may be DNA or RNA molecules. These nucleic acids may be used, for example, in methods for making ActRII-based ligand trap polypeptides of the present disclosure.

As used herein, isolated nucleic acid(s) refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

In certain instances, nucleic acids encoding ActRII polypeptides and GDF traps of the present disclosure are understood to include nucleic acids that are variants of any one of SEQ ID NOs: 7, 8, 12, 13, 27, 32, 39, 40, 42, 43, 46, 47, and 48. Variant nucleotide sequences include sequences that differ by one or more nucleotide substitutions, additions, or deletions including allelic variants, and therefore, will include coding sequence that differ from the nucleotide sequence designated in any one of SEQ ID NOs: 7, 8, 12, 13, 27, 32, 39, 40, 42, 43, 46, 47, and 48.

In certain instances, ActRII polypeptides and GDF traps of the present disclosure are encoded by isolated or recombinant nucleic acid sequences that are at least 80%, 85%, 90%, 95%, 97%, 98%, or 99% identical to SEQ ID NOs: 7, 8, 12, 13, 27, 32, 39, 40, 42, 43, 46, 47, and 48 In some instances, GDF traps of the present disclosure are not encoded by nucleic acid sequences that comprise or consist of any one of nucleotide sequences corresponding to any one of SEQ ID NOs: 7, 8, 12, 13, 27, and 32. One of ordinary skill in the art will appreciate that nucleic acid sequences that are at least 80%, 85%, 90%, 95%, 97%, 98%, or 99% identical to the sequences complementary to SEQ ID NOs: 7, 8, 12, 13, 27, 32, 39, 42, 47, and 48, and variants thereof, are also within the scope of the present disclosure. In further instances, the nucleic acid sequences of the disclosure can be isolated, recombinant, and/or fused with a heterologous nucleotide sequence, or in a DNA library.

In other instances, nucleic acids of the present disclosure also include nucleotide sequences that hybridize under highly stringent conditions to the nucleotide sequence designated in SEQ ID NOs: 7, 8, 12, 13, 27, 32, 39, 40, 42, 43, 46, 47, and 48, complement sequences of SEQ ID NOs: 7, 8, 12, 13, 27, 32, 39, 40, 42, 43, 46, 47, and 48, or fragments thereof. As discussed above, one of ordinary skill in the art will understand readily that appropriate stringency conditions which promote DNA hybridization can be varied. One of ordinary skill in the art will understand readily that appropriate stringency conditions which promote DNA hybridization can be varied. For example, one could perform the hybridization at 6.0 x sodium chloride/sodium citrate (SSC) at about 45 °C, followed by a wash of 2.0 x SSC at 50 °C. For example, the salt concentration in the wash step can be selected from a low stringency of about 2.0 x SSC at 50 °C to a high stringency of about 0.2 x SSC at 50 °C. In addition, the temperature in the wash step can be increased from low stringency conditions at room temperature, about 22 °C, to high stringency conditions at about 65 °C. Both temperature and salt may be varied, or temperature or salt concentration may be held constant while the other variable is changed. In one instance, the disclosure provides nucleic acids which hybridize under low stringency conditions of 6 x SSC at room temperature followed by a wash at 2 x SSC at room temperature.

Isolated nucleic acids which differ from the nucleic acids as set forth in SEQ ID NOs: 7, 8, 12, 13, 27, 32, 39, 40, 42, 43, 46, 47, and 48 due to degeneracy in the genetic code are also within the scope of the disclosure. For example, a number of amino acids are designated by more than one triplet. Codons that specify the same amino acid, or synonyms (for example, CAU and CAC are synonyms for histidine) may result in "silent" mutations which do not affect the amino acid sequence of the protein. However, it is expected that DNA sequence polymorphisms that do lead to changes in the amino acid sequences of the subject proteins will exist among mammalian cells. One skilled in the art will appreciate that these variations in one or more nucleotides (up to about 3-5% of the nucleotides) of the nucleic acids encoding a particular protein may exist among individuals of a given species due to natural allelic variation. Any and all such nucleotide variations and resulting amino acid polymorphisms are within the scope of this disclosure.

In certain instances, the recombinant nucleic acids of the present disclosure may be operably linked to one or more regulatory nucleotide sequences in an expression construct. Regulatory nucleotide sequences will generally be appropriate to the host cell used for expression. Numerous types of appropriate expression vectors and suitable regulatory sequences are known in the art for a variety of host cells. Typically, said one or more regulatory nucleotide sequences may include, but are not limited to, promoter sequences, leader or signal sequences, ribosomal binding sites, transcriptional start and termination sequences, translational start and termination sequences, and enhancer or activator sequences. Constitutive or inducible promoters as known in the art are contemplated by the disclosure. The promoters may be either naturally occurring promoters, or hybrid promoters that combine elements of more than one promoter. An expression construct may be present in a cell on an episome, such as a plasmid, or the expression construct may be inserted in a chromosome. In some instances, the expression vector contains a selectable marker gene to allow the selection of transformed host cells. Selectable marker genes are well known in the art and will vary with the host cell used.

In certain aspects of the present disclosure, the subject nucleic acid is provided in an expression vector comprising a nucleotide sequence encoding an ActRII polypeptide or a GDF trap and operably linked to at least one regulatory sequence. Regulatory sequences are art-recognized and are selected to direct expression of the ActRII or GDF trap polypeptide. Accordingly, the term regulatory sequence includes promoters, enhancers, and other expression control elements. Exemplary regulatory sequences are described in Goeddel; Gene Expression Technology: Methods in Enzymology, Academic Press, San Diego, CA (1990). For instance, any of a wide variety of expression control sequences that control the expression of a DNA sequence when operatively linked to it may be used in these vectors to express DNA sequences encoding an ActRII or GDF trap polypeptide. Such useful expression control sequences, include, for example, the early and late promoters of SV40, *tet* promoter, adenovirus or cytomegalovirus immediate early promoter, RSV promoters, the lac system, the *trp* system, the TAC or TRC system, T7 promoter whose expression is directed by T7 RNA polymerase, the major operator and promoter regions of phage lambda, the control regions for fd coat protein, the promoter for 3-phosphoglycerate kinase or other glycolytic enzymes, the promoters of acid phosphatase, *e.g.,* Pho5, the promoters of the yeast α-mating factors, the polyhedron promoter of the baculovirus system and other sequences known to control the expression of genes of prokaryotic or eukaryotic cells or their viruses, and various combinations thereof. It should be understood that the design of the expression vector may depend on such factors as the choice of the host cell to be transformed and/or the type of protein desired to be expressed. Moreover, the vector's copy number, the ability to control that copy number and the expression of any other protein encoded by the vector, such as antibiotic markers, should also be considered.

A recombinant nucleic acid of the present disclosure can be produced by ligating the cloned gene, or a portion thereof, into a vector suitable for expression in either prokaryotic cells, eukaryotic cells (yeast, avian, insect or mammalian), or both. Expression vehicles for production of a recombinant ActRII or GDF trap polypeptide include plasmids and other vectors. For instance, suitable vectors include plasmids of the following types: pBR322-derived plasmids, pEMBL-derived plasmids, pEX-derived plasmids, pBTac-derived plasmids and pUC-derived plasmids for expression in prokaryotic cells, such as *E. coli.*

Some mammalian expression vectors contain both prokaryotic sequences to facilitate the propagation of the vector in bacteria, and one or more eukaryotic transcription units that are expressed in eukaryotic cells. The pcDNAI/amp, pcDNAI/neo, pRc/CMV, pSV2gpt, pSV2neo, pSV2-dhfr, pTk2, pRSVneo, pMSG, pSVT7, pko-neo and pHyg derived vectors are examples of mammalian expression vectors suitable for transfection of eukaryotic cells. Some of these vectors are modified with sequences from bacterial plasmids, such as pBR322, to facilitate replication and drug resistance selection in both prokaryotic and eukaryotic cells. Alternatively, derivatives of viruses such as the bovine papilloma virus (BPV-1), or Epstein-Barr virus (pHEBo, pREP-derived and p205) can be used for transient expression of proteins in eukaryotic cells. Examples of other viral (including retroviral) expression systems can be found below in the description of gene therapy delivery systems. The various methods employed in the preparation of the plasmids and in transformation of host organisms are well known in the art. For other suitable expression systems for both prokaryotic and eukaryotic cells, as well as general recombinant procedures, see, *e.g.,* Molecular Cloning A Laboratory Manual, 3rd Ed., ed. by Sambrook, Fritsch and Maniatis (Cold Spring Harbor Laboratory Press, 2001). In some instances, it may be desirable to express the recombinant polypeptides by the use of a baculovirus expression system. Examples of such baculovirus expression systems include pVL-derived vectors (such as pVL1392, pVL1393 and pVL941), pAcUW-derived vectors (such as pAcUW1), and pBlueBac-derived vectors (such as the β-gal containing pBlueBac III).

In a preferred instance, a vector will be designed for production of the subject ActRII or GDF trap polypeptides in CHO cells, such as a Pcmv-Script vector (Stratagene, La Jolla, Calif.), pcDNA4 vectors (Invitrogen, Carlsbad, Calif.) and pCI-neo vectors (Promega, Madison, Wisc.). As will be apparent, the subject gene constructs can be used to cause expression of the subject ActRII polypeptides in cells propagated in culture, *e.g.,* to produce proteins, including fusion proteins or variant proteins, for purification.

This disclosure also pertains to a host cell transfected with a recombinant gene including a coding sequence for one or more of the subject ActRII or GDF trap polypeptides. The host cell may be any prokaryotic or eukaryotic cell. For example, an ActRII or GDF trap polypeptide of the disclosure may be expressed in bacterial cells such as *E. coli,* insect cells (*e.g*., using a baculovirus expression system), yeast, or mammalian cells [*e.g.* a Chinese hamster ovary (CHO) cell line]. Other suitable host cells are known to those skilled in the art.

Accordingly, the present disclosure further pertains to methods of producing the subject ActRII and GDF trap polypeptides. For example, a host cell transfected with an expression vector encoding an ActRII or GDF trap polypeptide can be cultured under appropriate conditions to allow expression of the ActRII or GDF trap polypeptide to occur. The polypeptide may be secreted and isolated from a mixture of cells and medium containing the polypeptide. Alternatively, the ActRII or GDF trap polypeptide may be retained cytoplasmically or in a membrane fraction and the cells harvested, lysed and the protein isolated. A cell culture includes host cells, media and other byproducts. Suitable media for cell culture are well known in the art. The subject polypeptides can be isolated from cell culture medium, host cells, or both, using techniques known in the art for purifying proteins, including ion-exchange chromatography, gel filtration chromatography, ultrafiltration, electrophoresis, immunoaffinity purification with antibodies specific for particular epitopes of the ActRII or GDF trap polypeptides, and affinity purification with an agent that binds to a domain fused to the ActRII or GDF trap polypeptide (*e.g.,* a protein A column may be used to purify an ActRII-Fc or GDF Trap-Fc fusion protein). In some instances, the ActRII or GDF trap polypeptide is a fusion protein containing a domain which facilitates its purification.

In some instances, purification is achieved by a series of column chromatography steps, including, for example, three or more of the following, in any order: protein A chromatography, Q sepharose chromatography, phenylsepharose chromatography, size exclusion chromatography, and cation exchange chromatography. The purification could be completed with viral filtration and buffer exchange. An ActRII-Fc or GDF trap-Fc protein may be purified to a purity of >90%, >95%, >96%, >98%, or >99% as determined by size exclusion chromatography and >90%, >95%, >96%, >98%, or >99% as determined by SDS PAGE. The target level of purity should be one that is sufficient to achieve desirable results in mammalian systems, particularly non-human primates, rodents (mice), and humans.

In another instance, a fusion gene coding for a purification leader sequence, such as a poly-(His)/enterokinase cleavage site sequence at the N-terminus of the desired portion of the recombinant ActRII or GDF trap polypeptide, can allow purification of the expressed fusion protein by affinity chromatography using a Ni²⁺ metal resin. The purification leader sequence can then be subsequently removed by treatment with enterokinase to provide the purified ActRII or GDF trap polypeptide. See, *e.g.,* Hochuli et al. (1987) J. Chromatography 411:177; and Janknecht et al. (1991) PNAS USA 88:8972.

Techniques for making fusion genes are well known. Essentially, the joining of various DNA fragments coding for different polypeptide sequences is performed in accordance with conventional techniques, employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another instance, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed to generate a chimeric gene sequence. See, *e.g.,* Current Protocols in Molecular Biology, eds. Ausubel et al., John Wiley & Sons: 1992.

### C. Antibody Antagonists

In certain aspects, the present disclosure relates to an antibody, or combination of antibodies, that antagonize ActRII activity (*e.g*., inhibition of ActRIIA and/or ActRIIB signaling transduction, such as SMAD 2/3 and/or SMAD 1/5/8 signaling). Such antibodies may bind to and inhibit one or more TGF-β family ligands (*e.g*., GDF8, GDF11, activin A, activin B, activin C, activin E, BMP6, BMP7, or Nodal) or one or more TGF-β family receptors (*e.g*., ActRIIA, ActRIIB, ALK4, or ALK5) particular, the disclosure provides methods of using an antibody ActRII antagonist or combination of antibody ActRII antagonists, alone or in combination with one or more erythropoiesis stimulating agents *(e.g.,* EPO) or other supportive therapies [*e.g*., transfusion of red blood cells or whole blood, iron chelation therapy, treatment with hydroxyurea, *etc.*], to, *e.g.,* increase red blood cell levels in a subject in need thereof, treat or prevent an anemia in a subject in need thereof, treat sickle-cell disease in a subject in need thereof, treat or prevent one or more complication of sickle-cell disease (*e.g*., anemia, anemia crisis, splenomegaly, pain crisis, chest syndrome, acute chest syndrome, blood transfusion requirement, organ damage, pain medicine (management) requirement, splenic sequestration crises, hyperhemolytic crisis, vaso-occlusion, vaso-occlusion crisis, acute myocardial infarction, sickle-cell chronic lung disease, thromboemboli, hepatic failure, hepatomegaly, hepatic sequestration, iron overload, splenic infarction, acute and/or chronic renal failure, pyelonephritis, aneurysm, ischemic stroke, intraparenchymal hemorrhage, subarachnoid hemorrhage, intraventricular hemorrhage, peripheral retinal ischemia, proliferative sickle retinopathy, vitreous hemorrhage, and/or priapism), and/or reduce blood transfusion burden in a subject in need thereof.

In certain instances, a preferred antibody ActRII antagonist of the disclosure is an antibody, or combination of antibodies, that binds to and/or inhibits activity of at least GDF11 (*e.g.,* GDF11-mediated activation of ActRIIA and/or ActRIIB signaling transduction, such as SMAD 2/3 signaling). Optionally, the antibody, or combination of antibodies, further binds to and/or inhibits activity of GDF8 (*e.g*., GDF8-mediated activation of ActRIIA and/or ActRIIB signaling transduction, such as SMAD 2/3 signaling), particularly in the case of a multispecific antibody that has binding affinity for both GDF11 and GDF8 or in the context of a combination of one or more anti-GDFl 1 antibodies and one or more anti-GDF8 antibodies. Optionally, an antibody, or combination of antibodies, of the disclosure does not substantially bind to and/or inhibit activity of activin A (*e.g*., activin A-mediated activation of ActRIIA or ActRIIB signaling transduction, such as SMAD 2/3 signaling). In some instances, an antibody, or combination of antibodies, of the disclosure that binds to and/or inhibits the activity of GDF11 and/or GDF8 further binds to and/or inhibits activity of one of more of activin A, activin B, activin AB, activin C, activin E, BMP6, BMP7, and Nodal (*e.g.,* activation of ActRIIA or ActRIIB SMAD 2/3 and/or SMAD 1/5/8 signaling), particularly in the case of a multispecific antibody that has binding affinity for multiple ActRII ligands or in the context of a combination of multiple antibodies - each having binding affinity for a different ActRII ligand.

In certain aspects, an ActRII antagonist of the present disclosure is an antibody, or combination of antibodies, that binds to and/or inhibits activity of at least GDF8 (*e.g.,* GDF8-mediated activation of ActRIIA and/or ActRIIB signaling transduction, such as SMAD 2/3 signaling). Optionally, the antibody, or combination of antibodies, further binds to and/or inhibits activity of GDF11 (*e.g.,* GDF11-mediated activation of ActRIIA and/or ActRIIB signaling transduction, such as SMAD 2/3 signaling), particularly in the case of a multispecific antibody that has binding affinity for both GDF8 and GDF11 or in the context of a combination of one or more anti-GDF8 antibodies and one or more anti-GDFl 1 antibodies. Optionally, an antibody, or combination of antibodies, of the disclosure does not substantially bind to and/or inhibit activity of activin A (*e.g*., activin A-mediated activation of ActRIIA or ActRIIB signaling transduction, such as SMAD 2/3 signaling). In some instances, an antibody, or combination of antibodies, of the disclosure that binds to and/or inhibits the activity of GDF8 and/or GDF11 further binds to and/or inhibits activity of one of more of activin A, activin B, activin AB, activin C, activin E, BMP6, BMP7, and Nodal (*e.g.,* activation of ActRIIA or ActRIIB signaling transduction, such as SMAD 2/3 and/or SMAD 1/5/8 signaling), particularly in the case of a multispecific antibody that has binding affinity for multiple ActRII ligands or in the context of a combination multiple antibodies - each having binding affinity for a different ActRII ligand.

In another aspect, an ActRII antagonist of the present disclosure is an antibody, or combination of antibodies, that binds to and/or inhibits activity of an ActRII receptor *(e.g.* an ActRIIA or ActRIIB receptor). In preferred instances, an anti-ActRII receptor antibody (*e.g.* an anti-ActRIIA or anti-ActRIIB receptor antibody), or combination of antibodies, of the disclosure binds to an ActRII receptor and prevents binding and/or activation of the ActRII receptor by at least GDF11 (*e.g.,* GDF11-mediated activation of ActRIIA and/or ActRIIB signaling transduction, such as SMAD 2/3 signaling). Optionally, an anti-ActRII receptor antibody, or combination of antibodies, of the disclosure further prevents binding and/or activation of the ActRII receptor by GDF8. Optionally, an anti-ActRII receptor antibody, or combination of antibodies, of the disclosure does not substantially inhibit activin A from binding to and/or activating an ActRII receptor. In some instances, an anti-ActRII receptor antibody, or combination of antibodies, of the disclosure that binds to an ActRII receptor and prevents binding and/or activation of the ActRII receptor by GDF11 and/or GDF8 further prevents binding and/or activation of the ActRII receptor by one or more of activin A, activin B, activin AB, activin C, activin E, BMP6, BMP7, and Nodal.

The term antibody is used herein in the broadest sense and encompasses various antibody structures, including but not limited to monoclonal antibodies, polyclonal antibodies, multispecific antibodies (*e.g*., bispecific antibodies), and antibody fragments so long as they exhibit the desired antigen-binding activity. An antibody fragment refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (*e.g*., scFv); and multispecific antibodies formed from antibody fragments. See, *e.g.,* Hudson et al. (2003) Nat. Med. 9:129-134; Plückthun, in The Pharmacology of Monoclonal Antibodies, vol. 113, Rosenburg and Moore eds., (Springer-Verlag, New York), pp. 269-315 (1994); WO 93/16185; and U.S. Pat. Nos. 5,571,894, 5,587,458, and 5,869,046. Antibodies disclosed herein may be polyclonal antibodies or monoclonal antibodies. In certain instances, the antibodies of the present disclosure comprise a label attached thereto and able to be detected (*e.g.,* the label can be a radioisotope, fluorescent compound, enzyme, or enzyme co-factor). In preferred instances, the antibodies of the present disclosure are isolated antibodies.

Diabodies are antibody fragments with two antigen-binding sites that may be bivalent or bispecific. See, *e.g.,* EP 404,097; WO 1993/01161; Hudson et al. (2003) Nat. Med. 9:129-134 (2003); and Hollinger et al. (1993) Proc. Natl. Acad. Sci. USA 90: 6444-6448. Triabodies and tetrabodies are also described in Hudson et al. (2003) Nat. Med. 9:129-134.

Single-domain antibodies are antibody fragments comprising all or a portion of the heavy-chain variable domain or all or a portion of the light-chain variable domain of an antibody. In certain instances, a single-domain antibody is a human single-domain antibody. *See, e.g.,* U.S. Pat. No. 6,248,516.

Antibody fragments can be made by various techniques, including but not limited to proteolytic digestion of an intact antibody as well as production by recombinant host cells (e.g., E. coli or phage), as described herein.

The antibodies herein may be of any class. The class of an antibody refers to the type of constant domain or constant region possessed by its heavy chain. There are five major classes of antibodies: IgA, IgD, IgE, IgG, and IgM, and several of these may be further divided into subclasses (isotypes), for example, IgG₁, IgG₂, IgG₃, IgG₄, IgA₁, and IgA₂. The heavy-chain constant domains that correspond to the different classes of immunoglobulins are called alpha, delta, epsilon, gamma, and mu.

In general, an antibody for use in the methods disclosed herein specifically binds to its target antigen, preferably with high binding affinity. Affinity may be expressed as a K_{D} value and reflects the intrinsic binding affinity (*e.g*., with minimized avidity effects). Typically, binding affinity is measured *in vitro,* whether in a cell-free or cell-associated setting. Any of a number of assays known in the art, including those disclosed herein, can be used to obtain binding affinity measurements including, for example, surface plasmon resonance (Biacore™ assay), radiolabeled antigen binding assay (RIA), and ELISA. In some instances, antibodies of the present disclosure bind to their target antigens (e.g. GDF11, GDF8, ActRIIA, ActRIIB, *etc*.) with at least a K_{D} of 1x 10⁻⁷ or stronger, 1x10⁻⁸ or stronger, 1x10⁻⁹ or stronger, 1x10⁻¹⁰ or stronger, 1x10⁻¹¹ or stronger, 1x10⁻¹² or stronger, 1x10⁻¹³ or stronger, or 1x10⁻¹⁴ or stronger.

In certain instances, K_{D} is measured by RIA performed with the Fab version of an antibody of interest and its target antigen as described by the following assay. Solution binding affinity of Fabs for the antigen is measured by equilibrating Fab with a minimal concentration of radiolabeled antigen (*e.g*., ¹²⁵I-labeled) in the presence of a titration series of unlabeled antigen, then capturing bound antigen with an anti-Fab antibody-coated plate [see, *e.g.,* Chen et al. (1999) J. Mol. Biol. 293:865-881]. To establish conditions for the assay, multi-well plates (*e.g.,* MICROTITER® from Thermo Scientific) are coated (*e.g.,* overnight) with a capturing anti-Fab antibody (*e.g*., from Cappel Labs) and subsequently blocked with bovine serum albumin, preferably at room temperature (approximately 23°C). In a non-adsorbent plate, radiolabeled antigen are mixed with serial dilutions of a Fab of interest [e.g., consistent with assessment of the anti-VEGF antibody, Fab-12, in Presta et al., (1997) Cancer Res. 57:4593-4599]. The Fab of interest is then incubated, preferably overnight but the incubation may continue for a longer period (*e.g*., about 65 hours) to ensure that equilibrium is reached. Thereafter, the mixtures are transferred to the capture plate for incubation, preferably at room temperature for about one hour. The solution is then removed and the plate is washed times several times, preferably with polysorbate 20 and PBS mixture. When the plates have dried, scintillant (*e.g*., MICROSCINT® from Packard) is added, and the plates are counted on a gamma counter (*e.g*., TOPCOUNT® from Packard).

According to another instance, K_{D} is measured using surface plasmon resonance assays using, for example a BIACORE® 2000 or a BIACORE® 3000 (Biacore, Inc., Piscataway, N.J.) with immobilized antigen CM5 chips at about 10 response units (RU). Briefly, carboxymethylated dextran biosensor chips (CM5, Biacore, Inc.) are activated with N-ethyl-N'-(3-dimethylaminopropyl)-carbodiimide hydrochloride (EDC) and N-hydroxysuccinimide (NHS) according to the supplier's instructions. For example, an antigen can be diluted with 10 mM sodium acetate, pH 4.8, to 5 µg/ml (about 0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20®) surfactant (PBST) at at a flow rate of approximately 25 µl/min. Association rates (kₒₙ) and dissociation rates (k_{off}) are calculated using, for example, a simple one-to-one Langmuir binding model (BIACORE® Evaluation Software version 3.2) by simultaneously fitting the association and dissociation sensorgrams. The equilibrium dissociation constant (K_{D}) is calculated as the ratio k_{off} / kₒₙ [see, *e.g.,* Chen et al., (1999) J. Mol. Biol. 293:865-881]. If the on-rate exceeds, for example, 10⁶ M⁻¹ s⁻¹ by the surface plasmon resonance assay above, then the on-rate can be determined by using a fluorescent quenching technique that measures the increase or decrease in fluorescence emission intensity (*e.g*., excitation=295 nm; emission=340 nm, 16 nm bandpass) of a 20 nM anti-antigen antibody (Fab form) in PBS in the presence of increasing concentrations of antigen as measured in a spectrometer, such as a stop-flow equipped spectrophometer (Aviv Instruments) or a 8000-series SLM-AMINCO® spectrophotometer (ThermoSpectronic) with a stirred cuvette.

As used herein, anti-GDFl 1 antibody generally refers to an antibody that is capable of binding to GDF11 with sufficient affinity such that the antibody is useful as a diagnostic and/or therapeutic agent in targeting GDF11. In certain instances, the extent of binding of an anti-GDFl 1 antibody to an unrelated, non-GDFl 1 protein is less than about 10%, 9%, 8%, 7%, 6%, 5%, 4%, 3%, 2%, or less than 1% of the binding of the antibody to GDF11 as measured, for example, by a radioimmunoassay (RIA). In certain instances, an anti-GDFl 1 antibody binds to an epitope of GDF11 that is conserved among GDF11 from different species. In certain preferred instances, an anti-GDFl 1 antibody of the present disclosure is an antagonist antibody that can inhibit GDF11 activity. For example, an anti-GDFl 1 antibody of the disclosure may inhibit GDF11 from binding to a cognate receptor *(e.g.,* ActRIIA or ActRIIB receptor) and/or inhibit GDF11-mediated signal transduction (activation) of a cognate receptor, such as SMAD2/3 signaling by ActRIIA and/or ActRIIB receptors. In some instances, anti-GDFl 1 antibodies of the present disclosure do not substantially bind to and/or inhibit activity of activin A. It should be noted that GDF11 has high sequence homology to GDF8 and therefore antibodies that bind and/or to GDF11, in some cases, may also bind to and/or inhibit GDF8.

An anti-GDF8 antibody refers to an antibody that is capable of binding to GDF8 with sufficient affinity such that the antibody is useful as a diagnostic and/or therapeutic agent in targeting GDF8. In certain instances, the extent of binding of an anti-GDF8 antibody to an unrelated, non-GDF8 protein is less than about 10%, 9%, 8%, 7%, 6%, 5%, 4%, 3%, 2%, or less than 1% of the binding of the antibody to GDF8 as measured, for example, by a radioimmunoassay (RIA). In certain instances, an anti-GDF8 antibody binds to an epitope of GDF8 that is conserved among GDF8 from different species. In preferred instances, an anti-GDF8 antibody of the present disclosure is an antagonist antibody that can inhibit GDF8 activity. For example, an anti-GDF8 antibody of the disclosure may inhibit GDF8 from binding to a cognate receptor (*e.g*., ActRIIA or ActRIIB receptor) and/or inhibit GDF8-mediated signal transduction (activation) of a cognate receptor, such as SMAD2/3 signaling by ActRIIA and/or ActRIIB receptors. In some instances, anti-GDF8 antibodies of the present disclosure do not substantially bind to and/or inhibit activity of activin A. It should be noted that GDF8 has high sequence homology to GDF11 and therefore antibodies that bind and/or to GDF8, in many cases, may also bind to and/or inhibit GDF11.

An anti-ActRIIA antibody refers to an antibody that is capable of binding to ActRIIA with sufficient affinity such that the antibody is useful as a diagnostic and/or therapeutic agent in targeting ActRIIA. In certain instances, the extent of binding of an anti-ActRIIA antibody to an unrelated, non-ActRIIA protein is less than about 10%, 9%, 8%, 7%, 6%, 5%, 4%, 3%, 2%, or less than 1% of the binding of the antibody to ActRIIA as measured, for example, by a radioimmunoassay (RIA). In certain instances, an anti-ActRIIA antibody binds to an epitope of ActRIIA that is conserved among ActRIIA from different species. In preferred instances, an anti-ActRIIA antibody of the present disclosure is an antagonist antibody that can inhibit ActRIIA activity. For example, an anti-ActRIIA antibody of the present disclosure may inhibit one or more ActRIIA ligands selected from activin A, activin B, activin AB, activin C, activin E, GDF11, GDF8, activin A, BMP6, and BMP7 from binding to the ActRIIA receptor and/or inhibit one of these ligands from activating ActRIIA signaling (*e.g*., SMAD2/3 and/or SMAD 1/5/8 ActRIIA signaling). In preferred instances, anti-ActRIIA antibodies of the present disclosure inhibit GDF11 from binding to the ActRIIA receptor and/or inhibit GDF11 from activating ActRIIA signaling. Optionally, anti-ActRIIA antibodies of the disclosure further inhibit GDF8 from binding to the ActRIIA receptor and/or inhibit GDF8 from activating ActRIIA signaling. Optionally, anti-ActRIIA antibodies of the present disclosure do not substantially inhibit activin A from binding to the ActRIIA receptor and/or do not substantially inhibit activin A-mediated activation of ActRIIA signaling. In some instances, an anti-ActRIIA antibody of the disclosure that inhibits GDF11 and/or GDF8 from binding to and/or activating an ActRIIA receptor further inhibits one or more of activin A, activin B, activin AB, activin C, activin E, activin A, GDF8, BMP6, and BMP7 from binding to and/or activating the ActRIIA receptor.

An anti-ActRIIB antibody refers to an antibody that is capable of binding to ActRIIB with sufficient affinity such that the antibody is useful as a diagnostic and/or therapeutic agent in targeting ActRIIB. In certain instances, the extent of binding of an anti-ActRIIB antibody to an unrelated, non-ActRIIB protein is less than about 10%, 9%, 8%, 7%, 6%, 5%, 4%, 3%, 2%, or less than 1% of the binding of the antibody to ActRIIB as measured, for example, by a radioimmunoassay (RIA). In certain instances, an anti-ActRIIB antibody binds to an epitope of ActRIIB that is conserved among ActRIIB from different species. In preferred instances, an anti-ActRIIB antibody of the present disclosure is an antagonist antibody that can inhibit ActRIIB activity. For example, an anti-ActRIIB antibody of the present disclosure may inhibit one or more ActRIIB ligands selected from activin A, activin B, activin AB, activin C, activin E, GDF11, GDF8, activin A, BMP6, and BMP7 from binding to the ActRIIB receptor and/or inhibit one of these ligands from activating ActRIIB signaling (*e.g*., SMAD2/3 and/or SMAD 1/5/8 ActRIIB signaling). In preferred instances, anti-ActRIIB antibodies of the present disclosure inhibit GDF11 from binding to the ActRIIB receptor and/or inhibit GDF11 from activating ActRIIB signaling. Optionally, anti-ActRIIB antibodies of the disclosure further inhibit GDF8 from binding to the ActRIIB receptor and/or inhibit GDF8 from activating ActRIIB signaling. Optionally, anti-ActRIIB antibodies of the present disclosure do not substantially inhibit activin A from binding to the ActRIIB receptor and/or do not substantially inhibit activin A-mediated activation of ActRIIB signaling. In some instances, an anti-ActRIIB antibody of the disclosure that inhibits GDF11 and/or GDF8 from binding to and/or activating an ActRIIB receptor further inhibits one or more of activin A, activin B, activin AB, activin C, activin E, activin A, GDF8, BMP6, and BMP7 from binding to and/or activating the ActRIIB receptor.

The nucleic acid and amino acid sequences of human GDF11, GDF8, activin A, activin B, activin AB, activin C, activin E, GDF8, BMP6, BMP7, ActRIIB, and ActRIIA are well known in the art and thus antibody antagonists for use in accordance with this disclosure may be routinely made by the skilled artisan based on the knowledge in the art and teachings provided herein.

In certain instances, an antibody provided herein (*e.g.,* an anti-GDFl 1 antibody, an anti-GDF8 antibody, an anti-ActRIIA antibody, or an anti-ActRIIB antibody) is a chimeric antibody. A chimeric antibody refers to an antibody in which a portion of the heavy and/or light chain is derived from a particular source or species, while the remainder of the heavy and/or light chain is derived from a different source or species. Certain chimeric antibodies are described, for example, in U.S. Pat. No. 4,816,567; and Morrison et al., (1984) Proc. Natl. Acad. Sci. USA, 81:6851-6855. In some instances, a chimeric antibody comprises a non-human variable region *(e.g.,* a variable region derived from a mouse, rat, hamster, rabbit, or non-human primate, such as a monkey) and a human constant region. In some instances, a chimeric antibody is a "class switched" antibody in which the class or subclass has been changed from that of the parent antibody. In general, chimeric antibodies include antigen-binding fragments thereof.

In certain instances, a chimeric antibody provided herein *(e.g.,* an anti-GDFl 1 antibody, an anti-GDF8 antibody, an anti-ActRIIA antibody, or an anti-ActRIIB antibody) is a humanized antibody. A humanized antibody refers to a chimeric antibody comprising amino acid residues from non-human hypervariable regions (HVRs) and amino acid residues from human framework regions (FRs). In certain instances, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, *e.g.,* a non-human antibody, refers to an antibody that has undergone humanization.

Humanized antibodies and methods of making them are reviewed, for example, in Almagro and Fransson (2008) Front. Biosci. 13:1619-1633 and are further described, for example, in Riechmann et al., (1988) Nature 332:323-329; Queen et al. (1989) Proc. Nat'1 Acad. Sci. USA 86:10029-10033; U.S. Pat. Nos. 5,821,337, 7,527,791, 6,982,321, and 7,087,409; Kashmiri et al., (2005) Methods 36:25-34 [describing SDR (a-CDR) grafting]; Padlan, Mol. Immunol. (1991) 28:489-498 (describing "resurfacing"); Dall'Acqua et al. (2005) Methods 36:43-60 (describing "FR shuffling"); Osbourn et al. (2005) Methods 36:61-68; and Klimka et al. Br. J. Cancer (2000) 83:252-260 (describing the "guided selection" approach to FR shuffling).

Human framework regions that may be used for humanization include but are not limited to: framework regions selected using the "best-fit" method [see, *e.g.,* Sims et al. (1993) J. Immunol. 151:2296 ]; framework regions derived from the consensus sequence of human antibodies of a particular subgroup of light-chain or heavy-chain variable regions [see, *e.g.,* Carter et al. (1992) Proc. Natl. Acad. Sci. USA, 89:4285; and Presta et al. (1993) J. Immunol., 151:2623]; human mature (somatically mutated) framework regions or human germline framework regions [see, *e.g.,* Almagro and Fransson (2008) Front. Biosci. 13:1619-1633]; and framework regions derived from screening FR libraries [see, *e.g.,* Baca et cd., (1997) J. Biol. Chem. 272:10678-10684; and Rosok et cd., (1996) J. Biol. Chem. 271:22611-22618].

In certain instances, an antibody provided herein (*e.g.,* an anti-GDFl 1 antibody, an anti-GDF8 antibody, an anti-ActRIIA antibody, or an anti-ActRIIB antibody) is a human antibody. Human antibodies can be produced using various techniques known in the art. Human antibodies are described generally in van Dijk and van de Winkel (2001) Curr. Opin. Pharmacol. 5: 368-74 and Lonberg (2008) Curr. Opin. Immunol. 20:450-459.

Human antibodies may be prepared by administering an immunogen (*e.g*., a GDF11 polypeptide, GDF8 polypeptide, an ActRIIA polypeptide, or an ActRIIB polypeptide) to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic animals, the endogenous immunoglobulin loci have generally been inactivated. For a review of methods for obtaining human antibodies from transgenic animals, see, for example, Lonberg (2005) Nat. Biotechnol. 23:1117-1125; U.S. Pat. Nos. 6,075,181 and 6,150,584 (describing XENOMOUSE™ technology); U.S. Pat. No. 5,770,429 (describing HuMab® technology); U.S. Pat. No. 7,041,870 (describing K-M MOUSE® technology); and U.S. Patent Application Publication No. 2007/0061900 (describing VelociMouse® technology). Human variable regions from intact antibodies generated by such animals may be further modified, for example, by combining with a different human constant region.

Human antibodies provided herein can also be made by hybridoma-based methods. Human myeloma and mouse-human heteromyeloma cell lines for the production of human monoclonal antibodies have been described [see, *e.g.,* Kozbor J. Immunol., (1984) 133: 3001; Brodeur et al. (1987) Monoclonal Antibody Production Techniques and Applications, pp. 51-63, Marcel Dekker, Inc., New York; and Boerner et al. (1991) J. Immunol., 147: 86]. Human antibodies generated via human B-cell hybridoma technology are also described in Li et al., (2006) Proc. Natl. Acad. Sci. USA, 103:3557-3562. Additional methods include those described, for example, in U.S. Pat. No. 7,189,826 (describing production of monoclonal human IgM antibodies from hybridoma cell lines) and Ni, Xiandai Mianyixue (2006) 26(4):265-268 (2006) (describing human-human hybridomas). Human hybridoma technology (Trioma technology) is also described in Vollmers and Brandlein (2005) Histol. Histopathol., 20(3):927-937 (2005) and Vollmers and Brandlein (2005) Methods Find Exp. Clin. Pharmacol., 27(3):185-91.

Human antibodies provided herein (*e.g.,* an anti-GDFl 1 antibody, an anti-activin B antibody, an anti-ActRIIA antibody, or an anti-ActRIIB antibody) may also be generated by isolating Fv clone variable-domain sequences selected from human-derived phage display libraries. Such variable-domain sequences may then be combined with a desired human constant domain. Techniques for selecting human antibodies from antibody libraries are described herein.

For example, antibodies of the present disclosure may be isolated by screening combinatorial libraries for antibodies with the desired activity or activities. A variety of methods are known in the art for generating phage-display libraries and screening such libraries for antibodies possessing the desired binding characteristics. Such methods are reviewed, for example, in Hoogenboom et al. (2001) in Methods in Molecular Biology 178:1-37, O'Brien et al., ed., Human Press, Totowa, N.J. and further described, for example, in the McCafferty et al. (1991) Nature 348:552-554; Clackson et al., (1991) Nature 352: 624-628; Marks et al. (1992) J. Mol. Biol. 222:581-597; Marks and Bradbury (2003) in Methods in Molecular Biology 248:161-175, Lo, ed., Human Press, Totowa, N.J.; Sidhu et al. (2004) J. Mol. Biol. 338(2):299-310; Lee et al. (2004) J. Mol. Biol. 340(5):1073-1093; Fellouse (2004) Proc. Natl. Acad. Sci. USA 101(34):12467-12472; and Lee et al. (2004) J. Immunol. Methods 284(1-2): 119-132.

In certain phage display methods, repertoires of VH and VL genes are separately cloned by polymerase chain reaction (PCR) and recombined randomly in phage libraries, which can then be screened for antigen-binding phage as described in Winter et al. (1994) Ann. Rev. Immunol., 12: 433-455. Phage typically display antibody fragments, either as single-chain Fv (scFv) fragments or as Fab fragments. Libraries from immunized sources provide high-affinity antibodies to the immunogen (*e.g*., GDF11, activin B, ActRIIA, or ActRIIB) without the requirement of constructing hybridomas. Alternatively, the naive repertoire can be cloned (*e.g.,* from human) to provide a single source of antibodies directed against a wide range of non-self and also self-antigens without any immunization as described by Griffiths et al. (1993) EMBO J, 12: 725-734. Finally, naive libraries can also be made synthetically by cloning un-rearranged V-gene segments from stem cells and using PCR primers containing random sequence to encode the highly variable CDR3 regions and to accomplish rearrangement in vitro, as described by Hoogenboom and Winter (1992) J. Mol. Biol., 227: 381-388. Patent publications describing human antibody phage libraries include, for example: U.S. Pat. No. 5,750,373, and U.S. Patent Publication Nos. 2005/0079574, 2005/0119455, 2005/0266000, 2007/0117126, 2007/0160598, 2007/0237764, 2007/0292936, and 2009/0002360.

In certain instances, an antibody provided herein is a multispecific antibody, for example, a bispecific antibody. Multispecific antibodies (typically monoclonal antibodies) have binding specificities for at least two different epitopes (*e.g*., two, three, four, five, or six or more) on one or more (*e.g*., two, three, four, five, six or more) antigens.

In certain instances, a multispecific antibody of the present disclosure comprises two or more binding specificities, with at least one of the binding specificities being for a GDF11 epitope, and optionally one or more additional binding specificities being for an epitope on a different ActRII ligand (*e.g.,* GDF8, activin A, activin B, activin AB, activin C, activin E, BMP6 BMP7 and/or Nodal) and/or an ActRII receptor (*e.g.,* an ActRIIA and/or ActRIIB receptor). In certain instances, multispecific antibodies may bind to two or more different epitopes of GDF11. Preferably a multispecific antibody of the disclosure that has binding affinity, in part, for a GDF11 epitope can be used to inhibit a GDF11 activity (*e.g*., the ability to bind to and/or activate an ActRIIA and/or ActRIIB receptor), and optionally inhibit the activity of one or more different ActRII ligands (*e.g*., GDF8, activin A, activin B, activin AB, activin C, activin E, BMP6, BMP7 and/or Nodal) and/or an ActRII receptor (*e.g.,* an ActRIIA or ActRIIB receptor). In certain preferred instances, multispecific antibodies of the present disclosure that bind to and/or inhibit GDF11 further bind to and/or inhibit at least GDF8. Optionally, multispecific antibodies of the disclosure that bind to and/or inhibit GDF11 do not substantially bind to and/or substantially inhibit activin A. In some instances, multispecific antibodies of the disclosure that bind to and/or inhibit GDF11 and GDD8 further bind to and/or inhibit one or more of activin A, activin B, activin AB, activin C, activin E, BMP6, BMP7 and/or Nodal.

In certain instances, a multispecific antibody of the present disclosure comprises two or more binding specificities, with at least one of the binding specificities being for a GDF8 epitope, and optionally one or more additional binding specificities being for an epitope on a different ActRII ligand *(e.g.,* GDF11, activin A, activin B, activin AB, activin C, activin E, BMP6, BMP7 and/or Nodal) and/or an ActRII receptor *(e.g.,* an ActRIIA and/or ActRIIB receptor). In certain instances, multispecific antibodies may bind to two or more different epitopes of GDF8. Preferably a multispecific antibody of the disclosure that has binding affinity, in part, for an GDF8 epitope can be used to inhibit an GDF8 activity (*e.g*., the ability to bind to and/or activate an ActRIIA and/or ActRIIB receptor), and optionally inhibit the activity of one or more different ActRII ligands *(e.g.,* GDF11, activin A, activin B, activin AB, activin C, activin E, BMP6, BMP7 and/or Nodal) and/or an ActRII receptor *(e.g.,* an ActRIIA or ActRIIB receptor). In certain preferred instances, multispecific antibodies of the present disclosure that bind to and/or inhibit GDF8 further bind to and/or inhibit at least GDF11. Optionally, multispecific antibodies of the disclosure that bind to and/or inhibit GDF8 do not substantially bind to and/or substantially inhibit activin A. In some instances, multispecific antibodies of the disclosure that bind to and/or inhibit GDF8 and GDF11 further bind to and/or inhibit one or more of activin A, activin B, activin AB, activin C, activin E, BMP6, BMP7 and/or Nodal.

Engineered antibodies with three or more functional antigen binding sites, including "octopus antibodies," are also included herein (see, *e.g.,* US 2006/0025576A1).

In certain instances, the antibodies disclosed herein *(e.g.,* an anti-GDFl 1 antibody, an anti-activin B antibody, an anti-ActRIIA antibody, or an anti-ActRIIB antibody) are monoclonal antibodies. Monoclonal antibody refers to an antibody obtained from a population of substantially homogeneous antibodies, *i.e.,* the individual antibodies comprising the population are identical and/or bind the same epitope, except for possible variant antibodies, *e.g.,* containing naturally occurring mutations or arising during production of a monoclonal antibody preparation, such variants generally being present in minor amounts. In contrast to polyclonal antibody preparations, which typically include different antibodies directed against different epitopes, each monoclonal antibody of a monoclonal antibody preparation is directed against a single epitope on an antigen. Thus, the modifier "monoclonal" indicates the character of the antibody as being obtained from a substantially homogeneous population of antibodies and is not to be construed as requiring production of the antibody by any particular method. For example, the monoclonal antibodies to be used in accordance with the present methods may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

For example, by using immunogens derived from GDF11 or GDF8, anti-protein/anti-peptide antisera or monoclonal antibodies can be made by standard protocols [see, *e.g.,* Antibodies: A Laboratory Manual (1988) ed. by Harlow and Lane, Cold Spring Harbor Press]. A mammal, such as a mouse, hamster, or rabbit can be immunized with an immunogenic form of the GDF11 or GDF8 polypeptide, an antigenic fragment which is capable of eliciting an antibody response, or a fusion protein. Techniques for conferring immunogenicity on a protein or peptide include conjugation to carriers or other techniques well known in the art. An immunogenic portion of a GDF11 or GDF8 polypeptide can be administered in the presence of adjuvant. The progress of immunization can be monitored by detection of antibody titers in plasma or serum. Standard ELISA or other immunoassays can be used with the immunogen as antigen to assess the levels of antibody production and/or level of binding affinity.

Following immunization of an animal with an antigenic preparation of GDF11 or GDF8, antisera can be obtained and, if desired, polyclonal antibodies can be isolated from the serum. To produce monoclonal antibodies, antibody-producing cells (lymphocytes) can be harvested from an immunized animal and fused by standard somatic cell fusion procedures with immortalizing cells such as myeloma cells to yield hybridoma cells. Such techniques are well known in the art, and include, for example, the hybridoma technique [see, *e.g.,* Kohler and Milstein (1975) Nature, 256: 495-497], the human B cell hybridoma technique [see, *e.g.,* Kozbar et al. (1983) Immunology Today, 4:72], and the EBV-hybridoma technique to produce human monoclonal antibodies [Cole et al. (1985) Monoclonal Antibodies and Cancer Therapy, Alan R. Liss, Inc. pp. 77-96]. Hybridoma cells can be screened immunochemically for production of antibodies specifically reactive with a GDF11 or GDF8 polypeptide, and monoclonal antibodies isolated from a culture comprising such hybridoma cells.

In certain instances, one or more amino acid modifications may be introduced into the Fc region of an antibody provided herein *(e.g.,* an anti-GDFl 1 antibody, an anti-activin B antibody, an anti-ActRIIA antibody, or an anti-ActRIIB antibody), thereby generating an Fc-region variant. The Fc-region variant may comprise a human Fc-region sequence (*e.g.,* a human IgG1, IgG2, IgG3 or IgG4 Fc region) comprising an amino acid modification (*e.g*., a substitution, deletion, and/or addition) at one or more amino acid positions.

For example, the present disclosure contemplates an antibody variant that possesses some but not all effector functions, which make it a desirable candidate for applications in which the half-life of the antibody *in vivo* is important yet for which certain effector functions [*e.g*., complement-dependent cytotoxicity (CDC) and antibody-dependent cellular cytotoxicity (ADCC)] are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcyR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in, for example, Ravetch and Kinet (1991) Annu. Rev. Immunol. 9:457-492. Non-limiting examples of *in vitro* assays to assess ADCC activity of a molecule of interest are described in U.S. Pat. No. 5,500,362; Hellstrom, I. et al. (1986) Proc. Nat'1 Acad. Sci. USA 83:7059-7063; Hellstrom, I et al. (1985) Proc. Nat'1 Acad. Sci. USA 82:1499-1502; U.S. Pat. No. 5,821,337; and Bruggemann, M. et al. (1987) J. Exp. Med. 166:1351-1361. Alternatively, non-radioactive assay methods may be employed (*e.g*., ACTF™, non-radioactive cytotoxicity assay for flow cytometry; CellTechnology, Inc. Mountain View, Calif.; and CytoTox 96® non-radioactive cytotoxicity assay, Promega, Madison, Wis.). Useful effector cells for such assays include peripheral blood mononuclear cells (PBMC) and natural killer (NK) cells. Alternatively, or additionally, ADCC activity of the molecule of interest may be assessed *in vivo,* for example, in an animal model such as that disclosed in Clynes et al. (1998) Proc. Nat'1 Acad. Sci. USA 95:652-656. C1q binding assays may also be carried out to confirm that the antibody is unable to bind C1q and hence lacks CDC activity [see, *e.g.,* C1q and C3c binding ELISA in WO 2006/029879 and WO 2005/100402]. To assess complement activation, a CDC assay may be performed [see, *e.g.,* Gazzano-Santoro et al. (1996) J. Immunol. Methods 202:163; Cragg, M. S. et al. (2003) Blood 101:1045-1052; and Cragg, M. S, and M. J. Glennie (2004) Blood 103:2738-2743]. FcRn binding and *in vivo* clearance/half-life determinations can also be performed using methods known in the art [see, *e.g.,* Petkova, S. B. et al. (2006) Int. Immunol. 18(12):1759-1769].

Antibodies of the present disclosure (*e.g*., an anti-GDFl 1 antibody, an anti-activin B antibody, an anti-ActRIIA antibody, or an anti-ActRIIB antibody) with reduced effector function include those with substitution of one or more of Fc region residues 238, 265, 269, 270, 297, 327 and 329 (U.S. Pat. No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (U.S. Pat. No. 7,332,581).

In certain instances, it may be desirable to create cysteine-engineered antibodies, *e.g.,* "thioMAbs," in which one or more residues of an antibody are substituted with cysteine residues. In particular instances, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain instances, any one or more of the following residues may be substituted with cysteine: V205 (Kabat numbering) of the light chain; A118 (EU numbering) of the heavy chain; and S400 (EU numbering) of the heavy-chain Fc region. Cysteine engineered antibodies may be generated as described, for example., in U.S. Pat. No. 7,521,541.

In addition, the techniques used to screen antibodies in order to identify a desirable antibody may influence the properties of the antibody obtained. For example, if an antibody is to be used for binding an antigen in solution, it may be desirable to test solution binding. A variety of different techniques are available for testing interaction between antibodies and antigens to identify particularly desirable antibodies. Such techniques include ELISAs, surface plasmon resonance binding assays (*e.g.*, the Biacore™ binding assay, Biacore AB, Uppsala, Sweden), sandwich assays (*e.g.*, the paramagnetic bead system of IGEN International, Inc., Gaithersburg, Maryland), western blots, immunoprecipitation assays, and immunohistochemistry.

In certain instances, amino acid sequence variants of the antibodies and/or the binding polypeptides provided herein are contemplated. For example, it may be desirable to improve the binding affinity and/or other biological properties of the antibody and/or binding polypeptide. Amino acid sequence variants of an antibody and/or binding polypeptides may be prepared by introducing appropriate modifications into the nucleotide sequence encoding the antibody and/or binding polypeptide, or by peptide synthesis. Such modifications include, for example, deletions from, and/or insertions into, and/or substitutions of residues within, the amino acid sequences of the antibody and/or binding polypeptide. Any combination of deletion, insertion, and substitution can be made to arrive at the final construct, provided that the final construct possesses the desired characteristics, *e.g*., target-binding (GDF11, GDF8, ActRIIA, and/or ActRIIB binding).

Alterations (*e.g.,* substitutions) may be made in HVRs, for example, to improve antibody affinity. Such alterations may be made in HVR "hotspots," *i.e.,* residues encoded by codons that undergo mutation at high frequency during the somatic maturation process (see, *e.g*., Chowdhury (2008) Methods Mol. Biol. 207:179-196 (2008)), and/or SDRs (a-CDRs), with the resulting variant VH or VL being tested for binding affinity. Affinity maturation by constructing and reselecting from secondary libraries has been described in the art [see, *e.g.,* Hoogenboom et al., in Methods in Molecular Biology 178:1-37, O'Brien et al., ed., Human Press, Totowa, N.J., (2001)]. In some instances of affinity maturation, diversity is introduced into the variable genes chosen for maturation by any of a variety of methods (*e.g.*, error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (*e.g*., 4-6 residues at a time) are randomized. HVR residues involved in antigen binding may be specifically identified, e.g., using alanine scanning mutagenesis or modeling. CDR-H3 and CDR-L3 in particular are often targeted.

In certain instances, substitutions, insertions, or deletions may occur within one or more HVRs so long as such alterations do not substantially reduce the ability of the antibody to bind to the antigen. For example, conservative alterations (*e.g.*, conservative substitutions as provided herein) that do not substantially reduce binding affinity may be made in HVRs. Such alterations may be outside of HVR "hotspots" or SDRs. In certain instances of the variant VH and VL sequences provided above, each HVR either is unaltered, or contains no more than one, two, or three amino acid substitutions.

A useful method for identification of residues or regions of the antibody and/or the binding polypeptide that may be targeted for mutagenesis is called "alanine scanning mutagenesis", as described by Cunningham and Wells (1989) Science, 244:1081-1085. In this method, a residue or group of target residues (*e.g*., charged residues such as arg, asp, his, lys, and glu) are identified and replaced by a neutral or negatively charged amino acid (*e.g.,* alanine or polyalanine) to determine whether the interaction of the antibody or binding polypeptide with antigen is affected. Further substitutions may be introduced at the amino acid locations demonstrating functional sensitivity to the initial substitutions. Alternatively, or additionally, a crystal structure of an antigen-antibody complex can be used to identify contact points between the antibody and antigen. Such contact residues and neighboring residues may be targeted or eliminated as candidates for substitution. Variants may be screened to determine whether they contain the desired properties.

Amino-acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include fusion of the N- or C-terminus of the antibody to an enzyme (*e.g.,* for ADEPT) or a polypeptide which increases the serum half-life of the antibody.

In certain instances, an antibody and/or binding polypeptide provided herein may be further modified to contain additional non-proteinaceous moieties that are known in the art and readily available. The moieties suitable for derivatization of the antibody and/or binding polypeptide include but are not limited to water-soluble polymers. Non-limiting examples of water-soluble polymers include, but are not limited to, polyethylene glycol (PEG), copolymers of ethylene glycol/propylene glycol, carboxymethyl cellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody and/or binding polypeptide may vary, and if more than one polymer are attached, they can be the same or different molecules. In general, the number and/or type of polymers used for derivatization can be determined based on considerations including, but not limited to, the particular properties or functions of the antibody and/or binding polypeptide to be improved, whether the antibody derivative and/or binding polypeptide derivative will be used in a therapy under defined conditions.

Any of the ActRII antagonist antibodies disclosed herein (*e.g.,* an anti-activin A antibody, an anti-activin B antibody, an anti-activin C antibody, an anti-activin E antibody, an anti-GDF11 antibody, an anti-GDF8 antibody, an anti-BMP6 antibody, an anti-BMP7 antibody, an anti-ActRIIA antibody, and/or or an anti-ActRIIB antibody) can be combined with one or more additional ActRII antagonist agents of the disclosure to achieve the desired effect (*e.g*., increase red blood cell levels and/or hemoglobin in a subject in need thereof, treat or prevent an anemia, treat sickle-cell disease, treat or prevent one or more complications of sickle-cell disease). For example, an ActRII antagonist antibody disclosed herein (*e.g.,* an anti-GDF11 antibody, an anti-activin B antibody, an anti-activin C antibody, an anti-activin E antibody, an anti-GDF11 antibody, an anti-GDF8 antibody, an anti-BMP6 antibody, an-anti-BMP7 antibody, an anti-ActRIIA antibody, or an anti-ActRIIB antibody) can be used in combination with i) one or more additional ActRII antagonist antibodies disclosed herein, ii) one or more ActRII polypeptides disclosed herein (*e.g*., ActRIIA and/or ActRIIB polypeptides), iii) one or more GDF traps disclosed herein; iv) one or more small-molecule ActRII antagonist disclosed herein (*e.g.,* a small molecule antagonist of one or more of GDF11, GDF8, activin A, activin B, activin AB, activin C, activin E, BMP6, BMP7, Nodal, ActRIIA, and/or ActRIIB); v) one or more polynucleotide ActRII antagonists disclosed herein (*e.g.,* a polynucleotide antagonist of one or more of GDF11, GDF8, activin A, activin B, activin AB, activin C, activin E, BMP6, BMP7, Nodal, ActRIIA, and/or ActRIIB); vi) one or more follistatin polypeptides disclosed herein; and/or vii) one or more FLRG polypeptides disclosed herein.

### D. Small-Molecule Antagonists

In another aspect, the present disclosure relates to a small molecule, or combination of small molecules, that antagonizes ActRII activity (*e.g*., inhibition of ActRIIA and/or ActRIIB signaling transduction, such as SMAD 2/3 and/or SMAD 1/5/8 signaling). In particular, the disclosure provides methods of using a small-molecule antagonist or combination of small antagonists of ActRII, alone or in combination with one or more erythropoiesis stimulating agents (*e.g.,* EPO) or other supportive therapies [*e.g.,* hematopoietic growth factors (*e.g.,* G-CSF or GM-CSF), transfusion of red blood cells or whole blood, iron chelation therapy, treatment with hydroxyurea, *etc*.], to, *e.g.,* increase red blood cell levels in a subject in need thereof, treat or prevent an anemia in a subject in need thereof, treat sickle-cell disease in a subject in need thereof, treat or prevent one or more complication of sickle-cell disease (*e.g*., anemia, anemia crisis, splenomegaly, pain crisis, chest syndrome, acute chest syndrome, blood transfusion requirement, organ damage, pain medicine requirement, splenic sequestration crises, hyperhemolytic crisis, vaso-occlusion, vaso-occlusion crisis, acute myocardial infarction, sickle-cell chronic lung disease, thromboemboli, hepatic failure, hepatomegaly, hepatic sequestration, iron overload, splenic infarction, acute and/or chronic renal failure, pyelonephritis, aneurysm, ischemic stroke, intraparenchymal hemorrhage, subarachnoid hemorrhage, intraventricular hemorrhage, peripheral retinal ischemia, proliferative sickle retinopathy, vitreous hemorrhage, priapism), and/or reduce blood transfusion burden in a subject in need thereof.

In some instances, a preferred ActRII antagonist of the present disclosure is a small-molecule antagonist, or combination of small-molecule antagonists, that direct or indirect inhibits at least GDF11 activity. Optionally, such a small-molecule antagonist, or combination of small-molecule antagonists, may further inhibit, either directly or indirectly, GDF8. Optionally, a small-molecule antagonist, or combination of small-molecule antagonists, of the present disclosure does not substantially inhibit activin A activity. In some instances, a small-molecule antagonist, or combination of small-molecule antagonists, of the present disclosure that inhibits, either directly or indirectly, GDF11 and/or GDF8 activity further inhibits, either directly or indirectly, activity of one or more of activin A, activin B, activin AB, activin C, activin E, BMP6, BMP7, Nodal, ActRIIA, and ActRIIB.

In certain instances, a small-molecule antagonist, or combination of small-molecule antagonists, of the present disclosure is an indirect inhibitor of one or more of GDF11, GDF8, activin A, activin B, activin AB, activin C, activin E, BMP6, Nodal, ActRIIA, and ActRIIB. For example, a small-molecule antagonist, or combination of small-molecule antagonists, of the present disclosure may inhibit the expression (*e.g*., transcription, translation, cellular secretion, or combinations thereof) of at least GDF11. Optionally, such a small-molecule antagonist, or combination of small-molecule antagonists, may further inhibit expression of GDF8. Optionally, a small-molecule antagonist, or combinations of small-molecule antagonists, of the disclosure does not substantially inhibit the expression of activin A. In some instances, a small-molecule antagonist, or combination of small-molecule antagonists, of the disclosure that inhibits expression of GDF11 and/or GDF8 may further inhibit the expression of one or more of activin A, activin B, activin AB, activin C, activin E, BMP6, BMP7, Nodal, ActRIIA, and ActRIIB.

In other instances, a small-molecule antagonist, or combination of small-molecule antagonists, of the present disclosure is direct inhibitor of one or more of GDF11, GDF8, activin A, activin B, activin AB, activin C, activin E, BMP6, BMP7, Nodal, ActRIIA, and ActRIIB. For example, a preferred small-molecule antagonist, or combination of small-molecule antagonists, of the present disclosure directly binds to and inhibits at least GDF11 activity (*e.g*. inhibits the ability GDF11 to bind to an ActRIIA and/or ActRIIB receptor; inhibits GDF11-mediated activation of the ActRIIA and/or ActRIIB signaling transduction, such as SMAD 2/3 signaling). Optionally, a small-molecule antagonist, or combinations of small-molecule antagonists, of the disclosure may further bind to and inhibit GDF8 activity (*e.g*. inhibits the ability of GDF8 to bind to an ActRIIA and/or ActRIIB receptor; inhibits GDF8-mediated activation of the ActRIIA and/or ActRIIB signaling transduction, such as SMAD 2/3 signaling). Optionally, a small-molecule antagonist, or combinations of small-molecule antagonists, of the disclosure does not substantially bind to or inhibit activin A activity (*e.g.* the ability of activin A to bind to an ActRIIA and/or ActRIIB receptor; activin A-mediated activation of the ActRIIA and/or ActRIIB signaling transduction, such as SMAD 2/3 signaling pathway). In some instances, a small-molecule antagonist, or combinations of small-molecule antagonists, of the disclosure that binds to and inhibits the activity of GDF11 and/or GDF8 further binds to and inhibits the activity of one or more of activin A, activin B, activin AB, activin C, activin E, BMP6, BMP7, Nodal, ActRIIA, and ActRIIB.

In some instances, a small-molecule antagonist, or combination of small-molecule antagonists, of the present disclosure directly binds to and inhibits at least GDF8 activity (*e.g.* inhibits the ability GDF8 to bind to an ActRIIA and/or ActRIIB receptor; inhibits GDF8-mediated activation of the ActRIIA and/or ActRIIB signaling transduction, such as SMAD 2/3 signaling). Optionally, a small-molecule antagonist, or combinations of small-molecule antagonists, of the disclosure may further bind to and inhibit GDF11 activity (*e.g.* inhibit the ability of GDF11 to bind to an ActRIIA and/or ActRIIB receptor; inhibit GDF11-mediated activation of the ActRIIA and/or ActRIIB signaling transduction, such as SMAD 2/3 signaling). Optionally, a small-molecule antagonist, or combinations of small-molecule antagonists, of the disclosure does not substantially bind to or inhibit activin A activity (*e.g.* the ability of activin A to bind to an ActRIIA and/or ActRIIB receptor; activin A-mediated activation of the ActRIIA and/or ActRIIB signaling transduction, SMAD 2/3 signaling). In some instances, a small-molecule antagonist, or combinations of small-molecule antagonists, of the disclosure that binds to and inhibits the activity of GDF8 and/or GDF11 further binds to and inhibits the activity of one or more of activin A, activin B, activin AB, activin C, activin E, BMP6, BMP7, Nodal, ActRIIA, and ActRIIB.

In some instances, a small-molecule antagonist, or combination of small-molecule antagonists, of the present disclosure directly binds to and inhibits at least ActRIIA activity (*e.g.* ActRII ligand-mediated activation of ActRIIA signaling transduction, such as SMAD 2/3 signaling). For example, a preferred small-molecule antagonist, or combination of small-molecule antagonists, of the disclosure binds to an ActRIIA receptor and inhibits at least GDF11 from binding to and/or activating the ActRIIA receptor. Optionally, such a small-molecule antagonist, or combination of small-molecule antagonists, may further inhibit GDF8 from binding to and/or activating the ActRIIA receptor. Optionally, a small-molecule antagonist, or combination of small-molecule antagonists, of the disclosure does not substantially inhibit activin A from binding to and/or activating an ActRIIA receptor. In some instances, a small-molecule antagonist, or combination of small-molecule antagonists, of the disclosure that inhibits GDF11 and/or GDF8 from binding to and/or activating the ActRIIA receptor further inhibits one or more of activin A, activin B, activin AB, activin C, activin E, BMP6, BMP7, and Nodal from binding to/and or activating the ActRIIA receptor.

In some instances, a small-molecule antagonist, or combination of small-molecule antagonists, of the present disclosure directly binds to and inhibits at least ActRIIB activity (*e.g.* ActRII ligand-mediated activation of ActRIIB signaling transduction, such as SMAD 2/3 signaling). For example, a preferred small-molecule antagonist, or combination of small-molecule antagonists, of the disclosure binds to an ActRIIB receptor and inhibits at least GDF11 from binding to and/or activating the ActRIIB receptor. Optionally, such a small-molecule antagonist, or combination of small-molecule antagonists, may further inhibit GDF8 from binding to and/or activating the ActRIIB receptor. Optionally, a small-molecule antagonist, or combination of small-molecule antagonists, of the disclosure does not substantially inhibit activin A from binding to and/or activating an ActRIIB receptor. In some instances, a small-molecule antagonist, or combination of small-molecule antagonists, of the disclosure that inhibits GDF11 and/or GDF8 from binding to and/or activating the ActRIIB receptor further inhibits one or more of activin A, activin B, activin AB, activin C, activin E, BMP6, BMP7, and Nodal from binding to/and or activating the ActRIIB receptor.

Binding organic small molecule antagonists of the present disclosure may be identified and chemically synthesized using known methodology (*see, e.g.,* PCT Publication Nos. WO 00/00823 and WO 00/39585). In general, small-molecule antagonists of the disclosure are usually less than about 2000 daltons in size, alternatively less than about 1500, 750, 500, 250 or 200 daltons in size, wherein such organic small molecules that are capable of binding, preferably specifically, to a polypeptide as described herein (*e.g.,* GDF11, GDF8, ActRIIA, and ActRIIB). Such small-molecule antagonists may be identified without undue experimentation using well-known techniques. In this regard, it is noted that techniques for screening organic small-molecule libraries for molecules that are capable of binding to a polypeptide target are well-known in the art (see, *e.g*., international patent publication Nos. WO00/00823 and WO00/39585).

Binding organic small molecules of the present disclosure may be, for example, aldehydes, ketones, oximes, hydrazones, semicarbazones, carbazides, primary amines, secondary amines, tertiary amines, N-substituted hydrazines, hydrazides, alcohols, ethers, thiols, thioethers, disulfides, carboxylic acids, esters, amides, ureas, carbamates, carbonates, ketals, thioketals, acetals, thioacetals, aryl halides, aryl sulfonates, alkyl halides, alkyl sulfonates, aromatic compounds, heterocyclic compounds, anilines, alkenes, alkynes, diols, amino alcohols, oxazolidines, oxazolines, thiazolidines, thiazolines, enamines, sulfonamides, epoxides, aziridines, isocyanates, sulfonyl chlorides, diazo compounds, and acid chlorides.

Any of the small-molecule ActRII antagonists disclosed herein (*e.g.,* a small-molecule antagonist of one or more of GDF11, GDF8, activin A, activin B, activin AB, activin C, activin E, BMP6, BMP7, Nodal, ActRIIA, and/or ActRIIB) can be combined with one or more additional ActRII antagonist agents of the disclosure to achieve the desired effect (*e.g*., increase red blood cell levels and/or hemoglobin in a subject in need thereof, treat or prevent an anemia, treat sickle-cell disease, treat or prevent one or more complications of sickle-cell disease). For example, a small-molecule ActRII antagonist disclosed herein (*e.g*., a small-molecule antagonist of one or more of GDF11, GDF8, activin A, activin B, activin AB, activin C, activin E, BMP6, BMP7, Nodal, ActRIIA, and/or ActRIIB) can be used in combination with i) one or more additional small molecule ActRII antagonists disclosed herein, ii) one or more ActRII polypeptides disclosed herein (*e.g*., ActRIIA and/or ActRIIB polypeptides), iii) one or more GDF traps disclosed herein; iv) one or more ActRII antagonist antibodies disclosed herein (*e.g.,* an anti-GDF11 antibody, an anti-activin B antibody, an anti-activin C antibody, an anti-activin E antibody, an anti-GDF11 antibody, an anti-GDF8 antibody, an anti-BMP6 antibody, an-anti-BMP7 antibody, an anti-ActRIIA antibody, or an anti-ActRIIB antibody); v) one or more polynucleotide ActRII antagonists disclosed herein (*e.g.,* a polynucleotide antagonist of one or more of GDF11, GDF8, activin A, activin B, activin AB, activin C, activin E, BMP6, BMP7, Nodal, ActRIIA, and/or ActRIIB); vi) one or more follistatin polypeptides disclosed herein; and/or vii) one or more FLRG polypeptides disclosed herein.

### E. Antagonist Polynucleotides

In another aspect, the present disclosure relates to a polynucleotide, or combination of polynucleotides, that antagonizes ActRII activity (*e.g*., inhibition of ActRIIA and/or ActRIIB signaling transduction, such as SMAD 2/3 and/or SMAD 1/5/8 signaling). In particular, the disclosure provides methods of using a polynucleotide ActRII antagonist or combination of polynucleotide ActRII antagonists, alone or in combination with one or more erythropoiesis stimulating agents (*e.g.,* EPO) or other supportive therapies [*e.g.,* hematopoietic growth factors (*e.g.,* G-CSF or GM-CSF), transfusion of red blood cells or whole blood, iron chelation therapy, treatment with hydroxyurea, *etc*.], to, *e.g.,* increase red blood cell levels in a subject in need thereof, treat or prevent an anemia in a subject in need thereof, treat sickle-cell disease in a subject in need thereof, treat or prevent one or more complication of sickle-cell disease (*e.g*., anemia, anemia crisis, splenomegaly, pain crisis, chest syndrome, acute chest syndrome, blood transfusion requirement, organ damage, pain medicine (management) requirement, splenic sequestration crises, hyperhemolytic crisis, vaso-occlusion, vaso-occlusion crisis, acute myocardial infarction, sickle-cell chronic lung disease, thromboemboli, hepatic failure, hepatomegaly, hepatic sequestration, iron overload, splenic infarction, acute and/or chronic renal failure, pyelonephritis, aneurysm, ischemic stroke, intraparenchymal hemorrhage, subarachnoid hemorrhage, intraventricular hemorrhage, peripheral retinal ischemia, proliferative sickle retinopathy, vitreous hemorrhage, and/or priapism), and/or reduce blood transfusion burden in a subject in need thereof.

In some instances, a polynucleotide ActRII antagonist, or combination of polynucleotide ActRII antagonists, of the present disclosure can be used to inhibit the activity and/or expression on one or more of GDF11, GDF8, activin A, activin B, activin AB, activin C, activin C, activin E, BMP6, BMP7, Nodal, ActRIIA, and/or ActRIIB. In certain preferred instances, a polynucleotide ActRII antagonist, or combination of polynucleotide ActRII antagonists, of the disclosure is a GDF-ActRII antagonist.

In some instances, a polynucleotide antagonist, or combination of polynucleotide antagonists, of the disclosure inhibits the activity and/or expression (*e.g*., transcription, translation, secretion, or combinations thereof) of at least GDF11. Optionally, such a polynucleotide antagonist, or combination of polynucleotide antagonists, may further inhibit the activity and/or expression of GDF8. Optionally, a polynucleotide antagonist, or combination of polynucleotide antagonists, of the disclosure does not substantially inhibit the activity and/or expression of activin A. In some instances, a polynucleotide antagonist, or combination of polynucleotide antagonists, of the disclosure that inhibits the activity and/or expression of GDF11 and/or GDF8 may further inhibit the activity and or expression of one or more of activin A, activin B, activin AB, activin C, activin E, BMP6, BMP7, Nodal, ActRIIA, and/or ActRIIB.

In some instances, a polynucleotide antagonist, or combination of polynucleotide antagonists, of the disclosure inhibits the activity and/or expression (*e.g.*, transcription, translation, secretion, or combinations thereof) of at least GDF8. Optionally, such polynucleotide antagonist, or combination of polynucleotide antagonists, may further inhibit the activity and/or expression of GDF11. Optionally, a polynucleotide antagonist, or combination of polynucleotide antagonists, of the disclosure does not substantially inhibit the activity and/or expression of activin A. In some instances, a polynucleotide antagonist, or combination of polynucleotide antagonists, of the disclosure that inhibits the activity and/or expression of GDF8 and/or GDF11 may further inhibit the activity and or expression of one or more of activin A, activin B, activin AB, activin C, activin E, BMP6, BMP7, Nodal, ActRIIA, and/or ActRIIB.

In some instances, a polynucleotide antagonist, or combination of polynucleotide antagonists, of the disclosure inhibits the activity and/or expression (*e.g*., transcription, translation, secretion, or combinations thereof) of at least ActRIIA. Optionally, a polynucleotide antagonist, or combination of polynucleotide antagonists, of the disclosure does not substantially inhibit the activity and/or expression of activin A. In some instances, a polynucleotide antagonist, or combination of polynucleotide antagonists, of the disclosure that inhibits the activity and/or expression of ActRIIA may further inhibit the activity and or expression of one or more of activin A, activin B, activin AB, activin C, activin E, BMP6, BMP7, Nodal, and/or ActRIIB.

In some instances, a polynucleotide antagonist, or combination of polynucleotide antagonists, of the disclosure inhibits the activity and/or expression (*e.g*., transcription, translation, secretion, or combinations thereof) of at least ActRIIB. Optionally, a polynucleotide antagonist, or combination of polynucleotide antagonists, of the disclosure does not substantially inhibit the activity and/or expression of activin A. In some instances, a polynucleotide antagonist, or combination of polynucleotide antagonists, of the disclosure that inhibits the activity and/or expression of ActRIIB may further inhibit the activity and or expression of one or more of activin A, activin B, activin AB, activin C, activin E, BMP6, BMP7, Nodal, and/or ActRIIA.

The polynucleotide antagonists of the present disclosure may be an antisense nucleic acid, an RNAi molecule [*e.g*., small interfering RNA (siRNA), small-hairpin RNA (shRNA), microRNA (miRNA)], an aptamer and/or a ribozyme. The nucleic acid and amino acid sequences of human GDF11, GDF8, activin A, activin B, activin C, activin E, BMP6, BMP7, Nodal, ActRIIA, and ActRIIB are known in the art and thus polynucleotide antagonists for use in accordance with methods of the present disclosure may be routinely made by the skilled artisan based on the knowledge in the art and teachings provided herein.

For example, antisense technology can be used to control gene expression through antisense DNA or RNA, or through triple-helix formation. Antisense techniques are discussed, for example, in Okano (1991) J. Neurochem. 56:560; Oligodeoxynucleotides as Antisense Inhibitors of Gene Expression, CRC Press, Boca Raton, Fla. (1988). Triple helix formation is discussed in, for instance, Cooney et al. (1988) Science 241:456; and Dervan et al., (1991)Science 251:1300. The methods are based on binding of a polynucleotide to a complementary DNA or RNA. In some instances, the antisense nucleic acids comprise a single-stranded RNA or DNA sequence that is complementary to at least a portion of an RNA transcript of a gene disclosed herein (*e.g.,* GDF11, GDF8, activin A, activin B, activin C, activin C, activin E, BMP6, BMP7, Nodal, ActRIIA, and ActRIIB). However, absolute complementarity, although preferred, is not required.

A sequence "complementary to at least a portion of an RNA," referred to herein, means a sequence having sufficient complementarity to be able to hybridize with the RNA, forming a stable duplex; in the case of double-stranded antisense nucleic acids of a gene disclosed herein (*e.g.,* GDF11, GDF8, activin A, activin B, activin C, activin E, BMP6, BMP7, Nodal, ActRIIA, and ActRIIB), a single strand of the duplex DNA may thus be tested, or triplex formation may be assayed. The ability to hybridize will depend on both the degree of complementarity and the length of the antisense nucleic acid. Generally, the larger the hybridizing nucleic acid, the more base mismatches with an RNA it may contain and still form a stable duplex (or triplex as the case may be). One skilled in the art can ascertain a tolerable degree of mismatch by use of standard procedures to determine the melting point of the hybridized complex.

Polynucleotides that are complementary to the 5' end of the message, for example, the 5'-untranslated sequence up to and including the AUG initiation codon, should work most efficiently at inhibiting translation. However, sequences complementary to the 3'-untranslated sequences of mRNAs have been shown to be effective at inhibiting translation of mRNAs as well [see, *e.g*., Wagner, R., (1994) Nature 372:333-335]. Thus, oligonucleotides complementary to either the 5'- or 3'-untranslated, noncoding regions of a gene of the disclosure (*e.g.*, GDF11, GDF8, activin A, activin B, activin C, activin E, BMP6, BMP7, Nodal, ActRIIA, and ActRIIB), could be used in an antisense approach to inhibit translation of an endogenous mRNA. Polynucleotides complementary to the 5'-untranslated region of the mRNA should include the complement of the AUG start codon. Antisense polynucleotides complementary to mRNA coding regions are less efficient inhibitors of translation but could be used in accordance with the methods of the present disclosure. Whether designed to hybridize to the 5'-untranslated, 3'-untranslated, or coding regions of an mRNA of the disclosure (*e.g.*, an GDF11, GDF8, activin A, activin B, activin C, activin E, BMP6, BMP7, Nodal, ActRIIA, and ActRIIB mRNA), antisense nucleic acids should be at least six nucleotides in length, and are preferably oligonucleotides ranging from 6 to about 50 nucleotides in length. In specific aspects, the oligonucleotide is at least 10 nucleotides, at least 17 nucleotides, at least 25 nucleotides, or at least 50 nucleotides.

In one instance, the antisense nucleic acid of the present disclosure (*e.g.,* a GDF11, GDF8, activin A, activin B, activin C, activin E, BMP6, BMP7, Nodal, ActRIIA, or ActRIIB antisense nucleic acid) is produced intracellularly by transcription from an exogenous sequence. For example, a vector or a portion thereof, is transcribed, producing an antisense nucleic acid (RNA) of a gene of the disclosure. Such a vector would contain a sequence encoding the desired antisense nucleic acid. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression in vertebrate cells. Expression of the sequence encoding desired genes of the instant disclosure, or fragments thereof, can be by any promoter known in the art to act in vertebrate, preferably human cells. Such promoters can be inducible or constitutive. Such promoters include, but are not limited to, the SV40 early promoter region [see, *e.g*., Benoist and Chambon (1981) Nature 29:304-310], the promoter contained in the 3' long terminal repeat of Rous sarcoma virus [see, *e.g.,* Yamamoto et al. (1980) Cell 22:787-797], the herpes thymidine promoter [see, *e.g.,* Wagner et al. (1981) Proc. Natl. Acad. Sci. U.S.A. 78:1441-1445], and the regulatory sequences of the metallothionein gene [see, *e.g.,* Brinster, et al. (1982) Nature 296:39-42].

In some instances, the polynucleotide antagonists are interfering RNA or RNAi molecules that target the expression of one or more of: GDF11, GDF8, activin A, activin B, activin C, activin E, BMP6, BMP7, Nodal, ActRIIA, and ActRIIB. RNAi refers to the expression of an RNA which interferes with the expression of the targeted mRNA. Specifically, RNAi silences a targeted gene via interacting with the specific mRNA through a siRNA (small interfering RNA). The ds RNA complex is then targeted for degradation by the cell. An siRNA molecule is a double-stranded RNA duplex of 10 to 50 nucleotides in length, which interferes with the expression of a target gene which is sufficiently complementary (*e.g.* at least 80% identity to the gene). In some instances, the siRNA molecule comprises a nucleotide sequence that is at least 85, 90, 95, 96, 97, 98, 99, or 100% identical to the nucleotide sequence of the target gene.

Additional RNAi molecules include short-hairpin RNA (shRNA); also short-interfering hairpin and microRNA (miRNA). The shRNA molecule contains sense and antisense sequences from a target gene connected by a loop. The shRNA is transported from the nucleus into the cytoplasm, and it is degraded along with the mRNA. Pol III or U6 promoters can be used to express RNAs for RNAi. Paddison et al. [Genes & Dev. (2002) 16:948-958, 2002] have used small RNA molecules folded into hairpins as a means to effect RNAi. Accordingly, such short hairpin RNA (shRNA) molecules are also advantageously used in the methods described herein. The length of the stem and loop of functional shRNAs varies; stem lengths can range anywhere from about 25 to about 30 nt, and loop size can range between 4 to about 25 nt without affecting silencing activity. While not wishing to be bound by any particular theory, it is believed that these shRNAs resemble the double-stranded RNA (dsRNA) products of the DICER RNase and, in any event, have the same capacity for inhibiting expression of a specific gene. The shRNA can be expressed from a lentiviral vector. An miRNA is a single-stranded RNA of about 10 to 70 nucleotides in length that are initially transcribed as pre-miRNA characterized by a "stem-loop" structure and which are subsequently processed into mature miRNA after further processing through the RISC.

Molecules that mediate RNAi, including without limitation siRNA, can be produced *in vitro* by chemical synthesis (Hohjoh, FEBS Lett 521:195-199, 2002), hydrolysis of dsRNA (Yang et al., Proc Natl Acad Sci USA 99:9942-9947, 2002), by *in vitro* transcription with T7 RNA polymerase (Donzeet et al., Nucleic Acids Res 30:e46, 2002; Yu et al., Proc Natl Acad Sci USA 99:6047-6052, 2002), and by hydrolysis of double-stranded RNA using a nuclease such as E. coli RNase III (Yang et al., Proc Natl Acad Sci USA 99:9942-9947, 2002).

According to another aspect, the disclosure provides polynucleotide antagonists including but not limited to, a decoy DNA, a double-stranded DNA, a single-stranded DNA, a complexed DNA, an encapsulated DNA, a viral DNA, a plasmid DNA, a naked RNA, an encapsulated RNA, a viral RNA, a double-stranded RNA, a molecule capable of generating RNA interference, or combinations thereof.

In some instances, the polynucleotide antagonists of the disclosure are aptamers. Aptamers are nucleic acid molecules, including double-stranded DNA and single-stranded RNA molecules, which bind to and form tertiary structures that specifically bind to a target molecule, such as a GDF11, GDF8, activin A, activin B, activin C, activin E, BMP6, BMP7, Nodal, ActRIIA, and ActRIIB polypeptide. The generation and therapeutic use of aptamers are well established in the art. See, *e.g.,* U.S. Pat. No. 5,475,096. Additional information on aptamers can be found in U.S. Patent Application Publication No. 20060148748. Nucleic acid aptamers are selected using methods known in the art, for example via the Systematic Evolution of Ligands by Exponential Enrichment (SELEX) process. SELEX is a method for the in vitro evolution of nucleic acid molecules with highly specific binding to target molecules as described in, *e.g.,* U.S. Pat. Nos. 5,475,096, 5,580,737, 5,567,588, 5,707,796, 5,763,177, 6,011,577, and 6,699,843. Another screening method to identify aptamers is described in U.S. Pat. No. 5,270,163. The SELEX process is based on the capacity of nucleic acids for forming a variety of two- and three-dimensional structures, as well as the chemical versatility available within the nucleotide monomers to act as ligands (form specific binding pairs) with virtually any chemical compound, whether monomeric or polymeric, including other nucleic acid molecules and polypeptides. Molecules of any size or composition can serve as targets. The SELEX method involves selection from a mixture of candidate oligonucleotides and step-wise iterations of binding, partitioning and amplification, using the same general selection scheme, to achieve desired binding affinity and selectivity. Starting from a mixture of nucleic acids, which can comprise a segment of randomized sequence, the SELEX method includes steps of contacting the mixture with the target under conditions favorable for binding; partitioning unbound nucleic acids from those nucleic acids which have bound specifically to target molecules; dissociating the nucleic acid-target complexes; amplifying the nucleic acids dissociated from the nucleic acid-target complexes to yield a ligand enriched mixture of nucleic acids. The steps of binding, partitioning, dissociating and amplifying are repeated through as many cycles as desired to yield highly specific high affinity nucleic acid ligands to the target molecule.

Typically, such binding molecules are separately administered to the animal [see, *e.g.,* O'Connor (1991) J. Neurochem. 56:560], but such binding molecules can also be expressed in vivo from polynucleotides taken up by a host cell and expressed *in vivo* [see, *e.g*., Oligodeoxynucleotides as Antisense Inhibitors of Gene Expression, CRC Press, Boca Raton, Fla. (1988)].

Any of the polynucleotide ActRII antagonists disclosed herein (*e.g.,* a polynucleotide antagonist of one or more of GDF11, GDF8, activin A, activin B, activin AB, activin C, activin E, BMP6, BMP7, Nodal, ActRIIA, and/or ActRIIB) can be combined with one or more additional ActRII antagonist agents of the disclosure to achieve the desired effect (*e.g*., increase red blood cell levels and/or hemoglobin in a subject in need thereof, treat or prevent an anemia, treat sickle-cell disease, treat or prevent one or more complications of sickle-cell disease). For example, an polynucleotide ActRII antagonist disclosed herein (*e.g.,* a polynucleotide antagonist of one or more of GDF11, GDF8, activin A, activin B, activin AB, activin C, activin E, BMP6, BMP7, Nodal, ActRIIA, and/or ActRIIB) can be used in combination with i) one or more additional polynucleotide ActRII antagonists disclosed herein, ii) one or more ActRII polypeptides disclosed herein (*e.g*., ActRIIA and/or ActRIIB polypeptides), iii) one or more GDF traps disclosed herein; iv) one or more ActRII antagonist antibodies disclosed herein (*e.g.,* an anti-GDF11 antibody, an anti-activin B antibody, an anti-activin C antibody, an anti-activin E antibody, an anti-GDF11 antibody, an anti-GDF8 antibody, an anti-BMP6 antibody, an-anti-BMP7 antibody, an anti-ActRIIA antibody, or an anti-ActRIIB antibody); v) one or more small molecule ActRII antagonists disclosed herein (*e.g.,* a small molecule antagonist of one or more of GDF11, GDF8, activin A, activin B, activin AB, activin C, activin E, BMP6, BMP7, Nodal, ActRIIA, and/or ActRIIB); vi) one or more follistatin polypeptides disclosed herein; and/or vii) one or more FLRG polypeptides disclosed herein.

### F. Other Antagonists

In other aspects, an agent for use in accordance with the methods disclosed herein is a follistatin polypeptide, which may be used alone or in combination with one or more erythropoiesis stimulating agents (e.g., EPO) or other supportive therapies [e.g., hematopoietic growth factors (e.g., G-CSF or GM-CSF), transfusion of red blood cells or whole blood, iron chelation therapy, treatment with hydroxyurea, etc.], to, increase red blood cell levels in a subject in need thereof, treat or prevent an anemia in a subject in need thereof, and methods of treating a sickle-cell disease in a subject in need thereof, and/or methods of treating or preventing one or more complications of sickle-cell disease), and/or reduce blood transfusion burden. The term "follistatin polypeptide" includes polypeptides comprising any naturally occurring polypeptide of follistatin as well as any variants thereof (including mutants, fragments, fusions, and peptidomimetic forms) that retain a useful activity, and further includes any functional monomer or multimer of follistatin. In certain preferred instances, follistatin polypeptides of the disclosure bind to and/or inhibit activin activity, particularly activin A (*e.g*., activin-mediated activation of ActRIIA and/or ActRIIB SMAD 2/3 signaling). Variants of follistatin polypeptides that retain activin binding properties can be identified based on previous studies involving follistatin and activin interactions. For example, WO2008/030367 discloses specific follistatin domains ("FSDs") that are shown to be important for activin binding. As shown below in SEQ ID NOs: 18-20, the follistatin N-terminal domain ("FSND" SEQ ID NO:18), FSD2 (SEQ ID NO: 20), and to a lesser extent FSD1 (SEQ ID NO: 19) represent exemplary domains within follistatin that are important for activin binding. In addition, methods for making and testing libraries of polypeptides are described above in the context of ActRII polypeptides, and such methods also pertain to making and testing variants of follistatin. Follistatin polypeptides include polypeptides derived from the sequence of any known follistatin having a sequence at least about 80% identical to the sequence of a follistatin polypeptide, and optionally at least 85%, 90%, 95%, 96%, 97%, 98%, 99% or greater identity. Examples of follistatin polypeptides include the mature follistatin polypeptide or shorter isoforms or other variants of the human follistatin precursor polypeptide (SEQ ID NO: 16) as described, for example, in WO2005/025601.

The human follistatin precursor polypeptide isoform FST344 is as follows:

1 mvrarhqpgg lcllllllcq fmedrsaqag ncwlrqakng rcqvlyktel
51skeeccstgr lstswteedv ndntlfkwmi fnggapncip cketcenvdc
101gpgkkcrmnk knkprcvcap dcsnitwkgp vcgldgktyr necallkarc
151keqpelevqy qgrckktcrd vfcpgsstcv vdqtnnaycv tcnricpepa
201sseqylcgnd gvtyssachl rkatcllgrs iglayegkci kakscediqc
251tggkkclwdf kvgrgrcslc delcpdsksd epvcasdnat yasecamkea
301 acssgvllev khsgscnsis edteeeeede dqdysfpiss ilew
(SEQ ID NO: 16; NCBI Reference No. NP_037541.1)

The signal peptide is underlined; also underlined above are the last 27 residues which represent the C-terminal extension distinguishing this follistatin isoform from the shorter follistatin isoform FST317 shown below.

The human follistatin precursor polypeptide isoform FST317 is as follows:

1MVRARHQPGG LCLLLLLLCQ FMEDRSAQAG NCWLRQAKNG RCQVLYKTEL

51SKEECCSTGR LSTSWTEEDV NDNTLFKWMI FNGGAPNCIP CKETCENVDC

101GPGKKCRMNK KNKPRCVCAP DCSNITWKGP VCGLDGKTYR NECALLKARC

151KEQPELEVQY QGRCKKTCRD VFCPGSSTCV VDQTNNAYCV TCNRICPEPA

201SSEQYLCGND GVTYSSACHL RKATCLLGRS IGLAYEGKCI KAKSCEDIQC

251TGGKKCLWDF KVGRGRCSLC DELCPDSKSD EPVCASDNAT YASECAMKEA

301ACSSGVLLEV KHSGSCN

(SEQ ID NO: 17; NCBI Reference No. NP_006341.1)

The signal peptide is underlined.

The follistatin N-terminal domain (FSND) sequence is as follows:

GNCWLRQAKNGRCQVLYKTELSKEECCSTGRLSTSWTEEDVNDNTLFKWM
IFNGGAPNCIPCK (SEQ ID NO: 18; FSND)

The FSD1 and FSD2 sequences are as follows:
ETCENVDCGPGKKCRMNKKNKPRCV (SEQ ID NO: 19; FSD1)
KTCRDVFCPGSSTCVVDQTNNAYCVT (SEQ ID NO: 20; FSD2)

In other aspects, an agent for use in accordance with the methods disclosed herein (*e.g*., methods for increasing red blood cell levels in a subject in need thereof, methods of treating or preventing an anemia in an subject in need thereof, and methods of treating or preventing a disorder/condition of sickle-cell disease) is a follistatin-like related gene (FLRG), also known as follistatin-related protein 3 (FSTL3). The term "FLRG polypeptide" includes polypeptides comprising any naturally occurring polypeptide of FLRG as well as any variants thereof (including mutants, fragments, fusions, and peptidomimetic forms) that retain a useful activity. In certain preferred instances, FLRG polypeptides of the disclosure bind to and/or inhibit activin activity, particularly activin A (*e.g.*, activin-mediated activation of ActRIIA and/or ActRIIB SMAD 2/3 signaling). Variants of FLRG polypeptides that retain activin binding properties can be identified using routine methods to assay FLRG and activin interactions (see, *e.g.,* US 6,537,966). In addition, methods for making and testing libraries of polypeptides are described above in the context of ActRII polypeptides and such methods also pertain to making and testing variants of FLRG. FLRG polypeptides include polypeptides derived from the sequence of any known FLRG having a sequence at least about 80% identical to the sequence of an FLRG polypeptide, and optionally at least 85%, 90%, 95%, 97%, 99% or greater identity.

The human FLRG precursor (follistatin-related protein 3 precursor) polypeptide is as follows:

1 MRPGAPGPLW PLPWGALAWA VGFVSSMGSG NPAPGGVCWL QQGQEATCSL

51VLQTDVTRAE CCASGNIDTA WSNLTHPGNK INLLGFLGLV HCLPCKDSCD

101GVECGPGKAC RMLGGRPRCE CAPDCSGLPA RLQVCGSDGA TYRDECELRA

151ARCRGHPDLS VMYRGRCRKS CEHVVCPRPQ SCVVDQTGSA HCVVCRAAPC

201PVPSSPGQEL CGNNNVTYIS SCHMRQATCF LGRSIGVRHA GSCAGTPEEP

251PGGESAEEEE NFV

(SEQ ID NO:21; NCBI Reference No. NP_005851.1)

The signal peptide is underlined.

In certain instances, functional variants or modified forms of the follistatin polypeptides and FLRG polypeptides include fusion proteins having at least a portion of the follistatin polypeptide or FLRG polypeptide and one or more fusion domains, such as, for example, domains that facilitate isolation, detection, stabilization or multimerization of the polypeptide. Suitable fusion domains are discussed in detail above with reference to the ActRII polypeptides. In some instance, an antagonist agent of the disclosure is a fusion protein comprising an activin-binding portion of a follistatin polypeptide fused to an Fc domain. In another instance, an antagonist agent of the disclosure is a fusion protein comprising an activin binding portion of an FLRG polypeptide fused to an Fc domain.

Any of the follistatin polypeptides disclosed herein may be combined with one or more additional ActRII antagonist agents of the disclosure to achieve the desired effect (*e.g.,* increase red blood cell levels and/or hemoglobin in a subject in need thereof, treat or prevent an anemia, treat sickle-cell disease, treat or prevent one or more complications of sickle-cell disease). For example, a follistatin polypeptide disclosed herein can be used in combination with i) one or more additional follistatin polypeptides disclosed herein, ii) one or more ActRII polypeptides disclosed herein (*e.g.*, ActRIIA and/or ActRIIB polypeptides), iii) one or more GDF traps disclosed herein; iv) one or more ActRII antagonist antibodies disclosed herein (*e.g.,* an anti-GDF11 antibody, an anti-activin B antibody, an anti-activin C antibody, an anti-activin E antibody, an anti-GDF11 antibody, an anti-GDF8 antibody, an anti-BMP6 antibody, an-anti-BMP7 antibody, an anti-ActRIIA antibody, or an anti-ActRIIB antibody); v) one or more small molecule ActRII antagonists disclosed herein (*e.g.,* a small molecule antagonist of one or more of GDF11, GDF8, activin A, activin B, activin AB, activin C, activin E, BMP6, BMP7, Nodal, ActRIIA, and/or ActRIIB); vi) one or more polynucleotide ActRII antagonists disclosed herein (*e.g.,* a polynucleotide antagonist of one or more of GDF11, GDF8, activin A, activin B, activin AB, activin C, activin E, BMP6, BMP7, Nodal, ActRIIA, and/or ActRIIB); and/or one or more FLRG polypeptides disclosed herein.

Similarly, any of the FLRG polypeptides disclosed herein may be combined with one or more additional ActRII antagonist agents of the disclosure to achieve the desired effect (*e.g.*, increase red blood cell levels and/or hemoglobin in a subject in need thereof, treat or prevent an anemia, treat sickle-cell disease, treat or prevent one or more complications of sickle-cell disease). For example, a FLRG polypeptide disclosed herein can be used in combination with i) one or more additional FLRG polypeptides disclosed herein, ii) one or more ActRII polypeptides disclosed herein (*e.g.*, ActRIIA and/or ActRIIB polypeptides), iii) one or more GDF traps disclosed herein; iv) one or more ActRII antagonist antibodies disclosed herein (*e.g.,* an anti-GDF11 antibody, an anti-activin B antibody, an anti-activin C antibody, an anti-activin E antibody, an anti-GDF11 antibody, an anti-GDF8 antibody, an anti-BMP6 antibody, an-anti-BMP7 antibody, an anti-ActRIIA antibody, or an anti-ActRIIB antibody); v) one or more small molecule ActRII antagonists disclosed herein (*e.g.,* a small molecule antagonist of one or more of GDF11, GDF8, activin A, activin B, activin AB, activin C, activin E, BMP6, BMP7, Nodal, ActRIIA, and/or ActRIIB); vi) one or more polynucleotide ActRII antagonists disclosed herein (*e.g.,* a polynucleotide antagonist of one or more of GDF11, GDF8, activin A, activin B, activin AB, activin C, activin E, BMP6, BMP7, Nodal, ActRIIA, and/or ActRIIB); and/or one or more follistatin polypeptides disclosed herein.

### 3. Screening Assays

In certain aspects, the present disclosure relates to the use of the subject ActRII polypeptides (*e.g*., ActRIIA and ActRIIB polypeptides) and GDF trap polypeptides to identify compounds (agents) which are agonist or antagonists of ActRIIB polypeptides. Compounds identified through this screening can be tested to assess their ability to modulate red blood cell, hemoglobin, and/or reticulocyte levels *in vivo* or *in vitro.* These compounds can be tested, for example, in animal models.

There are numerous approaches to screening for therapeutic agents for increasing red blood cell or hemoglobin levels by targeting ActRII signaling (*e.g*., ActRIIA and/or ActRIIB SMAD 2/3 and/or SMAD 1/5/8 signaling). In certain instances, high-throughput screening of compounds can be carried out to identify agents that perturb ActRII-mediated effects on a selected cell line. In certain instances, the assay is carried out to screen and identify compounds that specifically inhibit or reduce binding of an ActRII polypeptide or GDF trap polypeptide to its binding partner, such as an ActRII ligand (e.g., activin A, activin B, activin AB, activin C, Nodal, GDF8, GDF11 or BMP7). Alternatively, the assay can be used to identify compounds that enhance binding of an ActRII polypeptide or GDF trap polypeptide to its binding partner such as an ActRII ligand. In a further instance, the compounds can be identified by their ability to interact with an ActRII polypeptide or GDF trap polypeptide.

A variety of assay formats will suffice and, in light of the present disclosure, those not expressly described herein will nevertheless be comprehended by one of ordinary skill in the art. As described herein, the test compounds (agents) of the disclosure may be created by any combinatorial chemical method. Alternatively, the subject compounds may be naturally occurring biomolecules synthesized *in vivo* or *in vitro.* Compounds (agents) to be tested for their ability to act as modulators of tissue growth can be produced, for example, by bacteria, yeast, plants or other organisms (*e.g.,* natural products), produced chemically (*e.g.,* small molecules, including peptidomimetics), or produced recombinantly. Test compounds contemplated by the present disclosure include non-peptidyl organic molecules, peptides, polypeptides, peptidomimetics, sugars, hormones, and nucleic acid molecules. In certain instances, the test agent is a small organic molecule having a molecular weight of less than about 2,000 Daltons.

The test compounds of the disclosure can be provided as single, discrete entities, or provided in libraries of greater complexity, such as made by combinatorial chemistry. These libraries can comprise, for example, alcohols, alkyl halides, amines, amides, esters, aldehydes, ethers and other classes of organic compounds. Presentation of test compounds to the test system can be in either an isolated form or as mixtures of compounds, especially in initial screening steps. Optionally, the compounds may be optionally derivatized with other compounds and have derivatizing groups that facilitate isolation of the compounds. Non-limiting examples of derivatizing groups include biotin, fluorescein, digoxygenin, green fluorescent protein, isotopes, polyhistidine, magnetic beads, glutathione S-transferase (GST), photoactivatible crosslinkers or any combinations thereof.

In many drug-screening programs which test libraries of compounds and natural extracts, high-throughput assays are desirable in order to maximize the number of compounds surveyed in a given period of time. Assays which are performed in cell-free systems, such as may be derived with purified or semi-purified proteins, are often preferred as "primary" screens in that they can be generated to permit rapid development and relatively easy detection of an alteration in a molecular target which is mediated by a test compound. Moreover, the effects of cellular toxicity or bioavailability of the test compound can be generally ignored in the *in vitro* system, the assay instead being focused primarily on the effect of the drug on the molecular target as may be manifest in an alteration of binding affinity between an ActRII polypeptide or a GDF trap polypeptide and its binding partner (*e.g.,* an ActRII ligand).

Merely to illustrate, in an exemplary screening assay of the present disclosure, the compound of interest is contacted with an isolated and purified ActRIIB polypeptide which is ordinarily capable of binding to an ActRIIB ligand, as appropriate for the intention of the assay. To the mixture of the compound and ActRIIB polypeptide is then added to a composition containing an ActRIIB ligand (*e.g.,* GDF11). Detection and quantification of ActRIIB/ActRIIB ligand complexes provides a means for determining the compound's efficacy at inhibiting (or potentiating) complex formation between the ActRIIB polypeptide and its binding protein. The efficacy of the compound can be assessed by generating dose-response curves from data obtained using various concentrations of the test compound. Moreover, a control assay can also be performed to provide a baseline for comparison. For example, in a control assay, isolated and purified ActRIIB ligand is added to a composition containing the ActRIIB polypeptide, and the formation of ActRIIB/ActRIIB ligand complex is quantitated in the absence of the test compound. It will be understood that, in general, the order in which the reactants may be admixed can be varied, and can be admixed simultaneously. Moreover, in place of purified proteins, cellular extracts and lysates may be used to render a suitable cell-free assay system.

Complex formation between an ActRII polypeptide or GDF trap polypeptide and its binding protein may be detected by a variety of techniques. For instance, modulation of the formation of complexes can be quantitated using, for example, detectably labeled proteins such as radiolabeled (*e.g.,* ³²P, ³⁵S, ¹⁴C or ³H), fluorescently labeled (*e.g.,* FITC), or enzymatically labeled ActRII polypeptide or GDF trap polypeptide and/or its binding protein, by immunoassay, or by chromatographic detection.

In certain instances, the present disclosure contemplates the use of fluorescence polarization assays and fluorescence resonance energy transfer (FRET) assays in measuring, either directly or indirectly, the degree of interaction between an ActRII polypeptide of GDF trap polypeptide and its binding protein. Further, other modes of detection, such as those based on optical waveguides (see, *e.g.,* PCT Publication WO 96/26432 and U.S. Pat. No. 5,677,196), surface plasmon resonance (SPR), surface charge sensors, and surface force sensors, are compatible with many instances of the disclosure.

Moreover, the present disclosure contemplates the use of an interaction trap assay, also known as the "two-hybrid assay," for identifying agents that disrupt or potentiate interaction between an ActRII polypeptide or GDF trap polypeptide and its binding partner. See, *e.g.,* U.S. Pat. No. 5,283,317; Zervos et al. (1993) Cell 72:223-232; Madura et al. (1993) J Biol Chem 268:12046-12054; Bartel et al. (1993) Biotechniques 14:920-924; and Iwabuchi et al. (1993) Oncogene 8:1693-1696). In a specific instance, the present disclosure contemplates the use of reverse two-hybrid systems to identify compounds (*e.g*., small molecules or peptides) that dissociate interactions between an ActRII polypeptide or GDF trap and its binding protein [see, *e.g.,* Vidal and Legrain, (1999) Nucleic Acids Res 27:919-29; Vidal and Legrain, (1999) Trends Biotechnol 17:374-81; and U.S. Pat. Nos. 5,525,490; 5,955,280; and 5,965,368].

In certain instances, the subject compounds are identified by their ability to interact with an ActRII polypeptide or GDF trap polypeptide. The interaction between the compound and the ActRII polypeptide or GDF trap polypeptide may be covalent or non-covalent. For example, such interaction can be identified at the protein level using *in vitro* biochemical methods, including photo-crosslinking, radiolabeled ligand binding, and affinity chromatography [see, *e.g.,* Jakoby WB et al. (1974) Methods in Enzymology 46:1]. In certain cases, the compounds may be screened in a mechanism-based assay, such as an assay to detect compounds which bind to an ActRII polypeptide of GDF trap polypeptide. This may include a solid-phase or fluid-phase binding event. Alternatively, the gene encoding an ActRII polypeptide or GDF trap polypeptide can be transfected with a reporter system (*e.g.*, β-galactosidase, luciferase, or green fluorescent protein) into a cell and screened against the library preferably by high-throughput screening or with individual members of the library. Other mechanism-based binding assays may be used; for example, binding assays which detect changes in free energy. Binding assays can be performed with the target fixed to a well, bead or chip or captured by an immobilized antibody or resolved by capillary electrophoresis. The bound compounds may be detected usually using colorimetric endpoints or fluorescence or surface plasmon resonance.

### 4. Exemplary Therapeutic Uses

In certain aspects, an ActRII antagonist agent, or combination of ActRII antagonist agents, of the present disclosure can be used to increase red blood cell levels in a subject in need thereof, particularly mammals such as rodents, primates, and humans. In some instances, an ActRII antagonist agent, or combination of ActRII antagonist agents, of the present disclosure can be used to treat or prevent an anemia in a subject in need thereof, particularly mammals such as rodents, primates, and humans. In some instances, an ActRII antagonist agent, or combination of ActRII antagonist agents, of the present disclosure can be used treat sickle-cell disease, particularly mammals such as rodents, primates, and humans. In some instances, an ActRII antagonist agent, or combination of ActRII antagonist agents, of the present disclosure can be used treat or prevent anemia in a sickle-cell disease subject in need thereof, particularly mammals such as rodents, primates, and humans. In some instances, an ActRII antagonist agent, or combination of ActRII antagonist agents, of the present disclosure can be used treat or prevent one or more complications of sickle-cell disease (*e.g*., anemia, anemia crisis, splenomegaly, pain crisis, chest syndrome, acute chest syndrome, blood transfusion requirement, organ damage, pain medicine (management) requirement, splenic sequestration crises, hyperhemolytic crisis, vaso-occlusion, vaso-occlusion crisis, acute myocardial infarction, sickle-cell chronic lung disease, thromboemboli, hepatic failure, hepatomegaly, hepatic sequestration, iron overload, splenic infarction, acute and/or chronic renal failure, pyelonephritis, aneurysm, ischemic stroke, intraparenchymal hemorrhage, subarachnoid hemorrhage, intraventricular hemorrhage, peripheral retinal ischemia, proliferative sickle retinopathy, vitreous hemorrhage, and/or priapism) in a subject in need thereof, particularly mammals such as rodents, primates, and humans. In some instances, an ActRII antagonist agent, or combination of ActRII antagonist agents, of the present disclosure can be used treat or prevent vaso-occlusion crisis in a sickle-cell disease subject in need thereof, particularly mammals such as rodents, primates, and humans. In some instances, an ActRII antagonist agent, or combination of ActRII antagonist agents, of the present disclosure can be used treat or prevent pain crisis in a sickle-cell disease subject in need thereof, particularly mammals such as rodents, primates, and humans. In some instances, an ActRII antagonist agent, or combination of ActRII antagonist agents, of the present disclosure can be used treat or prevent an anemia crisis in a sickle-cell disease subject in need thereof, particularly mammals such as rodents, primates, and humans.

As used herein, a therapeutic that "prevents" a disorder or condition refers to a compound that, in a statistical sample, reduces the occurrence of the disorder or condition in the treated sample relative to an untreated control sample, or delays the onset or reduces the severity of one or more symptoms of the disorder or condition relative to the untreated control sample.

The term "treating" as used herein includes amelioration or elimination of the condition once it has been established. In either case, prevention or treatment may be discerned in the diagnosis provided by a physician or other health care provider and the intended result of administration of the therapeutic agent.

In general, treatment or prevention of a disease or condition as described in the present disclosure is achieved by administering one or more of the ActRII antagonists (*e.g*., an ActRIIA and/or ActRIIB antagonist) of the present disclosure in an effective amount. An effective amount of an agent refers to an amount effective, at dosages and for periods of time necessary, to achieve the desired therapeutic or prophylactic result. A "therapeutically effective amount" of an agent of the present disclosure may vary according to factors such as the disease state, age, sex, and weight of the individual, and the ability of the agent to elicit a desired response in the individual. A "prophylactically effective amount" refers to an amount effective, at dosages and for periods of time necessary, to achieve the desired prophylactic result.

Numerous genes contribute to classical sickle-cell disease (SCD; drepanocytosis). Primarily, sickle-cell disease is an inherited disorder caused by a mutation in the *β*-globin gene (a mutation of a glutamate to a valine at codon 6). See, *e.g.,* Kassim et al. (2013) Annu Rev Med, 64: 451-466. Sickle-cell anemia refers to the most common form of sickle-cell disease, with a homozygous mutation in the *β^{S}* allele (HbSS), affecting 60 to 70% of people with sickle-cell disease.

Because of the mutation in the *β*-globin gene, abnormal hemoglobin molecules are produced with a hydrophobic motif that is exposed when it is in a deoxygenated state [see, *e.g.,* Eaton et al. (1990) Adv Protein Chem, 40: 63-279; Steinberg, MH (1999) N Engl J Med 340(13): 1021-1030; and Ballas et al. (1992) Blood, 79(8): 2154-63]. Once exposed, the chains of the separate hemoglobin molecules polymerize, which results in damage to the red blood cell membrane and cellular dehydration. The membrane damage is manifested, in part, by a redistribution of membrane lipids leading to the expression of phosphatidylserine on the outer leaflet of the erythrocyte membrane [see, *e.g.,* (2002) Blood 99(5): 1564-1571]. Externalized phosphatidylserine promotes adhesion to both macrophages and activated endothelial cells, which contributes to vascular (vaso) occlusion. Thus, at low oxygen states, the red cell's hemoglobin precipitates into long crystals that cause it to elongate, morphologically switching into a "sickled" red blood cell. Both genotype and the extent and degree of deoxygenation contribute to the severity of hemoglobin polymerization. It has been demonstrated that the presence of fetal hemoglobin proportionally reduces the amount of pathological hemoglobin polymers and is protective from vaso-occlusive crises.

Most sickle-cell disease patients experience painful episodes call pain crises. A sickle-cell pain crisis refers to acute sickling-related pain that lasts for at least 1 hour (*e.g.,* at least 1, 2, 3, 4, 5, 6, or 10 hours) and optionally requires pain management therapy such as, *e.g.,* administration of one or more narcotic and/or non-steroid anti-inflammatory agent. A pain crisis typically results in patient admission to a medical facility for pain management therapy. Acute pain in patients with sickle-cell disease is generally ischemic in nature and can result from the occlusion of microvascular beds. Clinical data indicate that some patients with sickle-cell disease have from three to ten episodes of pain crisis per year. In many patients a pain-crisis episode will typically be resolved in about a week. In some cases, severe episodes may persist for several weeks or even months. Pain management in sickle-cell disease often requires administration of one or more opioid analgesics (*e.g.* hydromorphone, meperidine, *etc*.), non-steroidal anti-inflammatory drugs (*e.g.,* ketorolac tromethamine), and corticosteroids. In some instances, one or more ActRII antagonist agents of the disclosure, optionally combined with an EPO receptor activator and/or one or more additional therapies (*e.g.*, treatment with hydroxyurea), may be used to treat or prevent pain crisis in a sickle-cell disease patient. In some instances, one or more ActRII antagonist agents of the disclosure (*e.g.*, a GDF-ActRII antagonist, an ActRIIA polypeptide, an ActRIIB polypeptide, a GDF trap, *etc*.), optionally in combination with one or more agents and/or supportive therapies for treating sickle-cell disease, may be used to reduce the frequency of pain management (*e.g.,* treatment with one or more narcotics, non-steroid anti-inflammatory drugs, and/or corticosteroids) in a patient with sickle-cell disease. In some instances, one or more ActRII antagonist agents of the disclosure, optionally in combination with one or more agents and/or supportive therapies for treating sickle-cell disease, may be used to reduce the dosage amount of one or more pain management agents (*e.g.,* narcotics, non-steroid anti-inflammatory drugs, and/or corticosteroids) in a patient with sickle-cell disease.

Patients with SCD who receive frequent transfusions of red blood cells or whole blood are prone to develop transfusional iron overload, which may partly explain why transfusion dependency in SCD is associated with reduced likelihood of survival. Nevertheless, the use of iron chelation therapy in transfusion-dependent SCD patients remains controversial, because retrospective and registry data suggest chelated patients may live longer than unchelated patients, yet there are no prospective randomized trial data demonstrating a morbidity or mortality benefit from chelation, and currently approved agents are inconvenient (deferroxamine) or costly and poorly tolerated by many patients (deferasirox). Accordingly, one or more ActRII antagonist agents of the disclosure (*e.g.,* a GDF-ActRII antagonist, an ActRIIA polypeptide, an ActRIIB polypeptide, a GDF trap, *etc*.), optionally combined with an EPO receptor activator and/or one or more additional therapies (*e.g.*, treatment with hydroxyurea), may be used to reduce the frequency of blood transfusions or reduce the dosage of chelation therapy in SCD patients.

In certain aspects, one or more ActRII antagonist agents of the disclosure (*e.g.*, a GDF-ActRII antagonist, an ActRIIA polypeptide, an ActRIIB polypeptide, a GDF trap, *etc*.), optionally combined with an EPO receptor activator and/or one or more additional therapies, may be used to reduce liver iron content and/or prevent or reverse a hepatic complication of iron overload including, *e.g*., liver enlargement (hepatomegaly), liver fibrosis (increase in scar tissue), and cirrhosis (extensive scarring). In certain aspects, one or more ActRII antagonist agents of the disclosure (*e.g.*, a GDF-ActRII antagonist, an ActRIIA polypeptide, an ActRIIB polypeptide, a GDF trap, *etc*.), optionally combined with an EPO receptor activator and/or one or more additional therapies, may be used to prevent or reverse an endocrine complication of iron overload including, *e.g*., diabetes mellitus.

Vaso-occlusive crises are one of the clinical hallmarks of sickle-cell disease. See, *e.g.,* Rees et al. (2010) Lancet, 376: 2018-2031. Hypoxia, acidosis, inflammatory stress, and endothelial cell activation promote the entrapment of rigid, polymerized sickled erythrocytes and leukocytes within small vessels. Sickled red blood cells obstruct capillaries and restrict blood flow to the organ, leading to ischemia, pain, tissue necrosis, and damage to various organs. This can cause vascular obstruction, leading to tissue ischemia. Although polymerization and early membrane damage are initially reversible, repeated sickling episodes lead to irreversibly sickled erythrocytes, which can impact a variety of organ systems and lead to death. In some instances, one or more ActRII antagonist agents of the disclosure, optionally in combination with one or more agents and/or supportive therapies for treating sickle-cell disease, may be used to treat or prevent vaso-occlusive crisis in a sickle-cell disease patient. In some instances, one or more ActRII antagonist agents of the disclosure, optionally in combination with one or more agents and/or supportive therapies for treating sickle-cell disease, may be used to treat or prevent vaso-occlusion in a sickle-cell disease patient. In some instances, one or more ActRII antagonist agents of the disclosure, optionally in combination with one or more agents and/or supportive therapies for treating sickle-cell disease, may be used to treat or prevent a complication of vaso-occlusion in a sickle-cell disease patient. In some instances, one or more ActRII antagonist agents of the disclosure, (*e.g.*, a GDF-ActRII antagonist, an ActRIIA polypeptide, an ActRIIB polypeptide, a GDF trap, *etc*.), optionally combined with an EPO receptor activator and/or one or more additional therapies (*e.g*., treatment with hydroxyurea), may be used to treat or prevent vaso-occlusion pain in a sickle-cell disease patient.

Like vaso-occlusive complications, hemolytic anemia leads to significant morbidity in patients with sickle-cell disease. See, *e.g.,* Pakbaz et al. (2014) Hematol Oncol Clin N Am 28: 355-374; Kassim et al. (2013) Annu Rev Med 64: 451-466. Multiple factors contribute to chronic anemia in sickle-cell disease. As erythrocytes become deformed, antibodies are created to exposed antigens, which leads to increased destruction of erythrocytes, with an average lifespan of 17 days instead of 110 to 120 days. The release of hemoglobin during hemolysis inhibits nitric oxide signaling, leading to endothelial cell dysfunction and contributing to a hypercoagulable state. Chronic hemolysis contributes to anemia along with an impaired erythrocyte compensatory mechanism caused by hormone and vitamin deficiencies. Progressive renal disease is common in sickle-cell disease, leading to decreased erythropoietin and thus impaired stimulation of erythropoiesis. Folate and iron deficiency are common because of higher demand from erythrocyte production and increased urinary iron losses. All of these factors contribute to chronic anemia in sickle-cell disease patients. In some instances, one or more ActRII antagonist agents of the disclosure, optionally in combination with one or more agents and/or supportive therapies for treating sickle-cell disease, may be used to treat or prevent anemia in a sickle-cell disease patient. In some instances, one or more ActRII antagonist agents of the disclosure, (*e.g.*, a GDF-ActRII antagonist, an ActRIIA polypeptide, an ActRIIB polypeptide, a GDF trap, *etc*.), optionally combined with an EPO receptor activator and/or one or more additional therapies (*e.g.*, treatment with hydroxyurea), may be used to treat or prevent a complication of anemia in a sickle-cell disease patient.

Acute anemia, which can be severe and potentially fatal, is associated with a 10% to 15% mortality rate in patients with sickle-cell disease. In general, severe episodes are precipitated by three main causes: splenic sequestration crises, aplastic crises, or hyperhemolytic crises [see, *e.g.,* Ballas et al. (2010) Am J Hematol, 85: 6-13].

Splenic sequestration crises occur as a result of erythrocyte vaso-occlusion within the spleen, where a pooling of erythrocytes causes its rapid enlargement. As such, there is a decrease in circulating hemoglobin (*e.g.*, decreasing by 2 g/dL) and effective circulating volume, which may lead to hypovolemic shock. In some instances, one or more ActRII antagonist agents of the disclosure, optionally in combination with one or more agents and/or supportive therapies for treating sickle-cell disease, may be used to treat or prevent splenic sequestration crises in a sickle-cell disease patient. In some instances, one or more ActRII antagonist agents of the disclosure, optionally in combination with one or more agents and/or supportive therapies for treating sickle-cell disease, may be used to treat or prevent splenic sequestration of red blood cells in a sickle-cell disease patient. In some instances, one or more ActRII antagonist agents of the disclosure, (*e.g.*, a GDF-ActRII antagonist, an ActRIIA polypeptide, an ActRIIB polypeptide, a GDF trap, *etc*.) optionally combined with an EPO receptor activator and/or one or more additional therapies (*e.g.*, treatment with hydroxyurea), may be used to treat or prevent splenomegaly in a sickle-cell disease patient

Aplastic crises arise when erythropoiesis is impaired. Because of the constant overproduction of erythrocytes, an aplastic crisis can rapidly result in severe anemia. Infections, such as parvovirus B19, streptococci, salmonella, and Epstein-Barr virus, are common causes for the transient arrest of erythropoiesis. Circulating erythrocytes and reticulocytes are both decreased during aplastic crises. In some instances, one or more ActRII antagonist agents of the disclosure, optionally in combination with one or more agents and/or supportive therapies for treating sickle-cell disease, may be used to treat or prevent aplastic crises in a sickle-cell disease patient. In some instances, one or more ActRII antagonist agents of the disclosure (*e.g.*, a GDF-ActRII antagonist, an ActRIIA polypeptide, an ActRIIB polypeptide, a GDF trap, *etc*.), optionally combined with an EPO receptor activator and/or one or more additional therapies (*e.g*., treatment with hydroxyurea), may be used to treat or prevent aplastic anemia in a sickle-cell disease patient.

Hyperhemolysis occurs when there is a sudden exacerbation of anemia with reticulocytosis, without evidence of splenic sequestration. Hyperhemolytic crises have been documented in patients with multiple transfusions or in patients receiving intravenous immunoglobulin therapy. In some instances, one or more ActRII antagonist agents of the disclosure, optionally in combination with one or more agents and/or supportive therapies for treating sickle-cell disease, may be used to treat or prevent hyperhemolytic crises in a sickle-cell disease patient. In some instances, one or more ActRII antagonist agents of the disclosure, optionally in combination with one or more agents and/or supportive therapies for treating sickle-cell disease, may be used to treat or prevent hyperhemolytic anemia in a sickle-cell disease patient.

In certain aspects, ActRII antagonist agents of the disclosure, optionally in combination with one or more agents and/or supportive therapies for treating sickle-cell disease, may be used to treat or prevent a cardiac complication of sickle-cell disease. Typically, chronic anemia in sickle-cell disease causes a compensatory increased cardiac output. This, in turn, leads to cardiomegaly and left ventricular hypertrophy with left ventricular dysfunction. See, *e.g.,* Adebayo et al. (2002) Niger J Med, 11: 145-152; Sachdev et al. (2007) J Am Coll Cardiol, 49: 472-279; and Zilberman et al. (2007) Am J Hematol 82: 433-438. Acute myocardial infarction can occur, even without coronary artery disease, and is thus underdiagnosed in sickle-cell disease [see, *e.g.,* Pannu et al. (2008) Crit Pathw Cardio, 7: 133-138]. Cardiac arrhythmias and congestive heart failure have also been linked to premature death in sickle-cell disease patients [see, *e.g.,* Fitzhugh et al. (2010) Am J Hematol 85: 36-40]. In some instances, ActRII antagonist agents of the disclosure (*e.g.*, a GDF-ActRII antagonist, an ActRIIA polypeptide, an ActRIIB polypeptide, a GDF trap, *etc*.), optionally combined with an EPO receptor activator and/or one or more additional therapies (*e.g.*, treatment with hydroxyurea), may be used to treat or prevent one or more cardiac complications of sickle-cell disease including, *e.g*., increased cardiac output, cardiomegaly, cardiomyopathy, left ventricular hypertrophy, acute myocardial infarction, arrhythmia, and congestive heart failure.

In certain aspects, ActRII antagonist agents of the disclosure, optionally in combination with one or more agents and/or supportive therapies for treating sickle-cell disease, may be used to treat or prevent a pulmonary complication of sickle-cell disease. Sickle-cell disease frequently results in both acute and chronic pulmonary complications [see, *e.g.,* Rucknagel, DL (2001) Pediatr Pathol MOl Med, 20: 137-154; Haynes et al. (1986) Am J Med 80: 833-840]. Acute complications may include infection, pulmonary emboli from thrombi, bone marrow infarction, and fat emboli. Pulmonary dysfunction may occur because of local pain from rib and sternal infarctions, leading to hypoventilation and atelectasis with hypoxemia. Chronic complications include sickle-cell chronic lung disease and pulmonary hypertension. Acute chest syndrome (ACS) is unique to people with sickle disease and is defined by a new pulmonary infiltrate involving at least one complete lung segment, chest pain, and temperature above 38.5°C along with tachypnea, wheeze, or cough [see, *e.g.,* Vichinsky et al. (2000) N Engl J Med, 342: 1855-1865]. Development of pulmonary infarction, fat embolism, and infections may all contribute to ACS, and infection is a major cause of morbidity and mortality in patients with ACS.

Pulmonary hypertension is currently a major cause of morbidity and mortality in sickle-cell disease. See, *e.g.,* De Castro et al. (2008) Am J Hematol, 83: 19-25; Gladwin et al. (2004) N Engl J Med 350: 886-895. Pulmonary hypertension has been documented in 32% of adults with sickle-cell disease and is related to vaso-occlusive crises and hemolysis [see, *e.g.,* Machado et al. (2010) Chest, 137(6 supple): 30S-38S]. Cell-free hemoglobin from hemolysis is thought to decrease nitric oxide, a pulmonary vasodilator, contributing to vaso-occlusion [see, *e.g.,* Wood et al. (2008) Free Radic Biol Med 44: 1506-1528]. In some instances, ActRII antagonist agents of the disclosure (*e.g.*, a GDF-ActRII antagonist, an ActRIIA polypeptide, an ActRIIB polypeptide, a GDF trap, *etc*.), optionally combined with an EPO receptor activator and/or one or more additional therapies (*e.g*., treatment with hydroxyurea), may be used to treat or prevent one or more pulmonary complications of sickle-cell disease including, *e.g.,* fat or bone marrow emboli, pulmonary edema, sickle-cell lung disease, pulmonary hypertension, thromboemboli, and acute chest syndrome.

In certain aspects, ActRII antagonist agents of the disclosure, optionally in combination with one or more agents and/or supportive therapies for treating sickle-cell disease, may be used to treat or prevent a hepatic complication of sickle-cell disease. Liver pathology is common in sickle-cell disease, with hepatomegaly being observed in ∼90% of autopsy cases [see, *e.g.,* Bauer et al. (1980) Am J med 69: 833-837; Mills et al. (1988) Arch Pathol Lab Med 112: 290-294]. The effects of sickle-cell anemia on the liver include intrasinusoidal sickling with proximal sinusoidal dilation, Kupffer cell hyperplasia with erythrophagocytosis, and hemosiderosis. Focal necrosis, regenerative nodules, and cirrhosis have also been described in postmortem examinations. Vaso-occlusion can lead to sinusoidal obstruction and ischemia, resulting in acute sickle hepatic crises. Similar to splenic sequestration, erythrocytes can be sequestered within the liver, leading to acute anemia [see, *e.g.,* Lee et al. (1996) Postgrad Med J 72: 487-488]. Hepatic sequestration can also lead to intrahepatic cholestasis [see, *e.g.,* Shao et al. (1995) Am J Gastroenterol 90: 2045-2050]. Ischemia within hepatocytes from sickling episodes also leads to ballooning of erythrocytes and intracanalicular cholestasis. Some therapies used for treating sickle-cell disease also contribute to liver pathology. For example, frequent transfusions lead to increased iron deposition within Kupffer cells (which may lead to iron overload) and increase the risk of infection with blood-borne disease such as viral hepatitis. In some instances, ActRII antagonist agents of the disclosure (*e.g.*, a GDF-ActRII antagonist, an ActRIIA polypeptide, an ActRIIB polypeptide, a GDF trap, *etc*.), optionally combined with an EPO receptor activator and/or one or more additional therapies (*e.g.*, treatment with hydroxyurea), may be used to treat or prevent one or more hepatic complications of sickle-cell disease including, *e.g.,* hepatic failure, hepatomegaly, hepatic sequestration, intrahepatic cholestasis, cholelithiasis, and iron overload.

In certain aspects, ActRII antagonist agents of the disclosure, optionally in combination with one or more agents and/or supportive therapies for treating sickle-cell disease, may be used to treat or prevent a splenic complication of sickle-cell disease. Splenic sequestration, as previously discussed, occurs as a result of vaso-occlusion of erythrocytes within the spleen. Acute exacerbations result in splenomegaly and occasionally splenic infarction. More commonly, subclinical splenic sequestration may lead to the gradual loss of splenic function, leading to functional hyposplenia and asplenia. This, in turn, can lead to an increased susceptibility to sepsis as a result of encapsulated bacteria. In some instances, ActRII antagonist agents of the disclosure (*e.g.*, a GDF-ActRII antagonist, an ActRIIA polypeptide, an ActRIIB polypeptide, a GDF trap, *etc*.), optionally combined with an EPO receptor activator and/or one or more additional therapies (*e.g*., treatment with hydroxyurea), may be used to treat or prevent one or more splenic complications of sickle-cell disease including, *e.g*., acute or chronic splenic sequestration, splenomegaly, hyposplenia, asplenia, and splenic infarction.

In certain aspects, ActRII antagonist agents of the disclosure, optionally in combination with one or more agents and/or supportive therapies for treating sickle-cell disease, may be used to treat or prevent a renal complication of sickle-cell disease. Approximately twelve percent of people with sickle-cell disease develop renal failure [see, *e.g.,* Powars et al. (2205) Medicine 84: 363-376; Scheinman, JI (2009) Nat Clin Pract Nephrol 5: 78-88]. Vaso-occlusion within the vasa recta capillaries leads to microthrombotic infarction and extravasation of erythrocytes into the renal medulla. Blood becomes more viscous in the renal medulla because of low oxygen tension, low pH, and high osmolality and, if severe, can contribute to ischemia, infarction, and papillary necrosis. Repeated glomerular ischemia leads to glomerulosclerosis. Clinical consequences of ischemic damage include hematuria, proteinuria, decreased concentrating ability, renal tubular acidosis, abnormal proximal tubular function, acute and chronic renal failure, and urinary tract infections. In some instances, ActRII antagonist agents of the disclosure (*e.g.*, a GDF-ActRII antagonist, an ActRIIA polypeptide, an ActRIIB polypeptide, a GDF trap, *etc*.), optionally combined with an EPO receptor activator and/or one or more additional therapies (*e.g.,* treatment with hydroxyurea), may be used to treat or prevent one or more renal complications of sickle-cell disease including, *e.g*., acute and/or chronic renal failure, pyelonephritis, and renal medullary carcinoma.

In certain aspects, ActRII antagonist agents of the disclosure, optionally in combination with one or more agents and/or supportive therapies for treating sickle-cell disease, may be used to treat or prevent a bone and/or joint complication of sickle-cell disease. Bone and joint complications are a common complication in sickle-cell disease patients [see, *e.g.,* Hernigou et al. (1991) J Bone Join Surg Am, 73: 81-92]. Pain from the small bones in the hands and feet, dactylitis, occurs frequently in infants with sickle-cell disease. Long-term consequences of vaso-occlusion within bone marrow include infarcts, necrosis, and ultimately degenerative changes. Because of hyposplenia, bacterial infections are more common in sickle-cell disease. Infarcted bone and bone marrow are common sites of infection, leading to osteomyelitis and septic arthritis. Osteonecrosis, or avascular necrosis, occurs after infarction with bone and bone marrow. Infarctions are most common within long bones such as the humerus, tibia, and femur. Chronic weight bearing causes stress on abnormal femoral heads and leads to progressive joint destruction and arthritis. In some instances, ActRII antagonist agents of the disclosure (*e.g.,* a GDF-ActRII antagonist, an ActRIIA polypeptide, an ActRIIB polypeptide, a GDF trap, *etc*.), optionally combined with an EPO receptor activator and/or one or more additional therapies (*e.g.*, treatment with hydroxyurea), may be used to treat or prevent one or more bone and/or joint complications of sickle-cell disease including, *e.g*., infarction, necrosis, osteomyelitis, septic arthritis, osteonecrosis, and osteopenia.

In certain aspects, ActRII antagonist agents of the disclosure, optionally in combination with one or more agents and/or supportive therapies for treating sickle-cell disease, may be used to treat or prevent a neurological complication of sickle-cell disease. Approximately 25 percent of individuals with sickle-cell disease are affected by neurological injury [see, *e.g.,* Ohene-Frempong et al. (1998) Blood, 91: 288-294; Verduzco et al. (2009) Blood 114: 5117-5125]. The injuries may be acute or chronic. Cerebrovascular accidents are most common in adults, but depend on the genotype. A person with HbSS has the highest cerebrovascular risk, with a 24 percent likelihood of having a clinical stroke by the age of 45. Ischemic strokes are more common in children under 9 years of age, whereas hemorrhagic strokes are more common in adults. Ischemic strokes occur because of the occlusion of large intracranial arteries, leading to ischemia. The ischemia is secondary to occlusion of smaller vessels by rigid erythrocytes, exacerbated by chronic anemia, a hypercoagulable state, and flow-related hemodynamic injury to the arterial endothelium, further increasing the likelihood of erythrocyte adhesion. In contrast, hemorrhagic strokes may occur in intraventricular, intraparenchymal, and subarachnoid spaces [see, *e.g.,* Anson, et al. (1991) J Neurosurg, 75: 552-558]. Intraventricular hemorrhage may be associated with rupture of anterior cerebral artery aneurysms or direct extension of intraparenchymal hemorrhage into the lateral or third ventricle. In some instances, ActRII antagonist agents of the disclosure (*e.g.,* a GDF-ActRII antagonist, an ActRIIA polypeptide, an ActRIIB polypeptide, a GDF trap, *etc*.), optionally combined with an EPO receptor activator and/or one or more additional therapies (*e.g.*, treatment with hydroxyurea), may be used to treat or prevent one or more neurological complications of sickle-cell disease inclduing, *e.g*., aneurysm, ischemic stroke, intraparenchymal hemorrhage, subarachnoid hemorrhage, and intraventricular hemorrhage.

In certain aspects, ActRII antagonist agents of the disclosure, optionally in combination with one or more agents and/or supportive therapies for treating sickle-cell disease, may be used to treat or prevent an ophthalmic complication of sickle-cell disease. Eye complications in sickle-cell disease mainly affect the retina [see, *e.g.,* Downes et al. (2005) Opthalmology, 112: 1869-1875; Fadugbagbe et al. (2010) Ann Trop Paediatr 30: 19-26]. As a result of vaso-occlusive crises, peripheral retinal ischemia occurs. New blood vessels (sea-fan formations) form mostly near arteriovenous crossings and are known as proliferative sickle retinopathy. These new vessels can bleed easily, causing traction retinal detachments and ultimately blindness. Non-proliferative retinal changes are also more common in sickle-cell disease. In some instances, ActRII antagonist agents of the disclosure (*e.g.,* a GDF-ActRII antagonist, an ActRIIA polypeptide, an ActRIIB polypeptide, a GDF trap, *etc*.), optionally combined with an EPO receptor activator and/or one or more additional therapies (*e.g.,* treatment with hydroxyurea), may be used to treat or prevent one or more ophthalmic complications of sickle-cell disease including, *e.g*., peripheral retinal ischemia, proliferative sickle retinopathy, vitreous hemorrhage, retinal detachment, and non-proliferative retinal changes.

In certain aspects, ActRII antagonist agents of the disclosure may be administered to a subject in need thereof in combination with one or more additional agents (*e.g.,* hydroxyurea, an EPO antagonist, EPO, an opioid analgesic, a non-steroidal anti-inflammatory drug, a corticosteroid, an iron-chelating agent) or supportive therapies (*e.g.*, red blood cell transfusion) for treating sickle-cell disease or one or more complications of sickle-cell disease.

The mainstay of treatment for the majority of patients with sickle-cell disease is supportive. Current treatment options for patients with sickle-cell disease include antibiotics, pain management, intravenous fluids, blood transfusion, surgery, and compounds such as hydroxyurea.

Hydroxyurea (*e.g.* Droxia®)is an approved drug for treating sickle-cell disease. Hydroxyurea is an S-phase cytotoxic drug and is used for long-term therapy. It is believed to increase the levels of hemoglobin F which prevents formation of S-polymers and red cell sickling. It is also believed to increase NO production. A multi-center trial of hydroxyurea in adults with sickle-cell disease showed that hydroxyurea reduced the incidence of painful episodes by nearly half. However, presently hydroxyurea is used only in patients who suffer severe complications of sickle-cell disease and who are capable of following the daily dosage regimes. The general belief is that hydroxyurea therapy is effective only if given in a structured environment with a high potential for compliance. Unfortunately, many patients with sickle-cell disease are refractory to hydroxyurea. In some instances, the compositions for use of the present disclosure are for treating sickle-cell disease in a subject in need thereof comprising an ActRII antagonist of the disclosure for use in combination with hydroxyurea. In some instances, the methods of the present disclosure relate to compositions for use in treating or preventing one or more complications of sickle-cell disease in a subject in need thereof comprising an ActRII antagonist of the disclosure for use in combination with hydroxyurea.

In certain instances, one or more ActRII antagonist agents of the disclosure (*e.g.,* GDF-ActRII antagonist, an ActRIIA polypeptide, an ActRIIB polypeptide, a GDF trap, antibody *etc*.), optionally combined with an EPO receptor activator and/or one or more additional therapies (*e.g.*, treatment with hydroxyurea), may be used in combination with transfusion of either red blood cells or whole blood to treat anemia in patients with sickle-cell disease or one or more complications of sickle-cell disease. In patients who receive frequent transfusions of whole blood or red blood cells, normal mechanisms of iron homeostasis can be overwhelmed, eventually leading to toxic and potentially fatal accumulation of iron in vital tissues such as heart, liver, and endocrine glands. Regular red blood cell transfusions require exposure to various donor units of blood and hence a higher risk of alloimmunization. Difficulties with vascular access, availability of and compliance with iron chelation, and high cost are some of the reasons why it can be beneficial to limit the number of red blood cell transfusions]. In some instances, the compositions for use of the present disclosure relate to compositions for use in treating sickle-cell disease in a subject in need thereof comprising an ActRII antagonist of the disclosure for use in combination with one or more blood cell transfusions. In some instances, the compositions for use in of the present disclosure are for use in treating or preventing one or more complications of sickle-cell disease in a subject in need thereof comprising an ActRII antagonist of the disclosure for use in combination with one or more red blood cell transfusions. In some instances, treatment with one or more ActRII antagonists of the disclosure is effective at decreasing the transfusion requirement in a patient with sickle-cell disease, *e.g*., reduces the frequency and/or amount of blood transfusion required to effectively treat sickle-cell disease or one or more complications of sickle-cell disease.

In certain instances, one or more ActRII antagonist agents of the disclosure (*e.g.,* a GDF-ActRII antagonist, an ActRIIA polypeptide, an ActRIIB polypeptide, a GDF trap, *etc*.), optionally combined with an EPO receptor activator and/or one or more additional therapies (*e.g.*, treatment with hydroxyurea), may be used in combination with one or more iron-chelating molecules to promote iron excretion in the urine and/or stool and thereby prevent or reverse tissue iron overload in SCD patients. Effective iron-chelating agents should be able to selectively bind and neutralize ferric iron, the oxidized form of non-transferrin bound iron which likely accounts for most iron toxicity through catalytic production of hydroxyl radicals and oxidation products [see, *e.g*., Esposito et al. (2003) Blood 102:2670-2677]. These agents are structurally diverse, but all possess oxygen or nitrogen donor atoms able to form neutralizing octahedral coordination complexes with individual iron atoms in stoichiometries of 1:1 (hexadentate agents), 2:1 (tridentate), or 3:1 (bidentate) [Kalinowski et al. (2005) Pharmacol Rev 57:547-583]. In general, effective iron-chelating agents also are relatively low molecular weight (*e.g.*, less than 700 daltons), with solubility in both water and lipids to enable access to affected tissues. Specific examples of iron-chelating molecules include deferoxamine (also known as desferrioxamine B, desferoxamine B, DFO-B, DFOA, DFB, or desferal), a hexadentate agent of bacterial origin requiring daily parenteral administration, and the orally active synthetic agents deferiprone (bidentate; also known as Ferriprox™) and deferasirox (tridentate; also known as bis-hydroxyphenyl-triazole, ICL670, or Exjade™). Combination therapy consisting of same-day administration of two iron-chelating agents shows promise in patients unresponsive to chelation monotherapy and also in overcoming issues of poor patient compliance with dereroxamine alone [Cao et al. (2011) Pediatr Rep 3(2):e17; and Galanello et al. (2010) Ann NY Acad Sci 1202:79-86].

As shown herein, one or more ActRII antagonist agents of the disclosure (*e.g.,* a GDF-ActRII antagonist, an ActRIIA polypeptide, an ActRIIB polypeptide, a GDF trap, *etc*.), optionally combined with an EPO receptor activator and/or one or more additional therapies (*e.g.*, treatment with hydroxyurea), may be used to increase red blood cell, hemoglobin, or reticulocyte levels in healthy individuals and selected patient populations. Examples of appropriate patient populations include those with undesirably low red blood cell or hemoglobin levels, such as patients with anemia or sickle-cell disease and those at risk for developing undesirably low levels of red blood cells or hemoglobin, such as patients about to undergo major surgery or other procedures that may result in substantial blood loss. In some instances, a patient with adequate red blood cell levels is treated with one or more ActRII antagonist agents to increase red blood cell levels, and then blood is withdrawn and stored for later use in transfusions.

One or more ActRII antagonist agents of the disclosure (*e.g.,* a GDF-ActRII antagonist, an ActRIIA polypeptide, an ActRIIB polypeptide, a GDF trap, *etc*.), optionally combined with an EPO receptor activator and/or one or more additional supportive therapies (*e.g.,* treatment with hydroxyurea), may be used to increase red blood cell levels, hemoglobin levels, and/or hematocrit levels in a patient having an anemia (*e.g.,* a sickle-cell patient). When observing hemoglobin and/or hematocrit levels in humans, a level of less than normal for the appropriate age and gender category may be indicative of anemia, although individual variations are taken into account. For example, a hemoglobin level from 10-12.5 g/dl, and typically about 11.0 g/dl is considered to be within the normal range in healthy adults, although, in terms of therapy, a lower target level may cause fewer cardiovascular side effects. See, *e.g*., Jacobs et al. (2000) Nephrol Dial Transplant 15, 15-19. Alternatively, hematocrit levels (percentage of the volume of a blood sample occupied by the cells) can be used as a measure for anemia. Hematocrit levels for healthy individuals range from about 41-51% for adult males and from 35-45% for adult females. In certain instances, a patient may be treated with a dosing regimen intended to restore the patient to a target level of red blood cells, hemoglobin, and/or hematocrit. As hemoglobin and hematocrit levels vary from person to person, optimally, the target hemoglobin and/or hematocrit level can be individualized for each patient.

Anemia is frequently observed in patients having a tissue injury, an infection, and/or a chronic disease, particularly cancer. In some subjects, anemia is distinguished by low erythropoietin levels and/or an inadequate response to erythropoietin in the bone marrow. See, *e.g*., Adamson, 2008, Harrison's Principles of Internal Medicine, 17th ed.; McGraw Hill, New York, pp 628-634. Potential causes of anemia include, for example, blood-loss, nutritional deficits (*e.g.* reduced dietary intake of protein), medication reaction, various problems associated with the bone marrow, and many diseases. More particularly, anemia has been associated with a variety of disorders and conditions that include, for example, bone marrow transplantation; solid tumors (*e.g.*, breast cancer, lung cancer, and colon cancer); tumors of the lymphatic system (*e.g.*, chronic lymphocyte leukemia, non-Hodgkin's lymphoma, and Hodgkin's lymphoma); tumors of the hematopoietic system (*e.g.*, leukemia, a myelodysplastic syndrome and multiple myeloma); radiation therapy; chemotherapy (*e.g.*, platinum containing regimens); inflammatory and autoimmune diseases, including, but not limited to, rheumatoid arthritis, other inflammatory arthritides, systemic lupus erythematosis (SLE), acute or chronic skin diseases (*e.g.,* psoriasis), inflammatory bowel disease (*e.g.,* Crohn's disease and ulcerative colitis); acute or chronic renal disease or failure, including idiopathic or congenital conditions; acute or chronic liver disease; acute or chronic bleeding; situations where transfusion of red blood cells is not possible due to patient allo- or auto-antibodies and/or for religious reasons (*e.g.,* some Jehovah's Witnesses); infections (*e.g.,* malaria and osteomyelitis); hemoglobinopathies including, for example, sickle-cell disease (anemia), thalassemias; drug use or abuse (*e.g.*, alcohol misuse); pediatric patients with anemia from any cause to avoid transfusion; elderly patients; and patients with underlying cardiopulmonary disease and anemia who cannot receive transfusions due to concerns about circulatory overload. See, *e.g*., Adamson (2008) Harrison's Principles of Internal Medicine, 17th ed.; McGraw Hill, New York, pp 628-634. In some instances, one or more ActRII antagonist agents of the disclosure (*e.g.*, a GDF-ActRII antagonist, an ActRIIA polypeptide, an ActRIIB polypeptide, a GDF trap, *etc*.), optionally combined with an EPO receptor activator and/or one or more other additional therapies (*e.g*., treatment with hydroxyurea), may be used to treat or prevent anemia associated with one or more of the disorders or conditions disclosed herein.

Many factors can contribute to cancer-related anemia. Some are associated with the disease process itself and the generation of inflammatory cytokines such as interleukin-1, interferon-gamma, and tumor necrosis factor [Bron et al. (2001) Semin Oncol 28(Suppl 8):1-6]. Among its effects, inflammation induces the key iron-regulatory peptide hepcidin, thereby inhibiting iron export from macrophages and generally limiting iron availability for erythropoiesis [see, *e.g.,* Ganz (2007) J Am Soc Nephrol 18:394-400]. Blood loss through various routes can also contribute to cancer-related anemia. The prevalence of anemia due to cancer progression varies with cancer type, ranging from 5% in prostate cancer up to 90% in multiple myeloma. Cancer-related anemia has profound consequences for patients, including fatigue and reduced quality of life, reduced treatment efficacy, and increased mortality. In some instances, one or more ActRII antagonist agents of the disclosure (*e.g.*, a GDF-ActRII antagonist, an ActRIIA polypeptide, an ActRIIB polypeptide, a GDF trap, *etc*.), optionally combined with an EPO receptor activator, may be used to treat or prevent a cancer-related anemia.

A hypoproliferative anemia can result from primary dysfunction or failure of the bone marrow. Hypoproliferative anemias include: anemia of chronic disease, anemia of kidney disease, anemia associated with hypometabolic states, and anemia associated with cancer. In each of these types, endogenous erythropoietin levels are *inappropriately low* for the degree of anemia observed. Other hypoproliferative anemias include: early-stage iron-deficient anemia, and anemia caused by damage to the bone marrow. In these types, endogenous erythropoietin levels are *appropriately elevated* for the degree of anemia observed. Prominent examples would be myelosuppression caused by cancer and/or chemotherapeutic drugs or cancer radiation therapy. A broad review of clinical trials found that mild anemia can occur in 100% of patients after chemotherapy, while more severe anemia can occur in up to 80% of such patients [see, *e.g.,* Groopman et al. (1999) J Natl Cancer Inst 91:1616-1634]. Myelosuppressive drugs include, for example: 1) alkylating agents such as nitrogen mustards (*e.g.,* melphalan) and nitrosoureas (*e.g.,* streptozocin); 2) antimetabolites such as folic acid antagonists (*e.g.,* methotrexate), purine analogs (*e.g.,* thioguanine), and pyrimidine analogs (*e.g.*, gemcitabine); 3) cytotoxic antibiotics such as anthracyclines (*e.g.,* doxorubicin); 4) kinase inhibitors (*e.g.,* gefitinib); 5) mitotic inhibitors such as taxanes (*e.g.,* paclitaxel) and vinca alkaloids (*e.g.,* vinorelbine); 6) monoclonal antibodies (*e.g.,* rituximab); and 7) topoisomerase inhibitors (*e.g.,* topotecan and etoposide). In addition, conditions resulting in a hypometabolic rate can produce a mild-to-moderate hypoproliferative anemia. Among such conditions are endocrine deficiency states. For example, anemia can occur in Addison's disease, hypothyroidism, hyperparathyroidism, or males who are castrated or treated with estrogen. In some instances, one or more ActRII antagonist agents of the disclosure (*e.g.*, a GDF-ActRII antagonist, an ActRIIA polypeptide, an ActRIIB polypeptide, a GDF trap, *etc*.), optionally combined with an EPO receptor activator, may be used to treat or prevent a hyperproliferative anemia.

Chronic kidney disease is sometimes associated with hypoproliferative anemia, and the degree of the anemia varies in severity with the level of renal impairment. Such anemia is primarily due to inadequate production of erythropoietin and reduced survival of red blood cells. Chronic kidney disease usually proceeds gradually over a period of years or decades to end-stage (Stage-5) disease, at which point dialysis or kidney transplantation is required for patient survival. Anemia often develops early in this process and worsens as disease progresses. The clinical consequences of anemia of kidney disease are well-documented and include development of left ventricular hypertrophy, impaired cognitive function, reduced quality of life, and altered immune function [see, *e.g.,* Levin et al. (1999) Am J Kidney Dis 27:347-354; Nissenson (1992) Am J Kidney Dis 20(Suppl 1):21-24; Revicki et al. (1995) Am J Kidney Dis 25:548-554; Gafter et al., (1994) Kidney Int 45:224-231]. In some instances, one or more ActRII antagonist agents of the disclosure (*e.g.*, a GDF-ActRII antagonist, an ActRIIA polypeptide, an ActRIIB polypeptide, a GDF trap, *etc*.), optionally combined with an EPO receptor activator, may be used to treat or prevent anemia associated with acute or chronic renal disease or failure.

Anemia resulting from acute blood loss of sufficient volume, such as from trauma or postpartum hemorrhage, is known as acute post-hemorrhagic anemia. Acute blood loss initially causes hypovolemia without anemia since there is proportional depletion of RBCs along with other blood constituents. However, hypovolemia will rapidly trigger physiologic mechanisms that shift fluid from the extravascular to the vascular compartment, which results in hemodilution and anemia. If chronic, blood loss gradually depletes body iron stores and eventually leads to iron deficiency. In some instances, one or more ActRII antagonist agents of the disclosure (*e.g.*, a GDF-ActRII antagonist, an ActRIIA polypeptide, an ActRIIB polypeptide, a GDF trap, *etc*.), may be used to treat anemia resulting from acute blood loss.

Iron-deficiency anemia is the final stage in a graded progression of increasing iron deficiency which includes negative iron balance and iron-deficient erythropoiesis as intermediate stages. Iron deficiency can result from increased iron demand, decreased iron intake, or increased iron loss, as exemplified in conditions such as pregnancy, inadequate diet, intestinal malabsorption, acute or chronic inflammation, and acute or chronic blood loss. With mild-to-moderate anemia of this type, the bone marrow remains hypoproliferative, and RBC morphology is largely normal; however, even mild anemia can result in some microcytic hypochromic RBCs, and the transition to severe iron-deficient anemia is accompanied by hyperproliferation of the bone marrow and increasingly prevalent microcytic and hypochromic RBCs [see, *e.g*., Adamson (2008) Harrison's Principles of Internal Medicine, 17th ed.; McGraw Hill, New York, pp 628-634]. Appropriate therapy for iron-deficiency anemia depends on its cause and severity, with oral iron preparations, parenteral iron formulations, and RBC transfusion as major conventional options. In some instances, one or more ActRII antagonist agents of the disclosure (*e.g.*, a GDF-ActRII antagonist, an ActRIIA polypeptide, an ActRIIB polypeptide, a GDF trap, *etc*.), optionally combined with an EPO receptor activator, may be used to treat a chronic iron-deficiency.

Myelodysplastic syndrome (MDS) is a diverse collection of hematological conditions characterized by ineffective production of myeloid blood cells and risk of transformation to acute mylogenous leukemia. In MDS patients, blood stem cells do not mature into healthy red blood cells, white blood cells, or platelets. MDS disorders include, for example, refractory anemia, refractory anemia with ringed sideroblasts, refractory anemia with excess blasts, refractory anemia with excess blasts in transformation, refractory cytopenia with multilineage dysplasia, and myelodysplastic syndrome associated with an isolated 5q chromosome abnormality. As these disorders manifest as irreversible defects in both quantity and quality of hematopoietic cells, most MDS patients are afflicted with chronic anemia. Therefore, MDS patients eventually require blood transfusions and/or treatment with growth factors (e.g., erythropoietin or G-CSF) to increase red blood cell levels. However, many MDS patients develop side-effect due to frequency of such therapies. For example, patients who receive frequent red blood cell transfusion can have tissue and organ damage from the buildup of extra iron. Accordingly, one or more ActRII antagonist agents of the disclosure (*e.g.,* a GDF-ActRII antagonist, an ActRIIA polypeptide, an ActRIIB polypeptide, a GDF trap, *etc*.), optionally combined with an EPO receptor activator, may be used to treat patients having MDS. In certain instances, patients suffering from MDS may be treated using a one or more ActRII antagonist agents of the disclosure (*e.g.,* a GDF-ActRII antagonist, an ActRIIA polypeptide, an ActRIIB polypeptide, a GDF trap, *etc*.), optionally in combination with an EPO receptor activator. In other instances, patient suffering from MDS may be treated using a combination of one or more ActRII antagonist agents of the disclosure (*e.g.,* a GDF-ActRII antagonist, an ActRIIA polypeptide, an ActRIIB polypeptide, a GDF Trap, *etc*.) and one or more additional therapeutic agents for treating MDS including, for example, thalidomide, lenalidomide, azacitadine, decitabine, erythropoietins, deferoxamine, antithymocyte globulin, and filgrastrim (G-CSF).

As used herein, "in combination with" or "conjoint administration" refers to any form of administration such that additional therapies (*e.g.,* second, third, fourth, *etc*.) are still effective in the body (e.g., multiple compounds are simultaneously effective in the patient, which may include synergistic effects of those compounds). Effectiveness may not correlate to measurable concentration of the agent in blood, serum, or plasma. For example, the different therapeutic compounds can be administered either in the same formulation or in separate formulations, either concomitantly or sequentially, and on different schedules. Thus, an individual who receives such treatment can benefit from a combined effect of different therapies. One or more GDF11 and/or activin B antagonist agents (optionally further antagonists of one or more of GDF8, activin A, activin C, activin E, and BMP6) of the disclosure can be administered concurrently with, prior to, or subsequent to, one or more other additional agents or supportive therapies. In general, each therapeutic agent will be administered at a dose and/or on a time schedule determined for that particular agent. The particular combination to employ in a regimen will take into account compatibility of the antagonist of the present disclosure with the therapy and/or the desired therapeutic effect to be achieved.

In certain instances, on one or more ActRII antagonist agents of the disclosure (*e.g.*, a GDF-ActRII antagonist, an ActRIIA polypeptide, an ActRIIB polypeptide, a GDF trap, *etc*.) may be used in combination with hepcidin or a hepcidin agonist for treating sickle-cell disease, particularly sickle-cell disease complications associated with iron overload. A circulating polypeptide produced mainly in the liver, hepcidin is considered a master regulator of iron metabolism by virtue of its ability to induce the degradation of ferroportin, an iron-export protein localized on absorptive enterocytes, hepatocytes, and macrophages. Broadly speaking, hepcidin reduces availability of extracellular iron, so hepcidin agonists may be beneficial in the treatment of sickle-cell disease, particularly sickle-cell disease complications associated with iron overload.

One or more ActRII antagonist agents of the disclosure (*e.g.*, a GDF-ActRII antagonist, an ActRIIA polypeptide, an ActRIIB polypeptide, a GDF trap, *etc*.), optionally combined with an EPO receptor activator, would also be appropriate for treating anemias of disordered RBC maturation, which are characterized in part by undersized (microcytic), oversized (macrocytic), misshapen, or abnormally colored (hypochromic) RBCs.

In certain instances, the present disclosure provides compositions for use in treating or preventing anemia in an individual in need thereof comprising a therapeutically effective amount of one or more ActRII antagonist agents of the disclosure (*e.g*., a GDF-ActRII antagonist, an ActRIIA polypeptide, an ActRIIB polypeptide, a GDF trap, *etc*.) for use in combination with a EPO receptor activator (ESAs). In certain instances, one or more ActRII antagonist agents of the disclosure (*e.g.*, a GDF-ActRII antagonist, an ActRIIA polypeptide, an ActRIIB polypeptide, a GDF trap, *etc*.) may be used in combination with ESAs to reduce the required dose of these activators in patients that are susceptible to adverse effects of ESAs. These methods may be used for therapeutic and prophylactic treatments of a patient.

One or more ActRII antagonist agents of the disclosure (*e.g.*, a GDF-ActRII antagonist, an ActRIIA polypeptide, an ActRIIB polypeptide, a GDF trap, *etc*.) may be used in combination with EPO receptor activators to achieve an increase in red blood cells, particularly at lower dose ranges. This may be beneficial in reducing the known off-target effects and risks associated with high doses of EPO receptor activators. The primary adverse effects of ESAs include, for example, an excessive increase in the hematocrit or hemoglobin levels and polycythemia. Elevated hematocrit levels can lead to hypertension (more particularly aggravation of hypertension) and vascular thrombosis. Other adverse effects of ESAs which have been reported, some of which relate to hypertension, are headaches, influenza-like syndrome, obstruction of shunts, myocardial infarctions and cerebral convulsions due to thrombosis, hypertensive encephalopathy, and red cell blood cell aplasia. See, *e.g.,* Singibarti (1994) J. Clin Investig 72(suppl 6), S36-S43; Horl et al. (2000) Nephrol Dial Transplant 15(suppl 4), 51-56; Delanty et al. (1997) Neurology 49, 686-689; and Bunn (2002) N Engl J Med 346(7), 522-523).

Provided that antagonists of the present disclosure act by a different mechanism that ESAs, these antagonists may be useful for increasing red blood cell and hemoglobin levels in patients that do not respond well to ESAs. For example, an ActRII antagonist of the present disclosure may be beneficial for a patient in which administration of a normal to increased (>300 IU/kg/week) dose of ESA does not result in the increase of hemoglobin level up to the target level. Patients with an inadequate ESA response are found for all types of anemia, but higher numbers of non-responders have been observed particularly frequently in patients with cancers and patients with end-stage renal disease. An inadequate response to ESA can be either constitutive (observed upon the first treatment with ESA) or acquired (observed upon repeated treatment with ESA).

In certain instances, one or more ActRII antagonist agents of the disclosure (*e.g.*, a GDF-ActRII antagonist, an ActRIIA polypeptide, an ActRIIB polypeptide, a GDF trap, *etc*.), optionally combined with an EPO receptor activator and/or one or more additional therapies, may be used in combination with hepcidin, a hepcidin analog, or a hepcidin receptor activator for treating patients with SCD, particularly for complications associated with iron overload. A circulating polypeptide produced mainly in the liver, hepcidin is considered a master regulator of iron metabolism by virtue of its ability to induce the degradation of ferroportin, an iron-export protein localized on absorptive enterocytes, hepatocytes, and macrophages. In broad terms, hepcidin reduces availability of extracellular iron, so hepcidin, hepcidin analogs, or hepcidin receptor activators may be beneficial in the treatment of patients with SCD, particularly for complications associated with iron overload.

In certain instances, the present disclosure provides methods for managing a patient that has been treated with, or is a candidate to be treated with, one or more one or more ActRII antagonist agents of the disclosure (*e.g.*, a GDF-ActRII antagonist, an ActRIIA polypeptide, an ActRIIB polypeptide, a GDF trap, *etc*.) by measuring one or more hematologic parameters in the patient. The hematologic parameters may be used to evaluate appropriate dosing for a patient who is a candidate to be treated with the antagonist of the present disclosure, to monitor the hematologic parameters during treatment, to evaluate whether to adjust the dosage during treatment with one or more antagonist of the disclosure, and/or to evaluate an appropriate maintenance dose of one or more antagonists of the disclosure. If one or more of the hematologic parameters are outside the normal level, dosing with one or more ActRII antagonist agents of the disclosure (*e.g.*, a GDF-ActRII antagonist, an ActRIIA polypeptide, an ActRIIB polypeptide, a GDF trap, *etc*.) may be reduced, delayed or terminated.

Hematologic parameters that may be measured in accordance with the methods provided herein include, for example, red blood cell levels, blood pressure, iron stores, and other agents found in bodily fluids that correlate with increased red blood cell levels, using art recognized methods. Such parameters may be determined using a blood sample from a patient. Increases in red blood cell levels, hemoglobin levels, and/or hematocrit levels may cause increases in blood pressure.

In one instance, if one or more hematologic parameters are outside the normal range or on the high side of normal in a patient who is a candidate to be treated with one or more ActRII antagonist agents of the disclosure (*e.g.,* a GDF-ActRII antagonist, an ActRIIA polypeptide, an ActRIIB polypeptide, a GDF trap, *etc*.), then onset of administration of the one or more antagonists of the disclosure may be delayed until the hematologic parameters have returned to a normal or acceptable level either naturally or via therapeutic intervention. For example, if a candidate patient is hypertensive or pre-hypertensive, then the patient may be treated with a blood pressure lowering agent in order to reduce the patient's blood pressure. Any blood pressure lowering agent appropriate for the individual patient's condition may be used including, for example, diuretics, adrenergic inhibitors (including alpha blockers and beta blockers), vasodilators, calcium channel blockers, angiotensin-converting enzyme (ACE) inhibitors, or angiotensin II receptor blockers. Blood pressure may alternatively be treated using a diet and exercise regimen. Similarly, if a candidate patient has iron stores that are lower than normal, or on the low side of normal, then the patient may be treated with an appropriate regimen of diet and/or iron supplements until the patient's iron stores have returned to a normal or acceptable level. For patients having higher than normal red blood cell levels and/or hemoglobin levels, then administration of the one or more antagonists of the disclosure may be delayed until the levels have returned to a normal or acceptable level.

In certain embodiments, if one or more hematologic parameters are outside the normal range or on the high side of normal in a patient who is a candidate to be treated with one or more ActRII antagonist agents of the disclosure (*e.g.*, a GDF-ActRII antagonist, an ActRIIA polypeptide, an ActRIIB polypeptide, a GDF trap, *etc*.), then the onset of administration may not be delayed. However, the dosage amount or frequency of dosing of the one or more antagonists of the disclosure may be set at an amount that would reduce the risk of an unacceptable increase in the hematologic parameters arising upon administration of the one or more antagonists of the disclosure. Alternatively, a therapeutic regimen may be developed for the patient that combines one or more ActRII antagonist agents of the disclosure (*e.g*., a GDF-ActRII antagonist, an ActRIIA polypeptide, an ActRIIB polypeptide, a GDF trap, *etc*.) with a therapeutic agent that addresses the undesirable level of the hematologic parameter. For example, if the patient has elevated blood pressure, then a therapeutic regimen may be designed involving administration of one or more ActRII antagonist agents of the disclosure (*e.g.,* a GDF-ActRII antagonist, an ActRIIA polypeptide, an ActRIIB polypeptide, a GDF trap, *etc*.) and a blood pressure lowering agent. For a patient having lower than desired iron stores, a therapeutic regimen may be developed involving one or more ActRII antagonist agents of the disclosure (*e.g*., a GDF-ActRII antagonist, an ActRIIA polypeptide, an ActRIIB polypeptide, a GDF trap, *etc*.) and iron supplementation.

In one instance, baseline parameter(s) for one or more hematologic parameters may be established for a patient who is a candidate to be treated with one or more ActRII antagonist agents of the disclosure (*e.g*., a GDF-ActRII antagonist, an ActRIIA polypeptide, an ActRIIB polypeptide, a GDF trap, *etc*.) and an appropriate dosing regimen established for that patient based on the baseline value(s). Alternatively, established baseline parameters based on a patient's medical history could be used to inform an appropriate antagonist dosing regimen for a patient. For example, if a healthy patient has an established baseline blood pressure reading that is above the defined normal range it may not be necessary to bring the patient's blood pressure into the range that is considered normal for the general population prior to treatment with the one or more antagonist of the disclosure. A patient's baseline values for one or more hematologic parameters prior to treatment with one or more ActRII antagonist agents of the disclosure (*e.g.*, a GDF-ActRII antagonist, an ActRIIA polypeptide, an ActRIIB polypeptide, a GDF trap, *etc*.) may also be used as the relevant comparative values for monitoring any changes to the hematologic parameters during treatment with the one or more antagonists of the disclosure.

In certain instances, one or more hematologic parameters are measured in patients who are being treated with a one or more ActRII antagonist agents of the disclosure (*e.g*., a GDF-ActRII antagonist, an ActRIIA polypeptide, an ActRIIB polypeptide, a GDF trap, *etc*.). The hematologic parameters may be used to monitor the patient during treatment and permit adjustment or termination of the dosing with the one or more antagonists of the disclosure or additional dosing with another therapeutic agent. For example, if administration of one or more ActRII antagonist agents of the disclosure (*e.g*., a GDF-ActRII antagonist, an ActRIIA polypeptide, an ActRIIB polypeptide, a GDF trap, *etc*.) results in an increase in blood pressure, red blood cell level, or hemoglobin level, or a reduction in iron stores, then the dose of the one or more antagonists of the disclosure may be reduced in amount or frequency in order to decrease the effects of the one or more antagonists of the disclosure on the one or more hematologic parameters. If administration of one or more ActRII antagonist agents of the disclosure (*e.g.*, a GDF-ActRII antagonist, an ActRIIA polypeptide, an ActRIIB polypeptide, a GDF trap, *etc*.) results in a change in one or more hematologic parameters that is adverse to the patient, then the dosing of the one or more antagonists of the disclosure may be terminated either temporarily, until the hematologic parameter(s) return to an acceptable level, or permanently. Similarly, if one or more hematologic parameters are not brought within an acceptable range after reducing the dose or frequency of administration of the one or more antagonists of the disclosure, then the dosing may be terminated. As an alternative, or in addition to, reducing or terminating the dosing with the one or more antagonists of the disclosure, the patient may be dosed with an additional therapeutic agent that addresses the undesirable level in the hematologic parameter(s), such as, for example, a blood pressure lowering agent or an iron supplement. For example, if a patient being treated with one or more ActRII antagonist agents of the disclosure (*e.g.*, a GDF-ActRII antagonist, an ActRIIA polypeptide, an ActRIIB polypeptide, a GDF trap, *etc*.) has elevated blood pressure, then dosing with the one or more antagonists of the disclosure may continue at the same level and a blood-pressure-lowering agent is added to the treatment regimen, dosing with the one or more antagonist of the disclosure may be reduced (*e.g.,* in amount and/or frequency) and a blood-pressure-lowering agent is added to the treatment regimen, or dosing with the one or more antagonist of the disclosure may be terminated and the patient may be treated with a blood-pressure-lowering agent.

### 6. Pharmaceutical Compositions

In certain aspects, one or more ActRII antagonist agents of the disclosure (*e.g.,* a GDF-ActRII antagonist, an ActRIIA polypeptide, an ActRIIB polypeptide, a GDF trap, *etc*.) can be administered alone or as a component of a pharmaceutical formulation (also referred to as a therapeutic composition or pharmaceutical composition). A pharmaceutical formulation refers to a preparation which is in such form as to permit the biological activity of an active ingredient (*e.g.*, an agent of the present disclosure) contained therein to be effective and which contains no additional components which are unacceptably toxic to a subject to which the formulation would be administered. The subject compounds may be formulated for administration in any convenient way for use in human or veterinary medicine. For example, one or more agents of the present disclosure may be formulated with a pharmaceutically acceptable carrier. A pharmaceutically acceptable carrier refers to an ingredient in a pharmaceutical formulation, other than an active ingredient, which is generally nontoxic to a subject. A pharmaceutically acceptable carrier includes, but is not limited to, a buffer, excipient, stabilizer, and/or preservative. In general, pharmaceutical formulations for use in the present disclosure are in a pyrogen-free, physiologically-acceptable form when administered to a subject. Therapeutically useful agents other than those described herein, which may optionally be included in the formulation as described above, may be administered in combination with the subject agents in the compositions for use of the present disclosure.

Typically, compounds will be administered parenterally [*e.g.*, by intravenous (I.V.) injection, intraarterial injection, intraosseous injection, intramuscular injection, intrathecal injection, subcutaneous injection, or intradermal injection]. Pharmaceutical compositions suitable for parenteral administration may comprise one or more agents of the disclosure in combination with one or more pharmaceutically acceptable sterile isotonic aqueous or nonaqueous solutions, dispersions, suspensions or emulsions, or sterile powders which may be reconstituted into sterile injectable solutions or dispersions just prior to use. Injectable solutions or dispersions may contain antioxidants, buffers, bacteriostats, suspending agents, thickening agents, or solutes which render the formulation isotonic with the blood of the intended recipient. Examples of suitable aqueous and nonaqueous carriers which may be employed in the pharmaceutical formulations of the present disclosure include water, ethanol, polyols (*e.g.,* glycerol, propylene glycol, polyethylene glycol, *etc*.), vegetable oils (*e.g.,* olive oil), injectable organic esters (*e.g.*, ethyl oleate), and suitable mixtures thereof. Proper fluidity can be maintained, for example, by the use of coating materials (*e.g*., lecithin), by the maintenance of the required particle size in the case of dispersions, and by the use of surfactants.

In some instances, a compositions for use of the present disclosure is administered systemically, or locally, from an implant or device. Further, the pharmaceutical composition may be encapsulated or injected in a form for delivery to a target tissue site (*e.g.,* bone marrow or muscle). In certain instances, compositions of the present disclosure may include a matrix capable of delivering one or more of the agents of the present disclosure to a target tissue site (*e.g.,* bone marrow or muscle), providing a structure for the developing tissue and optimally capable of being resorbed into the body. For example, the matrix may provide slow release of one or more agents of the present disclosure. Such matrices may be formed of materials presently in use for other implanted medical applications.

The choice of matrix material may be based on one or more of: biocompatibility, biodegradability, mechanical properties, cosmetic appearance, and interface properties. The particular application of the subject compositions will define the appropriate formulation. Potential matrices for the compositions may be biodegradable and chemically defined calcium sulfate, tricalciumphosphate, hydroxyapatite, polylactic acid, and polyanhydrides. Other potential materials are biodegradable and biologically well-defined, including, for example, bone or dermal collagen. Further matrices are comprised of pure proteins or extracellular matrix components. Other potential matrices are non-biodegradable and chemically defined, including, for example, sintered hydroxyapatite, bioglass, aluminates, or other ceramics. Matrices may be comprised of combinations of any of the above mentioned types of material including, for example, polylactic acid and hydroxyapatite or collagen and tricalciumphosphate. The bioceramics may be altered in composition (*e.g.*, calcium-aluminate-phosphate) and processing to alter one or more of pore size, particle size, particle shape, and biodegradability.

In certain instances, pharmaceutical compositions of the present disclosure can be administered orally, for example, in the form of capsules, cachets, pills, tablets, lozenges (using a flavored basis such as sucrose and acacia or tragacanth), powders, granules, a solution or a suspension in an aqueous or non-aqueous liquid, an oil-in-water or water-in-oil liquid emulsion, or an elixir or syrup, or pastille (using an inert base, such as gelatin and glycerin, or sucrose and acacia), and/or a mouth wash, each containing a predetermined amount of a compound of the present disclosure and optionally one or more other active ingredients. A compound of the present disclosure and optionally one or more other active ingredients may also be administered as a bolus, electuary, or paste.

In solid dosage forms for oral administration (*e.g.*, capsules, tablets, pills, dragees, powders, and granules), one or more compounds of the present disclosure may be mixed with one or more pharmaceutically acceptable carriers including, for example, sodium citrate, dicalcium phosphate, a filler or extender (*e.g.,* a starch, lactose, sucrose, glucose, mannitol, and silicic acid), a binder (*e.g.* carboxymethylcellulose, an alginate, gelatin, polyvinyl pyrrolidone, sucrose, and acacia), a humectant (*e.g.,* glycerol), a disintegrating agent (*e.g.,* agar-agar, calcium carbonate, potato or tapioca starch, alginic acid, a silicate, and sodium carbonate), a solution retarding agent (*e.g.* paraffin), an absorption accelerator (*e.g.* a quaternary ammonium compound), a wetting agent (*e.g.,* cetyl alcohol and glycerol monostearate), an absorbent (*e.g.,* kaolin and bentonite clay), a lubricant (*e.g.,* a talc, calcium stearate, magnesium stearate, solid polyethylene glycols, sodium lauryl sulfate), a coloring agent, and mixtures thereof. In the case of capsules, tablets, and pills, the pharmaceutical formulation (composition) may also comprise a buffering agent. Solid compositions of a similar type may also be employed as fillers in soft and hard-filled gelatin capsules using one or more excipients including, e.g., lactose or a milk sugar as well as a high molecular-weight polyethylene glycol.

Liquid dosage forms for oral administration of the pharmaceutical composition may include pharmaceutically acceptable emulsions, microemulsions, solutions, suspensions, syrups, and elixirs. In addition to the active ingredient(s), the liquid dosage form may contain an inert diluent commonly used in the art including, for example, water or other solvent, a solubilizing agent and/or emulsifier [*e.g.*, ethyl alcohol, isopropyl alcohol, ethyl carbonate, ethyl acetate, benzyl alcohol, benzyl benzoate, propylene glycol, or 1,3-butylene glycol, an oil (*e.g.*, cottonseed, groundnut, corn, germ, olive, castor, and sesame oil), glycerol, tetrahydrofuryl alcohol, a polyethylene glycol, a fatty acid ester of sorbitan, and mixtures thereof]. Besides inert diluents, the oral formulation can also include an adjuvant including, for example, a wetting agent, an emulsifying and suspending agent, a sweetening agent, a flavoring agent, a coloring agent, a perfuming agent, a preservative agent, and combinations thereof.

Suspensions, in addition to the active compounds, may contain suspending agents including, for example, an ethoxylated isostearyl alcohol, polyoxyethylene sorbitol, a sorbitan ester, microcrystalline cellulose, aluminum metahydroxide, bentonite, agar-agar, tragacanth, and combinations thereof.

Prevention of the action and/or growth of microorganisms may be ensured by the inclusion of various antibacterial and antifungal agents including, for example, paraben, chlorobutanol, and phenol sorbic acid.

In certain instances, it may be desirable to include an isotonic agent including, for example, a sugar or sodium chloride into the compositions. In addition, prolonged absorption of an injectable pharmaceutical form may be brought about by the inclusion of an agent that delays absorption, including, for example, aluminum monostearate and gelatin.

It is understood that the dosage regimen will be determined by the attending physician considering various factors which modify the action of the one or more of the agents of the present disclosure. The various factors include, but are not limited to, the patient's red blood cell count, hemoglobin level, the desired target red blood cell count, the patient's age, the patient's sex, the patient's diet, the severity of any disease that may be contributing to a depressed red blood cell level, the time of administration, and other clinical factors. The addition of other known active agents to the final composition may also affect the dosage. Progress can be monitored by periodic assessment of one or more of red blood cell levels, hemoglobin levels, reticulocyte levels, and other indicators of the hematopoietic process.

In certain instances, the present disclosure also provides gene therapy for the *in vivo* production of one or more of the agents of the present disclosure. Such therapy would achieve its therapeutic effect by introduction of the agent sequences into cells or tissues having one or more of the disorders as listed above. Delivery of the agent sequences can be achieved, for example, by using a recombinant expression vector such as a chimeric virus or a colloidal dispersion system. Preferred therapeutic delivery of one or more of agent sequences of the disclosure is the use of targeted liposomes.

Various viral vectors which can be utilized for gene therapy as taught herein include adenovirus, herpes virus, vaccinia, or an RNA virus (*e.g.,* a retrovirus). The retroviral vector may be a derivative of a murine or avian retrovirus. Examples of retroviral vectors in which a single foreign gene can be inserted include, but are not limited to: Moloney murine leukemia virus (MoMuLV), Harvey murine sarcoma virus (HaMuSV), murine mammary tumor virus (MuMTV), and Rous sarcoma virus (RSV). A number of additional retroviral vectors can incorporate multiple genes. All of these vectors can transfer or incorporate a gene for a selectable marker so that transduced cells can be identified and generated. Retroviral vectors can be made target-specific by attaching, for example, a sugar, a glycolipid, or a protein. Preferred targeting is accomplished by using an antibody. Those of skill in the art will recognize that specific polynucleotide sequences can be inserted into the retroviral genome or attached to a viral envelope to allow target specific delivery of the retroviral vector containing one or more of the agents of the present disclosure.

Alternatively, tissue culture cells can be directly transfected with plasmids encoding the retroviral structural genes (gag, pol, and env), by conventional calcium phosphate transfection. These cells are then transfected with the vector plasmid containing the genes of interest. The resulting cells release the retroviral vector into the culture medium.

Another targeted delivery system for one or more of the agents of the present disclosure is a colloidal dispersion system. Colloidal dispersion systems include, for example, macromolecule complexes, nanocapsules, microspheres, beads, and lipid-based systems including oil-in-water emulsions, micelles, mixed micelles, and liposomes. In certain instances, the preferred colloidal system of this disclosure is a liposome. Liposomes are artificial membrane vesicles which are useful as delivery vehicles *in vitro* and *in vivo.* RNA, DNA, and intact virions can be encapsulated within the aqueous interior and be delivered to cells in a biologically active form [see, *e.g.,* Fraley, et al. (1981) Trends Biochem. Sci., 6:77]. Methods for efficient gene transfer using a liposome vehicle are known in the art [see, *e.g.,* Mannino, et al. (1988) Biotechniques, 6:682, 1988].

The composition of the liposome is usually a combination of phospholipids, which may include a steroid (*e.g.* cholesterol). The physical characteristics of liposomes depend on pH, ionic strength, and the presence of divalent cations. Other phospholipids or other lipids may also be used, including, for example a phosphatidyl compound (*e.g.*, phosphatidylglycerol, phosphatidylcholine, phosphatidylserine, phosphatidylethanolamine, sphingolipid, cerebroside, or a ganglioside), egg phosphatidylcholine, dipalmitoylphosphatidylcholine, and distearoylphosphatidylcholine. The targeting of liposomes is also possible based on, for example, organ specificity, cell specificity, and organelle specificity and is known in the art.

### EXEMPLIFICATION

The disclosure now being generally described, it will be more readily understood by reference to the following examples, which are included merely for purposes of illustration of certain instances of the present disclosure, and are not intended to limit the disclosure.

### Example 1: ActRIIa-Fc Fusion Proteins

Applicants constructed a soluble ActRIIA fusion protein that has the extracellular domain of human ActRIIa fused to a human or mouse Fc domain with a minimal linker in between. The constructs are referred to as ActRIIA-hFc and ActRIIA-mFc, respectively.

ActRIIA-hFc is shown below as purified from CHO cell lines (SEQ ID NO:22):

ILGRSETQECLFFNANWEKDRTNQTGVEPCYGDKDKRRHCFATWKNISGSIEI
VKQGCWLDDINCYDRTDCVEKKDSPEVYFCCCEGNMCNEKFSYFPEMEVTQPTSNP
VTPKPPTGGGTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCKVSN
KALPVPIEKTISKAKGQPREPQVYTLPPSREEMTKNQVSLTCLVKGFYPSDIAVEWES
NGQPENNYKTTPPVLDSDGSFFLYSKLTVDKSRWQQGNVFSCSVMHEALHNHYTQK
SLSLSPGK

The ActRIIA-hFc and ActRIIA-mFc proteins were expressed in CHO cell lines.

Three different leader sequences were considered:
(i) Honey bee mellitin (HBML): MKFLVNVALVFMVVYISYIYA (SEQ ID NO:23)
(ii) Tissue plasminogen activator (TPA): MDAMKRGLCCVLLLCGAVFVSP (SEQ ID NO:24)
(iii) Native: MGAAAKLAFAVFLISCSSGA (SEQ ID NO:25).

The selected form employs the TPA leader and has the following unprocessed amino acid sequence:

MDAMKRGLCCVLLLCGAVFVSPGAAILGRSETQECLFFNANWEKDRTNQTG
VEPCYGDKDKRRHCFATWKNISGSIEIVKQGCWLDDINCYDRTDCVEKKDSPEVYFC
CCEGNMCNEKFSYFPEMEVTQPTSNPVTPKPPTGGGTHTCPPCPAPELLGGPSVFLFP
PKPKDTLMISRTPEVTCVVVDVSHEDPEVKFNWYVDGVEVHNAKTKPREEQYNSTY
RVVSVLTVLHQDWLNGKEYKCKVSNKALPVPIEKTISKAKGQPREPQVYTLPPSREE
MTKNQVSLTCLVKGFYPSDIAVEWESNGQPENNYKTTPPVLDSDGSFFLYSKLTVDK
SRWQQGNVFSCSVMHEALHNHYTQKSLSLSPGK (SEQ ID NO:26)

This polypeptide is encoded by the following nucleic acid sequence:

ATGGATGCAATGAAGAGAGGGCTCTGCTGTGTGCTGCTGCTGTGTGGAGC
AGTCTTCGTTTCGCCCGGCGCCGCTATACTTGGTAGATCAGAAACTCAGGAGTGT
CTTTTTTTAATGCTAATTGGGAAAAGACAGAACCAATCAAACTGGTGTTGAACC
GTGTTATGGTGACAAAGATAAACGGCGGCATTGTTTTGCTACCTGGAAGAATATT
TCTGGTTCCATTGAATAGTGAAACAAGGTTGTTGGCTGGATGATATCAACTGCTA
TGACAGGACTGATTGTGTAGAAAAAAAAGACAGCCCTGAAGTATATTTCTGTTGC
TGTGAGGGCAATATGTGTAATGAAAAGTTTTCTTATTTTCCGGAGATGGAAGTCA
CACAGCCCACTTCAAATCCAGTTACACCTAAGCCACCCACCGGTGGTGGAACTCA
CACATGCCCACCGTGCCCAGCACCTGAACTCCTGGGGGGACCGTCAGTCTTCCTC
TTCCCCCCAAAACCCAAGGACACCCTCATGATCTCCCGGACCCCTGAGGTCACAT
GCGTGGTGGTGGACGTGAGCCACGAAGACCCTGAGGTCAAGTTCAACTGGTACG
TGGACGGCGTGGAGGTGCATAATGCCAAGACAAAGCCGCGGGAGGAGCAGTAC
AACAGCACGTACCGTGTGGTCAGCGTCCTCACCGTCCTGCACCAGGACTGGCTGA
ATGGCAAGGAGTACAAGTGCAAGGTCTCCAACAAAGCCCTCCCAGTCCCCATCG
AGAAAACCATCTCCAAAGCCAAAGGGCAGCCCCGAGAACCACAGGTGTACACCC
TGCCCCCATCCCGGGAGGAGATGACCAAGAACCAGGTCAGCCTGACCTGCCTGG
TCAAAGGCTTCTATCCCAGCGACATCGCCGTGGAGTGGGAGAGCAATGGGCAGC
CGGAGAACAACTACAAGACCACGCCTCCCGTGCTGGACTCCGACGGCTCCTTCTT
CCTCTATAGCAAGCTCACCGTGGACAAGAGCAGGTGGCAGCAGGGGAACGTCTT
CTCATGCTCCGTGATGCATGAGGCTCTGCACAACCACTACACGCAGAAGAGCCTC
TCCCTGTCTCCGGGTAAATGAGAATTC (SEQ ID NO:27)

Both ActRIIA-hFc and ActRIIA-mFc were remarkably amenable to recombinant expression. As shown in Figure 3, the protein was purified as a single, well-defined peak of protein. N-terminal sequencing revealed a single sequence of -ILGRSETQE (SEQ ID NO:34). Purification could be achieved by a series of column chromatography steps, including, for example, three or more of the following, in any order: protein A chromatography, Q sepharose chromatography, phenylsepharose chromatography, size exclusion chromatography, and cation exchange chromatography. The purification could be completed with viral filtration and buffer exchange. The ActRIIA-hFc protein was purified to a purity of >98% as determined by size exclusion chromatography and >95% as determined by SDS PAGE.

ActRIIA-hFc and ActRIIA-mFc showed a high affinity for ligands, particularly activin A. GDF-11 or activin A were immobilized on a Biacore™ CM5 chip using standard amine-coupling procedure. ActRIIA-hFc and ActRIIA-mFc proteins were loaded onto the system, and binding was measured. ActRIIA-hFc bound to activin with a dissociation constant (K_{D}) of 5 x 10⁻¹² and bound to GDF11 with a K_{D} of 9.96 x 10⁻⁹. See Figure 4. ActRIIA-mFc behaved similarly.

The ActRIIA-hFc was very stable in pharmacokinetic studies. Rats were dosed with 1 mg/kg, 3 mg/kg, or 10 mg/kg of ActRIIA-hFc protein, and plasma levels of the protein were measured at 24, 48, 72, 144 and 168 hours. In a separate study, rats were dosed at 1 mg/kg, 10 mg/kg, or 30 mg/kg. In rats, ActRIIA-hFc had an 11-14 day serum half-life, and circulating levels of the drug were quite high after two weeks (11 µg/ml, 110 µg/ml, or 304 µg/ml for initial administrations of 1 mg/kg, 10 mg/kg, or 30 mg/kg, respectively.) In cynomolgus monkeys, the plasma half-life was substantially greater than 14 days, and circulating levels of the drug were 25 µg/ml, 304 µg/ml, or 1440 µg/ml for initial administrations of 1 mg/kg, 10 mg/kg, or 30 mg/kg, respectively.

### Example 2: Characterization of an ActRIIA-hFc Protein

ActRIIA-hFc fusion protein was expressed in stably transfected CHO-DUKX B11 cells from a pAID4 vector (SV40 ori/enhancer, CMV promoter), using a tissue plasminogen leader sequence of SEQ ID NO:9. The protein, purified as described above in Example 1, had a sequence of SEQ ID NO:22. The Fc portion is a human IgG1 Fc sequence, as shown in SEQ ID NO:22. Protein analysis reveals that the ActRIIA-hFc fusion protein is formed as a homodimer with disulfide bonding.

The CHO-cell-expressed material has a higher affinity for activin B ligand than that reported for an ActRIIa-hFc fusion protein expressed in human 293 cells [see, del Re et al. (2004) J Biol Chem. 279(51):53126-53135]. Additionally, the use of the TPA leader sequence provided greater production than other leader sequences and, unlike ActRIIA-Fc expressed with a native leader, provided a highly pure N-terminal sequence. Use of the native leader sequence resulted in two major species of ActRIIA-Fc, each having a different N-terminal sequence.

### Example 3. ActRIIA-hFc Increases Red Blood Cell Levels in Non-Human Primates

The study employed four groups of five male and five female cynomolgus monkeys each, with three per sex per group scheduled for termination on Day 29, and two per sex per group scheduled for termination on Day 57. Each animal was administered the vehicle (Group 1) or ActRIIA-Fc at doses of 1, 10, or 30 mg/kg (Groups 2, 3 and 4, respectively) via intravenous (IV) injection on Days 1, 8, 15, and 22. The dose volume was maintained at 3 mL/kg. Various measures of red blood cell levels were assessed two days prior to the first administration and at days 15, 29, and 57 (for the remaining two animals) after the first administration.

The ActRIIA-hFc caused statistically significant increases in mean red blood cell parameters [red blood cell count (RBC), hemoglobin (HGB), and hematocrit (HCT)] for males and females, at all dose levels and time points throughout the study, with accompanying elevations in absolute and relative reticulocyte counts (ARTC; RTC). See Figures 5-8.

Statistical significance was calculated for each treatment group relative to the mean for the treatment group at baseline.

Notably, the increases in red blood cell counts and hemoglobin levels are roughly equivalent in magnitude to effects reported with erythropoietin. The onset of these effects is more rapid with ActRIIA-Fc than with erythropoietin.

Similar results were observed with rats and mice.

### Example 4: ActRIIA-hFc Increases Red Blood Cell Levels and Markers of Bone Formation in Human Patients

The ActRIIA-hFc fusion protein described in Example 1 was administered to human subjects in a randomized, double-blind, placebo-controlled study that was conducted to evaluate, primarily, the safety of the protein in healthy, postmenopausal women. Forty-eight subjects were randomized in cohorts of 6 to receive either a single dose of ActRIIA-hFc or placebo (5 active: 1 placebo). Dose levels ranged from 0.01 to 3.0 mg/kg intravenously (IV) and 0.03 to 0.1 mg/kg subcutaneously (SC). All subjects were followed for 120 days. In addition to pharmacokinetic (PK) analyses, the biologic activity of ActRIIA-hFc was also assessed by measurement of biochemical markers of bone formation and resorption as well as FSH levels.

To look for potential changes, hemoglobin and RBC numbers were examined in detail for all subjects over the course of the study and compared to the baseline levels. Platelet counts were compared over the same time as the control. There were no clinically significant changes from the baseline values over time for the platelet counts.

Pharmacokinetic (PK) analysis of ActRIIA-hFc revealed a linear profile with dose, and a mean half-life of approximately 25-32 days. The area-under-curve (AUC) for ActRIIA-hFc was linearly related to dose, and the absorption after SC dosing was essentially complete. See Figures 9 and 10. These data indicate that SC is a desirable approach to dosing because it provides equivalent bioavailability and serum-half life for the drug while avoiding the spike in serum concentrations of drug associated with the first few days of IV dosing (see Figure 10). ActRIIA-hFc caused a rapid, sustained dose-dependent increase in serum levels of bone-specific alkaline phosphatase (BAP), which is a marker for anabolic bone growth, and a dose-dependent decrease in C-terminal type 1 collagen telopeptide and tartrate-resistant acid phosphatase 5b levels, which are markers for bone resorption. Other markers such as P1NP showed inconclusive results. BAP levels showed near-saturating effects at the highest dosage of drug, indicating that half-maximal effects on this anabolic bone biomarker could be achieved at a dosage of 0.3 mg/kg, with increases ranging up to 3 mg/kg. Calculated as a relationship of pharmacodynamic effect to AUC for drug, the EC50 was 51,465 (day*ng/ml) (see Figure 11). These bone biomarker changes were sustained for approximately 120 days at the highest dose levels tested. There was also a dose-dependent decrease in serum FSH levels consistent with inhibition of activin.

Overall, there was a very small non-drug related reduction in hemoglobin over the first week of the study probably related to study phlebotomy in the 0.01 and 0.03 mg/kg groups whether given IV or SC. The 0.1 mg/kg SC and IV hemoglobin results were stable or showed modest increases by Day 8-15. At the 0.3 mg/kg IV dose level there was a clear increase in HGB levels seen as early as Day 2 and often peaking at Day 15-29 that was not seen in the placebo-treated subjects. At the 1.0 mg/kg IV dose and the 3.0 mg/kg IV dose, mean increases in hemoglobin of greater than 1 g/dl were observed in response to the single dose, with corresponding increases in RBC counts and hematocrit. These hematologic parameters peaked at about 60 days after the dose and substantial decrease by day 120. This indicates that dosing for the purpose of increasing red blood cell levels may be more effective if done at intervals less than 120 days (*i.e.,* prior to return to baseline), with dosing intervals of 90 days or less or 60 days or less may be desirable. For a summary of hematological changes, see Figures 12-15.

Overall, ActRIIA-hFc showed a dose-dependent effect on red blood cell counts and reticulocyte counts.

### Example 5: Treatment of an Anemic Patient with ActRIIA-hFc

A clinical study was designed to treat patients with multiple doses of ActRIIA-hFc, at three dose levels of 0.1 mg/kg, 0.3 mg/kg, and 1.0 mg/kg, with dosing to occur every 30 days. Normal healthy subjects in the trial exhibited an increase in hemoglobin and hematocrit that is consistent with the increases seen in the Phase I clinical trial reported in Example 4, except that in some instances the hemoglobin (Hg) and hematocrit (Hct) are elevated beyond the normal range. An anemic patient with hemoglobin levels of approximately 7.5 g/dL also received two doses at the 1 mg/kg level, resulting in a hemoglobin level of approximately 10.5 g/dL after two months. The patient's anemia was a microcytic anemia, thought to be caused by chronic iron deficiency.

ActRIIA-Fc fusion proteins have been further demonstrated to be effective in increasing red blood cell levels in various models of anemia including, for example, chemotherapy-induced anemia and anemia associated with chronic kidney disease (see, *e.g.,* U.S. Patent Application Publication No. 2010/0028331).

### Example 6: Alternative ActRIIA-Fc Proteins

A variety of ActRIIA variants that may be used according to the methods described herein are described in the International Patent Application published as WO2006/012627 (see e.g., pp. 55-58). An alternative construct may have a deletion of the C-terminal tail (the final 15 amino acids of the extracellular domain of ActRIIA. The sequence for such a construct is presented below (Fc portion underlined) (SEQ ID NO:28):

ILGRSETQECLFFNANWEKDRTNQTGVEPCYGDKDKRRHCFATWKNISGSIEIVKQG
CWLDDINCYDRTDCVEKKDSPEVYFCCCEGNMCNEKFSYFPEMTGGGTHTCPPCPA
PELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPVPIEKTISKAKGQPRE
PQVYTLPPSREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK

### Example 7: Generation of ActRIIB-Fc fusion proteins

Applicants constructed a soluble ActRIIB fusion protein that has the extracellular domain of human ActRIIB fused to a human or mouse Fc domain with a minimal linker (three glycine amino acids) in between. The constructs are referred to as ActRIIB-hFc and ActRIIB-mFc, respectively.

ActRIIB-hFcis shown below as purified from CHO cell lines (SEQ ID NO:29):

GRGEAETRECIYYNANWELERTNQSGLERCEGEQDKRLHCYASWRNSSGTIELVKK
GCWLDDFNCYDRQECVATEENPQVYFCCCEGNFCNERFTHLPEAGGPEVTYEPPPT
APTGGGTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKAL
PVPIEKTISKAKGQPREPQVYTLPPSREEMTKNQVSLTCLVKGFYPSDIAVEWESNGQ

PENNYKTTPPVLDSDGSFFLYSKLTVDKSRWQQGNVFSCSVMHEALHNHYTQKSLS
LSPGK

The ActRIIB-hFc and ActRIIB-mFc proteins were expressed in CHO cell lines. Three different leader sequences were considered:
(i) Honey bee mellitin (HBML): MKFLVNVALVFMVVYISYIYA (SEQ ID NO:23)
(ii) Tissue plasminogen activator (TPA): MDAMKRGLCCVLLLCGAVFVSP (SEQ ID NO:24)
(iii) Native: MGAAAKLAFAVFLISCSSGA (SEQ ID NO:30).

The selected form employs the TPA leader and has the following unprocessed amino acid sequence (SEQ ID NO: 31):

MDAMKRGLCCVLLLCGAVFVSPGASGRGEAETRECIYYNANWELERTNQSGLERCE
GEQDKRLHCYASWRNSSGTIELVKKGCWLDDFNCYDRQECVATEENPQVYFCCCE
GNFCNERFTHLPEAGGPEVTYEPPPTAPTGGGTHTCPPCPAPELLGGPSVFLFPPKPKD
TLMISRTPEVTCVVVDVSHEDPEVKFNWYVDGVEVHNAKTKPREEQYNSTYRVVSV
LTVLHQDWLNGKEYKCKVSNKALPVPIEKTISKAKGQPREPQVYTLPPSREEMTKNQ
VSLTCLVKGFYPSDIAVEWESNGQPENNYKTTPPVLDSDGSFFLYSKLTVDKSRWQQ
GNVFSCSVMHEALHNHYTQKSLSLSPGK

This polypeptide is encoded by the following nucleic acid sequence (SEQ ID NO:32):

A TGGATGCAAT GAAGAGAGGG CTCTGCTGTG TGCTGCTGCT GTGTGGAGCA
GTCTTCGTTT CGCCCGGCGC CTCTGGGCGT GGGGAGGCTG AGACACGGGA
GTGCATCTAC TACAACGCCA ACTGGGAGCT GGAGCGCACC AACCAGAGCG
GCCTGGAGCG CTGCGAAGGC GAGCAGGACA AGCGGCTGCA CTGCTACGCC
TCCTGGCGCA ACAGCTCTGG CACCATCGAG CTCGTGAAGA AGGGCTGCTG
GCTAGATGAC TTCAACTGCT ACGATAGGCA GGAGTGTGTG GCCACTGAGG
AGAACCCCCA GGTGTACTTC TGCTGCTGTG AAGGCAACTT CTGCAACGAG
CGCTTCACTC ATTTGCCAGA GGCTGGGGGC CCGGAAGTCA CGTACGAGCC
ACCCCCGACA GCCCCCACCG GTGGTGGAAC TCACACATGC CCACCGTGCC
CAGCACCTGA ACTCCTGGGG GGACCGTCAG TCTTCCTCTT CCCCCCAAAA
CCCAAGGACA CCCTCATGAT CTCCCGGACC CCTGAGGTCA CATGCGTGGT
GGTGGACGTG AGCCACGAAG ACCCTGAGGT CAAGTTCAAC TGGTACGTGG
ACGGCGTGGA GGTGCATAAT GCCAAGACAA AGCCGCGGGA GGAGCAGTAC
AACAGCACGT ACCGTGTGGT CAGCGTCCTC ACCGTCCTGC ACCAGGACTG
GCTGAATGGC AAGGAGTACA AGTGCAAGGT CTCCAACAAA GCCCTCCCAG

TCCCCATCGA GAAAACCATC TCCAAAGCCA AAGGGCAGCC CCGAGAACCA
CAGGTGTACA CCCTGCCCCC ATCCCGGGAG GAGATGACCA AGAACCAGGT
CAGCCTGACC TGCCTGGTCA AAGGCTTCTA TCCCAGCGAC ATCGCCGTGG
AGTGGGAGAG CAATGGGCAG CCGGAGAACA ACTACAAGAC CACGCCTCCC
GTGCTGGACT CCGACGGCTC CTTCTTCCTC TATAGCAAGC TCACCGTGGA
CAAGAGCAGG TGGCAGCAGG GGAACGTCTT CTCATGCTCC GTGATGCATG
AGGCTCTGCA CAACCACTAC ACGCAGAAGA GCCTCTCCCT GTCTCCGGGT
AAATGA

N-terminal sequencing of the CHO-cell-produced material revealed a major sequence of-GRGEAE (SEQ ID NO:33). Notably, other constructs reported in the literature begin with an -SGR... sequence.

Purification could be achieved by a series of column chromatography steps, including, for example, three or more of the following, in any order: protein A chromatography, Q sepharose chromatography, phenylsepharose chromatography, size exclusion chromatography, and cation exchange chromatography. The purification could be completed with viral filtration and buffer exchange.

ActRIIB-Fc fusion proteins were also expressed in HEK293 cells and COS cells. Although material from all cell lines and reasonable culture conditions provided protein with muscle-building activity *in vivo,* variability in potency was observed perhaps relating to cell line selection and/or culture conditions.

Applicants generated a series of mutations in the extracellular domain of ActRIIB and produced these mutant proteins as soluble fusion proteins between extracellular ActRIIB and an Fc domain. The background ActRIIB-Fc fusion has the sequence of SEQ ID NO:29.

Various mutations, including N- and C-terminal truncations, were introduced into the background ActRIIB-Fc protein. Based on the data presented in Example 1, it is expected that these constructs, if expressed with a TPA leader, will lack the N-terminal serine. Mutations were generated in ActRIIB extracellular domain by PCR mutagenesis. After PCR, fragments were purified through a Qiagen column, digested with SfoI and AgeI and gel purified. These fragments were ligated into expression vector pAID4 (see WO2006/012627) such that upon ligation it created fusion chimera with human IgG1. Upon transformation into E. coli DH5 alpha, colonies were picked and DNAs were isolated. For murine constructs (mFc), a murine IgG2a was substituted for the human IgG1. Sequences of all mutants were verified.

All of the mutants were produced in HEK293T cells by transient transfection. In summary, in a 500ml spinner, HEK293T cells were set up at 6x10⁵ cells/ml in Freestyle (Invitrogen) media in 250ml volume and grown overnight. Next day, these cells were treated with DNA:PEI (1:1) complex at 0.5 ug/ml final DNA concentration. After 4 hrs, 250 ml media was added and cells were grown for 7 days. Conditioned media was harvested by spinning down the cells and concentrated.

Mutants were purified using a variety of techniques, including, for example, a protein A column, and eluted with low pH (3.0) glycine buffer. After neutralization, these were dialyzed against PBS.

Mutants were also produced in CHO cells by similar methodology. Mutants were tested in binding assays and/or bioassays described in WO 2008/097541 and WO 2006/012627. In some instances, assays were performed with conditioned medium rather than purified proteins. Additional variations of ActRIIB are described in U.S. Patent No. 7,842,663.

### Example 8: ActRIIB-Fc Stimulates Erythropoiesis in Non-Human Primates

Cynomolgus monkeys were allocated into seven groups (6/sex/group) and administered ActRIIB(20-134)-hFc as a subcutaneous injection at dosages of 0.6, 3, or 15 mg/kg every 2 weeks or every 4 weeks over a 9-month period. The control group (6/sex/group) received the vehicle at the same dose volume (0.5 ml/kg/dose) as ActRIIB(20-134)-hFc-treated animals. Animals were monitored for changes in general clinical pathology parameters (*e.g*., hematology, clinical chemistry, coagulation, and urinalysis). Hematology, coagulation, and clinical chemistry parameters (including iron parameters, lipase, and amylase) were evaluated twice prior to initiation of dosing and on Days 59, 143, 199, 227, and on Days 267 (for groups dosed every 4 weeks) or 281 (for groups dosed every 2 weeks). The evaluations on Days 267/281 occurred 2 weeks after the final dose was administered.

Administration of ActRIIB(20-134)-hFc resulted in non-adverse, dose-related changes to hematology parameters in male and female monkeys. These changes included increased red blood cell count, reticulocyte count and red cell distribution width and decreased mean corpuscular volume, mean corpuscular hemoglobin, and platelet count. In males, RBC count was increased at all dose levels, and the magnitude of increase was generally comparable whether ActRIIB(20-134)-hFc was administered every 2 weeks or every 4 weeks. Mean RBC count was increased at all time points between Days 59 and 267/281 (except RBC count was not increased in group 2 males [0.6 mg/kg every 2 weeks] on Day 281). In females, RBC count was increased at ≥ 3 mg/kg every 2 weeks and the changes occurred between Days 143 and 281; at 15 mg/kg every 4 weeks mean RBC count was increased between Days 59 and 267.

These effects are consistent with a positive effect of ActRIIB(20-134)-hFc on stimulating erythropoiesis.

### Example 9: Generation of a GDF Trap

Applicants constructed a GDF trap as follows. A polypeptide having a modified extracellular domain of ActRIIB (amino acids 20-134 of SEQ ID NO: 1 with an L79D substitution) with greatly reduced activin A binding relative to GDF11 and/or myostatin (as a consequence of a leucine-to-aspartate substitution at position 79 in SEQ ID NO:1) was fused to a human or mouse Fc domain with a minimal linker (three glycine amino acids) in between. The constructs are referred to as ActRIIB(L79D 20-134)-hFc and ActRIIB(L79D 20-134)-mFc, respectively. Alternative forms with a glutamate rather than an aspartate at position 79 performed similarly (L79E). Alternative forms with an alanine rather than a valine at position 226 with respect to SEQ ID NO:36, below were also generated and performed equivalently in all respects tested. The aspartate at position 79 (relative to SEQ ID NO: 1, or position 60 relative to SEQ ID NO:36) is indicated with double underlining below. The valine at position 226 relative to SEQ ID NO:36 is also indicated by double underlining below.

The GDF trap ActRIIB(L79D 20-134)-hFc is shown below as purified from CHO cell lines (SEQ ID NO:36).

GRGEAETRECIYYNANWELERTNQSGLERCEGEQDKRLHCYASWRNSSGTIELVKK
GCWDDDFNCYDRQECVATEENPQVYFCCCEGNFCNERFTHLPEAGGPEVTYEPPPT
APTGGGTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKAL
PVPIEKTISKAKGQPREPQVYTLPPSREEMTKNQVSLTCLVKGFYPSDIAVEWESNGQ
PENNYKTTPPVLDSDGSFFLYSKLTVDKSRWQQGNVFSCSVMHEALHNHYTQKSLS
LSPGK

The ActRIIB-derived portion of the GDF trap has an amino acid sequence set forth below (SEQ ID NO: 37), and that portion could be used as a monomer or as a non-Fc fusion protein as a monomer, dimer, or greater-order complex.

GRGEAETRECIYYNANWELERTNQSGLERCEGEQDKRLHCYASWRNSSGTIELVKK
GCWDDDFNCYDRQECVATEENPQVYFCCCEGNFCNERFTHLPEAGGPEVTYEPPPT
APT (SEQ ID NO: 37)

The GDF trap protein was expressed in CHO cell lines. Three different leader sequences were considered:
(i) Honey bee melittin (HBML): MKFLVNVALVFMVVYISYIYA (SEQ ID NO:23)
(ii) Tissue plasminogen activator (TPA): MDAMKRGLCCVLLLCGAVFVSP (SEQ ID NO:24)
(iii) Native: MTAPWVALALLWGSLCAGS (SEQ ID NO:30).

The selected form employs the TPA leader and has the following unprocessed amino acid sequence:

MDAMKRGLCCVLLLCGAVFVSPGASGRGEAETRECIYYNANWELERTNQSGLERCE
GEQDKRLHCYASWRNSSGTIELVKKGCWDDDFNCYDRQECVATEENPQVYFCCCE
GNFCNERFTHLPEAGGPEVTYEPPPTAPTGGGTHTCPPCPAPELLGGPSVFLFPPKPKD
TLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVDKSRWQQ
GNVFSCSVMHEALHNHYTQKSLSLSPGK (SEQ ID NO:38)

This polypeptide is encoded by the following nucleic acid sequence (SEQ ID NO:39):

A TGGATGCAAT GAAGAGAGGG CTCTGCTGTG TGCTGCTGCT GTGTGGAGCA
GTCTTCGTTT CGCCCGGCGC CTCTGGGCGT GGGGAGGCTG AGACACGGGA
GTGCATCTAC TACAACGCCA ACTGGGAGCT GGAGCGCACC AACCAGAGCG
GCCTGGAGCG CTGCGAAGGC GAGCAGGACA AGCGGCTGCA CTGCTACGCC
TCCTGGCGCA ACAGCTCTGG CACCATCGAG CTCGTGAAGA AGGGCTGCTG
GGACGATGAC TTCAACTGCT ACGATAGGCA GGAGTGTGTG GCCACTGAGG
AGAACCCCCA GGTGTACTTC TGCTGCTGTG AAGGCAACTT CTGCAACGAG
CGCTTCACTC ATTTGCCAGA GGCTGGGGGC CCGGAAGTCA CGTACGAGCC
ACCCCCGACA GCCCCCACCG GTGGTGGAAC TCACACATGC CCACCGTGCC
CAGCACCTGA ACTCCTGGGG GGACCGTCAG TCTTCCTCTT CCCCCCAAAA
CCCAAGGACA CCCTCATGAT CTCCCGGACC CCTGAGGTCA CATGCGTGGT
GGTGGACGTG AGCCACGAAG ACCCTGAGGT CAAGTTCAAC TGGTACGTGG
ACGGCGTGGA GGTGCATAAT GCCAAGACAA AGCCGCGGGA GGAGCAGTAC
AACAGCACGT ACCGTGTGGT CAGCGTCCTC ACCGTCCTGC ACCAGGACTG

GCTGAATGGC AAGGAGTACA AGTGCAAGGT CTCCAACAAA GCCCTCCCAG
TCCCCATCGA GAAAACCATC TCCAAAGCCA AAGGGCAGCC CCGAGAACCA
CAGGTGTACA CCCTGCCCCC ATCCCGGGAG GAGATGACCA AGAACCAGGT
CAGCCTGACC TGCCTGGTCA AAGGCTTCTA TCCCAGCGAC ATCGCCGTGG
AGTGGGAGAG CAATGGGCAG CCGGAGAACA ACTACAAGAC CACGCCTCCC
GTGCTGGACT CCGACGGCTC CTTCTTCCTC TATAGCAAGC TCACCGTGGA
CAAGAGCAGG TGGCAGCAGG GGAACGTCTT CTCATGCTCC GTGATGCATG
AGGCTCTGCA CAACCACTAC ACGCAGAAGA GCCTCTCCCT GTCTCCGGGT
AAATGA

Purification could be achieved by a series of column chromatography steps, including, for example, three or more of the following, in any order: protein A chromatography, Q sepharose chromatography, phenylsepharose chromatography, size exclusion chromatography, and cation exchange chromatography. The purification could be completed with viral filtration and buffer exchange. In an example of a purification scheme, the cell culture medium is passed over a protein A column, washed in 150 mM Tris/NaCl (pH 8.0), then washed in 50 mM Tris/NaCl (pH 8.0) and eluted with 0.1 M glycine, pH 3.0. The low pH eluate is kept at room temperature for 30 minutes as a viral clearance step. The eluate is then neutralized and passed over a Q-sepharose ion-exchange column and washed in 50 mM Tris pH 8.0, 50 mM NaCl, and eluted in 50 mM Tris pH 8.0, with an NaCl concentration between 150 mM and 300 mM. The eluate is then changed into 50 mM Tris pH 8.0, 1.1 M ammonium sulfate and passed over a phenyl sepharose column, washed, and eluted in 50 mM Tris pH 8.0 with ammonium sulfate between 150 and 300 mM. The eluate is dialyzed and filtered for use.

Additional GDF traps (ActRIIB-Fc fusion proteins modified so as to reduce the ratio of activin A binding relative to myostatin or GDF11 binding) are described in WO 2008/097541 and WO 2006/012627.

### Example 10: Bioassav for GDF-11- and Activin-Mediated Signaling

An A-204 reporter gene assay was used to evaluate the effects of ActRIIB-Fc proteins and GDF traps on signaling by GDF-11 and activin A. Cell line: human rhabdomyosarcoma (derived from muscle). Reporter vector: pGL3(CAGA)12 (described in Dennler et al, 1998, EMBO 17: 3091-3100). The CAGA12 motif is present in TGF-beta responsive genes (*e.g.,* PAI-1 gene), so this vector is of general use for factors signaling through SMAD2 and 3.

Day 1: Split A-204 cells into 48-well plate.

Day 2: A-204 cells transfected with 10 ug pGL3(CAGA)12 or pGL3(CAGA)12(10 ug) + pRLCMV (1 µg) and Fugene.

Day 3: Add factors (diluted into medium + 0.1 % BSA). Inhibitors need to be preincubated with factors for 1 hr before adding to cells. Six hrs later, cells were rinsed with PBS and lysed.

This is followed by a luciferase assay. In the absence of any inhibitors, activin A showed 10-fold stimulation of reporter gene expression and an ED50 ∼ 2 ng/ml. GDF-11: 16 fold stimulation, ED50: ∼ 1.5 ng/ml.

ActRIIB(20-134) is a potent inhibitor of activin A, GDF-8, and GDF-11 activity in this assay. As described below, ActRIIB variants were also tested in this assay.

### Example 11: ActRIIB-Fc Variants, Cell-Based Activity

Activity of ActRIIB-Fc proteins and GDF traps was tested in a cell-based assay as described above. Results are summarized in the table below. Some variants were tested in different C-terminal truncation constructs. As discussed above, truncations of five or fifteen amino acids caused reduction in activity. The GDF traps (L79D and L79E variants) showed substantial loss of activin A inhibition while retaining almost wild-type inhibition of GDF-11.

### Soluble ActRIIB-Fc binding to GDF11 and Activin A:

| ActRIIB-Fc Variations | Portion of ActRIIB (corresponds to amino acids of SEQ ID NO:1) | GDF11 Inhibition Activity | Activin Inhibition Activity |
|---|---|---|---|
| R64 | 20-134 | +++ (approx. 10⁻⁸ M K_{I}) | +++ (approx. 10⁻⁸ M K_{I}) |
| A64 | 20-134 | + (approx. 10⁻⁶ M K_{I}) | + (approx. 10⁻⁶ M K_{I}) |
| R64 | 20-129 | +++ | +++ |
| R64 K74A | 20-134 | ++++ | ++++ |
| R64 A24N | 20-134 | +++ | +++ |
| R64 A24N | 20-119 | ++ | ++ |
| R64 A24N K74A | 20-119 | + | + |
| R64 L79P | 20-134 | + | + |
| R64 L79P K74A | 20-134 | + | + |
| R64 L79D | 20-134 | +++ | + |
| R64 L79E | 20-134 | +++ | + |
| R64K | 20-134 | +++ | +++ |
| R64K | 20-129 | +++ | +++ |
| R64 P129S P130A | 20-134 | +++ | +++ |
| R64N | 20-134 | + | + |

| | | | |
|---|---|---|---|
| + Poor activity (roughly 1x10⁻⁶ K_{I}) ++ Moderate activity (roughly 1x10⁻⁷ K_{I}) +++ Good (wild-type) activity (roughly 1x10⁻⁸ K_{I}) ++++ Greater than wild-type activity | | | |

Several variants have been assessed for serum half-life in rats. ActRIIB(20-134)-Fc has a serum half-life of approximately 70 hours. ActRIIB(A24N 20-134)-Fc has a serum half-life of approximately 100-150 hours. The A24N variant has activity in the cell-based assay (above) and *in vivo* assays (below) that is equivalent to the wild-type molecule. Coupled with the longer half-life, this means that over time an A24N variant will give greater effect per unit of protein than the wild-type molecule. The A24N variant, and any of the other variants tested above, may be combined with the GDF trap molecules, such as the L79D or L79E variants.

### Example 12: GDF-11 and Activin A Binding.

Binding of certain ActRIIB-Fc proteins and GDF traps to ligands was tested in a Biacore™ assay.

The ActRIIB-Fc variants or wild-type protein were captured onto the system using an anti-hFc antibody. Ligands were injected and flowed over the captured receptor proteins. Results are summarized in the tables below.

### Ligand-binding specificity IIB variants.

| | **GDF11** | | |
|---|---|---|---|
| **Protein** | **Kon (1/Ms)** | **Koff (1/s)** | **KD (M)** |
| ActRIIB(20-134)-hFc | 1.34e-6 | 1.13e-4 | 8.42e-11 |
| ActRIIB(A24N 20-134)-hFc | 1.21e-6 | 6.35e-5 | 5.19e-11 |
| ActRIIB(L79D 20-134)-hFc | 6.7e-5 | 4.39e-4 | 6.55e-10 |
| ActRIIB(L79E 20-134)-hFc | 3.8e-5 | 2.74e-4 | 7.16e-10 |
| ActRIIB(R64K 20-134)-hFc | 6.77e-5 | 2.41e-5 | 3.56e-11 |
| | | | |

| | **GDF8** | | |
|---|---|---|---|
| **Protein** | **Kon (1/Ms)** | **Koff (1/s)** | **KD (M)** |
| ActRIIB(20-134)-hFc | 3.69e-5 | 3.45e-5 | 9.35e-11 |
| ActRIIB(A24N 20-134)-hFc | | | |
| ActRIIB(L79D 20-134)-hFc | 3.85e-5 | 8.3e-4 | 2.15e-9 |
| ActRIIB(L79E 20-134)-hFc | 3.74e-5 | 9e-4 | 2.41e-9 |
| ActRIIB(R64K 20-134)-hFc | 2.25e-5 | 4.71e-5 | 2.1e-10 |
| ActRIIB(R64K 20-129)-hFc | 9.74e-4 | 2.09e-4 | 2.15e-9 |
| ActRIIB(P129S, P130R 20-134)-hFc | 1.08e-5 | 1.8e-4 | 1.67e-9 |
| ActRIIB(K74A 20-134)-hFc | 2.8e-5 | 2.03e-5 | 7.18e-11 |
| | | | |

| | Activin A | | |
|---|---|---|---|
| **Protein** | **Kon (1/Ms)** | **Koff (1/s)** | **KD (M)** |
| ActRIIB(20-134)-hFc | 5.94e6 | 1.59e-4 | 2.68e-11 |
| ActRIIB(A24N 20-134)-hFc | 3.34e6 | 3.46e-4 | 1.04e-10 |
| ActRIIB(L79D 20-134)-hFc | | | Low binding |
| ActRIIB(L79E 20-134)-hFc | | | Low binding |
| ActRIIB(R64K 20-134)-hFc | 6.82e6 | 3.25e-4 | 4.76e-11 |
| ActRIIB(R64K 20-129)-hFc | 7.46e6 | 6.28e-4 | 8.41e-11 |
| ActRIIB(P129S, P130R 20-134)-hFc | 5.02e6 | 4.17e-4 | 8.31e-11 |

These data obtained in a cell-free assay confirm the cell-based assay data, demonstrating that the A24N variant retains ligand-binding activity that is similar to that of the ActRIIB(20-134)-hFc molecule and that the L79D or L79E molecule retains myostatin and GDF11 binding but shows markedly decreased (non-quantifiable) binding to activin A.

Other variants have been generated and tested, as reported in WO2006/012627. See, *e.g.,* pp. 59-60, using ligands coupled to the device and flowing receptor over the coupled ligands. Notably, K74Y, K74F, K74I (and presumably other hydrophobic substitutions at K74, such as K74L), and D80I, cause a decrease in the ratio of activin A (ActA) binding to GDF11 binding, relative to the wild-type K74 molecule. A table of data with respect to these variants is reproduced below:

### Soluble ActRIIB-Fc variants binding to GDF11 and Activin A (Biacore™ assay)

| **ActRIIB** | **ActA** | **GDF11** |
|---|---|---|
| WT (64A) | KD=1.8e-7M (+) | KD= 2.6e-7M (+) |
| WT (64R) | na | KD= 8.6e-8M (+++) |
| +15tail | KD ∼2.6 e-8M (+++) | KD= 1.9e-8M (++++) |
| E37A | * | * |
| R40A | - | - |
| D54A | - | * |
| K55A | ++ | * |
| R56A | * | * |
| K74A | KD=4.35e-9 M | KD=5.3e-9M |
| K74Y | * | - |
| K74F | * | - |
| K74I | * | - |
| W78A | * | * |
| L79A | + | * |
| D80K | * | * |
| D80R | * | * |
| D80A | * | * |
| D80F | * | * |
| D80G | * | * |
| D80M | * | * |
| D80N | * | * |
| D80I | * | - |
| F82A | ++ | - |

| | | |
|---|---|---|
| * No observed binding -- < 1/5 WT binding - ∼ 1/2 WT binding + WT ++ < 2x increased binding +++ ∼5x increased binding ++++ ∼10x increased binding +++++ ∼ 40x increased binding | | |

### Example 13: A GDF Trap Increases Red Blood Cell Levels in vivo

Twelve-week-old male C57BL/6NTac mice were assigned to one of two treatment groups (N=10). Mice were dosed with either vehicle or with a variant ActRIIB polypeptide ("GDF trap") [ActRIIB(L79D 20-134)-hFc] by subcutaneous injection (SC) at 10 mg/kg twice per week for 4 weeks. At study termination, whole blood was collected by cardiac puncture into EDTA containing tubes and analyzed for cell distribution using an HM2 hematology analyzer (Abaxis, Inc).

### Group Designation

| **Group** | **N** | **Mice** | **Injection** | **Dose** (mg/kg) | **Route** | **Frequency** |
|---|---|---|---|---|---|---|
| 1 | 10 | C57BL/6 | PBS | 0 | SC | Twice/week |
| 2 | 10 | C57BL/6 | GDF trap [ActRIIB(L79D 20-134)-hFc] | 10 | SC | Twice/week |

Treatment with the GDF trap did not have a statistically significant effect on the number of white blood cells (WBC) compared to the vehicle controls. Red blood cell (RBC) numbers were increased in the treated group relative to the controls (see table below). Both the hemoglobin content (HGB) and hematocrit (HCT) were also increased due to the additional red blood cells. The average width of the red blood cells (RDWc) was higher in the treated animals, indicating an increase in the pool of immature red blood cells. Therefore, treatment with the GDF trap leads to increases in red blood cells, with no distinguishable effects on white blood cell populations.

### Hematology Results

| | **RBC 10¹²/L** | **HGB (g/dL)** | **HCT (%)** | **RDWc (%)** |
|---|---|---|---|---|
| **PBS** | 10.7 ± 0.1 | 14.8 ± 0.6 | 44.8 ± 0.4 | 17.0 ± 0.1 |
| **GDF trap** | 12.4 ± 0.4** | 17.0 ± 0.7* | 48.8 ± 1.8* | 18.4 ± 0.2** |

| | | | | |
|---|---|---|---|---|
| *=p<0.05, **= p<0.01 | | | | |

### Example 14: A GDF Trap is Superior to ActRIIB-Fc for Increasing Red Blood Cell Levels in vivo.

Nineteen-week-old male C57BL/6NTac mice were randomly assigned to one of three groups. Mice were dosed with vehicle (10 mM Tris-buffered saline, TBS), wild-type ActRIIB(20-134)-mFc, or GDF trap ActRIIB(L79D 20-134)-hFc by subcutaneous injection twice per week for three weeks. Blood was collected by cheek bleed at baseline and after three weeks of dosing and analyzed for cell distribution using a hematology analyzer (HM2, Abaxis, Inc.)

Treatment with ActRIIB-Fc or the GDF trap did not have a significant effect on white blood cell (WBC) numbers compared to vehicle controls. The red blood cell count (RBC), hematocrit (HCT), and hemoglobin levels were all elevated in mice treated with GDF trap compared to either the controls or the wild-type construct (see table below). Therefore, in a direct comparison, the GDF trap promotes increases in red blood cells to a significantly greater extent than a wild-type ActRIIB-Fc protein. In fact, in this experiment, the wild-type ActRIIB-Fc protein did not cause a statistically significant increase in red blood cells, suggesting that longer or higher dosing would be needed to reveal this effect.

### Hematology Results after three weeks of dosing

| | **RBC (10¹²/ml)** | **HCT %** | **HGB g/dL** |
|---|---|---|---|
| **TBS** | 11.06 ± 0.46 | 46.78 ± 1.9 | 15.7 ± 0.7 |
| **ActRIIB-mFc** | 11.64 ± 0.09 | 49.03 ± 0.3 | 16.5 ± 1.5 |
| **GDF trap** | 13.19 ± 0.2** | 53.04 ±0.8** | 18.4 ± 0.3** |

| | | | |
|---|---|---|---|
| **=p<0.01 | | | |

### Example 15: Generation of a GDF Trap with Truncated ActRIIB Extracellular Domain

As described in Example 9, a GDF trap referred to as ActRIIB(L79D 20-134)-hFc was generated by N-terminal fusion of TPA leader to the ActRIIB extracellular domain (residues 20-134 in SEQ ID NO:1) containing a leucine-to-aspartate substitution (at residue 79 in SEQ ID NO:1) and C-terminal fusion of human Fc domain with minimal linker (three glycine residues) (Figure 16). A nucleotide sequence corresponding to this fusion protein is shown in Figure 17.

A GDF trap with truncated ActRIIB extracellular domain, referred to as ActRIIB(L79D 25-131)-hFc, was generated by N-terminal fusion of TPA leader to truncated extracellular domain (residues 25-131 in SEQ ID NO:1) containing a leucine-to-aspartate substitution (at residue 79 in SEQ ID NO:1) and C-terminal fusion of human Fc domain with minimal linker (three glycine residues) (Figure 18). A nucleotide sequence corresponding to this fusion protein is shown in Figure 19.

### Example 16: Selective Ligand Binding by GDF Trap with Double-Truncated ActRIIB Extracelluar Domain

The affinity of GDF traps and other ActRIIB-hFc proteins for several ligands was evaluated *in vitro* with a Biacore™ instrument. Results are summarized in the table below. Kd values were obtained by steady-state affinity fit due to the very rapid association and dissociation of the complex, which prevented accurate determination of kₒₙ and k_{off}.

### Ligand Selectivity of ActRIIB-hFc Variants:

| **Fusion Construct** | **Activin A (Kd e-11)** | **Activin B (Kd e-11)** | **GDF11 (Kd e-11)** |
|---|---|---|---|
| ActRIIB(L79 20-134)-hFc | 1.6 | 1.2 | 3.6 |
| ActRIIB(L79D 20-134)-hFc | 1350.0 | 78.8 | 12.3 |
| ActRIIB(L79 25-131)-hFc | 1.8 | 1.2 | 3.1 |
| ActRIIB(L79D 25-131)-hFc | 2290.0 | 62.1 | 7.4 |

The GDF trap with a truncated extracellular domain, ActRIIB(L79D 25-131)-hFc, equaled or surpassed the ligand selectivity displayed by the longer variant, ActRIIB(L79D 20-134)-hFc, with pronounced loss of activin A binding, partial loss of activin B binding, and nearly full retention of GDF11 binding compared to ActRIIB-hFc counterparts lacking the L79D substitution. Note that truncation alone (without L79D substitution) did not alter selectivity among the ligands displayed here [compare ActRIIB(L79 25-131)-hFc with ActRIIB(L79 20-134)-hFc].

### Example 17: Generation of ActRIIB(L79D 25-131)-hFc with Alternative Nucleotide Sequences

To generate ActRIIB(L79D 25-131)-hFc, the human ActRIIB extracellular domain with an aspartate substitution at native position 79 (SEQ ID NO:1) and with N-terminal and C-terminal truncations (residues 25-131 in SEQ ID NO: 1) was fused N-terminally with a TPA leader sequence instead of the native ActRIIB leader and C-terminally with a human Fc domain via a minimal linker (three glycine residues) (Figure 18). One nucleotide sequence encoding this fusion protein is shown in Figure 19 (SEQ ID NO: 42), and an alternative nucleotide sequence encoding exactly the same fusion protein is shown in Figure 22 (SEQ ID NO: 46). This protein was expressed and purified using the methodology described in Example 9.

### Example 18: GDF Trap with a Truncated ActRIIB Extracellular Domain Increases Proliferation of Erythroid Progenitors in Mice

ActRIIB(L79D 25-131)-hFc was evaluated to determine its effect on proliferation of erythroid progenitors. Male C57BL/6 mice (8 weeks old) were treated with ActRIIB(L79D 25-131)-hFc (10 mg/kg, s.c.; n = 6) or vehicle (TBS; n = 6) on Days 1 and 4, then euthanized on Day 8 for collection of spleens, tibias, femurs, and blood. Cells of the spleen and bone marrow were isolated, diluted in Iscove's modified Dulbecco's medium containing 5% fetal bovine serum, suspended in specialized methylcellulose-based medium, and cultured for either 2 or 12 days to assess levels of clonogenic progenitors at the colony-forming unit-erythroid (CFU-E) and burst forming unit-erythroid (BFU-E) stages, respectively. Methylcellulose-based medium for BFU-E determination (MethoCult M3434, Stem Cell Technologies) included recombinant murine stem cell factor, interleukin-3, and interleukin-6, which were not present in methylcellulose medium for CFU-E determination (MethoCult M3334, Stem Cell Technologies), while both media contained erythropoietin, among other constituents. For both BFU-E and CFU-E, the number of colonies were determined in duplicate culture plates derived from each tissue sample, and statistical analysis of the results was based on the number of mice per treatment group.

Spleen-derived cultures from mice treated with ActRIIB(L79D 25-131)-hFc had twice the number of CFU-E colonies as did corresponding cultures from control mice (P < 0.05), whereas the number of BFU-E colonies did not differ significantly with treatment *in vivo.* The number of CFU-E or BFU-E colonies from bone marrow cultures also did not differ significantly with treatment. As expected, increased numbers of CFU-E colonies in spleen-derived cultures were accompanied by highly significant (P < 0.001) changes in red blood cell level (11.6% increase), hemoglobin concentration (12% increase), and hematocrit level (11.6% increase) at euthanasia in mice treated with ActRIIB(L79D 25-131)-hFc compared to controls. These results indicate that *in vivo* administration of a GDF trap with truncated ActRIIB extracellular domain can stimulate proliferation of erythroid progenitors as part of its overall effect to increase red blood cell levels.

GDF trap fusion proteins have been further demonstrated to be effective in increasing red blood cell levels in various models of anemia including, for example, chemotherapy-induced anemia, nephrectomy-induced anemia, and in a blood loss anemia (see, e.g., International Patent Application Publication No. WO 2010/019261).

### Example 19: GDF Trap with Truncated ActRIIB Extracellular Domain Increases Levels of Red Blood Cells in Non-Human Primates

Two GDF Traps, ActRIIB(L79D 20-134)-hFc and ActRIIB(L79D 25-131)-hFc, were evaluated for their ability to stimulate red blood cell production in cynomolgus monkeys. Monkeys were treated subcutaneously with GDF trap (10 mg/kg; n = 4 males/4 females), or vehicle (n = 2 males/2 females) on Days 1 and 8. Blood samples were collected on Days 1 (pretreatment baseline), 3, 8, 15, 29, and 44, and were analyzed for red blood cell levels (Figure 24), hematocrit (Figure 25), hemoglobin levels (Figure 26), and reticulocyte levels (Figure 27). Vehicle-treated monkeys exhibited decreased levels of red blood cells, hematocrit, and hemoglobin at all post-treatment time points, an expected effect of repeated blood sampling. In contrast, treatment with ActRIIB(L79D 20-134)-hFc or ActRIIB(L79D 25-13 1)-hFc increased these parameters by the first post-treatment time point (Day 3) and maintained them at substantially elevated levels for the duration of the study (Figures 24-26). Importantly, reticulocyte levels in monkeys treated with ActRIIB(L79D 20-134)-hFc or ActRIIB(L79D 25-13 1)-hFc were substantially increased at Days 8, 15, and 29 compared to vehicle (Figure 27). This result demonstrates that GDF trap treatment increased production of red blood cell precursors, resulting in elevated red blood cell levels.

Taken together, these data demonstrate that truncated GDF traps, as well as a full-length variants, can be used as selective antagonists of GDF11 and potentially related ligands to increase red blood cell formation *in vivo.*

### Example 20: GDF Trap Derived from ActRIIB5

Others have reported an alternate, soluble form of ActRIIB (designated ActRIIB5), in which exon 4, including the ActRIIB transmembrane domain, has been replaced by a different C-terminal sequence (see, *e.g.,* WO 2007/053775).

The sequence of native human ActRIIB5 without its leader is as follows:

GRGEAETRECIYYNANWELERTNQSGLERCEGEQDKRLHCYASWRNSSGTIELVK
KGCWLDDFNCYDRQECVATEENPQVYFCCCEGNFCNERFTHLPEAGGPEGPWAST
TIPSGGPEATAAAGDQGSGALWLCLEGPAHE (SEQ ID NO:49)

An leucine-to-aspartate substitution, or other acidic substitutions, may be performed at native position 79 (underlined) as described to construct the variant ActRIIB5(L79D), which has the following sequence:

GRGEAETRECIYYNANWELERTNQSGLERCEGEQDKRLHCYASWRNSSGTIELVK
KGCWDDDFNCYDRQECVATEENPQVYFCCCEGNFCNERFTHLPEAGGPEGPWAST
TIPSGGPEATAAAGDQGSGALWLCLEGPAHE (SEQ ID NO:50)

This variant may be connected to human Fc (double underline) with a TGGG linker (single underline) to generate a human ActRIIB5(L79D)-hFc fusion protein with the following sequence:

GRGEAETRECIYYNANWELERTNQSGLERCEGEQDKRLHCYASWRNSSGTIELVK
KGCWDDDFNCYDRQECVATEENPQVYFCCCEGNFCNERFTHLPEAGGPEGPWAST
TIPSGGPEATAAAGDQGSGALWLCLEGPAHETGGGTHTCPPCPAPELLGGPSVFL
FPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLP
PSREEMTKNQVSLTCLVKGFYPSDIAVEWESNGQPENNYKTTPPVLDSDGSFFLY
SKLTVDKSRWQQGNVFSCSVMHEALHNHYTQKSLSLSPGK (SEQ ID NO:51).

This construct may be expressed in CHO cells.

### Example 21: Effects in Mice of Combined Treatment with EPO and a GDF Trap with a Truncated ActRIIB Extracellular Domain

EPO induces formation of red blood cells by increasing the proliferation of erythroid precursors, whereas GDF traps could potentially affect formation of red blood cells in ways that complement or enhance EPO's effects. Therefore, Applicants investigated the effect of combined treatment with EPO and ActRIIB(L79D 25-131)-hFc on erythropoietic parameters. Male C57BL/6 mice (9 weeks old) were given a single i.p. injection of recombinant human EPO alone (epoetin alfa, 1800 units/kg), ActRIIB(L79D 25-131)-hFc alone (10 mg/kg), both EPO and ActRIIB(L79D 25-131)-hFc, or vehicle (Tris-buffered saline). Mice were euthanized 72 h after dosing for collection of blood, spleens, and femurs.

Spleens and femurs were processed to obtain erythroid precursor cells for flow cytometric analysis. After removal, the spleen was minced in Iscove's modified Dulbecco's medium containing 5% fetal bovine serum and mechanically dissociated by pushing through a 70-µm cell strainer with the plunger from a sterile 1-mL syringe. Femurs were cleaned of any residual muscle or connective tissue and ends were trimmed to permit collection of marrow by flushing the remaining shaft with Iscove's modified Dulbecco's medium containing 5% fetal bovine serum through a 21-gauge needle connected to a 3-mL syringe. Cell suspensions were centrifuged (2000 rpm for 10 min) and the cell pellets resuspended in PBS containing 5% fetal bovine serum. Cells (10⁶) from each tissue were incubated with anti-mouse IgG to block nonspecific binding, then incubated with fluorescently labeled antibodies against mouse cell-surface markers CD71 (transferrin receptor) and Ter119 (an antigen associated with cell-surface glycophorin A), washed, and analyzed by flow cytrometry. Dead cells in the samples were excluded from analysis by counterstaining with propidium iodide. Erythroid differentiation in spleen or bone marrow was assessed by the degree of CD71 labeling, which decreases over the course of differentiation, and Ter119 labeling, which is increased during terminal erythroid differentiation beginning with the proerythroblast stage (Socolovsky et al., 2001, Blood 98:3261-3273; Ying et al., 2006, Blood 108:123-133). Thus, flow cytometry was used to determine the number of proerythroblasts (CD71^{high}Ter119^{low}), basophilic erythroblasts (CD71^{high}Ter119^{high}), polychromatophilic + orthochromatophilic erythroblasts (CD71^{med}Ter119^{high}), and late orthochromatophilic erythroblasts + reticulocytes (CD71^{low}Ter119^{high}), as described.

Combined treatment with EPO and ActRIIB(L79D 25-131)-hFc led to a surprisingly vigorous increase in red blood cells. In the 72-h time frame of this experiment, neither EPO nor ActRIIB(L79D 25-131)-hFc alone increased hematocrit significantly compared to vehicle, whereas combined treatment with the two agents led to a nearly 25% increase in hematocrit that was unexpectedly synergistic, i.e., greater than the sum of their separate effects (Figure 28). Synergy of this type is generally considered evidence that individual agents are acting through different cellular mechanisms. Similar results were also observed for hemoglobin concentrations (Figure 29) and red blood cell concentrations (Figure 30), each of which was also increased synergistically by combined treatment.

Analysis of erythroid precursor levels revealed a more complex pattern. In the mouse, the spleen is considered the primary organ responsible for inducible ("stress") erythropoiesis. Flow cytometric analysis of splenic tissue at 72 h revealed that EPO markedly altered the erythropoietic precursor profile compared to vehicle, increasing the number of basophilic erythroblasts by more than 170% at the expense of late precursors (late orthochromatophilic erythroblasts + reticulocytes), which decreased by more than one third (Figure 31). Importantly, combined treatment increased basophilic erythroblasts significantly compared to vehicle, but to a lesser extent than EPO alone, while supporting undiminished maturation of late-stage precursors (Figure 31). Thus, combined treatment with EPO and ActRIIB(L79D 25-131)-hFc increased erythropoiesis through a balanced enhancement of precursor proliferation and maturation. In contrast to spleen, the precursor cell profile in bone marrow after combined treatment did not differ appreciably from that after EPO alone. Applicants predict from the splenic precursor profile that combined treatment would lead to increased reticulocyte levels and would be accompanied by sustained elevation of mature red blood cell levels if the experiment were extended beyond 72 h.

Taken together, these findings demonstrate that a GDF trap with a truncated ActRIIB extracellular domain can be administered in combination with EPO to synergistically increase red blood cell formation in vivo. Acting through a complementary but undefined mechanism, a GDF trap can moderate the strong proliferative effect of an EPO receptor activator alone and still permit target levels of red blood cells to be attained with lower doses of an EPO receptor activator, thereby avoiding potential adverse effects or other problems associated with higher levels of EPO receptor activation.

### Example 22: GDF Trap Increases Red Blood Cell Levels and Improves Red Blood Cell Morphology in Sickle-Cell Disease Model

Applicants investigated the effect of ActRIIB(L79D 25-131)-mFc on red blood cell (RBC) formation in a mouse model of sickle-cell disease (SCD) in which the mouse hemoglobin genes (α/α and β/β) have been replaced with the human sickle hemoglobin genes (α/α, γ/γ, and β^{S}/β^{S}). Mice homozygous for the human β^{S} allele exhibit the major features (*e.g.,* severe hemolytic anemia, irreversibly sickled red cells, vascular (vaso) occlusion, and multi-organ pathology) found in humans with sickle-cell disease [see, *e.g.,* Wu et al., (2006) Blood, 108(4): 1183-1188; Ryan et al. (1997) Science 278: 873-876].

SCD mice (β^{S}/β^{S}) at 3 months of age were randomly assigned to receive ActRIIB(L79D 25-131)-mFc (1 mg/kg or 10 mg/kg) or vehicle [Tris-buffered saline (TBS)] by subcutaneous injections twice weekly. Non-symptomatic compound heterozygote (β/β^{S}) litermates dosed with vehicle served as additional controls (Wt animals). At baseline, SCD mice had reduced RBC levels (-28%, P<0.01) and hemoglobin levels (-14.5%, P<0.05) and increased reticulocyte levels (+50%, P<0.001) compared to the compound heterozygote mice, demonstrating that the SCD mice were severely anemic.

Following one month of treatment, subjects were assessed for changes in various red blood cell parameters. Treatment of SCD mice with ActRIIB(L79D 25-131)-mFc for four weeks (1 mg/kg) increased RBC levels markedly (+15.2%, p < 0.01) compared to vehicle-treated SCD mice, thereby reducing the anemia observed in this model (Figures 32 and 33). ActRIIB(L79D 25-13 1)-mFc treatment-associated increases in hematocrit and hemoglobin concentrations were also observed (Figure 33) as well as significant decreases in mean corpuscular volume, RDC distribution width, reticulocyte numbers, and reactive oxygen species (Figure 34), which are all consistent with improved red blood cell half-life. Surprisingly, treatment of SCD mice with ActRIIB(L79D 25-13 1)-mFc for 6 weeks (1 mg/kg) resulted in a substantial decrease in phosphatidylserine (PS) exposure in peripheral blood cells (-14%, P = 0.08), as determined by scramblase enzyme assay and annexin-V assay, indicating a trend toward improved membrane phospholipid asymmetry compared to vehicle-treated subjects.

Following three months of treatment, subjects were observed to have improvements in additional blood chemistry parameters. In particular, treatment of SCD mice with ActRIIB(L79D 25-13 1)-mFc for 12 weeks (1 mg/kg) significantly decreased bilirubin (total) levels (-17.0%, p < 0.05), blood urea nitrogen levels (-19.2%, p < 0.05), and cell free hemoglobin (-30.7%, p = 0.06) compared to vehicle-treated SCD mice. These data indicate that GDF trap-treated subjects have decreased levels of red blood cell hemolysis in comparision to vehicle-treated subjects, which is consistent with the increase of red blood cell levels observed as early as one month following the start of ActRIIB(L79D 25-13 1)-mFc therapy. Annexin-V assays demonstrated a significant decrease in phosphatidylserine (PS) exposure in peripheral blood cells (-13.4%, p = 0.06) after three months of therapy in comparison to vehicle-treated subjects. Moreover, blood smears performed after three months of treatment (1 mg/kg) showed fewer irreversibly sickle-formed red blood cells in ActRIIB(L79D 25-131)-mFc-treated mice (-66.5%, p < 0.0001; enumerated from approximately 2000 cells per group) compared to mice treated with vehicle alone. These data indicate a qualitative improvement in red blood cell morphology following ActRIIB(L79D 25-13 1)-mFc treatment, which is consistent with the scramblase enzyme assay and annexin-V assay data obtained after one and three months of ActRIIB(L79D 25-13 1)-mFc treatment. Furthermore, treatment of SCD mice with the GDF trap for 3 months (1 mg/kg) also resulted in a significant decrease in spleen weight (-20.5%, p < 0.05) compared to vehicle-treated SCD mice. These data indicate that ActRIIB(L79D 25-13 1)-mFc may be useful in the treatment of other complications associated with sickle-cell disease including, for example, splenic sequestration of red blood cells, which can result in splenic sequestration crisis and/or spenomegaly.

Hemolysis and sickling of red blood cells in SCD patients results in anemia and reduced oxygen transport to various tissues. Such hypoxic conditions frequently result in vascular congestion, damage, and necrosis, which can ultimately lead to end organ damage in SCD patients. During the course of this study, it was observed that ActRIIB(L79D 25-131)-mFc significantly improves the oxygen carrying capacity of hemoglobin and prevents end organ damage in SCD mice.

After six weeks of therapy [10 mg/kg of ActRIIB(L79D 25-131)-mFc, s.c., twice weekly], GDF trap treated SCD mice had increased hemoglobin levels (+18.4%, p < 0.05) compared to vehicle-treated SCD mice. Blood samples were further assessed for differences in hemoglobin oxygen carrying properties. It was observed that GDF trap treated SCD mice have increased oxyhemoglobin (O₂Hb) saturation levels (+41.3%, p=0.06) and decreased carboxyhemoglobin (COHb) saturation levels (-25.2%, p < 0.05) compared to vehicle-treated SCD mice. Moreover, ActRIIB(L79D 25-131)-mFc treatment (10 mg/kg) resulted in increased blood oxygen content (O₂Ct) and saturation (SO₂) compared to control subjects (+65.2%, p=0.05, O₂Ct; +40.9%, p=0.06, SO₂). Based on these measurements, it was determined that the hemoglobin oxygen carrying capacity (O₂Cap) of GDF trap treated SCD mice is significantly higher (+14.04%, p < 0.05) than that of vehicle-treated mice. These data therefore indicate that ActRII antagonist therapy can be used to improve oxygenation of tissues/organs in SCD patients.

In addition to oxygen carrying capacity, end organ damage was assessed in GDF trap- [10 mg/kg of ActRIIB(L79D 25-131)-mFc, s.c., twice weekly] and vehicle-treated SCD mice. Histological analysis of GDF trap treated SCD mice showed a substantial reduction in vascular congestion and damage in spleens and kidneys (corticomedullary junction shown) compared to vehicle-treated SCD mice. See Figure 35. In addition, ActRIIB(L79D 25-131)-mFc treatment was shown to reduce alveolar thickening in the lungs. See black arrows in Figure 35. These results are consistent with the reduced sickling of red-blood cells and increased oxygen carrying capacity observed in GDF trap treated SCD mice. Accordingly, the data indicate that ActRII antagonists can be used to treat or prevent end organ damage in SCD patients.

Applicants have demonstrated that a GDF trap comprising an ActRIIB extracellular domain can provide various therapeutic benefits in a murine model of SCD. In addition to increasing RBC levels and improving various blood parameters (*e.g*., increased oxygen carrying capacity), GDF trap therapy improved RBC morphology (*i.e.,* reduced numbers of sickle shaped cells), reduced enlargement of the spleen, and reduced end organ damage in SCD patients. Accordingly, the data presented herein indicate that ActRII antagonists (*e.g*., GDF trap polypeptides) can be used to treat anemia as well as non-anemia complications of sickle-cell disease (e.g., complications arising from vaso-occlusion). Moreover, unlike red blood cell transfusions, which are inherently a source of exogenous iron, ActRII antagonists (*e.g.,* GDF trap polypeptides) can raise RBC levels by promoting use of endogenous iron stores via erythropoiesis and thus avoid iron overloading and its negative consequences.

### Example 23: Effects of a GDF Trap on Acute Chest Syndrome

As Acute chest syndrome (ACS) is one of the most common causes of hospitalization in SCD patients, Applicants investigated the effects of ActRIIB(L79D 25-131)-mFc in a mouse model of ACS. SCD mice were placed into one of two groups: treatment with ActRIIB(L79D 25-131)-mFc (10 mg/kg, twice weekly, s.c.); and treatment with TBS vehicle (control). After three months of treatment, hemin was injected into the ActRIIB(L79D 25-131)-mFc and the vehicle treated mice to induce hemolysis and ACS. See, *e.g.,* Samit Ghosh et al., (2013) J Clin Invest, 123(11): 4809-4820. ActRIIB(L79D 25-131)-mFc pre-treatment significantly extended survival time (3-fold) SCD mice compared to vehicle pre-treatment. These data indicate that ActRII antagonists (e.g., GDF trap polypeptides) can be used to reduce acute intravascular hemolysis and provide increased resistance to naturally occurring ACS episodes in SCD patients.

### Example 24: Effects of Combined Hydroxyurea and GDF Trap Treatment in SCD Mice

In a further study, Applicants investigated the effect of ActRIIB(L79D 25-131)-mFc and hydroxyurea (HU) combination therapy in a mouse model of SCD. SCD mice (β^{S}/β^{S}) were placed into four treatment groups: i) treatment with ActRIIB(L79D 25-131)-mFc (10 mg/kg, twice weekly, s.c.); ii) treatment with ActRIIB(L79D 25-131)-mFc (10 mg/kg, twice weekly, s.c.) and HU (100 mg/kg, twice weekly, i.p.); iii) treatment with HU (100 mg/kg, twice weekly, i.p.); and iv) TBS vehicle (control) for three months. Non-symptomatic compound heterozygote (β/β^{S}) litermates were treated similarly and used as controls to confirm disease in SCD (β^{S}/β^{S}) mice. At study baseline, SCD mice had reduced RBC levels (-28%, *P*<*0.01*) and hemoglobin levels (-14.5%, *P*<*0.05*) and increased reticulocytes levels (+50%, *P*<*0.001*) compared to compound heterozygote mice, indicating hemolysis characteristic of sickle-cell disease. Following one month of treatment, subjects were assessed for changes in various parameters.

As observed in the previous experiments, ActRIIB(L79D 25-131)-mFc (alone) treatment resulted in significant increases in red blood cell levels (+20%, p<0.01) and decreases in reticulocytes (-30%, p<0.05) compared to vehicle-treated SCD mice. Data from the combined ActRIIB(L79D 25-13 1)-mFc and HU treatment demonstrated additive beneficial effects in SCD mice compared to monotherapy. For example, the combination of ActRIIB(L79D 25-13 1)-mFc and HU resulted in a greater reduction in annexin V/PS exposure on peripheral blood cells than treatment with HU alone [-35.6% (p<0.001) vs.-22.2%, respectively, all relative to vehicle-treated SCD mice], indicating a greater improvement in membrane phospholipid asymmetry in SCD mice receiving the combination therapy. The combination of ActRIIB(L79D 25-13 1)-mFc and HU also resulted in a greater reduction in spleen size (-50.7%, p<0.05) compared to monotherapy with either HU (-20.2%, p<0.05) or ActRIIB(L79D 25-13 1)-mFc (-16.4%, p<0.05), all relative to vehicle-treated SCD mice. These finding show that combination therapy has a greater effect on reducing splenomegaly in SCD subjects compared to HU or ActRIIB(L79D 25-13 1)-mFc monotherapy. In addition, a greater reduction in bilirubin levels was observed mice receiving the combination therapy (-55.0%, p<0.01) compared to monotherapy with either HU (-48.4%, p<0.05) or ActRIIB(L79D 25-13 1)-mFc (-40.8%, p<0.05), all relative to vehicle-treated SCD mice. These finding show that combination therapy has a greater effect on reducing hemolysis in SCD subjects compared to HU or ActRIIB(L79D 25-13 1)-mFc monotherapy. Histological analysis further demonstrated a greater reduction in vascular congestion and damage in spleens and kidneys of mice receiving the ActRIIB(L79D 25-13 1)-mFc and HU combination therapy compared to mice receiving vehicle or HU monotherapy. See Figure 36. In addition, the histology demonstrated a greater reduction in alveolar thickening within the lungs of mice receiving the ActRIIB(L79D 25-13 1)-mFc and HU combination therapy compared to mice receiving the vehicle or HU monotherapy. See black arrows in Figure 36.

Taken together, data presented herein demonstrate that a GDF trap is efficacious as a monotherapy as well as effective as part of a combination therapy with HU in improving SCD disease pathology. In fact, the data indicate that a GDF trap may be administered in combination with HU to synergistically treat SCD.

## Claims

1. A composition for use in a method of treating or preventing a complication of sickle-cell disease in a subject, the method comprising administering the composition to a subject, wherein the composition comprises an ActRII antagonist, wherein the complication is selected from the group consisting of pain crisis, vaso-occlusion and vaso-occlusive crisis, wherein the ActRII antagonist comprises a polypeptide that comprises an amino acid sequence that is at least 90% identical to the sequence of amino acids 29-109 of SEQ ID NO: 1, wherein the polypeptide comprises an acidic amino acid at the position corresponding to position 79 of SEQ ID NO: 1; wherein the polypeptide binds to GDF8 and/or GDF11.

2. The composition for use according to claim 1, wherein the complication is pain crisis.

3. The composition for use according to claim 1, wherein the complication of sickle-cell disease is vaso-occlusive crisis.

4. The composition for use according to any one of claims 1 to 3, wherein the polypeptide ccomprises an amino acid sequence that is at least 95%, 96%, 97%, 98%, or 99% identical to the sequence of amino acids 29-109 of SEQ ID NO:1.

5. The composition for use according to any one of claims 1 to 4, wherein the acidic amino acid is a glutamic acid or an aspartic acid.

6. The composition for use according to claim 4, wherein the acidic amino acid is a glutamic acid.

7. The composition for use according to claim 4, wherein the acidic amino acid is an aspartic acid.

8. The composition for use according to any one of claim 1 to 4, wherein the polypeptide is:
a polypeptide comprising the amino acid sequence of SEQ ID NO:44 or comprising an amino acid sequence that is at least 95% identical to the amino acid sequence of SEQ ID NO:44; or
a polypeptide comprising the amino acid sequence of SEQ ID NO:45 or comprising an amino acid sequence that is at least 95% identical to the amino acid sequence of SEQ ID NO:45.

9. The composition for use according to any one of claims 1 to 4 or 8, wherein the polypeptide comprises the amino acid sequence of SEQ ID NO: 45.

10. The composition for use according to any one of claims 1 to 4 or 8, wherein the polypeptide comprises the amino acid sequence of SEQ ID NO: 44.

11. The composition for use according to any one of claims 1 to 10, wherein the polypeptide is a fusion protein further comprising one or more heterologous polypeptide domains that enhance one or more of: *in vivo* half-life, *in vitro* half-life, administration, tissue localization or distribution, formation of protein complexes, and purification, wherein the heterologous polypeptide domain is selected from: an immunoglobulin Fc domain and a serum albumin.

12. The composition for use according to claim 11, wherein the immunoglobulin Fc domain:
a. is an IgG1 Fc domain; or
b. comprises an amino acid sequence selected from SEQ ID NO: 15 or 16.

13. The composition for use according to claim 11 or 12, wherein the fusion protein further comprises a linker domain positioned between the polypeptide domain and the immunoglobulin Fc domain.

14. The composition for use according to any one of claims 1 to 13, wherein the polypeptide comprises one or more amino acid modifications selected from: a glycosylated amino acid, a PEGylated amino acid, a farnesylated amino acid, an acetylated amino acid, a biotinylated amino acid, an amino acid conjugated to a lipid moiety, and amino acid conjugated to an organic derivatizing agent.

15. The composition for use according to any one of claims 1 to 14, wherein the method further comprises administering one or more supportive therapy for sickle-cell disease, wherein the supportive therapy:
a) is transfusion with red blood cells;
b) comprises administration of an iron-chelating agent or multiple iron-chelating agents;
c) comprises administration of erythropoietin, epoetin alfa, epoetin beta, epoetin delta, epoetin omega, darbepoetin alfa, or methoxy-polyethylene-glycol epoetin beta; and/or
d) comprises administration of hydroxyurea.

16. The composition for use according to claim 15, wherein the supportive therapy comprises administration of hydroxyurea.

## Patentansprüche

1. Zusammensetzung zur Verwendung in einem Verfahren zur Behandlung oder Prävention einer Komplikation der Sichelzellenanämie bei einem Patienten, wobei das Verfahren ein Verabreichen der Zusammensetzung an einen Patienten umfasst, wobei die Zusammensetzung einen ActRII-Antagonisten umfasst, wobei die Komplikation aus der Gruppe bestehend aus Schmerzkrise, Okklusion von Blutgefäßen und kritischer Okklusion von Blutgefäßen ausgewählt ist, wobei der ActRII-Antagonist ein Polypeptid umfasst, das eine Aminosäuresequenz umfasst, die mindestens zu 90 % mit der Sequenz der Aminosäuren 29-109 der SEQ ID NO: 1 identisch ist, wobei das Polypeptid eine saure Aminosäure an der Position, die der Position 79 der SEQ ID NO: 1 entspricht, umfasst; wobei das Polypeptid an GDF8 und/oder GDF11 bindet.

2. Zusammensetzung zur Verwendung nach Anspruch 1, wobei es sich bei der Komplikation um eine Schmerzkrise handelt.

3. Zusammensetzung zur Verwendung nach Anspruch 1, wobei es sich bei der Komplikation der Sichelzellenanämie um eine kritische Gefäßokklusion handelt.

4. Zusammensetzung zur Verwendung nach einem der Ansprüche 1 bis 3, wobei das Polypeptid eine Aminosäuresequenz umfasst, die mindestens zu 95 %, 96 %, 97 %, 98 % oder 99 % mit der Sequenz der Aminosäuren 29-109 der SEQ ID NO: 1 identisch ist.

5. Zusammensetzung zur Verwendung nach einem der Ansprüche 1 bis 4, wobei es sich bei der sauren Aminosäure um eine Glutaminsäure oder eine Asparaginsäure handelt.

6. Zusammensetzung zur Verwendung nach Anspruch 4, wobei es sich bei der sauren Aminosäure um eine Glutaminsäure handelt.

7. Zusammensetzung zur Verwendung nach Anspruch 4, wobei es sich bei der sauren Aminosäure um eine Asparaginsäure handelt.

8. Zusammensetzung zur Verwendung nach einem der Ansprüche 1 bis 4, wobei es sich bei dem Polypeptid um Folgendes handelt:
ein Polypeptid, das die Aminosäuresequenz der SEQ ID NO: 44 umfasst oder eine Aminosäuresequenz umfasst, die mindestens zu 95 % mit der Aminosäuresequenz der SEQ ID NO: 44 identisch ist; oder
ein Polypeptid, das die Aminosäuresequenz der SEQ ID NO: 45 umfasst oder eine Aminosäuresequenz umfasst, die mindestens zu 95 % mit der Aminosäuresequenz der SEQ ID NO: 45 identisch ist.

9. Zusammensetzung zur Verwendung nach einem der Ansprüche 1 bis 4 oder 8, wobei das Polypeptid die Aminosäuresequenz der SEQ ID NO: 45 umfasst.

10. Zusammensetzung zur Verwendung nach einem der Ansprüche 1 bis 4 oder 8, wobei das Polypeptid die Aminosäuresequenz der SEQ ID NO: 44 umfasst.

11. Zusammensetzung zur Verwendung nach einem der Ansprüche 1 bis 10, wobei es sich bei dem Polypeptid um ein Fusionsprotein handelt, das ferner eine oder mehrere heterologe Polypeptid-Domänen umfasst, die eines oder mehrere des Folgenden verbessern: *in*-*vivo*-Halbwertszeit, *in*-*vitro*-Halbwertszeit, Verabreichung, Gewebelokalisierung oder -verteilung, Bildung von Proteinkomplexen und Reinigung, wobei die heterologe Polypeptid-Domäne aus Folgendem ausgewählt ist: einer Immunoglobulin-Fc-Domäne und einem Serumalbumin.

12. Zusammensetzung zur Verwendung nach Anspruch 11, wobei die Immunoglobulin-Fc-Domäne:
a. eine IgG1-Fc-Domäne ist; oder
b. eine aus den SEQ ID NO: 15 oder 16 ausgewählte Aminosäuresequenz umfasst.

13. Zusammensetzung zur Verwendung nach Anspruch 11 oder 12, wobei das Fusionsprotein ferner eine Linker-Domäne umfasst, die zwischen der Polypeptid-Domäne und der Immunoglobulin-Fc-Domäne positioniert ist.

14. Zusammensetzung zur Verwendung nach einem der Ansprüche 1 bis 13, wobei das Polypeptid eine oder mehrere Aminosäuresequenzmodifikationen umfasst, die aus Folgendem ausgewählt sind: einer glycosilierten Aminosäure, einer PEGylierten Aminosäure, einer farnesylierten Aminosäure, einer acetylierten Aminosäure, einer biotinylierten Aminosäure, einer an einen Lipidrest konjugierten Aminosäure und einer an ein organisches Derivatisierungsmittel konjugierten Aminosäure.

15. Zusammensetzung zur Verwendung nach einem der Ansprüche 1 bis 14, wobei das Verfahren ferner ein Verabreichen einer oder mehrerer Begleittherapien gegen Sichelzellenanämie umfasst, wobei die Begleittherapie:
a) eine Transfusion mit Erythrozyten ist;
b) ein Verabreichen eines Eisenchelatbildners oder mehrerer Eisenchelatbildner umfasst;
c) ein Verabreichen von Erythropoietin, Epoetin alfa, Epoetin beta, Epoetin delta, Epoetin omega, Darbepoetin alfa oder Methoxy-Polyethylenglycol-Epoetin beta umfasst; und/oder
d) ein Verabreichen von Hydroxyharnstoff umfasst.

16. Zusammensetzung zur Verwendung nach Anspruch 15, wobei die Begleittherapie ein Verabreichen von Hydroxyharnstoff umfasst.

## Revendications

1. Composition à utiliser dans un procédé de traitement ou de prévention d'une complication de la drépanocytose chez un sujet, le procédé comprenant l'administration de la composition à un sujet, dans laquelle la composition comprend un antagoniste d'ActRII, dans laquelle la complication est choisie dans le groupe comprenant une crise de douleur, une vaso-occlusion et une crise vaso-occlusive, dans laquelle l'antagoniste d'ActRII comprend un polypeptide qui comprend une séquence d'acides aminés qui est au moins à 90 % identique à la séquence d'acides aminés 29-109 de la SEQ ID NO: 1, dans laquelle le polypeptide comprend un acide aminé acide au niveau de la position correspondant à la position 79 de la SEQ ID NO: 1, dans laquelle le polypeptide se lie au GDF8 et/ou au GDF11.

2. Composition à utiliser selon la revendication 1, dans laquelle la complication est une crise de douleur.

3. Composition à utiliser selon la revendication 1, dans laquelle la complication de la drépanocytose est une crise vaso-occlusive.

4. Composition à utiliser selon l'une quelconque des revendications 1 à 3, dans laquelle le polypeptide comprend une séquence d'acides aminés qui est au moins à 95 %, 96 %, 97 %, 98 % ou 99 % identique à la séquence d'acides aminés 29-109 de la SEQ ID NO: 1.

5. Composition à utiliser selon l'une quelconque des revendications 1 à 4, dans laquelle l'acide aminé acide est un acide glutamique ou un acide aspartique.

6. Composition à utiliser selon la revendication 4, dans laquelle l'acide aminé acide est un acide glutamique.

7. Composition à utiliser selon la revendication 4, dans laquelle l'acide aminé acide est un acide aspartique.

8. Composition à utiliser selon l'une quelconque des revendications 1 à 4, dans laquelle le polypeptide est :
un polypeptide comprenant la séquence d'acides aminés de la SEQ ID NO: 44 ou comprenant une séquence d'acides aminés qui est au moins à 95% identique à la séquence d'acides aminés de la SEQ ID NO: 44; ou
un polypeptide comprenant la séquence d'acides aminés de la SEQ ID NO: 45 ou comprenant une séquence d'acides aminés qui est au moins à 95 % identique à la séquence d'acides aminés de la SEQ ID NO: 45.

9. Composition à utiliser selon l'une quelconque des revendications 1 à 4 ou 8, dans laquelle le polypeptide comprend la séquence d'acides aminés de la SEQ ID NO: 45.

10. Composition à utiliser selon l'une quelconque des revendications 1 à 4 ou 8, dans laquelle le polypeptide comprend la séquence d'acides aminés de la SEQ ID NO: 44.

11. Composition à utiliser selon l'une quelconque des revendications 1 à 10, dans laquelle le polypeptide est une protéine de fusion comprenant en outre un ou plusieurs domaines polypeptidiques hétérologues qui améliorent l'un ou plusieurs parmi : la demi-vie *in vivo,* la demi-vie *in vitro,* l'administration, la localisation ou distribution tissulaire, la formation de complexes protéiques et la purification, dans laquelle le domaine polypeptidique hétérologue est choisi parmi : un domaine Fc d'immunoglobuline et une albumine sérique.

12. Composition à utiliser selon la revendication 11, dans laquelle le domaine Fc d'immunoglobuline :
a. est un domaine IgGl Fc ; ou
b. comprend une séquence d'acides aminés choisie parmi les SEQ ID NO: 15 ou 16.

13. Composition à utiliser selon la revendication 11 ou 12, dans laquelle la protéine de fusion comprend en outre un domaine de liaison positionné entre le domaine polypeptidique et le domaine Fc d'immunoglobuline.

14. Composition à utiliser selon l'une quelconque des revendications 1 à 13, dans laquelle le polypeptide comprend une ou plusieurs modifications d'acides aminés choisies parmi : un acide aminé glycosylé, un acide aminé PEGylé, un acide aminé farnésylé, un acide aminé acétylé, un acide aminé biotinylé, un acide aminé conjugué à une fraction lipidique et un acide aminé conjugué à un agent de dérivation organique.

15. Composition à utiliser selon l'une quelconque des revendications 1 à 14, dans laquelle le procédé comprend en outre l'administration d'une ou de plusieurs thérapies de soutien pour la drépanocytose, dans laquelle la thérapie de soutien :
a) est une transfusion de globules rouges ;
b) comprend l'administration d'un agent chélateur du fer ou de plusieurs agents chélateurs du fer ;
c) comprend l'administration d'érythropoïétine, d'époétine alfa, d'époétine bêta, d'époétine delta, d'époétine oméga, de darbépoétine alfa ou de méthoxy-polyéthylène-glycol époétine bêta ; et/ou
d) comprend l'administration d'hydroxyurée.

16. Composition à utiliser selon la revendication 15, dans laquelle la thérapie de soutien comprend l'administration d'hydroxyurée.
